(19) Europäisches Patentamt
European Patent Office
Office européen des brevets

(11) EP 1 259 627 B1

(12) EUROPEAN PATENT SPECIFICATION

(45) Date of publication and mention of the grant of the patent:
**24.01.2007 Bulletin 2007/04**

(21) Application number: **01914109.2**

(22) Date of filing: **28.02.2001**

(51) Int Cl.:
***C12N 15/70*** *(2006.01)* ***C07K 14/22*** *(2006.01)*
***C07K 19/00*** *(2006.01)*

(86) International application number:
**PCT/IB2001/000452**

(87) International publication number:
**WO 2001/064922 (07.09.2001 Gazette 2001/36)**

(54) **HETEROLOGOUS EXPRESSION OF NEISSERIAL PROTEINS**

HETEROLOGE EXPRESSION VON NEISSERIA PROTEINE

EXPRESSION HETEROLOGUE DE PROTEINES ISSUES DU GONOCOQUE

(84) Designated Contracting States:
**AT BE CH CY DE DK ES FI FR GB GR IE IT LI LU MC NL PT SE TR**

(30) Priority: **28.02.2000 GB 0004695**
**13.11.2000 GB 0027675**

(43) Date of publication of application:
**27.11.2002 Bulletin 2002/48**

(60) Divisional application:
**06076718.3**

(73) Proprietor: **Novartis Vaccines and Diagnostics S.r.l.**
**53100 Siena SI (IT)**

(72) Inventors:
- **ARICO, Maria, Beatrice, c/o Chiron SpA I-53100 Siena (IT)**
- **COMANDUCCI, Maurizio, c/o Chiron SpA I-53100 Siena (IT)**
- **GALEOTTI, Cesira, c/o Chiron SpA I-53100 Siena (IT)**
- **MASIGNANI, Vega, c/o Chiron SpA I-53100 Siena (IT)**
- **GUILIANI, Marzia Monica, c/o Chiron SpA I-53100 Siena (IT)**
- **PIZZA, Mariagrazia, c/o Chiron SpA I-53100 Siena (IT)**

(74) Representative: **Marshall, Cameron John et al**
**Carpmaels & Ransford**
**43 Bloomsbury Square**
**London WC1A 2RA (GB)**

(56) References cited:

**WO-A-97/13860** **WO-A-97/28273**
**WO-A-99/36544** **WO-A2-99/57280**
**FR-A- 2 720 408** **US-A- 6 013 267**
**US-A- 6 028 049**

- **GENEVIÈVE RENAULD-MONG[1]NIE ET AL.: "Identification of human transferrin-binding sites within meningococcal transferrin-binding protein B" JOURNAL OF BACTERIOLOGY, vol. 179, no. 20, October 1997 (1997-10), pages 6400-6407, XP002168798**
- **LEGRAIN M ET AL: "PRODUCTION OF LIPIDATED MENINGOCOCCAL TRANSFERRIN BINDING PROTEIN 2 IN ESCHERICHIA COLI" PROTEIN EXPRESSION AND PURIFICATION, ACADEMIC PRESS, US, vol. 6, October 1995 (1995-10), pages 570-578, XP000996792 ISSN: 1046-5928**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 259 627 B1

SEQUENCE LISTING

<110> Chiron SpA

<120> Heterologous Expression of Neisserial Proteins

<130> P024049WO

<140> PCT/IB01/00452
<141> 2001-02-28

<150> 0004695.3
<151> 2000-02-28

<150> 0027675.8
<151> 2000-11-13

<160> 620

<170> SeqWin99, version 1.02

<210> 1
<211> 441
<212> PRT
<213> Neisseria meningitidis

<400> 1

```
Met Lys Lys Tyr Leu Phe Arg Ala Ala Leu Tyr Gly Ile Ala Ala Ala
1               5                   10                  15

Ile Leu Ala Ala Cys Gln Ser Lys Ser Ile Gln Thr Phe Pro Gln Pro
            20                  25                  30

Asp Thr Ser Val Ile Asn Gly Pro Asp Arg Pro Val Gly Ile Pro Asp
        35                  40                  45

Pro Ala Gly Thr Thr Val Gly Gly Gly Gly Ala Val Tyr Thr Val Val
    50                  55                  60

Pro His Leu Ser Leu Pro His Trp Ala Ala Gln Asp Phe Ala Lys Ser
65                  70                  75                  80

Leu Gln Ser Phe Arg Leu Gly Cys Ala Asn Leu Lys Asn Arg Gln Gly
                85                  90                  95

Trp Gln Asp Val Cys Ala Gln Ala Phe Gln Thr Pro Val His Ser Phe
            100                 105                 110

Gln Ala Lys Gln Phe Phe Glu Arg Tyr Phe Thr Pro Trp Gln Val Ala
        115                 120                 125

Gly Asn Gly Ser Leu Ala Gly Thr Val Thr Gly Tyr Tyr Glu Pro Val
    130                 135                 140

Leu Lys Gly Asp Asp Arg Arg Thr Ala Gln Ala Arg Phe Pro Ile Tyr
145                 150                 155                 160
```

Gly Ile Pro Asp Asp Phe Ile Ser Val Pro Leu Pro Ala Gly Leu Arg
165 170 175

Ser Gly Lys Ala Leu Val Arg Ile Arg Gln Thr Gly Lys Asn Ser Gly
180 185 190

Thr Ile Asp Asn Thr Gly Gly Thr His Thr Ala Asp Leu Ser Arg Phe
195 200 205

Pro Ile Thr Ala Arg Thr Thr Ala Ile Lys Gly Arg Phe Glu Gly Ser
210 215 220

Arg Phe Leu Pro Tyr His Thr Arg Asn Gln Ile Asn Gly Gly Ala Leu
225 230 235 240

Asp Gly Lys Ala Pro Ile Leu Gly Tyr Ala Glu Asp Pro Val Glu Leu
245 250 255

Phe Phe Met His Ile Gln Gly Ser Gly Arg Leu Lys Thr Pro Ser Gly
260 265 270

Lys Tyr Ile Arg Ile Gly Tyr Ala Asp Lys Asn Glu His Pro Tyr Val
275 280 285

Ser Ile Gly Arg Tyr Met Ala Asp Lys Gly Tyr Leu Lys Leu Gly Gln
290 295 300

Thr Ser Met Gln Gly Ile Lys Ala Tyr Met Arg Gln Asn Pro Gln Arg
305 310 315 320

Leu Ala Glu Val Leu Gly Gln Asn Pro Ser Tyr Ile Phe Phe Arg Glu
325 330 335

Leu Ala Gly Ser Ser Asn Asp Gly Pro Val Gly Ala Leu Gly Thr Pro
340 345 350

Leu Met Gly Glu Tyr Ala Gly Ala Val Asp Arg His Tyr Ile Thr Leu
355 360 365

Gly Ala Pro Leu Phe Val Ala Thr Ala His Pro Val Thr Arg Lys Ala
370 375 380

Leu Asn Arg Leu Ile Met Ala Gln Asp Thr Gly Ser Ala Ile Lys Gly
385 390 395 400

Ala Val Arg Val Asp Tyr Phe Trp Gly Tyr Gly Asp Glu Ala Gly Glu
405 410 415

Leu Ala Gly Lys Gln Lys Thr Thr Gly Tyr Val Trp Gln Leu Leu Pro
420 425 430

Asn Gly Met Lys Pro Glu Tyr Arg Pro
435 440

<210> 2
<211> 420
<212> PRT
<213> Neisseria meningitidis

<400> 2

```
Gln Ser Lys Ser Ile Gln Thr Phe Pro Gln Pro Asp Thr Ser Val Ile
1               5                   10                  15

Asn Gly Pro Asp Arg Pro Val Gly Ile Pro Asp Pro Ala Gly Thr Thr
            20                  25                  30

Val Gly Gly Gly Gly Ala Val Tyr Thr Val Val Pro His Leu Ser Leu
        35                  40                  45

Pro His Trp Ala Ala Gln Asp Phe Ala Lys Ser Leu Gln Ser Phe Arg
    50                  55                  60

Leu Gly Cys Ala Asn Leu Lys Asn Arg Gln Gly Trp Gln Asp Val Cys
65                  70                  75                  80

Ala Gln Ala Phe Gln Thr Pro Val His Ser Phe Gln Ala Lys Gln Phe
                85                  90                  95

Phe Glu Arg Tyr Phe Thr Pro Trp Gln Val Ala Gly Asn Gly Ser Leu
            100                 105                 110

Ala Gly Thr Val Thr Gly Tyr Tyr Glu Pro Val Leu Lys Gly Asp Asp
        115                 120                 125

Arg Arg Thr Ala Gln Ala Arg Phe Pro Ile Tyr Gly Ile Pro Asp Asp
    130                 135                 140

Phe Ile Ser Val Pro Leu Pro Ala Gly Leu Arg Ser Gly Lys Ala Leu
145                 150                 155                 160

Val Arg Ile Arg Gln Thr Gly Lys Asn Ser Gly Thr Ile Asp Asn Thr
                165                 170                 175

Gly Gly Thr His Thr Ala Asp Leu Ser Arg Phe Pro Ile Thr Ala Arg
            180                 185                 190

Thr Thr Ala Ile Lys Gly Arg Phe Glu Gly Ser Arg Phe Leu Pro Tyr
        195                 200                 205

His Thr Arg Asn Gln Ile Asn Gly Gly Ala Leu Asp Gly Lys Ala Pro
    210                 215                 220

Ile Leu Gly Tyr Ala Glu Asp Pro Val Glu Leu Phe Phe Met His Ile
225                 230                 235                 240

Gln Gly Ser Gly Arg Leu Lys Thr Pro Ser Gly Lys Tyr Ile Arg Ile
                245                 250                 255

Gly Tyr Ala Asp Lys Asn Glu His Pro Tyr Val Ser Ile Gly Arg Tyr
            260                 265                 270

Met Ala Asp Lys Gly Tyr Leu Lys Leu Gly Gln Thr Ser Met Gln Gly
        275                 280                 285
```

Ile Lys Ala Tyr Met Arg Gln Asn Pro Gln Arg Leu Ala Glu Val Leu
290 295 300

Gly Gln Asn Pro Ser Tyr Ile Phe Phe Arg Glu Leu Ala Gly Ser Ser
305 310 315 320

Asn Asp Gly Pro Val Gly Ala Leu Gly Thr Pro Leu Met Gly Glu Tyr
325 330 335

Ala Gly Ala Val Asp Arg His Tyr Ile Thr Leu Gly Ala Pro Leu Phe
340 345 350

Val Ala Thr Ala His Pro Val Thr Arg Lys Ala Leu Asn Arg Leu Ile
355 360 365

Met Ala Gln Asp Thr Gly Ser Ala Ile Lys Gly Ala Val Arg Val Asp
370 375 380

Tyr Phe Trp Gly Tyr Gly Asp Glu Ala Gly Glu Leu Ala Gly Lys Gln
385 390 395 400

Lys Thr Thr Gly Tyr Val Trp Gln Leu Leu Pro Asn Gly Met Lys Pro
405 410 415

Glu Tyr Arg Pro
420

<210> 3
<211> 440
<212> PRT
<213> Artificial Sequence

<220>
<223> 919

<400> 3

```
Met Lys Thr Phe Phe Lys Thr Leu Ser Ala Ala Ala Leu Ala Leu Ile
1               5                   10                  15

Leu Ala Ala Cys Gln Ser Lys Ser Ile Gln Thr Phe Pro Gln Pro Asp
            20                  25                  30

Thr Ser Val Ile Asn Gly Pro Asp Arg Pro Val Gly Ile Pro Asp Pro
        35                  40                  45

Ala Gly Thr Thr Val Gly Gly Gly Gly Ala Val Tyr Thr Val Val Pro
    50                  55                  60

His Leu Ser Leu Pro His Trp Ala Ala Gln Asp Phe Ala Lys Ser Leu
65                  70                  75                  80

Gln Ser Phe Arg Leu Gly Cys Ala Asn Leu Lys Asn Arg Gln Gly Trp
                85                  90                  95

Gln Asp Val Cys Ala Gln Ala Phe Gln Thr Pro Val His Ser Phe Gln
            100                 105                 110
```

Ala Lys Gln Phe Phe Glu Arg Tyr Phe Thr Pro Trp Gln Val Ala Gly
115 120 125

Asn Gly Ser Leu Ala Gly Thr Val Thr Gly Tyr Tyr Glu Pro Val Leu
130 135 140

Lys Gly Asp Asp Arg Arg Thr Ala Gln Ala Arg Phe Pro Ile Tyr Gly
145 150 155 160

Ile Pro Asp Asp Phe Ile Ser Val Pro Leu Pro Ala Gly Leu Arg Ser
165 170 175

Gly Lys Ala Leu Val Arg Ile Arg Gln Thr Gly Lys Asn Ser Gly Thr
180 185 190

Ile Asp Asn Thr Gly Gly Thr His Thr Ala Asp Leu Ser Arg Phe Pro
195 200 205

Ile Thr Ala Arg Thr Thr Ala Ile Lys Gly Arg Phe Glu Gly Ser Arg
210 215 220

Phe Leu Pro Tyr His Thr Arg Asn Gln Ile Asn Gly Gly Ala Leu Asp
225 230 235 240

Gly Lys Ala Pro Ile Leu Gly Tyr Ala Glu Asp Pro Val Glu Leu Phe
245 250 255

Phe Met His Ile Gln Gly Ser Gly Arg Leu Lys Thr Pro Ser Gly Lys
260 265 270

Tyr Ile Arg Ile Gly Tyr Ala Asp Lys Asn Glu His Pro Tyr Val Ser
275 280 285

Ile Gly Arg Tyr Met Ala Asp Lys Gly Tyr Leu Lys Leu Gly Gln Thr
290 295 300

Ser Met Gln Gly Ile Lys Ser Tyr Met Arg Gln Asn Pro Gln Arg Leu
305 310 315 320

Ala Glu Val Leu Gly Gln Asn Pro Ser Tyr Ile Phe Phe Arg Glu Leu
325 330 335

Ala Gly Ser Ser Asn Asp Gly Pro Val Gly Ala Leu Gly Thr Pro Leu
340 345 350

Met Gly Glu Tyr Ala Gly Ala Val Asp Arg His Tyr Ile Thr Leu Gly
355 360 365

Ala Pro Leu Phe Val Ala Thr Ala His Pro Val Thr Arg Lys Ala Leu
370 375 380

Asn Arg Leu Ile Met Ala Gln Asp Thr Gly Ser Ala Ile Lys Gly Ala
385 390 395 400

Val Arg Val Asp Tyr Phe Trp Gly Tyr Gly Asp Glu Ala Gly Glu Leu
405 410 415

Ala Gly Lys Gln Lys Thr Thr Gly Tyr Val Trp Gln Leu Leu Pro Asn
420 425 430

Gly Met Lys Pro Glu Tyr Arg Pro
435 440

<210> 4
<211> 58
<212> PRT
<213> Artificial Sequence

<220>
<223> 907-2.pep

<400> 4

Glu Arg Arg Arg Leu Leu Val Asn Ile Gln Tyr Glu Ser Ser Arg Ala
1 5 10 15

Gly Leu Asp Thr Gln Ile Val Leu Gly Leu Ile Glu Val Glu Ser Ala
20 25 30

Phe Arg Gln Tyr Ala Ile Ser Gly Val Gly Ala Arg Gly Leu Met Gln
35 40 45

Val Met Pro Phe Trp Lys Asn Tyr Ile Gly
50 55

<210> 5
<211> 60
<212> PRT
<213> Artificial Sequence

<220>
<223> Escherichia coli

<400> 5

Glu Arg Phe Pro Leu Ala Tyr Asn Asp Leu Phe Lys Arg Tyr Thr Ser
1 5 10 15

Gly Lys Glu Ile Pro Gln Ser Tyr Ala Met Ala Ile Ala Arg Gln Glu
20 25 30

Ser Ala Trp Asn Pro Lys Val Lys Ser Pro Val Gly Ala Ser Gly Leu
35 40 45

Met Gln Ile Met Pro Gly Thr Ala Thr His Thr Val
50 55 60

<210> 6
<211> 120
<212> PRT
<213> Artificial Sequence

<220>
<223> 922.pep

<400> 6

```
Val Ala Gln Lys Tyr Gly Val Pro Ala Glu Leu Ile Val Ala Val Ile
1               5                   10                  15

Gly Ile Glu Thr Asn Tyr Gly Lys Asn Thr Gly Ser Phe Arg Val Ala
            20                  25                  30

Asp Ala Leu Ala Thr Leu Gly Phe Asp Tyr Pro Arg Arg Ala Gly Phe
        35                  40                  45

Phe Gln Lys Glu Leu Val Glu Leu Leu Lys Leu Ala Lys Glu Glu Gly
    50                  55                  60

Gly Asp Val Phe Ala Phe Lys Gly Ser Tyr Ala Gly Ala Met Gly Met
65                  70                  75                  80

Pro Gln Phe Met Pro Ser Ser Tyr Arg Lys Trp Ala Val Asp Tyr Asp
                85                  90                  95

Gly Asp Gly His Arg Asp Ile Trp Gly Asn Val Gly Asp Val Ala Ala
            100                 105                 110

Ser Val Ala Asn Tyr Met Lys Gln
        115                 120
```

<210> 7
<211> 119
<212> PRT
<213> Artificial Sequence

<220>
<223> Escherichia coli

<400> 7

```
Ala Trp Gln Val Tyr Gly Val Pro Pro Glu Ile Ile Val Gly Ile Ile
1               5                   10                  15

Gly Val Glu Thr Arg Trp Gly Arg Val Met Gly Lys Thr Arg Ile Leu
            20                  25                  30

Asp Ala Leu Ala Thr Leu Ser Phe Asn Tyr Pro Arg Arg Ala Glu Tyr
        35                  40                  45

Phe Ser Gly Glu Leu Glu Thr Phe Leu Leu Met Ala Arg Asp Glu Gln
    50                  55                  60

Asp Asp Pro Leu Asn Leu Lys Gly Ser Phe Ala Gly Ala Met Gly Tyr
65                  70                  75                  80

Gly Gln Phe Met Pro Ser Ser Tyr Lys Gln Tyr Ala Val Asp Phe Ser
                85                  90                  95

Gly Asp Gly His Ile Asn Leu Trp Asp Pro Val Asp Ala Ile Gly Ser
            100                 105                 110

Val Ala Asn Tyr Phe Lys Ala
        115
```

<210> 8
<211> 194
<212> PRT
<213> Artificial Sequence

<220>
<223> 919.pep

<400> 8

```
Ala Leu Asp Gly Lys Ala Pro Ile Leu Gly Tyr Ala Glu Asp Pro Val
1               5                   10                  15

Glu Leu Phe Phe Met His Ile Gln Gly Ser Gly Arg Leu Lys Thr Pro
            20                  25                  30

Ser Gly Lys Tyr Ile Arg Ile Gly Tyr Ala Asp Lys Asn Glu His Pro
        35                  40                  45

Tyr Val Ser Ile Gly Arg Tyr Met Ala Asp Lys Gly Tyr Leu Lys Leu
    50                  55                  60

Gly Gln Thr Ser Met Gln Gly Ile Lys Ser Tyr Met Arg Gln Asn Pro
65                  70                  75                  80

Gln Arg Leu Ala Glu Val Leu Gly Gln Asn Pro Ser Tyr Ile Phe Phe
                85                  90                  95

Arg Glu Leu Ala Gly Ser Ser Asn Asp Gly Pro Val Gly Ala Leu Gly
            100                 105                 110

Thr Pro Leu Met Gly Glu Tyr Ala Gly Ala Val Asp Arg His Tyr Ile
        115                 120                 125

Thr Leu Gly Ala Pro Leu Phe Val Ala Thr Ala His Pro Val Thr Arg
    130                 135                 140

Lys Ala Leu Asn Arg Leu Ile Met Ala Gln Asp Thr Gly Ser Ala Ile
145                 150                 155                 160

Lys Gly Ala Val Arg Val Asp Tyr Phe Trp Gly Tyr Gly Asp Glu Ala
                165                 170                 175

Gly Glu Leu Ala Gly Lys Gln Lys Thr Thr Gly Tyr Val Trp Gln Leu
            180                 185                 190

Leu Pro
```

<210> 9
<211> 196
<212> PRT
<213> Escherichia coli

<400> 9

```
Ala Leu Ser Asp Lys Tyr Ile Leu Ala Tyr Ser Asn Ser Leu Met Asp
1               5                   10                  15
```

Asn Phe Ile Met Asp Val Gln Gly Ser Gly Tyr Ile Asp Phe Gly Asp
20 25 30

Gly Ser Pro Leu Asn Phe Phe Ser Tyr Ala Gly Lys Asn Gly His Ala
35 40 45

Tyr Arg Ser Ile Gly Lys Val Leu Ile Asp Arg Gly Glu Val Lys Lys
50 55 60

Glu Asp Met Ser Met Gln Ala Ile Arg His Trp Gly Glu Thr His Ser
65 70 75 80

Glu Ala Glu Val Arg Glu Leu Leu Glu Gln Asn Pro Ser Phe Val Phe
85 90 95

Phe Lys Pro Gln Ser Phe Ala Pro Val Lys Gly Ala Ser Ala Val Pro
100 105 110

Leu Val Gly Arg Ala Ser Val Ala Ser Asp Arg Ser Ile Ile Pro Pro
115 120 125

Gly Thr Thr Leu Leu Ala Glu Val Pro Leu Leu Asp Asn Asn Gly Lys
130 135 140

Phe Asn Gly Gln Tyr Glu Leu Arg Leu Met Val Ala Leu Asp Val Gly
145 150 155 160

Gly Ala Ile Lys Gly Gln His Phe Asp Ile Tyr Gln Gly Ile Gly Pro
165 170 175

Glu Ala Gly His Arg Ala Gly Trp Tyr Asn His Tyr Gly Arg Val Trp
180 185 190

Val Leu Lys Thr
195

<210> 10
<211> 28
<212> DNA
<213> Artificial Sequence

<220>
<223> Oligonucleotide

<400> 10
cgaagacccc gtcggtcttt tttttatg 28

<210> 11
<211> 28
<212> DNA
<213> Artificial Sequence

<220>
<223> Oligonucleotide

<400> 11
gtgcataaaa aaaagaccga cggggtct 28

<210> 12
<211> 25
<212> DNA
<213> Artificial Sequence

<220>
<223> Oligonucleotide

<400> 12
aacgcctcgc cggtgttttg ggtca 25

<210> 13
<211> 25
<212> DNA
<213> Artificial Sequence

<220>
<223> Oligonucleotide

<400> 13
tttgacccaa aacaccggcg aggcg 25

<210> 14
<211> 26
<212> DNA
<213> Artificial Sequence

<220>
<223> Oligonucleotide

<400> 14
tgccggcgca gtcggtcggc actaca 26

<210> 15
<211> 26
<212> DNA
<213> Artificial Sequence

<220>
<223> Oligonucleotide

<400> 15
taatgtagtg ccgaccgact gcgccg 26

<210> 16
<211> 25
<212> DNA
<213> Artificial Sequence

<220>
<223> Oligonucleotide

<400> 16
tgattgaggt gggtagcgcg ttccg 25

<210> 17
<211> 25
<212> DNA

<213> Artificial Sequence

<220>
<223> Oligonucleotide

<400> 17
ggcggaacgc gctacccacc tcaat 25

<210> 18
<211> 34
<212> DNA
<213> Artificial Sequence

<220>
<223> Oligonucleotide

<400> 18
ccggaattct tatgaaaaaa atcatcttcg ccgc 34

<210> 19
<211> 32
<212> DNA
<213> Artificial Sequence

<220>
<223> Oligonucleotide

<400> 19
gcccaagctt ttattgtttg gctgcctcga tt 32

<210> 20
<211> 37
<212> DNA
<213> Artificial Sequence

<220>
<223> Oligonucleotide

<400> 20
ccggaattct tatgtcgccc gatgttaaat cggcgga 37

<210> 21
<211> 32
<212> DNA
<213> Artificial Sequence

<220>
<223> Oligonucleotide

<400> 21
gcccaagctt tcaatcctgc tcttttttgc cg 32

<210> 22
<211> 34
<212> DNA
<213> Artificial Sequence

<220>

<223> Oligonucleotide

<400> 22
ccggaattct tatgagccaa gatatggcgg cagt 34

<210> 23
<211> 32
<212> DNA
<213> Artificial Sequence

<220>
<223> Oligonucleotide

<400> 23
gcccaagctt tcaatcctgc tcttttttgc cg 32

<210> 24
<211> 34
<212> DNA
<213> Artificial Sequence

<220>
<223> Oligonucleotide

<400> 24
ccggaattct tatgtccgcc gaatccgcaa atca 34

<210> 25
<211> 32
<212> DNA
<213> Artificial Sequence

<220>
<223> Oligonucleotide

<400> 25
gcccaagctt tcaatcctgc tcttttttgc cg 32

<210> 26
<211> 36
<212> DNA
<213> Artificial Sequence

<220>
<223> Oligonucleotide

<400> 26
ccggaattct tatgggaagg gttgatttgg ctaatg 36

<210> 27
<211> 32
<212> DNA
<213> Artificial Sequence

<220>
<223> Oligonucleotide

<400> 27

gcccaagctt tcaatcctgc tcttttttgc cg 32

<210> 28
<211> 36
<212> DNA
<213> Artificial Sequence

<220>
<223> Oligonucleotide

<400> 28
ccggaattct tatgtcagat ttggcaaacg attctt 36

<210> 29
<211> 35
<212> DNA
<213> Artificial Sequence

<220>
<223> Oligonucleotide

<400> 29
gcccaagctt ttacgtatca tatttcacgt gcttc 35

<210> 30
<211> 37
<212> DNA
<213> Artificial Sequence

<220>
<223> Oligonucleotide

<400> 30
ccggaattct tatgtcgccc gatgttaaat cggcgga 37

<210> 31
<211> 35
<212> DNA
<213> Artificial Sequence

<220>
<223> Oligonucleotide

<400> 31
gcccaagctt ttacgtatca tatttcacgt gcttc 35

<210> 32
<211> 36
<212> DNA
<213> Artificial Sequence

<220>
<223> Oligonucleotide

<400> 32
ccggaattct tatgcaaagc aagagcatcc aaacct 36

<210> 33

<211> 30
<212> DNA
<213> Artificial Sequence

<220>
<223> Oligonucleotide

<400> 33
gcccaagctt ttacgggcgg tattcgggct 30

<210> 34
<211> 29
<212> DNA
<213> Artificial Sequence

<220>
<223> Oligonucleotide

<400> 34
ccggaattca tatgaaacac tttccatcc 29

<210> 35
<211> 28
<212> DNA
<213> Artificial Sequence

<220>
<223> Oligonucleotide

<400> 35
gcccaagctt ttaccactcg taattgac 28

<210> 36
<211> 29
<212> DNA
<213> Artificial Sequence

<220>
<223> Oligonucleotide

<400> 36
ccggaattca tatggccaca agcgacgac 29

<210> 37
<211> 28
<212> DNA
<213> Artificial Sequence

<220>
<223> Oligonucleotide

<400> 37
gcccaagctt ttaccactcg taattgac 28

<210> 38
<211> 29
<212> DNA
<213> Artificial Sequence

<220>
<223> Oligonucleotide

<400> 38
ccggaattct tatgaaacac tttccatcc 29

<210> 39
<211> 31
<212> DNA
<213> Artificial Sequence

<220>
<223> Oligonucleotide

<400> 39
gcccaagctt tcaacccacg ttgtaaggtt g 31

<210> 40
<211> 30
<212> DNA
<213> Artificial Sequence

<220>
<223> Oligonucleotide

<400> 40
ccggaattct tatggccaca aacgacgacg 30

<210> 41
<211> 31
<212> DNA
<213> Artificial Sequence

<220>
<223> Oligonucleotide

<400> 41
gcccaagctt tcaacccacg ttgtaaggtt g 31

<210> 42
<211> 34
<212> DNA
<213> Artificial Sequence

<220>
<223> Oligonucleotide

<400> 42
ccggaattct tatggccacc tacaaagtgg acga 34

<210> 43
<211> 32
<212> DNA
<213> Artificial Sequence

<220>
<223> Oligonucleotide

<400> 43
gcccaagctt ttattgtttg gctgcctcga tt 32

<210> 44
<211> 32
<212> DNA
<213> Artificial Sequence

<220>
<223> Oligonucleotide

<400> 44
cgcggatccg ctagccccga tgttaaatcg gc 32

<210> 45
<211> 31
<212> DNA
<213> Artificial Sequence

<220>
<223> Oligonucleotide

<400> 45
cccgctcgag tcaatcctgc tcttttttgc c 31

<210> 46
<211> 32
<212> DNA
<213> Artificial Sequence

<220>
<223> Oligonucleotide

<400> 46
cgcggatccg ctagccaaga tatggcggca gt 32

<210> 47
<211> 32
<212> DNA
<213> Artificial Sequence

<220>
<223> Oligonucleotide

<400> 47
cgcggatccg ctagcgccga atccgcaaat ca 32

<210> 48
<211> 32
<212> DNA
<213> Artificial Sequence

<220>
<223> Oligonucleotide

<400> 48
cgcgctagcg gaagggttga tttggctaat gg 32

<210> 49
<211> 34
<212> DNA
<213> Artificial Sequence

<220>
<223> Oligonucleotide

<400> 49
gggaattcca tatgggcatt tcccgcaaaa tatc 34

<210> 50
<211> 32
<212> DNA
<213> Artificial Sequence

<220>
<223> Oligonucleotide

<400> 50
cccgctcgag ttacgtatca tatttcacgt gc 32

<210> 51
<211> 34
<212> DNA
<213> Artificial Sequence

<220>
<223> Oligonucleotide

<400> 51
gggaattcca tatgggcatt tcccgcaaaa tatc 34

<210> 52
<211> 33
<212> DNA
<213> Artificial Sequence

<220>
<223> Oligonucleotide

<400> 52
cccgctcgag ttattctatg ccttgtgcgg cat 33

<210> 53
<211> 32
<212> DNA
<213> Artificial Sequence

<220>
<223> Oligonucleotide

<400> 53
cgcggatccc atatggccac aagcgacgac ga 32

<210> 54
<211> 28
<212> DNA

<213> Artificial Sequence

<220>
<223> Oligonucleotide

<400> 54
cccgctcgag ttaccactcg taattgac 28

<210> 55
<211> 28
<212> DNA
<213> Artificial Sequence

<220>
<223> Oligonucleotide

<400> 55
cgcggatccc atatggccac aaacgacg 28

<210> 56
<211> 35
<212> DNA
<213> Artificial Sequence

<220>
<223> Oligonucleotide

<400> 56
cccgctcgag tcatttagca atattatctt tgttc 35

<210> 57
<211> 33
<212> DNA
<213> Artificial Sequence

<220>
<223> Oligonucleotide

<400> 57
cgcggatccc atatgaaagc aaacagtgcc gac 33

<210> 58
<211> 28
<212> DNA
<213> Artificial Sequence

<220>
<223> Oligonucleotide

<400> 58
cccgctcgag ttaccactcg taattgac 28

<210> 59
<211> 28
<212> DNA
<213> Artificial Sequence

<220>

<223> Oligonucleotide

<400> 59
cgcggatccc atatggccac aaacgacg 28

<210> 60
<211> 29
<212> DNA
<213> Artificial Sequence

<220>
<223> Oligonucleotide

<400> 60
cccgctcgag ttaacccacg ttgtaaggt 29

<210> 61
<211> 33
<212> DNA
<213> Artificial Sequence

<220>
<223> Oligonucleotide

<400> 61
cgcggatccc atatgatgaa acactttcca tcc 33

<210> 62
<211> 29
<212> DNA
<213> Artificial Sequence

<220>
<223> Oligonucleotide

<400> 62
cccgctcgag ttaacccacg ttgtaaggt 29

<210> 63
<211> 28
<212> DNA
<213> Artificial Sequence

<220>
<223> Oligonucleotide

<400> 63
cgcggatccc atatggccac aaacgacg 28

<210> 64
<211> 32
<212> DNA
<213> Artificial Sequence

<220>
<223> Oligonucleotide

<400> 64

cccgctcgag tcagtctgac actgttttat cc 32

<210> 65
<211> 32
<212> DNA
<213> Artificial Sequence

<220>
<223> Oligonucleotide

<400> 65
cgcggatccg ctagccccga tgttaaatcg gc 32

<210> 66
<211> 27
<212> DNA
<213> Artificial Sequence

<220>
<223> Oligonucleotide

<400> 66
cccgctcgag ttacgggcgg tattcgg 27

<210> 67
<211> 32
<212> DNA
<213> Artificial Sequence

<220>
<223> Oligonucleotide

<400> 67
cgcggatccg ctagccccga tgttaaatcg gc 32

<210> 68
<211> 32
<212> DNA
<213> Artificial Sequence

<220>
<223> Oligonucleotide

<400> 68
cccgctcgag ttacgtatca tatttcacgt gc 32

<210> 69
<211> 32
<212> DNA
<213> Artificial Sequence

<220>
<223> Oligonucleotide

<400> 69
cgcggatccg ctagccccga tgttaaatcg gc 32

<210> 70

<211> 28
<212> DNA
<213> Artificial Sequence

<220>
<223> Oligonucleotide

<400> 70
cccgctcgag ttaccactcg taattgac 28

<210> 71
<211> 1457
<212> PRT
<213> Neisseria meningitidis

<400> 71

Met Lys Thr Thr Asp Lys Arg Thr Thr Glu Thr His Arg Lys Ala Pro
1 5 10 15

Lys Thr Gly Arg Ile Arg Phe Ser Pro Ala Tyr Leu Ala Ile Cys Leu
20 25 30

Ser Phe Gly Ile Leu Pro Gln Ala Trp Ala Gly His Thr Tyr Phe Gly
35 40 45

Ile Asn Tyr Gln Tyr Tyr Arg Asp Phe Ala Glu Asn Lys Gly Lys Phe
50 55 60

Ala Val Gly Ala Lys Asp Ile Glu Val Tyr Asn Lys Lys Gly Glu Leu
65 70 75 80

Val Gly Lys Ser Met Thr Lys Ala Pro Met Ile Asp Phe Ser Val Val
85 90 95

Ser Arg Asn Gly Val Ala Ala Leu Val Gly Asp Gln Tyr Ile Val Ser
100 105 110

Val Ala His Asn Gly Gly Tyr Asn Asn Val Asp Phe Gly Ala Glu Gly
115 120 125

Arg Asn Pro Asp Gln His Arg Phe Thr Tyr Lys Ile Val Lys Arg Asn
130 135 140

Asn Tyr Lys Ala Gly Thr Lys Gly His Pro Tyr Gly Gly Asp Tyr His

145 150 155 160

Met Pro Arg Leu His Lys Phe Val Thr Asp Ala Glu Pro Val Glu Met
165 170 175

Thr Ser Tyr Met Asp Gly Arg Lys Tyr Ile Asp Gln Asn Asn Tyr Pro
180 185 190

Asp Arg Val Arg Ile Gly Ala Gly Arg Gln Tyr Trp Arg Ser Asp Glu
195 200 205

Asp Glu Pro Asn Asn Arg Glu Ser Ser Tyr His Ile Ala Ser Ala Tyr
210 215 220

Ser Trp Leu Val Gly Gly Asn Thr Phe Ala Gln Asn Gly Ser Gly Gly
225 230 235 240

Gly Thr Val Asn Leu Gly Ser Glu Lys Ile Lys His Ser Pro Tyr Gly
245 250 255

Phe Leu Pro Thr Gly Gly Ser Phe Gly Asp Ser Gly Ser Pro Met Phe
260 265 270

Ile Tyr Asp Ala Gln Lys Gln Lys Trp Leu Ile Asn Gly Val Leu Gln
275 280 285

Thr Gly Asn Pro Tyr Ile Gly Lys Ser Asn Gly Phe Gln Leu Val Arg
290 295 300

Lys Asp Trp Phe Tyr Asp Glu Ile Phe Ala Gly Asp Thr His Ser Val
305 310 315 320

Phe Tyr Glu Pro Arg Gln Asn Gly Lys Tyr Ser Phe Asn Asp Asp Asn
325 330 335

Asn Gly Thr Gly Lys Ile Asn Ala Lys His Glu His Asn Ser Leu Pro
340 345 350

Asn Arg Leu Lys Thr Arg Thr Val Gln Leu Phe Asn Val Ser Leu Ser
355 360 365

Glu Thr Ala Arg Glu Pro Val Tyr His Ala Ala Gly Gly Val Asn Ser
370 375 380

Tyr Arg Pro Arg Leu Asn Asn Gly Glu Asn Ile Ser Phe Ile Asp Glu
385 390 395 400

Gly Lys Gly Glu Leu Ile Leu Thr Ser Asn Ile Asn Gln Gly Ala Gly
405 410 415

Gly Leu Tyr Phe Gln Gly Asp Phe Thr Val Ser Pro Glu Asn Asn Glu
420 425 430

Thr Trp Gln Gly Ala Gly Val His Ile Ser Glu Asp Ser Thr Val Thr
435 440 445

Trp Lys Val Asn Gly Val Ala Asn Asp Arg Leu Ser Lys Ile Gly Lys

```
        450                     455                     460

    Gly Thr Leu His Val Gln Ala Lys Gly Glu Asn Gln Gly Ser Ile Ser
    465                 470                 475                 480

    Val Gly Asp Gly Thr Val Ile Leu Asp Gln Gln Ala Asp Asp Lys Gly
                    485                 490                 495

    Lys Lys Gln Ala Phe Ser Glu Ile Gly Leu Val Ser Gly Arg Gly Thr
                500                 505                 510

    Val Gln Leu Asn Ala Asp Asn Gln Phe Asn Pro Asp Lys Leu Tyr Phe
            515                 520                 525

    Gly Phe Arg Gly Gly Arg Leu Asp Leu Asn Gly His Ser Leu Ser Phe
        530                 535                 540

    His Arg Ile Gln Asn Thr Asp Glu Gly Ala Met Ile Val Asn His Asn
    545                 550                 555                 560

    Gln Asp Lys Glu Ser Thr Val Thr Ile Thr Gly Asn Lys Asp Ile Ala
                    565                 570                 575

    Thr Thr Gly Asn Asn Asn Ser Leu Asp Ser Lys Lys Glu Ile Ala Tyr
                580                 585                 590

    Asn Gly Trp Phe Gly Glu Lys Asp Thr Thr Lys Thr Asn Gly Arg Leu
            595                 600                 605

    Asn Leu Val Tyr Gln Pro Ala Ala Glu Asp Arg Thr Leu Leu Leu Ser
        610                 615                 620

    Gly Gly Thr Asn Leu Asn Gly Asn Ile Thr Gln Thr Asn Gly Lys Leu
    625                 630                 635                 640

    Phe Phe Ser Gly Arg Pro Thr Pro His Ala Tyr Asn His Leu Asn Asp
                    645                 650                 655

    His Trp Ser Gln Lys Glu Gly Ile Pro Arg Gly Glu Ile Val Trp Asp
                660                 665                 670

    Asn Asp Trp Ile Asn Arg Thr Phe Lys Ala Glu Asn Phe Gln Ile Lys
            675                 680                 685

    Gly Gly Gln Ala Val Val Ser Arg Asn Val Ala Lys Val Lys Gly Asp
        690                 695                 700

    Trp His Leu Ser Asn His Ala Gln Ala Val Phe Gly Val Ala Pro His
    705                 710                 715                 720

    Gln Ser His Thr Ile Cys Thr Arg Ser Asp Trp Thr Gly Leu Thr Asn
                    725                 730                 735

    Cys Val Glu Lys Thr Ile Thr Asp Asp Lys Val Ile Ala Ser Leu Thr
                740                 745                 750

    Lys Thr Asp Ile Ser Gly Asn Val Asp Leu Ala Asp His Ala His Leu
```

755 760 765

Asn Leu Thr Gly Leu Ala Thr Leu Asn Gly Asn Leu Ser Ala Asn Gly
770 775 780

Asp Thr Arg Tyr Thr Val Ser His Asn Ala Thr Gln Asn Gly Asn Leu
785 790 795 800

Ser Leu Val Gly Asn Ala Gln Ala Thr Phe Asn Gln Ala Thr Leu Asn
805 810 815

Gly Asn Thr Ser Ala Ser Gly Asn Ala Ser Phe Asn Leu Ser Asp His
820 825 830

Ala Val Gln Asn Gly Ser Leu Thr Leu Ser Gly Asn Ala Lys Ala Asn
835 840 845

Val Ser His Ser Ala Leu Asn Gly Asn Val Ser Leu Ala Asp Lys Ala
850 855 860

Val Phe His Phe Glu Ser Ser Arg Phe Thr Gly Gln Ile Ser Gly Gly
865 870 875 880

Lys Asp Thr Ala Leu His Leu Lys Asp Ser Glu Trp Thr Leu Pro Ser
885 890 895

Gly Thr Glu Leu Gly Asn Leu Asn Leu Asp Asn Ala Thr Ile Thr Leu
900 905 910

Asn Ser Ala Tyr Arg His Asp Ala Ala Gly Ala Gln Thr Gly Ser Ala
915 920 925

Thr Asp Ala Pro Arg Arg Arg Ser Arg Arg Ser Arg Arg Ser Leu Leu
930 935 940

Ser Val Thr Pro Pro Thr Ser Val Glu Ser Arg Phe Asn Thr Leu Thr
945 950 955 960

Val Asn Gly Lys Leu Asn Gly Gln Gly Thr Phe Arg Phe Met Ser Glu
965 970 975

Leu Phe Gly Tyr Arg Ser Asp Lys Leu Lys Leu Ala Glu Ser Ser Glu
980 985 990

Gly Thr Tyr Thr Leu Ala Val Asn Asn Thr Gly Asn Glu Pro Ala Ser
995 1000 1005

Leu Glu Gln Leu Thr Val Val Glu Gly Lys Asp Asn Lys Pro Leu Ser
1010 1015 1020

Glu Asn Leu Asn Phe Thr Leu Gln Asn Glu His Val Asp Ala Gly Ala
1025 1030 1035 1040

Trp Arg Tyr Gln Leu Ile Arg Lys Asp Gly Glu Phe Arg Leu His Asn
1045 1050 1055

Pro Val Lys Glu Gln Glu Leu Ser Asp Lys Leu Gly Lys Ala Glu Ala

1060 1065 1070

Lys Lys Gln Ala Glu Lys Asp Asn Ala Gln Ser Leu Asp Ala Leu Ile
1075 1080 1085

Ala Ala Gly Arg Asp Ala Val Glu Lys Thr Glu Ser Val Ala Glu Pro
1090 1095 1100

Ala Arg Gln Ala Gly Gly Glu Asn Val Gly Ile Met Gln Ala Glu Glu
1105 1110 1115 1120

Glu Lys Lys Arg Val Gln Ala Asp Lys Asp Thr Ala Leu Ala Lys Gln
1125 1130 1135

Arg Glu Ala Glu Thr Arg Pro Ala Thr Thr Ala Phe Pro Arg Ala Arg
1140 1145 1150

Arg Ala Arg Arg Asp Leu Pro Gln Leu Gln Pro Gln Pro Gln Pro Gln
1155 1160 1165

Pro Gln Arg Asp Leu Ile Ser Arg Tyr Ala Asn Ser Gly Leu Ser Glu
1170 1175 1180

Phe Ser Ala Thr Leu Asn Ser Val Phe Ala Val Gln Asp Glu Leu Asp
1185 1190 1195 1200

Arg Val Phe Ala Glu Asp Arg Arg Asn Ala Val Trp Thr Ser Gly Ile
1205 1210 1215

Arg Asp Thr Lys His Tyr Arg Ser Gln Asp Phe Arg Ala Tyr Arg Gln
1220 1225 1230

Gln Thr Asp Leu Arg Gln Ile Gly Met Gln Lys Asn Leu Gly Ser Gly
1235 1240 1245

Arg Val Gly Ile Leu Phe Ser His Asn Arg Thr Glu Asn Thr Phe Asp
1250 1255 1260

Asp Gly Ile Gly Asn Ser Ala Arg Leu Ala His Gly Ala Val Phe Gly
1265 1270 1275 1280

Gln Tyr Gly Ile Asp Arg Phe Tyr Ile Gly Ile Ser Ala Gly Ala Gly
1285 1290 1295

Phe Ser Ser Gly Ser Leu Ser Asp Gly Ile Gly Gly Lys Ile Arg Arg
1300 1305 1310

Arg Val Leu His Tyr Gly Ile Gln Ala Arg Tyr Arg Ala Gly Phe Gly
1315 1320 1325

Gly Phe Gly Ile Glu Pro His Ile Gly Ala Thr Arg Tyr Phe Val Gln
1330 1335 1340

Lys Ala Asp Tyr Arg Tyr Glu Asn Val Asn Ile Ala Thr Pro Gly Leu
1345 1350 1355 1360

Ala Phe Asn Arg Tyr Arg Ala Gly Ile Lys Ala Asp Tyr Ser Phe Lys

1365 1370 1375

Pro Ala Gln His Ile Ser Ile Thr Pro Tyr Leu Ser Leu Ser Tyr Thr
1380 1385 1390

Asp Ala Ala Ser Gly Lys Val Arg Thr Arg Val Asn Thr Ala Val Leu
1395 1400 1405

Ala Gln Asp Phe Gly Lys Thr Arg Ser Ala Glu Trp Gly Val Asn Ala
1410 1415 1420

Glu Ile Lys Gly Phe Thr Leu Ser Leu His Ala Ala Ala Ala Lys Gly
1425 1430 1435 1440

Pro Gln Leu Glu Ala Gln His Ser Ala Gly Ile Lys Leu Gly Tyr Arg
1445 1450 1455

Trp

<210> 72
<211> 21
<212> PRT
<213> Escherichia coli

<400> 72

Met Lys Lys Thr Ala Ile Ala Ile Ala Val Ala Leu Ala Gly Phe Ala
1 5 10 15

Thr Val Ala Gln Ala
20

<210> 73
<211> 1439
<212> PRT
<213> Neisseria meningitidis

<400> 73

Met Lys Lys Thr Ala Ile Ala Ile Ala Val Ala Leu Ala Gly Phe Ala
1 5 10 15

Thr Val Ala Gln Ala Ala Ser Ala Gly His Thr Tyr Phe Gly Ile Asn
20 25 30

Tyr Gln Tyr Tyr Arg Asp Phe Ala Glu Asn Lys Gly Lys Phe Ala Val
35 40 45

Gly Ala Lys Asp Ile Glu Val Tyr Asn Lys Lys Gly Glu Leu Val Gly
50 55 60

Lys Ser Met Thr Lys Ala Pro Met Ile Asp Phe Ser Val Val Ser Arg
65 70 75 80

Asn Gly Val Ala Ala Leu Val Gly Asp Gln Tyr Ile Val Ser Val Ala
85 90 95

His Asn Gly Gly Tyr Asn Asn Val Asp Phe Gly Ala Glu Gly Arg Asn

100 105 110

Pro Asp Gln His Arg Phe Thr Tyr Lys Ile Val Lys Arg Asn Asn Tyr
115 120 125

Lys Ala Gly Thr Lys Gly His Pro Tyr Gly Gly Asp Tyr His Met Pro
130 135 140

Arg Leu His Lys Phe Val Thr Asp Ala Glu Pro Val Glu Met Thr Ser
145 150 155 160

Tyr Met Asp Gly Arg Lys Tyr Ile Asp Gln Asn Asn Tyr Pro Asp Arg
165 170 175

Val Arg Ile Gly Ala Gly Arg Gln Tyr Trp Arg Ser Asp Glu Asp Glu
180 185 190

Pro Asn Asn Arg Glu Ser Ser Tyr His Ile Ala Ser Ala Tyr Ser Trp
195 200 205

Leu Val Gly Gly Asn Thr Phe Ala Gln Asn Gly Ser Gly Gly Gly Thr
210 215 220

Val Asn Leu Gly Ser Glu Lys Ile Lys His Ser Pro Tyr Gly Phe Leu
225 230 235 240

Pro Thr Gly Gly Ser Phe Gly Asp Ser Gly Ser Pro Met Phe Ile Tyr
245 250 255

Asp Ala Gln Lys Gln Lys Trp Leu Ile Asn Gly Val Leu Gln Thr Gly
260 265 270

Asn Pro Tyr Ile Gly Lys Ser Asn Gly Phe Gln Leu Val Arg Lys Asp
275 280 285

Trp Phe Tyr Asp Glu Ile Phe Ala Gly Asp Thr His Ser Val Phe Tyr
290 295 300

Glu Pro Arg Gln Asn Gly Lys Tyr Ser Phe Asn Asp Asp Asn Asn Gly
305 310 315 320

Thr Gly Lys Ile Asn Ala Lys His Glu His Asn Ser Leu Pro Asn Arg
325 330 335

Leu Lys Thr Arg Thr Val Gln Leu Phe Asn Val Ser Leu Ser Glu Thr
340 345 350

Ala Arg Glu Pro Val Tyr His Ala Ala Gly Gly Val Asn Ser Tyr Arg
355 360 365

Pro Arg Leu Asn Asn Gly Glu Asn Ile Ser Phe Ile Asp Glu Gly Lys
370 375 380

Gly Glu Leu Ile Leu Thr Ser Asn Ile Asn Gln Gly Ala Gly Gly Leu
385 390 395 400

Tyr Phe Gln Gly Asp Phe Thr Val Ser Pro Glu Asn Asn Glu Thr Trp

405 410 415

Gln Gly Ala Gly Val His Ile Ser Glu Asp Ser Thr Val Thr Trp Lys
420 425 430

Val Asn Gly Val Ala Asn Asp Arg Leu Ser Lys Ile Gly Lys Gly Thr
435 440 445

Leu His Val Gln Ala Lys Gly Glu Asn Gln Gly Ser Ile Ser Val Gly
450 455 460

Asp Gly Thr Val Ile Leu Asp Gln Gln Ala Asp Asp Lys Gly Lys Lys
465 470 475 480

Gln Ala Phe Ser Glu Ile Gly Leu Val Ser Gly Arg Gly Thr Val Gln
485 490 495

Leu Asn Ala Asp Asn Gln Phe Asn Pro Asp Lys Leu Tyr Phe Gly Phe
500 505 510

Arg Gly Gly Arg Leu Asp Leu Asn Gly His Ser Leu Ser Phe His Arg
515 520 525

Ile Gln Asn Thr Asp Glu Gly Ala Met Ile Val Asn His Asn Gln Asp
530 535 540

Lys Glu Ser Thr Val Thr Ile Thr Gly Asn Lys Asp Ile Ala Thr Thr
545 550 555 560

Gly Asn Asn Asn Ser Leu Asp Ser Lys Lys Glu Ile Ala Tyr Asn Gly
565 570 575

Trp Phe Gly Glu Lys Asp Thr Thr Lys Thr Asn Gly Arg Leu Asn Leu
580 585 590

Val Tyr Gln Pro Ala Ala Glu Asp Arg Thr Leu Leu Leu Ser Gly Gly
595 600 605

Thr Asn Leu Asn Gly Asn Ile Thr Gln Thr Asn Gly Lys Leu Phe Phe
610 615 620

Ser Gly Arg Pro Thr Pro His Ala Tyr Asn His Leu Asn Asp His Trp
625 630 635 640

Ser Gln Lys Glu Gly Ile Pro Arg Gly Glu Ile Val Trp Asp Asn Asp
645 650 655

Trp Ile Asn Arg Thr Phe Lys Ala Glu Asn Phe Gln Ile Lys Gly Gly
660 665 670

Gln Ala Val Val Ser Arg Asn Val Ala Lys Val Lys Gly Asp Trp His
675 680 685

Leu Ser Asn His Ala Gln Ala Val Phe Gly Val Ala Pro His Gln Ser
690 695 700

His Thr Ile Cys Thr Arg Ser Asp Trp Thr Gly Leu Thr Asn Cys Val

705 710 715 720

Glu Lys Thr Ile Thr Asp Asp Lys Val Ile Ala Ser Leu Thr Lys Thr
725 730 735

Asp Ile Ser Gly Asn Val Asp Leu Ala Asp His Ala His Leu Asn Leu
740 745 750

Thr Gly Leu Ala Thr Leu Asn Gly Asn Leu Ser Ala Asn Gly Asp Thr
755 760 765

Arg Tyr Thr Val Ser His Asn Ala Thr Gln Asn Gly Asn Leu Ser Leu
770 775 780

Val Gly Asn Ala Gln Ala Thr Phe Asn Gln Ala Thr Leu Asn Gly Asn
785 790 795 800

Thr Ser Ala Ser Gly Asn Ala Ser Phe Asn Leu Ser Asp His Ala Val
805 810 815

Gln Asn Gly Ser Leu Thr Leu Ser Gly Asn Ala Lys Ala Asn Val Ser
820 825 830

His Ser Ala Leu Asn Gly Asn Val Ser Leu Ala Asp Lys Ala Val Phe
835 840 845

His Phe Glu Ser Ser Arg Phe Thr Gly Gln Ile Ser Gly Gly Lys Asp
850 855 860

Thr Ala Leu His Leu Lys Asp Ser Glu Trp Thr Leu Pro Ser Gly Thr
865 870 875 880

Glu Leu Gly Asn Leu Asn Leu Asp Asn Ala Thr Ile Thr Leu Asn Ser
885 890 895

Ala Tyr Arg His Asp Ala Ala Gly Ala Gln Thr Gly Ser Ala Thr Asp
900 905 910

Ala Pro Arg Arg Arg Ser Arg Arg Ser Arg Arg Ser Leu Leu Ser Val
915 920 925

Thr Pro Pro Thr Ser Val Glu Ser Arg Phe Asn Thr Leu Thr Val Asn
930 935 940

Gly Lys Leu Asn Gly Gln Gly Thr Phe Arg Phe Met Ser Glu Leu Phe
945 950 955 960

Gly Tyr Arg Ser Asp Lys Leu Lys Leu Ala Glu Ser Ser Glu Gly Thr
965 970 975

Tyr Thr Leu Ala Val Asn Asn Thr Gly Asn Glu Pro Ala Ser Leu Glu
980 985 990

Gln Leu Thr Val Val Glu Gly Lys Asp Asn Lys Pro Leu Ser Glu Asn
995 1000 1005

Leu Asn Phe Thr Leu Gln Asn Glu His Val Asp Ala Gly Ala Trp Arg

1010 1015 1020

Tyr Gln Leu Ile Arg Lys Asp Gly Glu Phe Arg Leu His Asn Pro Val
1025 1030 1035 1040

Lys Glu Gln Glu Leu Ser Asp Lys Leu Gly Lys Ala Glu Ala Lys Lys
1045 1050 1055

Gln Ala Glu Lys Asp Asn Ala Gln Ser Leu Asp Ala Leu Ile Ala Ala
1060 1065 1070

Gly Arg Asp Ala Val Glu Lys Thr Glu Ser Val Ala Glu Pro Ala Arg
1075 1080 1085

Gln Ala Gly Gly Glu Asn Val Gly Ile Met Gln Ala Glu Glu Glu Lys
1090 1095 1100

Lys Arg Val Gln Ala Asp Lys Asp Thr Ala Leu Ala Lys Gln Arg Glu
1105 1110 1115 1120

Ala Glu Thr Arg Pro Ala Thr Thr Ala Phe Pro Arg Ala Arg Arg Ala
1125 1130 1135

Arg Arg Asp Leu Pro Gln Leu Gln Pro Gln Pro Gln Pro Gln Pro Gln
1140 1145 1150

Arg Asp Leu Ile Ser Arg Tyr Ala Asn Ser Gly Leu Ser Glu Phe Ser
1155 1160 1165

Ala Thr Leu Asn Ser Val Phe Ala Val Gln Asp Glu Leu Asp Arg Val
1170 1175 1180

Phe Ala Glu Asp Arg Arg Asn Ala Val Trp Thr Ser Gly Ile Arg Asp
1185 1190 1195 1200

Thr Lys His Tyr Arg Ser Gln Asp Phe Arg Ala Tyr Arg Gln Gln Thr
1205 1210 1215

Asp Leu Arg Gln Ile Gly Met Gln Lys Asn Leu Gly Ser Gly Arg Val
1220 1225 1230

Gly Ile Leu Phe Ser His Asn Arg Thr Glu Asn Thr Phe Asp Asp Gly
1235 1240 1245

Ile Gly Asn Ser Ala Arg Leu Ala His Gly Ala Val Phe Gly Gln Tyr
1250 1255 1260

Gly Ile Asp Arg Phe Tyr Ile Gly Ile Ser Ala Gly Ala Gly Phe Ser
1265 1270 1275 1280

Ser Gly Ser Leu Ser Asp Gly Ile Gly Gly Lys Ile Arg Arg Arg Val
1285 1290 1295

Leu His Tyr Gly Ile Gln Ala Arg Tyr Arg Ala Gly Phe Gly Gly Phe
1300 1305 1310

Gly Ile Glu Pro His Ile Gly Ala Thr Arg Tyr Phe Val Gln Lys Ala

1315 1320 1325

Asp Tyr Arg Tyr Glu Asn Val Asn Ile Ala Thr Pro Gly Leu Ala Phe
1330 1335 1340

Asn Arg Tyr Arg Ala Gly Ile Lys Ala Asp Tyr Ser Phe Lys Pro Ala
1345 1350 1355 1360

Gln His Ile Ser Ile Thr Pro Tyr Leu Ser Leu Ser Tyr Thr Asp Ala
1365 1370 1375

Ala Ser Gly Lys Val Arg Thr Arg Val Asn Thr Ala Val Leu Ala Gln
1380 1385 1390

Asp Phe Gly Lys Thr Arg Ser Ala Glu Trp Gly Val Asn Ala Glu Ile
1395 1400 1405

Lys Gly Phe Thr Leu Ser Leu His Ala Ala Ala Ala Lys Gly Pro Gln
1410 1415 1420

Leu Glu Ala Gln His Ser Ala Gly Ile Lys Leu Gly Tyr Arg Trp
1425 1430 1435

<210> 74
<211> 164
<212> PRT
<213> Neisseria meningitidis

<400> 74

Met Lys Lys Asn Ile Leu Glu Phe Trp Val Gly Leu Phe Val Leu Ile
1               5                   10                  15

Gly Ala Ala Ala Val Ala Phe Leu Ala Phe Arg Val Ala Gly Gly Ala
            20                  25                  30

Ala Phe Gly Gly Ser Asp Lys Thr Tyr Ala Val Tyr Ala Asp Phe Gly
        35                  40                  45

Asp Ile Gly Gly Leu Lys Val Asn Ala Pro Val Lys Ser Ala Gly Val
    50                  55                  60

Leu Val Gly Arg Val Gly Ala Ile Gly Leu Asp Pro Lys Ser Tyr Gln
65                  70                  75                  80

Ala Arg Val Arg Leu Asp Leu Asp Gly Lys Tyr Gln Phe Ser Ser Asp
                85                  90                  95

Val Ser Ala Gln Ile Leu Thr Ser Gly Leu Leu Gly Glu Gln Tyr Ile
            100                 105                 110

Gly Leu Gln Gln Gly Gly Asp Thr Glu Asn Leu Ala Ala Gly Asp Thr
        115                 120                 125

Ile Ser Val Thr Ser Ser Ala Met Val Leu Glu Asn Leu Ile Gly Lys
    130                 135                 140

Phe Met Thr Ser Phe Ala Glu Lys Asn Ala Asp Gly Gly Asn Ala Glu

145                 150                 155                 160

Lys Ala Ala Glu

<210> 75
<211> 21
<212> PRT
<213> Erwinia carotovora

<400> 75

Met Lys Tyr Leu Leu Pro Thr Ala Ala Ala Gly Leu Leu Leu Ala Ala
1               5                   10                  15

Gln Pro Ala Met Ala
            20

<210> 76
<211> 608
<212> PRT
<213> Neisseria meningitidis ORF46

<400> 76

```
Leu Gly Ile Ser Arg Lys Ile Ser Leu Ile Leu Ser Ile Leu Ala Val
1               5                   10                  15

Cys Leu Pro Met His Ala His Ala Ser Asp Leu Ala Asn Asp Ser Phe
            20                  25                  30

Ile Arg Gln Val Leu Asp Arg Gln His Phe Glu Pro Asp Gly Lys Tyr
        35                  40                  45

His Leu Phe Gly Ser Arg Gly Glu Leu Ala Glu Arg Ser Gly His Ile
    50                  55                  60

Gly Leu Gly Lys Ile Gln Ser His Gln Leu Gly Asn Leu Met Ile Gln
65                  70                  75                  80

Gln Ala Ala Ile Lys Gly Asn Ile Gly Tyr Ile Val Arg Phe Ser Asp
                85                  90                  95

His Gly His Glu Val His Ser Pro Phe Asp Asn His Ala Ser His Ser
            100                 105                 110

Asp Ser Asp Glu Ala Gly Ser Pro Val Asp Gly Phe Ser Leu Tyr Arg
        115                 120                 125

Ile His Trp Asp Gly Tyr Glu His His Pro Ala Asp Gly Tyr Asp Gly
    130                 135                 140

Pro Gln Gly Gly Gly Tyr Pro Ala Pro Lys Gly Ala Arg Asp Ile Tyr
145                 150                 155                 160

Ser Tyr Asp Ile Lys Gly Val Ala Gln Asn Ile Arg Leu Asn Leu Thr
                165                 170                 175

Asp Asn Arg Ser Thr Gly Gln Arg Leu Ala Asp Arg Phe His Asn Ala
```

```
            180                     185                     190

Gly Ser Met Leu Thr Gln Gly Val Gly Asp Gly Phe Lys Arg Ala Thr
        195                     200                     205

Arg Tyr Ser Pro Glu Leu Asp Arg Ser Gly Asn Ala Ala Glu Ala Phe
    210                     215                     220

Asn Gly Thr Ala Asp Ile Val Lys Asn Ile Ile Gly Ala Ala Gly Glu
225                     230                     235                 240

Ile Val Gly Ala Gly Asp Ala Val Gln Gly Ile Ser Glu Gly Ser Asn
                245                     250                     255

Ile Ala Val Met His Gly Leu Gly Leu Leu Ser Thr Glu Asn Lys Met
            260                     265                     270

Ala Arg Ile Asn Asp Leu Ala Asp Met Ala Gln Leu Lys Asp Tyr Ala
        275                     280                     285

Ala Ala Ala Ile Arg Asp Trp Ala Val Gln Asn Pro Asn Ala Ala Gln
    290                     295                     300

Gly Ile Glu Ala Val Ser Asn Ile Phe Met Ala Ala Ile Pro Ile Lys
305                     310                     315                 320

Gly Ile Gly Ala Val Arg Gly Lys Tyr Gly Leu Gly Gly Ile Thr Ala
                325                     330                     335

His Pro Ile Lys Arg Ser Gln Met Gly Ala Ile Ala Leu Pro Lys Gly
            340                     345                     350

Lys Ser Ala Val Ser Asp Asn Phe Ala Asp Ala Ala Tyr Ala Lys Tyr
        355                     360                     365

Pro Ser Pro Tyr His Ser Arg Asn Ile Arg Ser Asn Leu Glu Gln Arg
    370                     375                     380

Tyr Gly Lys Glu Asn Ile Thr Ser Ser Thr Val Pro Pro Ser Asn Gly
385                     390                     395                 400

Lys Asn Val Lys Leu Ala Asp Gln Arg His Pro Lys Thr Gly Val Pro
                405                     410                     415

Phe Asp Gly Lys Gly Phe Pro Asn Phe Glu Lys His Val Lys Tyr Asp
            420                     425                     430

Thr Lys Leu Asp Ile Gln Glu Leu Ser Gly Gly Gly Ile Pro Lys Ala
        435                     440                     445

Lys Pro Val Ser Asp Ala Lys Pro Arg Trp Glu Val Asp Arg Lys Leu
    450                     455                     460

Asn Lys Leu Thr Thr Arg Glu Gln Val Glu Lys Asn Val Gln Glu Ile
465                     470                     475                 480

Arg Asn Gly Asn Lys Asn Ser Asn Phe Ser Gln His Ala Gln Leu Glu
```

485 490 495

Arg Glu Ile Asn Lys Leu Lys Ser Ala Asp Glu Ile Asn Phe Ala Asp
500 505 510

Gly Met Gly Lys Phe Thr Asp Ser Met Asn Asp Lys Ala Phe Ser Arg
515 520 525

Leu Val Lys Ser Val Lys Glu Asn Gly Phe Thr Asn Pro Val Val Glu
530 535 540

Tyr Val Glu Ile Asn Gly Lys Ala Tyr Ile Val Arg Gly Asn Asn Arg
545 550 555 560

Val Phe Ala Ala Glu Tyr Leu Gly Arg Ile His Glu Leu Lys Phe Lys
565 570 575

Lys Val Asp Phe Pro Val Pro Asn Thr Ser Trp Lys Asn Pro Thr Asp
580 585 590

Val Leu Asn Glu Ser Gly Asn Val Lys Arg Pro Arg Tyr Arg Ser Lys
595 600 605

<210> 77
<211> 584
<212> PRT
<213> Artificial Sequence

<220>
<223> ORF46-2

<400> 77

```
Ser Asp Leu Ala Asn Asp Ser Phe Ile Arg Gln Val Leu Asp Arg Gln
1               5                   10                  15

His Phe Glu Pro Asp Gly Lys Tyr His Leu Phe Gly Ser Arg Gly Glu
            20                  25                  30

Leu Ala Glu Arg Ser Gly His Ile Gly Leu Gly Lys Ile Gln Ser His
        35                  40                  45

Gln Leu Gly Asn Leu Met Ile Gln Gln Ala Ala Ile Lys Gly Asn Ile
    50                  55                  60

Gly Tyr Ile Val Arg Phe Ser Asp His Gly His Glu Val His Ser Pro
65                  70                  75                  80

Phe Asp Asn His Ala Ser His Ser Asp Ser Asp Glu Ala Gly Ser Pro
                85                  90                  95

Val Asp Gly Phe Ser Leu Tyr Arg Ile His Trp Asp Gly Tyr Glu His
            100                 105                 110

His Pro Ala Asp Gly Tyr Asp Gly Pro Gln Gly Gly Gly Tyr Pro Ala
        115                 120                 125
```

Pro Lys Gly Ala Arg Asp Ile Tyr Ser Tyr Asp Ile Lys Gly Val Ala
130 135 140

Gln Asn Ile Arg Leu Asn Leu Thr Asp Asn Arg Ser Thr Gly Gln Arg
145 150 155 160

Leu Ala Asp Arg Phe His Asn Ala Gly Ser Met Leu Thr Gln Gly Val
165 170 175

Gly Asp Gly Phe Lys Arg Ala Thr Arg Tyr Ser Pro Glu Leu Asp Arg
180 185 190

Ser Gly Asn Ala Ala Glu Ala Phe Asn Gly Thr Ala Asp Ile Val Lys
195 200 205

Asn Ile Ile Gly Ala Ala Gly Glu Ile Val Gly Ala Gly Asp Ala Val
210 215 220

Gln Gly Ile Ser Glu Gly Ser Asn Ile Ala Val Met His Gly Leu Gly
225 230 235 240

Leu Leu Ser Thr Glu Asn Lys Met Ala Arg Ile Asn Asp Leu Ala Asp
245 250 255

Met Ala Gln Leu Lys Asp Tyr Ala Ala Ala Ala Ile Arg Asp Trp Ala
260 265 270

Val Gln Asn Pro Asn Ala Ala Gln Gly Ile Glu Ala Val Ser Asn Ile
275 280 285

Phe Met Ala Ala Ile Pro Ile Lys Gly Ile Gly Ala Val Arg Gly Lys
290 295 300

Tyr Gly Leu Gly Gly Ile Thr Ala His Pro Ile Lys Arg Ser Gln Met
305 310 315 320

Gly Ala Ile Ala Leu Pro Lys Gly Lys Ser Ala Val Ser Asp Asn Phe
325 330 335

Ala Asp Ala Ala Tyr Ala Lys Tyr Pro Ser Pro Tyr His Ser Arg Asn
340 345 350

Ile Arg Ser Asn Leu Glu Gln Arg Tyr Gly Lys Glu Asn Ile Thr Ser
355 360 365

Ser Thr Val Pro Pro Ser Asn Gly Lys Asn Val Lys Leu Ala Asp Gln
370 375 380

Arg His Pro Lys Thr Gly Val Pro Phe Asp Gly Lys Gly Phe Pro Asn
385 390 395 400

Phe Glu Lys His Val Lys Tyr Asp Thr Lys Leu Asp Ile Gln Glu Leu
405 410 415

Ser Gly Gly Gly Ile Pro Lys Ala Lys Pro Val Ser Asp Ala Lys Pro
420 425 430

Arg Trp Glu Val Asp Arg Lys Leu Asn Lys Leu Thr Thr Arg Glu Gln
435 440 445

Val Glu Lys Asn Val Gln Glu Ile Arg Asn Gly Asn Lys Asn Ser Asn
450 455 460

Phe Ser Gln His Ala Gln Leu Glu Arg Glu Ile Asn Lys Leu Lys Ser
465 470 475 480

Ala Asp Glu Ile Asn Phe Ala Asp Gly Met Gly Lys Phe Thr Asp Ser
485 490 495

Met Asn Asp Lys Ala Phe Ser Arg Leu Val Lys Ser Val Lys Glu Asn
500 505 510

Gly Phe Thr Asn Pro Val Val Glu Tyr Val Glu Ile Asn Gly Lys Ala
515 520 525

Tyr Ile Val Arg Gly Asn Asn Arg Val Phe Ala Ala Glu Tyr Leu Gly
530 535 540

Arg Ile His Glu Leu Lys Phe Lys Lys Val Asp Phe Pro Val Pro Asn
545 550 555 560

Thr Ser Trp Lys Asn Pro Thr Asp Val Leu Asn Glu Ser Gly Asn Val
565 570 575

Lys Arg Pro Arg Tyr Arg Ser Lys
580

<210> 78
<211> 364
<212> PRT
<213> Neisseria meningitidis

<400> 78

Met Ser Met Lys His Phe Pro Ala Lys Val Leu Thr Thr Ala Ile Leu
1 5 10 15

Ala Thr Phe Cys Ser Gly Ala Leu Ala Ala Thr Ser Asp Asp Asp Val
20 25 30

Lys Lys Ala Ala Thr Val Ala Ile Val Ala Ala Tyr Asn Asn Gly Gln
35 40 45

Glu Ile Asn Gly Phe Lys Ala Gly Glu Thr Ile Tyr Asp Ile Gly Glu
50 55 60

Asp Gly Thr Ile Thr Gln Lys Asp Ala Thr Ala Ala Asp Val Glu Ala
65 70 75 80

Asp Asp Phe Lys Gly Leu Gly Leu Lys Lys Val Val Thr Asn Leu Thr
85 90 95

Lys Thr Val Asn Glu Asn Lys Gln Asn Val Asp Ala Lys Val Lys Ala
100 105 110

Ala Glu Ser Glu Ile Glu Lys Leu Thr Thr Lys Leu Ala Asp Thr Asp
115 120 125

Ala Ala Leu Ala Asp Thr Asp Ala Ala Leu Asp Glu Thr Thr Asn Ala
130 135 140

Leu Asn Lys Leu Gly Glu Asn Ile Thr Thr Phe Ala Glu Glu Thr Lys
145 150 155 160

Thr Asn Ile Val Lys Ile Asp Glu Lys Leu Glu Ala Val Ala Asp Thr
165 170 175

Val Asp Lys His Ala Glu Ala Phe Asn Asp Ile Ala Asp Ser Leu Asp
180 185 190

Glu Thr Asn Thr Lys Ala Asp Glu Ala Val Lys Thr Ala Asn Glu Ala
195 200 205

Lys Gln Thr Ala Glu Glu Thr Lys Gln Asn Val Asp Ala Lys Val Lys
210 215 220

Ala Ala Glu Thr Ala Ala Gly Lys Ala Glu Ala Ala Ala Gly Thr Ala
225 230 235 240

Asn Thr Ala Ala Asp Lys Ala Glu Ala Val Ala Ala Lys Val Thr Asp
245 250 255

Ile Lys Ala Asp Ile Ala Thr Asn Lys Ala Asp Ile Ala Lys Asn Ser
260 265 270

Ala Arg Ile Asp Ser Leu Asp Lys Asn Val Ala Asn Leu Arg Lys Glu
275 280 285

Thr Arg Gln Gly Leu Ala Glu Gln Ala Ala Leu Ser Gly Leu Phe Gln
290 295 300

Pro Tyr Asn Val Gly Arg Phe Asn Val Thr Ala Ala Val Gly Gly Tyr
305 310 315 320

Lys Ser Glu Ser Ala Val Ala Ile Gly Thr Gly Phe Arg Phe Thr Glu
325 330 335

Asn Phe Ala Ala Lys Ala Gly Val Ala Val Gly Thr Ser Ser Gly Ser
340 345 350

Ser Ala Ala Tyr His Val Gly Val Asn Tyr Glu Trp
355 360

<210> 79
<211> 427
<212> PRT
<213> Neisseria meningitidis

<400> 79

Met Phe Glu Arg Ser Val Ile Ala Met Ala Cys Ile Phe Ala Leu Ser
1               5                   10                  15

```
Ala Cys Gly Gly Gly Gly Gly Gly Ser Pro Asp Val Lys Ser Ala Asp
            20                  25                  30

Thr Leu Ser Lys Pro Ala Ala Pro Val Val Ala Glu Lys Glu Thr Glu
        35                  40                  45

Val Lys Glu Asp Ala Pro Gln Ala Gly Ser Gln Gly Gln Gly Ala Pro
    50                  55                  60

Ser Thr Gln Gly Ser Gln Asp Met Ala Ala Val Ser Ala Glu Asn Thr
65                  70                  75                  80

Gly Asn Gly Gly Ala Ala Thr Thr Asp Lys Pro Lys Asn Glu Asp Glu
                85                  90                  95

Gly Pro Gln Asn Asp Met Pro Gln Asn Ser Ala Glu Ser Ala Asn Gln
            100                 105                 110

Thr Gly Asn Asn Gln Pro Ala Asp Ser Ser Asp Ser Ala Pro Ala Ser
        115                 120                 125

Asn Pro Ala Pro Ala Asn Gly Gly Ser Asn Phe Gly Arg Val Asp Leu
    130                 135                 140

Ala Asn Gly Val Leu Ile Asp Gly Pro Ser Gln Asn Ile Thr Leu Thr
145                 150                 155                 160

His Cys Lys Gly Asp Ser Cys Asn Gly Asp Asn Leu Leu Asp Glu Glu
                165                 170                 175

Ala Pro Ser Lys Ser Glu Phe Glu Asn Leu Asn Glu Ser Glu Arg Ile
            180                 185                 190

Glu Lys Tyr Lys Lys Asp Gly Lys Ser Asp Lys Phe Thr Asn Leu Val
        195                 200                 205

Ala Thr Ala Val Gln Ala Asn Gly Thr Asn Lys Tyr Val Ile Ile Tyr
    210                 215                 220

Lys Asp Lys Ser Ala Ser Ser Ser Ser Ala Arg Phe Arg Arg Ser Ala
225                 230                 235                 240

Arg Ser Arg Arg Ser Leu Pro Ala Glu Met Pro Leu Ile Pro Val Asn
                245                 250                 255

Gln Ala Asp Thr Leu Ile Val Asp Gly Glu Ala Val Ser Leu Thr Gly
            260                 265                 270

His Ser Gly Asn Ile Phe Ala Pro Glu Gly Asn Tyr Arg Tyr Leu Thr
        275                 280                 285

Tyr Gly Ala Glu Lys Leu Pro Gly Gly Ser Tyr Ala Leu Arg Val Gln
    290                 295                 300

Gly Glu Pro Ala Lys Gly Glu Met Leu Ala Gly Thr Ala Val Tyr Asn
305                 310                 315                 320
```

Gly Glu Val Leu His Phe His Thr Glu Asn Gly Arg Pro Tyr Pro Thr
325 330 335

Arg Gly Arg Phe Ala Ala Lys Val Asp Phe Gly Ser Lys Ser Val Asp
340 345 350

Gly Ile Ile Asp Ser Gly Asp Asp Leu His Met Gly Thr Gln Lys Phe
355 360 365

Lys Ala Ala Ile Asp Gly Asn Gly Phe Lys Gly Thr Trp Thr Glu Asn
370 375 380

Gly Gly Gly Asp Val Ser Gly Arg Phe Tyr Gly Pro Ala Gly Glu Glu
385 390 395 400

Val Ala Gly Lys Tyr Ser Tyr Arg Pro Thr Asp Ala Glu Lys Gly Gly
405 410 415

Phe Gly Val Phe Ala Gly Lys Lys Glu Gln Asp
420 425

<210> 80
<211> 410
<212> PRT
<213> Artificial Sequence

<220>
<223> 287untagged

<400> 80

Cys Gly Gly Gly Gly Gly Gly Ser Pro Asp Val Lys Ser Ala Asp Thr
1 5 10 15

Leu Ser Lys Pro Ala Ala Pro Val Val Ala Glu Lys Glu Thr Glu Val
20 25 30

Lys Glu Asp Ala Pro Gln Ala Gly Ser Gln Gly Gln Gly Ala Pro Ser
35 40 45

Thr Gln Gly Ser Gln Asp Met Ala Ala Val Ser Ala Glu Asn Thr Gly
50 55 60

Asn Gly Gly Ala Ala Thr Thr Asp Lys Pro Lys Asn Glu Asp Glu Gly
65 70 75 80

Pro Gln Asn Asp Met Pro Gln Asn Ser Ala Glu Ser Ala Asn Gln Thr
85 90 95

Gly Asn Asn Gln Pro Ala Asp Ser Ser Asp Ser Ala Pro Ala Ser Asn
100 105 110

Pro Ala Pro Ala Asn Gly Gly Ser Asn Phe Gly Arg Val Asp Leu Ala
115 120 125

Asn Gly Val Leu Ile Asp Gly Pro Ser Gln Asn Ile Thr Leu Thr His
130 135 140

Cys Lys Gly Asp Ser Cys Asn Gly Asp Asn Leu Leu Asp Glu Glu Ala
145 150 155 160

Pro Ser Lys Ser Glu Phe Glu Asn Leu Asn Glu Ser Glu Arg Ile Glu
165 170 175

Lys Tyr Lys Lys Asp Gly Lys Ser Asp Lys Phe Thr Asn Leu Val Ala
180 185 190

Thr Ala Val Gln Ala Asn Gly Thr Asn Lys Tyr Val Ile Ile Tyr Lys
195 200 205

Asp Lys Ser Ala Ser Ser Ser Ser Ala Arg Phe Arg Arg Ser Ala Arg
210 215 220

Ser Arg Arg Ser Leu Pro Ala Glu Met Pro Leu Ile Pro Val Asn Gln
225 230 235 240

Ala Asp Thr Leu Ile Val Asp Gly Glu Ala Val Ser Leu Thr Gly His
245 250 255

Ser Gly Asn Ile Phe Ala Pro Glu Gly Asn Tyr Arg Tyr Leu Thr Tyr
260 265 270

Gly Ala Glu Lys Leu Pro Gly Gly Ser Tyr Ala Leu Arg Val Gln Gly
275 280 285

Glu Pro Ala Lys Gly Glu Met Leu Ala Gly Thr Ala Val Tyr Asn Gly
290 295 300

Glu Val Leu His Phe His Thr Glu Asn Gly Arg Pro Tyr Pro Thr Arg
305 310 315 320

Gly Arg Phe Ala Ala Lys Val Asp Phe Gly Ser Lys Ser Val Asp Gly
325 330 335

Ile Ile Asp Ser Gly Asp Asp Leu His Met Gly Thr Gln Lys Phe Lys
340 345 350

Ala Ala Ile Asp Gly Asn Gly Phe Lys Gly Thr Trp Thr Glu Asn Gly
355 360 365

Gly Gly Asp Val Ser Gly Arg Phe Tyr Gly Pro Ala Gly Glu Glu Val
370 375 380

Ala Gly Lys Tyr Ser Tyr Arg Pro Thr Asp Ala Glu Lys Gly Gly Phe
385 390 395 400

Gly Val Phe Ala Gly Lys Lys Glu Gln Asp
405 410

<210> 81
<211> 9
<212> PRT
<213> Artificial Sequence

<220>

<223> 920L N-terminal

<400> 81

His Arg Val Trp Val Glu Thr Ala His
1 5

<210> 82
<211> 16
<212> PRT
<213> Artificial Sequence

<220>
<223> 953L N-terminal

<400> 82

Ala Thr Tyr Lys Val Asp Glu Tyr His Ala Asn Ala Arg Phe Ala Phe
1 5 10 15

<210> 83
<211> 9
<212> PRT
<213> Artificial Sequence

<220>
<223> 519.1L N-terminal

<400> 83

Met Glu Phe Phe Ile Ile Leu Leu Ala
1 5

<210> 84
<211> 488
<212> PRT
<213> Artificial Sequence

<220>
<223> deltaG287

<400> 84

```
Met Phe Lys Arg Ser Val Ile Ala Met Ala Cys Ile Phe Ala Leu Ser
1               5                   10                  15

Ala Cys Gly Gly Gly Gly Gly Gly Ser Pro Asp Val Lys Ser Ala Asp
            20                  25                  30

Thr Leu Ser Lys Pro Ala Ala Pro Val Val Ser Glu Lys Glu Thr Glu
        35                  40                  45

Ala Lys Glu Asp Ala Pro Gln Ala Gly Ser Gln Gly Gln Gly Ala Pro
    50                  55                  60

Ser Ala Gln Gly Ser Gln Asp Met Ala Ala Val Ser Glu Glu Asn Thr
65                  70                  75                  80

Gly Asn Gly Gly Ala Val Thr Ala Asp Asn Pro Lys Asn Glu Asp Glu
                85                  90                  95
```

Val Ala Gln Asn Asp Met Pro Gln Asn Ala Ala Gly Thr Asp Ser Ser
100 105 110

Thr Pro Asn His Thr Pro Asp Pro Asn Met Leu Ala Gly Asn Met Glu
115 120 125

Asn Gln Ala Thr Asp Ala Gly Glu Ser Ser Gln Pro Ala Asn Gln Pro
130 135 140

Asp Met Ala Asn Ala Ala Asp Gly Met Gln Gly Asp Asp Pro Ser Ala
145 150 155 160

Gly Gly Gln Asn Ala Gly Asn Thr Ala Ala Gln Gly Ala Asn Gln Ala
165 170 175

Gly Asn Asn Gln Ala Ala Gly Ser Ser Asp Pro Ile Pro Ala Ser Asn
180 185 190

Pro Ala Pro Ala Asn Gly Gly Ser Asn Phe Gly Arg Val Asp Leu Ala
195 200 205

Asn Gly Val Leu Ile Asp Gly Pro Ser Gln Asn Ile Thr Leu Thr His
210 215 220

Cys Lys Gly Asp Ser Cys Ser Gly Asn Asn Phe Leu Asp Glu Glu Val
225 230 235 240

Gln Leu Lys Ser Glu Phe Glu Lys Leu Ser Asp Ala Asp Lys Ile Ser
245 250 255

Asn Tyr Lys Lys Asp Gly Lys Asn Asp Lys Phe Val Gly Leu Val Ala
260 265 270

Asp Ser Val Gln Met Lys Gly Ile Asn Gln Tyr Ile Ile Phe Tyr Lys
275 280 285

Pro Lys Pro Thr Ser Phe Ala Arg Phe Arg Arg Ser Ala Arg Ser Arg
290 295 300

Arg Ser Leu Pro Ala Glu Met Pro Leu Ile Pro Val Asn Gln Ala Asp
305 310 315 320

Thr Leu Ile Val Asp Gly Glu Ala Val Ser Leu Thr Gly His Ser Gly
325 330 335

Asn Ile Phe Ala Pro Glu Gly Asn Tyr Arg Tyr Leu Thr Tyr Gly Ala
340 345 350

Glu Lys Leu Pro Gly Gly Ser Tyr Ala Leu Arg Val Gln Gly Glu Pro
355 360 365

Ala Lys Gly Glu Met Leu Ala Gly Ala Ala Val Tyr Asn Gly Glu Val
370 375 380

Leu His Phe His Thr Glu Asn Gly Arg Pro Tyr Pro Thr Arg Gly Arg
385 390 395 400

Phe Ala Ala Lys Val Asp Phe Gly Ser Lys Ser Val Asp Gly Ile Ile
405 410 415

Asp Ser Gly Asp Asp Leu His Met Gly Thr Gln Lys Phe Lys Ala Ala
420 425 430

Ile Asp Gly Asn Gly Phe Lys Gly Thr Trp Thr Glu Asn Gly Ser Gly
435 440 445

Asp Val Ser Gly Lys Phe Tyr Gly Pro Ala Gly Glu Glu Val Ala Gly
450 455 460

Lys Tyr Ser Tyr Arg Pro Thr Asp Ala Glu Lys Gly Gly Phe Gly Val
465 470 475 480

Phe Ala Gly Lys Lys Glu Gln Asp
485

<210> 85
<211> 712
<212> PRT
<213> Artificial Sequence

<220>
<223> TBP2

<400> 85

```
Met Asn Asn Pro Leu Val Asn Gln Ala Ala Met Val Leu Pro Val Phe
1               5                   10                  15

Leu Leu Ser Ala Cys Leu Gly Gly Gly Gly Ser Phe Asp Leu Asp Ser
            20                  25                  30

Val Asp Thr Glu Ala Pro Arg Pro Ala Pro Lys Tyr Gln Asp Val Phe
        35                  40                  45

Ser Glu Lys Pro Gln Ala Gln Lys Asp Gln Gly Gly Tyr Gly Phe Ala
    50                  55                  60

Met Arg Leu Lys Arg Arg Asn Trp Tyr Pro Gln Ala Lys Glu Asp Glu
65                  70                  75                  80

Val Lys Leu Asp Glu Ser Asp Trp Glu Ala Thr Gly Leu Pro Asp Glu
                85                  90                  95

Pro Lys Glu Leu Pro Lys Arg Gln Lys Ser Val Ile Glu Lys Val Glu
            100                 105                 110

Thr Asp Ser Asp Asn Asn Ile Tyr Ser Ser Pro Tyr Leu Lys Pro Ser
        115                 120                 125

Asn His Gln Asn Gly Asn Thr Gly Asn Gly Ile Asn Gln Pro Lys Asn
    130                 135                 140

Gln Ala Lys Asp Tyr Glu Asn Phe Lys Tyr Val Tyr Ser Gly Trp Phe
145                 150                 155                 160
```

```
Tyr Lys His Ala Lys Arg Glu Phe Asn Leu Lys Val Glu Pro Lys Ser
                165                 170                 175

Ala Lys Asn Gly Asp Asp Gly Tyr Ile Phe Tyr His Gly Lys Glu Pro
            180                 185                 190

Ser Arg Gln Leu Pro Ala Ser Gly Lys Ile Thr Tyr Lys Gly Val Trp
        195                 200                 205

His Phe Ala Thr Asp Thr Lys Lys Gly Gln Lys Phe Arg Glu Ile Ile
    210                 215                 220

Gln Pro Ser Lys Ser Gln Gly Asp Arg Tyr Ser Gly Phe Ser Gly Asp
225                 230                 235                 240

Asp Gly Glu Glu Tyr Ser Asn Lys Asn Lys Ser Thr Leu Thr Asp Gly
                245                 250                 255

Gln Glu Gly Tyr Gly Phe Thr Ser Asn Leu Glu Val Asp Phe His Asn
            260                 265                 270

Lys Lys Leu Thr Gly Lys Leu Ile Arg Asn Asn Ala Asn Thr Asp Asn
        275                 280                 285

Asn Gln Ala Thr Thr Thr Gln Tyr Tyr Ser Leu Glu Ala Gln Val Thr
    290                 295                 300

Gly Asn Arg Phe Asn Gly Lys Ala Thr Ala Thr Asp Lys Pro Gln Gln
305                 310                 315                 320

Asn Ser Glu Thr Lys Glu His Pro Phe Val Ser Asp Ser Ser Ser Leu
                325                 330                 335

Ser Gly Gly Phe Phe Gly Pro Gln Gly Glu Glu Leu Gly Phe Arg Phe
            340                 345                 350

Leu Ser Asp Asp Gln Lys Val Ala Val Val Gly Ser Ala Lys Thr Lys
        355                 360                 365

Asp Lys Pro Ala Asn Gly Asn Thr Ala Ala Ala Ser Gly Gly Thr Asp
    370                 375                 380

Ala Ala Ala Ser Asn Gly Ala Ala Gly Thr Ser Ser Glu Asn Gly Lys
385                 390                 395                 400

Leu Thr Thr Val Leu Asp Ala Val Glu Leu Lys Leu Gly Asp Lys Glu
                405                 410                 415

Val Gln Lys Leu Asp Asn Phe Ser Asn Ala Ala Gln Leu Val Val Asp
            420                 425                 430

Gly Ile Met Ile Pro Leu Leu Pro Glu Ala Ser Glu Ser Gly Asn Asn
        435                 440                 445

Gln Ala Asn Gln Gly Thr Asn Gly Gly Thr Ala Phe Thr Arg Lys Phe
    450                 455                 460
```

Asp His Thr Pro Glu Ser Asp Lys Lys Asp Ala Gln Ala Gly Thr Gln
465 470 475 480

Thr Asn Gly Ala Gln Thr Ala Ser Asn Thr Ala Gly Asp Thr Asn Gly
485 490 495

Lys Thr Lys Thr Tyr Glu Val Glu Val Cys Cys Ser Asn Leu Asn Tyr
500 505 510

Leu Lys Tyr Gly Met Leu Thr Arg Lys Asn Ser Lys Ser Ala Met Gln
515 520 525

Ala Gly Glu Ser Ser Ser Gln Ala Asp Ala Lys Thr Glu Gln Val Glu
530 535 540

Gln Ser Met Phe Leu Gln Gly Glu Arg Thr Asp Glu Lys Glu Ile Pro
545 550 555 560

Ser Glu Gln Asn Ile Val Tyr Arg Gly Ser Trp Tyr Gly Tyr Ile Ala
565 570 575

Asn Asp Lys Ser Thr Ser Trp Ser Gly Asn Ala Ser Asn Ala Thr Ser
580 585 590

Gly Asn Arg Ala Glu Phe Thr Val Asn Phe Ala Asp Lys Lys Ile Thr
595 600 605

Gly Thr Leu Thr Ala Asp Asn Arg Gln Glu Ala Thr Phe Thr Ile Asp
610 615 620

Gly Asn Ile Lys Asp Asn Gly Phe Glu Gly Thr Ala Lys Thr Ala Glu
625 630 635 640

Ser Gly Phe Asp Leu Asp Gln Ser Asn Thr Thr Arg Thr Pro Lys Ala
645 650 655

Tyr Ile Thr Asp Ala Lys Val Gln Gly Gly Phe Tyr Gly Pro Lys Ala
660 665 670

Glu Glu Leu Gly Gly Trp Phe Ala Tyr Pro Gly Asp Lys Gln Thr Lys
675 680 685

Asn Ala Thr Asn Ala Ser Gly Asn Ser Ser Ala Thr Val Val Phe Gly
690 695 700

Ala Lys Arg Gln Gln Pro Val Arg
705 710

<210> 86
<211> 274
<212> PRT
<213> Artificial Sequence

<220>
<223> 741

<400> 86

Val Asn Arg Thr Ala Phe Cys Cys Leu Ser Leu Thr Thr Ala Leu Ile
1               5                   10                  15

Leu Thr Ala Cys Ser Ser Gly Gly Gly Gly Val Ala Ala Asp Ile Gly
            20                  25                  30

Ala Gly Leu Ala Asp Ala Leu Thr Ala Pro Leu Asp His Lys Asp Lys
        35                  40                  45

Gly Leu Gln Ser Leu Thr Leu Asp Gln Ser Val Arg Lys Asn Glu Lys
    50                  55                  60

Leu Lys Leu Ala Ala Gln Gly Ala Glu Lys Thr Tyr Gly Asn Gly Asp
65                  70                  75                  80

Ser Leu Asn Thr Gly Lys Leu Lys Asn Asp Lys Val Ser Arg Phe Asp
                85                  90                  95

Phe Ile Arg Gln Ile Glu Val Asp Gly Gln Leu Ile Thr Leu Glu Ser
            100                 105                 110

Gly Glu Phe Gln Val Tyr Lys Gln Ser His Ser Ala Leu Thr Ala Phe
        115                 120                 125

Gln Thr Glu Gln Ile Gln Asp Ser Glu His Ser Gly Lys Met Val Ala
    130                 135                 140

Lys Arg Gln Phe Arg Ile Gly Asp Ile Ala Gly Glu His Thr Ser Phe
145                 150                 155                 160

Asp Lys Leu Pro Glu Gly Gly Arg Ala Thr Tyr Arg Gly Thr Ala Phe
                165                 170                 175

Gly Ser Asp Asp Ala Gly Gly Lys Leu Thr Tyr Thr Ile Asp Phe Ala
            180                 185                 190

Ala Lys Gln Gly Asn Gly Lys Ile Glu His Leu Lys Ser Pro Glu Leu
        195                 200                 205

Asn Val Asp Leu Ala Ala Ala Asp Ile Lys Pro Asp Gly Lys Arg His
    210                 215                 220

Ala Val Ile Ser Gly Ser Val Leu Tyr Asn Gln Ala Glu Lys Gly Ser
225                 230                 235                 240

Tyr Ser Leu Gly Ile Phe Gly Gly Lys Ala Gln Glu Val Ala Gly Ser
                245                 250                 255

Ala Glu Val Lys Thr Val Asn Gly Ile Arg His Ile Gly Leu Ala Ala
            260                 265                 270

Lys Gln

<210> 87
<211> 1082
<212> PRT
<213> Artificial Sequence

<220>
<223> 983

<400> 87

Met Arg Thr Thr Pro Thr Phe Pro Thr Lys Thr Phe Lys Pro Thr Ala
1               5                   10                  15

Met Ala Leu Ala Val Ala Thr Thr Leu Ser Ala Cys Leu Gly Gly Gly
            20                  25                  30

Gly Gly Gly Thr Ser Ala Pro Asp Phe Asn Ala Gly Gly Thr Gly Ile
        35                  40                  45

Gly Ser Asn Ser Arg Ala Thr Thr Ala Lys Ser Ala Ala Val Ser Tyr
    50                  55                  60

Ala Gly Ile Lys Asn Glu Met Cys Lys Asp Arg Ser Met Leu Cys Ala
65                  70                  75                  80

Gly Arg Asp Asp Val Ala Val Thr Asp Arg Asp Ala Lys Ile Asn Ala
                85                  90                  95

Pro Pro Pro Asn Leu His Thr Gly Asp Phe Pro Asn Pro Asn Asp Ala
            100                 105                 110

Tyr Lys Asn Leu Ile Asn Leu Lys Pro Ala Ile Glu Ala Gly Tyr Thr
        115                 120                 125

Gly Arg Gly Val Glu Val Gly Ile Val Asp Thr Gly Glu Ser Val Gly
    130                 135                 140

Ser Ile Ser Phe Pro Glu Leu Tyr Gly Arg Lys Glu His Gly Tyr Asn
145                 150                 155                 160

Glu Asn Tyr Lys Asn Tyr Thr Ala Tyr Met Arg Lys Glu Ala Pro Glu
                165                 170                 175

Asp Gly Gly Gly Lys Asp Ile Glu Ala Ser Phe Asp Asp Glu Ala Val
            180                 185                 190

Ile Glu Thr Glu Ala Lys Pro Thr Asp Ile Arg His Val Lys Glu Ile
        195                 200                 205

Gly His Ile Asp Leu Val Ser His Ile Ile Gly Gly Arg Ser Val Asp
    210                 215                 220

Gly Arg Pro Ala Gly Gly Ile Ala Pro Asp Ala Thr Leu His Ile Met
225                 230                 235                 240

Asn Thr Asn Asp Glu Thr Lys Asn Glu Met Met Val Ala Ala Ile Arg
                245                 250                 255

Asn Ala Trp Val Lys Leu Gly Glu Arg Gly Val Arg Ile Val Asn Asn
            260                 265                 270

Ser Phe Gly Thr Thr Ser Arg Ala Gly Thr Ala Asp Leu Phe Gln Ile
275 280 285

Ala Asn Ser Glu Glu Gln Tyr Arg Gln Ala Leu Leu Asp Tyr Ser Gly
290 295 300

Gly Asp Lys Thr Asp Glu Gly Ile Arg Leu Met Gln Gln Ser Asp Tyr
305 310 315 320

Gly Asn Leu Ser Tyr His Ile Arg Asn Lys Asn Met Leu Phe Ile Phe
325 330 335

Ser Thr Gly Asn Asp Ala Gln Ala Gln Pro Asn Thr Tyr Ala Leu Leu
340 345 350

Pro Phe Tyr Glu Lys Asp Ala Gln Lys Gly Ile Ile Thr Val Ala Gly
355 360 365

Val Asp Arg Ser Gly Glu Lys Phe Lys Arg Glu Met Tyr Gly Glu Pro
370 375 380

Gly Thr Glu Pro Leu Glu Tyr Gly Ser Asn His Cys Gly Ile Thr Ala
385 390 395 400

Met Trp Cys Leu Ser Ala Pro Tyr Glu Ala Ser Val Arg Phe Thr Arg
405 410 415

Thr Asn Pro Ile Gln Ile Ala Gly Thr Ser Phe Ser Ala Pro Ile Val
420 425 430

Thr Gly Thr Ala Ala Leu Leu Leu Gln Lys Tyr Pro Trp Met Ser Asn
435 440 445

Asp Asn Leu Arg Thr Thr Leu Leu Thr Thr Ala Gln Asp Ile Gly Ala
450 455 460

Val Gly Val Asp Ser Lys Phe Gly Trp Gly Leu Leu Asp Ala Gly Lys
465 470 475 480

Ala Met Asn Gly Pro Ala Ser Phe Pro Phe Gly Asp Phe Thr Ala Asp
485 490 495

Thr Lys Gly Thr Ser Asp Ile Ala Tyr Ser Phe Arg Asn Asp Ile Ser
500 505 510

Gly Thr Gly Gly Leu Ile Lys Lys Gly Gly Ser Gln Leu Gln Leu His
515 520 525

Gly Asn Asn Thr Tyr Thr Gly Lys Thr Ile Ile Glu Gly Gly Ser Leu
530 535 540

Val Leu Tyr Gly Asn Asn Lys Ser Asp Met Arg Val Glu Thr Lys Gly
545 550 555 560

Ala Leu Ile Tyr Asn Gly Ala Ala Ser Gly Gly Ser Leu Asn Ser Asp
565 570 575

Gly Ile Val Tyr Leu Ala Asp Thr Asp Gln Ser Gly Ala Asn Glu Thr
580 585 590

Val His Ile Lys Gly Ser Leu Gln Leu Asp Gly Lys Gly Thr Leu Tyr
595 600 605

Thr Arg Leu Gly Lys Leu Leu Lys Val Asp Gly Thr Ala Ile Ile Gly
610 615 620

Gly Lys Leu Tyr Met Ser Ala Arg Gly Lys Gly Ala Gly Tyr Leu Asn
625 630 635 640

Ser Thr Gly Arg Arg Val Pro Phe Leu Ser Ala Ala Lys Ile Gly Gln
645 650 655

Asp Tyr Ser Phe Phe Thr Asn Ile Glu Thr Asp Gly Gly Leu Leu Ala
660 665 670

Ser Leu Asp Ser Val Glu Lys Thr Ala Gly Ser Glu Gly Asp Thr Leu
675 680 685

Ser Tyr Tyr Val Arg Arg Gly Asn Ala Ala Arg Thr Ala Ser Ala Ala
690 695 700

Ala His Ser Ala Pro Ala Gly Leu Lys His Ala Val Glu Gln Gly Gly
705 710 715 720

Ser Asn Leu Glu Asn Leu Met Val Glu Leu Asp Ala Ser Glu Ser Ser
725 730 735

Ala Thr Pro Glu Thr Val Glu Thr Ala Ala Ala Asp Arg Thr Asp Met
740 745 750

Pro Gly Ile Arg Pro Tyr Gly Ala Thr Phe Arg Ala Ala Ala Ala Val
755 760 765

Gln His Ala Asn Ala Ala Asp Gly Val Arg Ile Phe Asn Ser Leu Ala
770 775 780

Ala Thr Val Tyr Ala Asp Ser Thr Ala Ala His Ala Asp Met Gln Gly
785 790 795 800

Arg Arg Leu Lys Ala Val Ser Asp Gly Leu Asp His Asn Gly Thr Gly
805 810 815

Leu Arg Val Ile Ala Gln Thr Gln Gln Asp Gly Gly Thr Trp Glu Gln
820 825 830

Gly Gly Val Glu Gly Lys Met Arg Gly Ser Thr Gln Thr Val Gly Ile
835 840 845

Ala Ala Lys Thr Gly Glu Asn Thr Thr Ala Ala Ala Thr Leu Gly Met
850 855 860

Gly Arg Ser Thr Trp Ser Glu Asn Ser Ala Asn Ala Lys Thr Asp Ser
865 870 875 880

Ile Ser Leu Phe Ala Gly Ile Arg His Asp Ala Gly Asp Ile Gly Tyr
885 890 895

Leu Lys Gly Leu Phe Ser Tyr Gly Arg Tyr Lys Asn Ser Ile Ser Arg
900 905 910

Ser Thr Gly Ala Asp Glu His Ala Glu Gly Ser Val Asn Gly Thr Leu
915 920 925

Met Gln Leu Gly Ala Leu Gly Gly Val Asn Val Pro Phe Ala Ala Thr
930 935 940

Gly Asp Leu Thr Val Glu Gly Gly Leu Arg Tyr Asp Leu Leu Lys Gln
945 950 955 960

Asp Ala Phe Ala Glu Lys Gly Ser Ala Leu Gly Trp Ser Gly Asn Ser
965 970 975

Leu Thr Glu Gly Thr Leu Val Gly Leu Ala Gly Leu Lys Leu Ser Gln
980 985 990

Pro Leu Ser Asp Lys Ala Val Leu Phe Ala Thr Ala Gly Val Glu Arg
995 1000 1005

Asp Leu Asn Gly Arg Asp Tyr Thr Val Thr Gly Gly Phe Thr Gly Ala
1010 1015 1020

Thr Ala Ala Thr Gly Lys Thr Gly Ala Arg Asn Met Pro His Thr Arg
1025 1030 1035 1040

Leu Val Ala Gly Leu Gly Ala Asp Val Glu Phe Gly Asn Gly Trp Asn
1045 1050 1055

Gly Leu Ala Arg Tyr Ser Tyr Ala Gly Ser Lys Gln Tyr Gly Asn His
1060 1065 1070

Ser Gly Arg Val Gly Val Gly Tyr Arg Phe
1075 1080

<210> 88
<211> 2505
<212> DNA
<213> Artificial Sequence

<220>
<223> deltaG287-919

<400> 88

```
atggctagcc ccgatgttaa atcggcggac acgctgtcaa aaccggccgc tcctgttgtt    60
gctgaaaaag agacagaggt aaaagaagat gcgccacagg caggttctca aggacagggc   120
gcgccatcca cacaaggcag ccaagatatg gcggcagttt cggcagaaaa tacaggcaat   180
ggcggtgcgg caacaacgga caaacccaaa aatgaagacg agggaccgca aaatgatatg   240
ccgcaaaatt ccgccgaatc cgcaaatcaa acagggaaca accaacccgc cgattcttca   300
gattccgccc ccgcgtcaaa ccctgcacct gcgaatggcg gtagcaattt tggaagggtt   360
gatttggcta atggcgtttt gattgatggg ccgtcgcaaa atataacgtt gacccactgt   420
aaaggcgatt cttgtaatgg tgataattta ttggatgaag aagcaccgtc aaaatcagaa   480

tttgaaaatt taaatgagtc tgaacgaatt gagaaatata agaaagatgg gaaaagcgat   540
aaatttacta atttggttgc gacagcagtt caagctaatg gaactaacaa atatgtcatc   600
atttataaag acaagtccgc ttcatcttca tctgcgcgat tcaggcgttc tgcacggtcg   660
aggaggtcgc ttcctgccga gatgccgcta atccccgtca atcaggcgga tacgctgatt   720
gtcgatgggg aagcggtcag cctgacgggg cattccggca atatcttcgc gcccgaaggg   780
aattaccggt atctgactta cggggcggaa aaattgcccg gcggatcgta tgccctccgt   840
gtgcaaggcg aaccggcaaa aggcgaaatg cttgctggca cggccgtgta caacggcgaa   900
gtgctgcatt ttcatacgga aaacggccgt ccgtacccga ctagaggcag gtttgccgca   960
aaagtcgatt tcggcagcaa atctgtggac ggcattatcg acagcggcga tgatttgcat  1020
atgggtacgc aaaaattcaa agccgccatc gatggaaacg gctttaaggg gacttggacg  1080
gaaaatggcg gcggggatgt ttccggaagg ttttacggcc cggccggcga ggaagtggcg  1140
ggaaaataca gctatcgccc gacagatgcg gaaaagggcg gattcggcgt gtttgccggc  1200
aaaaaagagc aggatggatc cggaggagga ggatgccaaa gcaagagcat ccaaaccttt  1260
ccgcaacccg acacatccgt catcaacggc ccggaccggc cggtcggcat ccccgacccc  1320
gccggaacga cggtcggcgg cggcggggcc gtctataccg ttgtaccgca cctgtccctg  1380
ccccactggg cggcgcagga tttcgccaaa agcctgcaat ccttccgcct cggctgcgcc  1440
aatttgaaaa accgccaagg ctggcaggat gtgtgcgccc aagcctttca aacccccgtc  1500
cattcctttc aggcaaaaca gtttttgaa cgctatttca cgccgtggca ggttgcaggc   1560
aacggaagcc ttgccggtac ggttaccggc tattacgagc cggtgctgaa gggcgacgac  1620
aggcggacgg cacaagcccg cttcccgatt tacggtattc ccgacgattt tatctccgtc  1680
cccctgcctg ccggtttgcg gagcggaaaa gcccttgtcc gcatcaggca gacgggaaaa  1740
aacagcggca caatcgacaa taccggcggc acacataccg ccgacctctc ccgattcccc  1800
atcaccgcgc gcacaacggc aatcaaaggc aggtttgaag gaagccgctt cctcccctac  1860
cacacgcgca accaaatcaa cggcggcgcg cttgacggca aagccccgat actcggttac  1920
gccgaagacc ccgtcgaact ttttttatg cacatccaag gctcgggccg tctgaaaacc   1980
ccgtccggca aatacatccg catcggctat gccgacaaaa acgaacatcc ctacgtttcc  2040
atcggacgct atatggcgga caaaggctac ctcaagctcg ggcagacctc gatgcagggc  2100
atcaaagcct atatgcggca aaatccgcaa cgcctcgccg aagttttggg tcaaaacccc  2160
agctatatct tttccgcgga gcttgccgga agcagcaatg acggtcccgt cggcgcactg  2220
ggcacgccgt tgatggggga atatgccggc gcagtcgacc ggcactacat taccttgggc  2280
gcgcccttat ttgtcgccac cgcccatccg gttacccgca aagccctcaa ccgcctgatt  2340
atggcgcagg ataccggcag cgcgattaaa ggcgcggtgc gcgtggatta tttttgggga  2400
tacggcgacg aagccggcga acttgccggc aaacagaaaa ccacgggtta cgtctggcag  2460
ctcctaccca acggtatgaa gcccgaatac cgcccgtaac tcgag                   2505
```

<210> 89

<211> 832

<212> PRT

<213> Artificial Sequence

<220>

<223> deltaG287-919

<400> 89

Met Ala Ser Pro Asp Val Lys Ser Ala Asp Thr Leu Ser Lys Pro Ala
1 5 10 15

Ala Pro Val Val Ala Glu Lys Glu Thr Glu Val Lys Glu Asp Ala Pro
20 25 30

Gln Ala Gly Ser Gln Gly Gln Gly Ala Pro Ser Thr Gln Gly Ser Gln
35 40 45

Asp Met Ala Ala Val Ser Ala Glu Asn Thr Gly Asn Gly Gly Ala Ala
50 55 60

Thr Thr Asp Lys Pro Lys Asn Glu Asp Glu Gly Pro Gln Asn Asp Met

65 70 75 80

Pro Gln Asn Ser Ala Glu Ser Ala Asn Gln Thr Gly Asn Asn Gln Pro
85 90 95

Ala Asp Ser Ser Asp Ser Ala Pro Ala Ser Asn Pro Ala Pro Ala Asn
100 105 110

Gly Gly Ser Asn Phe Gly Arg Val Asp Leu Ala Asn Gly Val Leu Ile
115 120 125

Asp Gly Pro Ser Gln Asn Ile Thr Leu Thr His Cys Lys Gly Asp Ser
130 135 140

Cys Asn Gly Asp Asn Leu Leu Asp Glu Glu Ala Pro Ser Lys Ser Glu
145 150 155 160

Phe Glu Asn Leu Asn Glu Ser Glu Arg Ile Glu Lys Tyr Lys Lys Asp
165 170 175

Gly Lys Ser Asp Lys Phe Thr Asn Leu Val Ala Thr Ala Val Gln Ala
180 185 190

Asn Gly Thr Asn Lys Tyr Val Ile Ile Tyr Lys Asp Lys Ser Ala Ser
195 200 205

Ser Ser Ser Ala Arg Phe Arg Arg Ser Ala Arg Ser Arg Arg Ser Leu
210 215 220

Pro Ala Glu Met Pro Leu Ile Pro Val Asn Gln Ala Asp Thr Leu Ile
225 230 235 240

Val Asp Gly Glu Ala Val Ser Leu Thr Gly His Ser Gly Asn Ile Phe
245 250 255

Ala Pro Glu Gly Asn Tyr Arg Tyr Leu Thr Tyr Gly Ala Glu Lys Leu
260 265 270

Pro Gly Gly Ser Tyr Ala Leu Arg Val Gln Gly Glu Pro Ala Lys Gly
275 280 285

Glu Met Leu Ala Gly Thr Ala Val Tyr Asn Gly Glu Val Leu His Phe
290 295 300

His Thr Glu Asn Gly Arg Pro Tyr Pro Thr Arg Gly Arg Phe Ala Ala
305 310 315 320

Lys Val Asp Phe Gly Ser Lys Ser Val Asp Gly Ile Ile Asp Ser Gly
325 330 335

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser

370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Cys Gln Ser Lys Ser
405 410 415

Ile Gln Thr Phe Pro Gln Pro Asp Thr Ser Val Ile Asn Gly Pro Asp
420 425 430

Arg Pro Val Gly Ile Pro Asp Pro Ala Gly Thr Thr Val Gly Gly Gly
435 440 445

Gly Ala Val Tyr Thr Val Val Pro His Leu Ser Leu Pro His Trp Ala
450 455 460

Ala Gln Asp Phe Ala Lys Ser Leu Gln Ser Phe Arg Leu Gly Cys Ala
465 470 475 480

Asn Leu Lys Asn Arg Gln Gly Trp Gln Asp Val Cys Ala Gln Ala Phe
485 490 495

Gln Thr Pro Val His Ser Phe Gln Ala Lys Gln Phe Phe Glu Arg Tyr
500 505 510

Phe Thr Pro Trp Gln Val Ala Gly Asn Gly Ser Leu Ala Gly Thr Val
515 520 525

Thr Gly Tyr Tyr Glu Pro Val Leu Lys Gly Asp Asp Arg Arg Thr Ala
530 535 540

Gln Ala Arg Phe Pro Ile Tyr Gly Ile Pro Asp Asp Phe Ile Ser Val
545 550 555 560

Pro Leu Pro Ala Gly Leu Arg Ser Gly Lys Ala Leu Val Arg Ile Arg
565 570 575

Gln Thr Gly Lys Asn Ser Gly Thr Ile Asp Asn Thr Gly Gly Thr His
580 585 590

Thr Ala Asp Leu Ser Arg Phe Pro Ile Thr Ala Arg Thr Thr Ala Ile
595 600 605

Lys Gly Arg Phe Glu Gly Ser Arg Phe Leu Pro Tyr His Thr Arg Asn
610 615 620

Gln Ile Asn Gly Gly Ala Leu Asp Gly Lys Ala Pro Ile Leu Gly Tyr
625 630 635 640

Ala Glu Asp Pro Val Glu Leu Phe Phe Met His Ile Gln Gly Ser Gly
645 650 655

Arg Leu Lys Thr Pro Ser Gly Lys Tyr Ile Arg Ile Gly Tyr Ala Asp
660 665 670

Lys Asn Glu His Pro Tyr Val Ser Ile Gly Arg Tyr Met Ala Asp Lys

675 680 685

Gly Tyr Leu Lys Leu Gly Gln Thr Ser Met Gln Gly Ile Lys Ala Tyr
690 695 700

Met Arg Gln Asn Pro Gln Arg Leu Ala Glu Val Leu Gly Gln Asn Pro
705 710 715 720

Ser Tyr Ile Phe Phe Arg Glu Leu Ala Gly Ser Ser Asn Asp Gly Pro
725 730 735

Val Gly Ala Leu Gly Thr Pro Leu Met Gly Glu Tyr Ala Gly Ala Val
740 745 750

Asp Arg His Tyr Ile Thr Leu Gly Ala Pro Leu Phe Val Ala Thr Ala
755 760 765

His Pro Val Thr Arg Lys Ala Leu Asn Arg Leu Ile Met Ala Gln Asp
770 775 780

Thr Gly Ser Ala Ile Lys Gly Ala Val Arg Val Asp Tyr Phe Trp Gly
785 790 795 800

Tyr Gly Asp Glu Ala Gly Glu Leu Ala Gly Lys Gln Lys Thr Thr Gly
805 810 815

Tyr Val Trp Gln Leu Leu Pro Asn Gly Met Lys Pro Glu Tyr Arg Pro
820 825 830

<210> 90
<211> 1746
<212> DNA
<213> Artificial Sequence

<220>
<223> deltaG287-953

<400> 90

```
atggctagcc ccgatgttaa atcggcggac acgctgtcaa aaccggccgc tcctgttgtt   60
gctgaaaaag agacagaggt aaaagaagat gcgccacagg caggttctca aggacagggc  120
gcgccatcca cacaaggcag ccaagatatg gcggcagttt cggcagaaaa tacaggcaat  180
ggcggtgcgg caacaacgga caaacccaaa aatgaagacg agggaccgca aaatgatatg  240
ccgcaaaatt ccgccgaatc cgcaaatcaa acagggaaca accaacccgc cgattcttca  300
gattccgccc ccgcgtcaaa ccctgcacct gcgaatggcg gtagcaattt tggaagggtt  360
gatttggcta atggcgtttt gattgatggg ccgtcgcaaa atataacgtt gacccactgt  420
aaaggcgatt cttgtaatgg tgataattta ttggatgaag aagcaccgtc aaaatcagaa  480
tttgaaaatt taaatgagtc tgaacgaatt gagaaatata agaaagatgg gaaaagcgat  540
aaatttacta atttggttgc gacagcagtt caagctaatg gaactaacaa atatgtcatc  600
atttataaag acaagtccgc ttcatcttca tctgcgcgat tcaggcgttc tgcacggtcg  660
aggaggtcgc ttcctgccga gatgccgcta atccccgtca atcaggcgga tacgctgatt  720
gtcgatgggg aagcggtcag cctgacgggg cattccggca atatcttcgc gcccgaaggg  780
aattaccggt atctgactta cggggcggaa aaattgcccg gcggatcgta tgccctccgt  840
gtgcaaggcg aaccggcaaa aggcgaaatg cttgctggca cggccgtgta caacggcgaa  900
gtgctgcatt ttcatacgga aaacggccgt ccgtacccga ctagaggcag gtttgccgca  960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080

gaaaatggcg gcggggatgt ttccggaagg ttttacggcc cggccggcga ggaagtggcg 1140
ggaaaataca gctatcgccc gacagatgcg gaaaagggcg gattcggcgt gtttgccggc 1200
aaaaaagagc aggatggatc cggaggagga ggagccacct acaaagtgga cgaatatcac 1260
gccaacgccc gtttcgccat cgaccatttc aacaccagca ccaacgtcgg cggtttttac 1320
ggtctgaccg gttccgtcga gttcgaccaa gcaaaacgcg acggtaaaat cgacatcacc 1380
atccccgttg ccaacctgca aagcggttcg caacacttta ccgaccacct gaaatcagcc 1440
gacatcttcg atgccgccca atatccggac atccgctttg tttccaccaa attcaacttc 1500
aacggcaaaa aactggtttc cgttgacggc aacctgacca tgcacggcaa aaccgccccc 1560
gtcaaactca aagccgaaaa attcaactgc taccaaagcc cgatggcgaa aaccgaagtt 1620
tgcggcggcg acttcagcac caccatcgac cgcaccaaat ggggcgtgga ctacctcgtt 1680
aacgttggta tgaccaaaag cgtccgcatc gacatccaaa tcgaggcagc caaacaataa 1740
ctcgag                                                             1746
```

<210> 91
<211> 579
<212> PRT
<213> Artificial Sequence

<220>
<223> deltaG287-953

<400> 91

```
Met Ala Ser Pro Asp Val Lys Ser Ala Asp Thr Leu Ser Lys Pro Ala
1               5                   10                  15

Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190
```

Asn Gly Thr Asn Lys Tyr Val Ile Ile Tyr Lys Asp Lys Ser Ala Ser
195 200 205

Ser Ser Ser Ala Arg Phe Arg Arg Ser Ala Arg Ser Arg Arg Ser Leu
210 215 220

Pro Ala Glu Met Pro Leu Ile Pro Val Asn Gln Ala Asp Thr Leu Ile
225 230 235 240

Val Asp Gly Glu Ala Val Ser Leu Thr Gly His Ser Gly Asn Ile Phe
245 250 255

Ala Pro Glu Gly Asn Tyr Arg Tyr Leu Thr Tyr Gly Ala Glu Lys Leu
260 265 270

Pro Gly Gly Ser Tyr Ala Leu Arg Val Gln Gly Glu Pro Ala Lys Gly
275 280 285

Glu Met Leu Ala Gly Thr Ala Val Tyr Asn Gly Glu Val Leu His Phe
290 295 300

His Thr Glu Asn Gly Arg Pro Tyr Pro Thr Arg Gly Arg Phe Ala Ala
305 310 315 320

Lys Val Asp Phe Gly Ser Lys Ser Val Asp Gly Ile Ile Asp Ser Gly
325 330 335

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser
370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Ala Thr Tyr Lys Val
405 410 415

Asp Glu Tyr His Ala Asn Ala Arg Phe Ala Ile Asp His Phe Asn Thr
420 425 430

Ser Thr Asn Val Gly Gly Phe Tyr Gly Leu Thr Gly Ser Val Glu Phe
435 440 445

Asp Gln Ala Lys Arg Asp Gly Lys Ile Asp Ile Thr Ile Pro Val Ala
450 455 460

Asn Leu Gln Ser Gly Ser Gln His Phe Thr Asp His Leu Lys Ser Ala
465 470 475 480

Asp Ile Phe Asp Ala Ala Gln Tyr Pro Asp Ile Arg Phe Val Ser Thr
485 490 495

Lys Phe Asn Phe Asn Gly Lys Lys Leu Val Ser Val Asp Gly Asn Leu
500 505 510

Thr Met His Gly Lys Thr Ala Pro Val Lys Leu Lys Ala Glu Lys Phe
515 520 525

Asn Cys Tyr Gln Ser Pro Met Ala Lys Thr Glu Val Cys Gly Gly Asp
530 535 540

Phe Ser Thr Thr Ile Asp Arg Thr Lys Trp Gly Val Asp Tyr Leu Val
545 550 555 560

Asn Val Gly Met Thr Lys Ser Val Arg Ile Asp Ile Gln Ile Glu Ala
565 570 575

Ala Lys Gln

<210> 92
<211> 2388
<212> DNA
<213> Artificial Sequence

<220>
<223> deltaG287-961

<400> 92

```
atggctagcc ccgatgttaa atcggcggac acgctgtcaa aaccggccgc tcctgttgtt   60
gctgaaaaag agacagaggt aaaagaagat gcgccacagg caggttctca aggacagggc   120
gcgccatcca cacaaggcag ccaagatatg gcggcagttt cggcagaaaa tacaggcaat   180
ggcggtgcgg caacaacgga caaacccaaa aatgaagacg agggaccgca aaatgatatg   240
ccgcaaaatt ccgccgaatc cgcaaatcaa acagggaaca accaacccgc cgattcttca   300
gattccgccc ccgcgtcaaa ccctgcacct gcgaatggcg gtagcaattt tggaagggtt   360
gatttggcta atggcgtttt gattgatggg ccgtcgcaaa atataacgtt gacccactgt   420
aaaggcgatt cttgtaatgg tgataattta ttggatgaag aagcaccgtc aaaatcagaa   480
tttgaaaatt taaatgagtc tgaacgaatt gagaaatata agaaagatgg gaaaagcgat   540
aaatttacta atttggttgc gacagcagtt caagctaatg gaactaacaa atatgtcatc   600
atttataaag acaagtccgc ttcatcttca tctgcgcgat tcaggcgttc tgcacggtcg   660
aggaggtcgc ttcctgccga gatgccgcta atccccgtca atcaggcgga tacgctgatt   720
gtcgatgggg aagcggtcag cctgacgggg cattccggca atatcttcgc gcccgaaggg   780
aattaccggt atctgactta cggggcggaa aaattgcccg gcggatcgta tgccctccgt   840
gtgcaaggcg aaccggcaaa aggcgaaatg cttgctggca cggccgtgta caacggcgaa   900
gtgctgcatt ttcatacgga aaacggccgt ccgtacccga ctagaggcag gtttgccgca   960
aaagtcgatt tcggcagcaa atctgtggac ggcattatcg acagcggcga tgatttgcat   1020
atgggtacgc aaaaattcaa agccgccatc gatggaaacg gctttaaggg gacttggacg   1080
gaaaatggcg gcggggatgt ttccggaagg ttttacggcc cggccggcga ggaagtggcg   1140
ggaaaataca gctatcgccc gacagatgcg gaaaagggcg gattcggcgt gtttgccggc   1200
aaaaaagagc aggatggatc cggaggagga ggagccacaa acgacgacga tgttaaaaaa   1260
gctgccactg tggccattgc tgctgcctac aacaatggcc aagaaatcaa cggtttcaaa   1320
gctggagaga ccatctacga cattgatgaa gacggcacaa ttaccaaaaa agacgcaact   1380
gcagccgatg ttgaagccga cgactttaaa ggtctgggtc tgaaaaaagt cgtgactaac   1440
ctgaccaaaa ccgtcaatga aaacaaacaa aacgtcgatg ccaaagtaaa agctgcagaa   1500
tctgaaatag aaaagttaac aaccaagtta gcagacactg atgccgcttt agcagatact   1560
gatgccgctc tggatgcaac caccaacgcc ttgaataaat tgggagaaaa tataacgaca   1620
tttgctgaag agactaagac aaatatcgta aaaattgatg aaaaattaga agccgtggct   1680
gataccgtcg acaagcatgc cgaagcattc aacgatatcg ccgattcatt ggatgaaacc   1740

aacactaagg cagacgaagc cgtcaaaacc gccaatgaag ccaaacagac ggccgaagaa   1800
accaaacaaa acgtcgatgc caaagtaaaa gctgcagaaa ctgcagcagg caaagccgaa   1860
gctgccgctg gcacagctaa tactgcagcc gacaaggccg aagctgtcgc tgcaaaagtt   1920
accgacatca aagctgatat cgctacgaac aaagataata ttgctaaaaa agcaaacagt   1980
gccgacgtgt acaccagaga agagtctgac agcaaatttg tcagaattga tggtctgaac   2040
gctactaccg aaaaattgga cacacgcttg gcttctgctg aaaaatccat tgccgatcac   2100
gatactcgcc tgaacggttt ggataaaaca gtgtcagacc tgcgcaaaga aacccgccaa   2160
ggccttgcag aacaagccgc gctctccggt ctgttccaac cttacaacgt gggtcggttc   2220
aatgtaacgg ctgcagtcgg cggctacaaa tccgaatcgg cagtcgccat cggtaccggc   2280
ttccgcttta ccgaaaactt tgccgccaaa gcaggcgtgg cagtcggcac ttcgtccggt   2340
tcttccgcag cctaccatgt cggcgtcaat tacgagtggt aactcgag                2388
```

<210> 93
<211> 793
<212> PRT
<213> Artificial Sequence

<220>
<223> deltaG287-961

<400> 93

Met Ala Ser Pro Asp Val Lys Ser Ala Asp Thr Leu Ser Lys Pro Ala
1               5                   10                  15

Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190

Asn Gly Thr Asn Lys Tyr Val Ile Ile Tyr Lys Asp Lys Ser Ala Ser
195 200 205

Ser Ser Ser Ala Arg Phe Arg Arg Ser Ala Arg Ser Arg Arg Ser Leu
210 215 220

Pro Ala Glu Met Pro Leu Ile Pro Val Asn Gln Ala Asp Thr Leu Ile
225 230 235 240

Val Asp Gly Glu Ala Val Ser Leu Thr Gly His Ser Gly Asn Ile Phe
245 250 255

Ala Pro Glu Gly Asn Tyr Arg Tyr Leu Thr Tyr Gly Ala Glu Lys Leu
260 265 270

Pro Gly Gly Ser Tyr Ala Leu Arg Val Gln Gly Glu Pro Ala Lys Gly
275 280 285

Glu Met Leu Ala Gly Thr Ala Val Tyr Asn Gly Glu Val Leu His Phe
290 295 300

His Thr Glu Asn Gly Arg Pro Tyr Pro Thr Arg Gly Arg Phe Ala Ala
305 310 315 320

Lys Val Asp Phe Gly Ser Lys Ser Val Asp Gly Ile Ile Asp Ser Gly
325 330 335

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser
370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Ala Thr Asn Asp Asp
405 410 415

Asp Val Lys Lys Ala Ala Thr Val Ala Ile Ala Ala Ala Tyr Asn Asn
420 425 430

Gly Gln Glu Ile Asn Gly Phe Lys Ala Gly Glu Thr Ile Tyr Asp Ile
435 440 445

Asp Glu Asp Gly Thr Ile Thr Lys Lys Asp Ala Thr Ala Ala Asp Val
450 455 460

Glu Ala Asp Asp Phe Lys Gly Leu Gly Leu Lys Lys Val Val Thr Asn
465 470 475 480

Leu Thr Lys Thr Val Asn Glu Asn Lys Gln Asn Val Asp Ala Lys Val
485 490 495

Lys Ala Ala Glu Ser Glu Ile Glu Lys Leu Thr Thr Lys Leu Ala Asp
500 505 510

Thr Asp Ala Ala Leu Ala Asp Thr Asp Ala Ala Leu Asp Ala Thr Thr
515 520 525

Asn Ala Leu Asn Lys Leu Gly Glu Asn Ile Thr Thr Phe Ala Glu Glu
530 535 540

Thr Lys Thr Asn Ile Val Lys Ile Asp Glu Lys Leu Glu Ala Val Ala
545 550 555 560

Asp Thr Val Asp Lys His Ala Glu Ala Phe Asn Asp Ile Ala Asp Ser
565 570 575

Leu Asp Glu Thr Asn Thr Lys Ala Asp Glu Ala Val Lys Thr Ala Asn
580 585 590

Glu Ala Lys Gln Thr Ala Glu Glu Thr Lys Gln Asn Val Asp Ala Lys
595 600 605

Val Lys Ala Ala Glu Thr Ala Ala Gly Lys Ala Glu Ala Ala Ala Gly
610 615 620

Thr Ala Asn Thr Ala Ala Asp Lys Ala Glu Ala Val Ala Ala Lys Val
625 630 635 640

Thr Asp Ile Lys Ala Asp Ile Ala Thr Asn Lys Asp Asn Ile Ala Lys
645 650 655

Lys Ala Asn Ser Ala Asp Val Tyr Thr Arg Glu Glu Ser Asp Ser Lys
660 665 670

Phe Val Arg Ile Asp Gly Leu Asn Ala Thr Thr Glu Lys Leu Asp Thr
675 680 685

Arg Leu Ala Ser Ala Glu Lys Ser Ile Ala Asp His Asp Thr Arg Leu
690 695 700

Asn Gly Leu Asp Lys Thr Val Ser Asp Leu Arg Lys Glu Thr Arg Gln
705 710 715 720

Gly Leu Ala Glu Gln Ala Ala Leu Ser Gly Leu Phe Gln Pro Tyr Asn
725 730 735

Val Gly Arg Phe Asn Val Thr Ala Ala Val Gly Gly Tyr Lys Ser Glu
740 745 750

Ser Ala Val Ala Ile Gly Thr Gly Phe Arg Phe Thr Glu Asn Phe Ala
755 760 765

Ala Lys Ala Gly Val Ala Val Gly Thr Ser Ser Gly Ser Ser Ala Ala
770 775 780

Tyr His Val Gly Val Asn Tyr Glu Trp
785 790

<210> 94
<211> 2700
<212> DNA
<213> Artificial Sequence

<220>
<223> deltaG287NZ-919

<400> 94

```
atggctagcc ccgatgtcaa gtcggcggac acgctgtcaa aacctgccgc ccctgttgtt 60
tctgaaaaag agacagaggc aaaggaagat gcgccacagg caggttctca aggacagggc 120
gcgccatccg cacaaggcgg tcaagatatg gcggcggttt cggaagaaaa tacaggcaat 180
ggcggtgcgg cagcaacgga caaacccaaa aatgaagacg agggggcgca aaatgatatg 240
ccgcaaaatg ccgccgatac agatagtttg acaccgaatc acaccccggc ttcgaatatg 300
ccggccggaa atatggaaaa ccaagcaccg gatgccgggg aatcggagca gccggcaaac 360
caaccggata tggcaaatac ggcggacgga atgcagggtg acgatccgtc ggcaggcggg 420
gaaaatgccg gcaatacggc tgcccaaggt acaaatcaag ccgaaaacaa tcaaaccgcc 480
ggttctcaaa atcctgcctc ttcaaccaat cctagcgcca cgaatagcgg tggtgatttt 540
ggaaggacga acgtgggcaa ttctgttgtg attgacgggc cgtcgcaaaa tataacgttg 600
acccactgta aaggcgattc ttgtagtggc aataatttct tggatgaaga agtacagcta 660
aaatcagaat ttgaaaaatt aagtgatgca gacaaaataa gtaattacaa gaaagatggg 720
aagaatgacg ggaagaatga taaatttgtc ggtttggttg ccgatagtgt gcagatgaag 780
ggaatcaatc aatatattat cttttataaa cctaaaccca cttcatttgc gcgatttagg 840
cgttctgcac ggtcgaggcg gtcgcttccg gccgagatgc cgctgattcc cgtcaatcag 900
gcggatacgc tgattgtcga tggggaagcg gtcagcctga cggggcattc cggcaatatc 960
ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggatg ccaaagcaag 1440
agcatccaaa cctttccgca acccgacaca tccgtcatca acggcccgga ccggccggtc 1500
ggcatccccg accccgccgg aacgacggtc ggcggcggcg gggccgtcta taccgttgta 1560
ccgcacctgt ccctgcccca ctgggcggcg caggatttcg ccaaaagcct gcaatccttc 1620
cgcctcggct gcgccaattt gaaaaaccgc caaggctggc aggatgtgtg cgcccaagcc 1680
tttcaaaccc ccgtccattc ctttcaggca aaacagtttt ttgaacgcta tttcacgccg 1740
tggcaggttg caggcaacgg aagccttgcc ggtacggtta ccggctatta cgagccggtg 1800
ctgaagggcg acgacaggcg gacggcacaa gcccgcttcc cgatttacgg tattcccgac 1860
gattttatct ccgtcccct gcctgccggt ttgcggagcg gaaaagccct tgtccgcatc 1920
aggcagacgg gaaaaaacag cggcacaatc gacaataccg gcggcacaca taccgccgac 1980
ctctcccgat tccccatcac cgcgcgcaca acggcaatca aaggcaggtt tgaaggaagc 2040
cgcttcctcc cctaccacac gcgcaaccaa atcaacggcg gcgcgcttga cggcaaagcc 2100
ccgatactcg gttacgccga agaccccgtc gaactttttt ttatgcacat ccaaggctcg 2160
ggccgtctga aaaccccgtc cggcaaatac atccgcatcg gctatgccga caaaaacgaa 2220
catccctacg tttccatcgg acgctatatg gcggacaaag gctacctcaa gctcgggcag 2280
acctcgatgc agggcatcaa agcctatatg cggcaaaatc cgcaacgcct cgccgaagtt 2340
ttgggtcaaa accccagcta tatctttttc cgcgagcttg ccggaagcag caatgacggt 2400
cccgtcggcg cactgggcac gccgttgatg gggggaatatg ccggcgcagt cgaccggcac 2460
tacattacct tgggcgcgcc cttatttgtc gccaccgccc atccggttac ccgcaaagcc 2520
ctcaaccgcc tgattatggc gcaggatacc ggcagcgcga ttaaaggcgc ggtgcgcgtg 2580
gattattttt ggggatacgg cgacgaagcc ggcgaacttg ccggcaaaca gaaaaccacg 2640
ggttacgtct ggcagctcct acccaacggt atgaagcccg aataccgccc gtaaaagctt 2700
```

<210> 95
<211> 897
<212> PRT
<213> Artificial Sequence

<220>
<223> deltaG287NZ-919

<400> 95

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
    210                 215                 220

Glu Lys Leu Ser Asp Ala Asp Lys Ile Ser Asn Tyr Lys Lys Asp Gly
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270
```

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu
290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

Ser Tyr Arg Pro Thr Asp Ala Glu Lys Gly Gly Phe Gly Val Phe Ala
450 455 460

Gly Lys Lys Glu Gln Asp Gly Ser Gly Gly Gly Gly Cys Gln Ser Lys
465 470 475 480

Ser Ile Gln Thr Phe Pro Gln Pro Asp Thr Ser Val Ile Asn Gly Pro
485 490 495

Asp Arg Pro Val Gly Ile Pro Asp Pro Ala Gly Thr Thr Val Gly Gly
500 505 510

Gly Gly Ala Val Tyr Thr Val Val Pro His Leu Ser Leu Pro His Trp
515 520 525

Ala Ala Gln Asp Phe Ala Lys Ser Leu Gln Ser Phe Arg Leu Gly Cys
530 535 540

Ala Asn Leu Lys Asn Arg Gln Gly Trp Gln Asp Val Cys Ala Gln Ala
545 550 555 560

Phe Gln Thr Pro Val His Ser Phe Gln Ala Lys Gln Phe Phe Glu Arg
565 570 575

Tyr Phe Thr Pro Trp Gln Val Ala Gly Asn Gly Ser Leu Ala Gly Thr
580 585 590

Val Thr Gly Tyr Tyr Glu Pro Val Leu Lys Gly Asp Asp Arg Arg Thr
595 600 605

Ala Gln Ala Arg Phe Pro Ile Tyr Gly Ile Pro Asp Asp Phe Ile Ser
610 615 620

Val Pro Leu Pro Ala Gly Leu Arg Ser Gly Lys Ala Leu Val Arg Ile
625 630 635 640

Arg Gln Thr Gly Lys Asn Ser Gly Thr Ile Asp Asn Thr Gly Gly Thr
645 650 655

His Thr Ala Asp Leu Ser Arg Phe Pro Ile Thr Ala Arg Thr Thr Ala
660 665 670

Ile Lys Gly Arg Phe Glu Gly Ser Arg Phe Leu Pro Tyr His Thr Arg
675 680 685

Asn Gln Ile Asn Gly Gly Ala Leu Asp Gly Lys Ala Pro Ile Leu Gly
690 695 700

Tyr Ala Glu Asp Pro Val Glu Leu Phe Phe Met His Ile Gln Gly Ser
705 710 715 720

Gly Arg Leu Lys Thr Pro Ser Gly Lys Tyr Ile Arg Ile Gly Tyr Ala
725 730 735

Asp Lys Asn Glu His Pro Tyr Val Ser Ile Gly Arg Tyr Met Ala Asp
740 745 750

Lys Gly Tyr Leu Lys Leu Gly Gln Thr Ser Met Gln Gly Ile Lys Ala
755 760 765

Tyr Met Arg Gln Asn Pro Gln Arg Leu Ala Glu Val Leu Gly Gln Asn
770 775 780

Pro Ser Tyr Ile Phe Phe Arg Glu Leu Ala Gly Ser Ser Asn Asp Gly
785 790 795 800

Pro Val Gly Ala Leu Gly Thr Pro Leu Met Gly Glu Tyr Ala Gly Ala
805 810 815

Val Asp Arg His Tyr Ile Thr Leu Gly Ala Pro Leu Phe Val Ala Thr
820 825 830

Ala His Pro Val Thr Arg Lys Ala Leu Asn Arg Leu Ile Met Ala Gln
835 840 845

Asp Thr Gly Ser Ala Ile Lys Gly Ala Val Arg Val Asp Tyr Phe Trp
850 855 860

Gly Tyr Gly Asp Glu Ala Gly Glu Leu Ala Gly Lys Gln Lys Thr Thr
865 870 875 880

Gly Tyr Val Trp Gln Leu Leu Pro Asn Gly Met Lys Pro Glu Tyr Arg
885 890 895

Pro

<210> 96
<211> 1941
<212> DNA
<213> Artificial Sequence

<220>
<223> deltaG287NZ-953

<400> 96

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc  120
gcgccatccg cacaaggcgg tcaagatatg gcggcggttt cggaagaaaa tacaggcaat  180
ggcggtgcgg cagcaacgga caaacccaaa aatgaagacg agggggcgca aaatgatatg  240
ccgcaaaatg ccgccgatac agatagtttg acaccgaatc acaccccggc ttcgaatatg  300
ccggccggaa atatggaaaa ccaagcaccg gatgccgggg aatcggagca gccggcaaac  360
caaccggata tggcaaatac ggcggacgga atgcagggtg acgatccgtc ggcaggcggg  420
gaaaatgccg gcaatacggc tgcccaaggt acaaatcaag ccgaaaacaa tcaaaccgcc  480
ggttctcaaa atcctgcctc ttcaaccaat cctagcgcca cgaatagcgg tggtgatttt  540
ggaaggacga acgtgggcaa ttctgttgtg attgacgggc cgtcgcaaaa tataacgttg  600
acccactgta aaggcgattc ttgtagtggc aataatttct tggatgaaga agtacagcta  660
aaatcagaat ttgaaaaatt aagtgatgca gacaaaataa gtaattacaa gaaagatggg  720
aagaatgacg ggaagaatga taaatttgtc ggtttggttg ccgatagtgt gcagatgaag  780
ggaatcaatc aatatattat cttttataaa cctaaaccca cttcatttgc gcgatttagg  840
cgttctgcac ggtcgaggcg gtcgcttccg gccgagatgc cgctgattcc cgtcaatcag  900
gcggatacgc tgattgtcga tggggaagcg gtcagcctga cggggcattc cggcaatatc  960
ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggagc cacctacaaa 1440
gtggacgaat atcacgccaa cgcccgtttc gccatcgacc atttcaacac cagcaccaac 1500
gtcggcggtt tttacggtct gaccggttcc gtcgagttcg accaagcaaa acgcgacggt 1560
aaaatcgaca tcaccatccc cgttgccaac ctgcaaagcg gttcgcaaca ctttaccgac 1620
cacctgaaat cagccgacat cttcgatgcc gcccaatatc cggacatccg ctttgtttcc 1680
accaaattca acttcaacgg caaaaaactg gtttccgttg acggcaacct gaccatgcac 1740
ggcaaaaccg cccccgtcaa actcaaagcc gaaaaattca actgctacca aagcccgatg 1800
gcgaaaaccg aagtttgcgg cggcgacttc agcaccacca tcgaccgcac caaatggggc 1860
gtggactacc tcgttaacgt tggtatgacc aaaagcgtcc gcatcgacat ccaaatcgag 1920
gcagccaaac aataaaagct t                                           1941
```

<210> 97
<211> 644
<212> PRT
<213> Artificial Sequence

<220>
<223> deltaG287NZ-953

<400> 97

Met Ala Ser Pro Asp Val Lys Ser Ala Asp Thr Leu Ser Lys Pro Ala
1 5 10 15

Ala Pro Val Val Ser Glu Lys Glu Thr Glu Ala Lys Glu Asp Ala Pro
20 25 30

Gln Ala Gly Ser Gln Gly Gln Gly Ala Pro Ser Ala Gln Gly Gly Gln
35 40 45

Asp Met Ala Ala Val Ser Glu Glu Asn Thr Gly Asn Gly Gly Ala Ala
50 55 60

Ala Thr Asp Lys Pro Lys Asn Glu Asp Glu Gly Ala Gln Asn Asp Met
65 70 75 80

Pro Gln Asn Ala Ala Asp Thr Asp Ser Leu Thr Pro Asn His Thr Pro
85 90 95

Ala Ser Asn Met Pro Ala Gly Asn Met Glu Asn Gln Ala Pro Asp Ala
100 105 110

Gly Glu Ser Glu Gln Pro Ala Asn Gln Pro Asp Met Ala Asn Thr Ala
115 120 125

Asp Gly Met Gln Gly Asp Asp Pro Ser Ala Gly Gly Glu Asn Ala Gly
130 135 140

Asn Thr Ala Ala Gln Gly Thr Asn Gln Ala Glu Asn Asn Gln Thr Ala
145 150 155 160

Gly Ser Gln Asn Pro Ala Ser Ser Thr Asn Pro Ser Ala Thr Asn Ser
165 170 175

Gly Gly Asp Phe Gly Arg Thr Asn Val Gly Asn Ser Val Val Ile Asp
180 185 190

Gly Pro Ser Gln Asn Ile Thr Leu Thr His Cys Lys Gly Asp Ser Cys
195 200 205

Ser Gly Asn Asn Phe Leu Asp Glu Glu Val Gln Leu Lys Ser Glu Phe
210 215 220

Glu Lys Leu Ser Asp Ala Asp Lys Ile Ser Asn Tyr Lys Lys Asp Gly
225 230 235 240

Lys Asn Asp Gly Lys Asn Asp Lys Phe Val Gly Leu Val Ala Asp Ser
245 250 255

Val Gln Met Lys Gly Ile Asn Gln Tyr Ile Ile Phe Tyr Lys Pro Lys
260 265 270

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu

290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

Ser Tyr Arg Pro Thr Asp Ala Glu Lys Gly Gly Phe Gly Val Phe Ala
450 455 460

Gly Lys Lys Glu Gln Asp Gly Ser Gly Gly Gly Gly Ala Thr Tyr Lys
465 470 475 480

Val Asp Glu Tyr His Ala Asn Ala Arg Phe Ala Ile Asp His Phe Asn
485 490 495

Thr Ser Thr Asn Val Gly Gly Phe Tyr Gly Leu Thr Gly Ser Val Glu
500 505 510

Phe Asp Gln Ala Lys Arg Asp Gly Lys Ile Asp Ile Thr Ile Pro Val
515 520 525

Ala Asn Leu Gln Ser Gly Ser Gln His Phe Thr Asp His Leu Lys Ser
530 535 540

Ala Asp Ile Phe Asp Ala Ala Gln Tyr Pro Asp Ile Arg Phe Val Ser
545 550 555 560

Thr Lys Phe Asn Phe Asn Gly Lys Lys Leu Val Ser Val Asp Gly Asn
565 570 575

Leu Thr Met His Gly Lys Thr Ala Pro Val Lys Leu Lys Ala Glu Lys
580 585 590

Phe Asn Cys Tyr Gln Ser Pro Met Ala Lys Thr Glu Val Cys Gly Gly

595 600 605

Asp Phe Ser Thr Thr Ile Asp Arg Thr Lys Trp Gly Val Asp Tyr Leu
610 615 620

Val Asn Val Gly Met Thr Lys Ser Val Arg Ile Asp Ile Gln Ile Glu
625 630 635 640

Ala Ala Lys Gln

<210> 98
<211> 2583
<212> DNA
<213> Artificial Sequence

<220>
<223> deltaG287NZ-961

<400> 98

atggctagcc ccgatgtcaa gtcggcggac acgctgtcaa aacctgccgc ccctgttgtt 60
tctgaaaaag agacagaggc aaaggaagat gcgccacagg caggttctca aggacagggc 120
gcgccatccg cacaaggcgg tcaagatatg gcggcggttt cggaagaaaa tacaggcaat 180
ggcggtgcgg cagcaacgga caaacccaaa aatgaagacg aggggggcgca aaatgatatg 240
ccgcaaaatg ccgccgatac agatagtttg acaccgaatc acaccccggc ttcgaatatg 300
ccggccggaa atatggaaaa ccaagcaccg gatgccgggg aatcggagca gccggcaaac 360
caaccggata tggcaaatac ggcggacgga atgcagggtg acgatccgtc ggcaggcggg 420
gaaaatgccg gcaatacggc tgcccaaggt acaaatcaag ccgaaaacaa tcaaaccgcc 480
ggttctcaaa atcctgcctc ttcaaccaat cctagcgcca cgaatagcgg tggtgatttt 540
ggaaggacga acgtgggcaa ttctgttgtg attgacgggc cgtcgcaaaa tataacgttg 600
acccactgta aaggcgattc ttgtagtggc aataatttct tggatgaaga agtacagcta 660
aaatcagaat ttgaaaaatt aagtgatgca gacaaaataa gtaattacaa gaaagatggg 720
aagaatgacg ggaagaatga taaatttgtc ggtttggttg ccgatagtgt gcagatgaag 780
ggaatcaatc aatatattat cttttataaa cctaaaccca cttcatttgc gcgatttagg 840
cgttctgcac ggtcgaggcg gtcgcttccg gccgagatgc cgctgattcc cgtcaatcag 900
gcggatacgc tgattgtcga tggggaagcg gtcagcctga cggggcattc cggcaatatc 960
ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggagc cacaaacgac 1440
gacgatgtta aaaaagctgc cactgtggcc attgctgctg cctacaacaa tggccaagaa 1500
atcaacggtt tcaaagctgg agagaccatc tacgacattg atgaagacgg cacaattacc 1560
aaaaaagacg caactgcagc cgatgttgaa gccgacgact ttaaaggtct gggtctgaaa 1620
aaagtcgtga ctaacctgac caaaaccgtc aatgaaaaca aacaaaacgt cgatgccaaa 1680
gtaaaagctg cagaatctga aatagaaaag ttaacaacca agttagcaga cactgatgcc 1740
gctttagcag atactgatgc cgctctggat gcaaccacca acgccttgaa taaattggga 1800
gaaaatataa cgacatttgc tgaagagact aagacaaata tcgtaaaaat tgatgaaaaa 1860
ttagaagccg tggctgatac cgtcgacaag catgccgaag cattcaacga tatcgccgat 1920
tcattggatg aaaccaacac taaggcagac gaagccgtca aaaccgccaa tgaagccaaa 1980
cagacggccg aagaaaccaa acaaaacgtc gatgccaaag taaaagctgc agaaactgca 2040
gcaggcaaag ccgaagctgc cgctggcaca gctaatactg cagccgacaa ggccgaagct 2100
gtcgctgcaa aagttaccga catcaaagct gatatcgcta cgaacaaaga taatattgct 2160
aaaaaagcaa acagtgccga cgtgtacacc agagaagagt ctgacagcaa atttgtcaga 2220

attgatggtc tgaacgctac taccgaaaaa ttggacacac gcttggcttc tgctgaaaaa 2280
tccattgccg atcacgatac tcgcctgaac ggtttggata aaacagtgtc agacctgcgc 2340
aaagaaaccc gccaaggcct tgcagaacaa gccgcgctct ccggtctgtt ccaaccttac 2400
aacgtgggtc ggttcaatgt aacggctgca gtcggcggct acaaatccga atcggcagtc 2460
gccatcggta ccggcttccg ctttaccgaa aactttgccg ccaaagcagg cgtggcagtc 2520
ggcacttcgt ccggttcttc cgcagcctac catgtcggcg tcaattacga gtggtaaaag 2580
ctt 2583

<210> 99
<211> 858
<212> PRT
<213> Artificial Sequence

<220>
<223> deltaG287NZ-961

<400> 99

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
```

```
    210                 215                 220

Glu Lys Leu Ser Asp Ala Asp Lys Ile Ser Asn Tyr Lys Lys Asp Gly
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270

Pro Thr Ser Phe Ala Arg Phe Arg Arg Ser Ala Arg Ser Arg Arg Ser
        275                 280                 285

Leu Pro Ala Glu Met Pro Leu Ile Pro Val Asn Gln Ala Asp Thr Leu
    290                 295                 300

Ile Val Asp Gly Glu Ala Val Ser Leu Thr Gly His Ser Gly Asn Ile
305                 310                 315                 320

Phe Ala Pro Glu Gly Asn Tyr Arg Tyr Leu Thr Tyr Gly Ala Glu Lys
                325                 330                 335

Leu Pro Gly Gly Ser Tyr Ala Leu Arg Val Gln Gly Glu Pro Ser Lys
            340                 345                 350

Gly Glu Met Leu Ala Gly Thr Ala Val Tyr Asn Gly Glu Val Leu His
        355                 360                 365

Phe His Thr Glu Asn Gly Arg Pro Ser Pro Ser Arg Gly Arg Phe Ala
    370                 375                 380

Ala Lys Val Asp Phe Gly Ser Lys Ser Val Asp Gly Ile Ile Asp Ser
385                 390                 395                 400

Gly Asp Gly Leu His Met Gly Thr Gln Lys Phe Lys Ala Ala Ile Asp
                405                 410                 415

Gly Asn Gly Phe Lys Gly Thr Trp Thr Glu Asn Gly Gly Gly Asp Val
            420                 425                 430

Ser Gly Lys Phe Tyr Gly Pro Ala Gly Glu Glu Val Ala Gly Lys Tyr
        435                 440                 445

Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Ala Thr Asn Asp
465                 470                 475                 480

Asp Asp Val Lys Lys Ala Ala Thr Val Ala Ile Ala Ala Ala Tyr Asn
                485                 490                 495

Asn Gly Gln Glu Ile Asn Gly Phe Lys Ala Gly Glu Thr Ile Tyr Asp
            500                 505                 510

Ile Asp Glu Asp Gly Thr Ile Thr Lys Lys Asp Ala Thr Ala Ala Asp
```

515 520 525

Val Glu Ala Asp Asp Phe Lys Gly Leu Gly Leu Lys Lys Val Val Thr
530 535 540

Asn Leu Thr Lys Thr Val Asn Glu Asn Lys Gln Asn Val Asp Ala Lys
545 550 555 560

Val Lys Ala Ala Glu Ser Glu Ile Glu Lys Leu Thr Thr Lys Leu Ala
565 570 575

Asp Thr Asp Ala Ala Leu Ala Asp Thr Asp Ala Ala Leu Asp Ala Thr
580 585 590

Thr Asn Ala Leu Asn Lys Leu Gly Glu Asn Ile Thr Thr Phe Ala Glu
595 600 605

Glu Thr Lys Thr Asn Ile Val Lys Ile Asp Glu Lys Leu Glu Ala Val
610 615 620

Ala Asp Thr Val Asp Lys His Ala Glu Ala Phe Asn Asp Ile Ala Asp
625 630 635 640

Ser Leu Asp Glu Thr Asn Thr Lys Ala Asp Glu Ala Val Lys Thr Ala
645 650 655

Asn Glu Ala Lys Gln Thr Ala Glu Glu Thr Lys Gln Asn Val Asp Ala
660 665 670

Lys Val Lys Ala Ala Glu Thr Ala Ala Gly Lys Ala Glu Ala Ala Ala
675 680 685

Gly Thr Ala Asn Thr Ala Ala Asp Lys Ala Glu Ala Val Ala Ala Lys
690 695 700

Val Thr Asp Ile Lys Ala Asp Ile Ala Thr Asn Lys Asp Asn Ile Ala
705 710 715 720

Lys Lys Ala Asn Ser Ala Asp Val Tyr Thr Arg Glu Glu Ser Asp Ser
725 730 735

Lys Phe Val Arg Ile Asp Gly Leu Asn Ala Thr Thr Glu Lys Leu Asp
740 745 750

Thr Arg Leu Ala Ser Ala Glu Lys Ser Ile Ala Asp His Asp Thr Arg
755 760 765

Leu Asn Gly Leu Asp Lys Thr Val Ser Asp Leu Arg Lys Glu Thr Arg
770 775 780

Gln Gly Leu Ala Glu Gln Ala Ala Leu Ser Gly Leu Phe Gln Pro Tyr
785 790 795 800

Asn Val Gly Arg Phe Asn Val Thr Ala Ala Val Gly Gly Tyr Lys Ser
805 810 815

Glu Ser Ala Val Ala Ile Gly Thr Gly Phe Arg Phe Thr Glu Asn Phe

820 825 830

Ala Ala Lys Ala Gly Val Ala Val Gly Thr Ser Ser Gly Ser Ser Ala
835 840 845

Ala Tyr His Val Gly Val Asn Tyr Glu Trp
850 855

<210> 100
<211> 4425
<212> DNA
<213> Artificial Sequence

<220>
<223> deltaG983-ORF46.1

<400> 100

```
atgacttctg cgcccgactt caatgcaggc ggtaccggta tcggcagcaa cagcagagca 60
acaacagcga aatcagcagc agtatcttac gccggtatca agaacgaaat gtgcaaagac 120
agaagcatgc tctgtgccgg tcgggatgac gttgcggtta cagacaggga tgccaaaatc 180
aatgcccccc ccccgaatct gcataccgga gactttccaa acccaatga cgcatacaag 240
aatttgatca acctcaaacc tgcaattgaa gcaggctata caggacgcgg ggtagaggta 300
ggtatcgtcg acacaggcga atccgtcggc agcatatcct ttcccgaact gtatggcaga 360
aaagaacacg gctataacga aaattacaaa aactatacgg cgtatatgcg gaaggaagcg 420
cctgaagacg gaggcggtaa agacattgaa gcttctttcg acgatgaggc cgttatagag 480
actgaagcaa agccgacgga tatccgccac gtaaaagaaa tcggacacat cgatttggtc 540
tcccatatta ttggcgggcg ttccgtggac ggcagacctg caggcggtat tgcgcccgat 600
gcgacgctac acataatgaa tacgaatgat gaaaccaaga acgaaatgat ggttgcagcc 660
atccgcaatg catgggtcaa gctgggcgaa cgtggcgtgc gcatcgtcaa taacagtttt 720
ggaacaacat cgagggcagg cactgccgac cttttccaaa tagccaattc ggaggagcag 780
taccgccaag cgttgctcga ctattccggc ggtgataaaa cagacgaggg tatccgcctg 840
atgcaacaga gcgattacgg caacctgtcc taccacatcc gtaataaaaa catgcttttc 900
atcttttcga caggcaatga cgcacaagct cagcccaaca catatgccct attgccattt 960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
```

```
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgac ggtggcggag gcactggatc ctcagatttg 3180
gcaaacgatt cttttatccg gcaggttctc gaccgtcagc atttcgaacc cgacgggaaa 3240
taccacctat tcggcagcag gggggaactt gccgagcgca gcggccatat cggattggga 3300
aaaatacaaa gccatcagtt gggcaacctg atgattcaac aggcggccat taaaggaaat 3360
atcggctaca ttgtccgctt ttccgatcac gggcacgaag tccattcccc cttcgacaac 3420
catgcctcac attccgattc tgatgaagcc ggtagtcccg ttgacggatt tagcctttac 3480
cgcatccatt gggacggata cgaacaccat cccgccgacg gctatgacgg gccacagggc 3540
ggcggctatc ccgctcccaa aggcgcgagg gatatataca gctacgacat aaaaggcgtt 3600
gcccaaaata tccgcctcaa cctgaccgac aaccgcagca ccggacaacg gcttgccgac 3660
cgtttccaca atgccggtag tatgctgacg caaggagtag gcgacggatt caaacgcgcc 3720
acccgataca gccccgagct ggacagatcg ggcaatgccg ccgaagcctt caacggcact 3780
gcagatatcg ttaaaaacat catcggcgcg gcaggagaaa ttgtcggcgc aggcgatgcc 3840
gtgcagggca taagcgaagg ctcaaacatt gctgtcatgc acggcttggg tctgctttcc 3900
accgaaaaca agatggcgcg catcaacgat ttggcagata tggcgcaact caaagactat 3960
gccgcagcag ccatccgcga ttgggcagtc caaaacccca atgccgcaca aggcatagaa 4020
gccgtcagca atatctttat ggcagccatc cccatcaaag ggattggagc tgttcgggga 4080
aaatacggct tgggcggcat cacggcacat cctatcaagc ggtcgcagat gggcgcgatc 4140
gcattgccga aagggaaatc cgccgtcagc gacaattttg ccgatgcggc atacgccaaa 4200
tacccgtccc cttaccattc ccgaaatatc cgttcaaact tggagcagcg ttacggcaaa 4260
gaaaacatca cctcctcaac cgtgccgccg tcaaacggca aaaatgtcaa actggcagac 4320
caacgccacc cgaagacagg cgtaccgttt gacggtaaag ggtttccgaa ttttgagaag 4380
cacgtgaaat atgatacgct cgagcaccac caccaccacc actga 4425
```

<210> 101
<211> 1474
<212> PRT
<213> Artificial Sequence

<220>
<223> deltaG983-ORF46.1

<400> 101

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
```

65 70 75 80

Asn Leu Ile Asn Leu Lys Pro Ala Ile Glu Ala Gly Tyr Thr Gly Arg
85 90 95

Gly Val Glu Val Gly Ile Val Asp Thr Gly Glu Ser Val Gly Ser Ile
100 105 110

Ser Phe Pro Glu Leu Tyr Gly Arg Lys Glu His Gly Tyr Asn Glu Asn
115 120 125

Tyr Lys Asn Tyr Thr Ala Tyr Met Arg Lys Glu Ala Pro Glu Asp Gly
130 135 140

Gly Gly Lys Asp Ile Glu Ala Ser Phe Asp Asp Glu Ala Val Ile Glu
145 150 155 160

Thr Glu Ala Lys Pro Thr Asp Ile Arg His Val Lys Glu Ile Gly His
165 170 175

Ile Asp Leu Val Ser His Ile Ile Gly Gly Arg Ser Val Asp Gly Arg
180 185 190

Pro Ala Gly Gly Ile Ala Pro Asp Ala Thr Leu His Ile Met Asn Thr
195 200 205

Asn Asp Glu Thr Lys Asn Glu Met Met Val Ala Ala Ile Arg Asn Ala
210 215 220

Trp Val Lys Leu Gly Glu Arg Gly Val Arg Ile Val Asn Asn Ser Phe
225 230 235 240

Gly Thr Thr Ser Arg Ala Gly Thr Ala Asp Leu Phe Gln Ile Ala Asn
245 250 255

Ser Glu Glu Gln Tyr Arg Gln Ala Leu Leu Asp Tyr Ser Gly Gly Asp
260 265 270

Lys Thr Asp Glu Gly Ile Arg Leu Met Gln Gln Ser Asp Tyr Gly Asn
275 280 285

Leu Ser Tyr His Ile Arg Asn Lys Asn Met Leu Phe Ile Phe Ser Thr
290 295 300

Gly Asn Asp Ala Gln Ala Gln Pro Asn Thr Tyr Ala Leu Leu Pro Phe
305 310 315 320

Tyr Glu Lys Asp Ala Gln Lys Gly Ile Ile Thr Val Ala Gly Val Asp
325 330 335

Arg Ser Gly Glu Lys Phe Lys Arg Glu Met Tyr Gly Glu Pro Gly Thr
340 345 350

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn

370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn

675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala

980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Asp Gly Gly Gly Gly Thr Gly
1045 1050 1055

Ser Ser Asp Leu Ala Asn Asp Ser Phe Ile Arg Gln Val Leu Asp Arg
1060 1065 1070

Gln His Phe Glu Pro Asp Gly Lys Tyr His Leu Phe Gly Ser Arg Gly
1075 1080 1085

Glu Leu Ala Glu Arg Ser Gly His Ile Gly Leu Gly Lys Ile Gln Ser
1090 1095 1100

His Gln Leu Gly Asn Leu Met Ile Gln Gln Ala Ala Ile Lys Gly Asn
1105 1110 1115 1120

Ile Gly Tyr Ile Val Arg Phe Ser Asp His Gly His Glu Val His Ser
1125 1130 1135

Pro Phe Asp Asn His Ala Ser His Ser Asp Ser Asp Glu Ala Gly Ser
1140 1145 1150

Pro Val Asp Gly Phe Ser Leu Tyr Arg Ile His Trp Asp Gly Tyr Glu
1155 1160 1165

His His Pro Ala Asp Gly Tyr Asp Gly Pro Gln Gly Gly Gly Tyr Pro
1170 1175 1180

Ala Pro Lys Gly Ala Arg Asp Ile Tyr Ser Tyr Asp Ile Lys Gly Val
1185 1190 1195 1200

Ala Gln Asn Ile Arg Leu Asn Leu Thr Asp Asn Arg Ser Thr Gly Gln
1205 1210 1215

Arg Leu Ala Asp Arg Phe His Asn Ala Gly Ser Met Leu Thr Gln Gly
1220 1225 1230

Val Gly Asp Gly Phe Lys Arg Ala Thr Arg Tyr Ser Pro Glu Leu Asp
1235 1240 1245

Arg Ser Gly Asn Ala Ala Glu Ala Phe Asn Gly Thr Ala Asp Ile Val
1250 1255 1260

Lys Asn Ile Ile Gly Ala Ala Gly Glu Ile Val Gly Ala Gly Asp Ala
1265 1270 1275 1280

Val Gln Gly Ile Ser Glu Gly Ser Asn Ile Ala Val Met His Gly Leu

1285 1290 1295

Gly Leu Leu Ser Thr Glu Asn Lys Met Ala Arg Ile Asn Asp Leu Ala
1300 1305 1310

Asp Met Ala Gln Leu Lys Asp Tyr Ala Ala Ala Ala Ile Arg Asp Trp
1315 1320 1325

Ala Val Gln Asn Pro Asn Ala Ala Gln Gly Ile Glu Ala Val Ser Asn
1330 1335 1340

Ile Phe Met Ala Ala Ile Pro Ile Lys Gly Ile Gly Ala Val Arg Gly
1345 1350 1355 1360

Lys Tyr Gly Leu Gly Gly Ile Thr Ala His Pro Ile Lys Arg Ser Gln
1365 1370 1375

Met Gly Ala Ile Ala Leu Pro Lys Gly Lys Ser Ala Val Ser Asp Asn
1380 1385 1390

Phe Ala Asp Ala Ala Tyr Ala Lys Tyr Pro Ser Pro Tyr His Ser Arg
1395 1400 1405

Asn Ile Arg Ser Asn Leu Glu Gln Arg Tyr Gly Lys Glu Asn Ile Thr
1410 1415 1420

Ser Ser Thr Val Pro Pro Ser Asn Gly Lys Asn Val Lys Leu Ala Asp
1425 1430 1435 1440

Gln Arg His Pro Lys Thr Gly Val Pro Phe Asp Gly Lys Gly Phe Pro
1445 1450 1455

Asn Phe Glu Lys His Val Lys Tyr Asp Thr Leu Glu His His His His
1460 1465 1470

His His

<210> 102
<211> 3939
<212> DNA
<213> Artificial Sequence

<220>
<223> deltaG983-741

<400> 102

```
atgacttctg cgcccgactt caatgcaggc ggtaccggta tcggcagcaa cagcagagca   60
acaacagcga aatcagcagc agtatcttac gccggtatca agaacgaaat gtgcaaagac  120
agaagcatgc tctgtgccgg tcgggatgac gttgcggtta cagacaggga tgccaaaatc  180
aatgcccccc ccccgaatct gcataccgga gactttccaa acccaaatga cgcatacaag  240
aatttgatca acctcaaacc tgcaattgaa gcaggctata caggacgcgg ggtagaggta  300
ggtatcgtcg acacaggcga atccgtcggc agcatatcct ttcccgaact gtatggcaga  360
aaagaacacg gctataacga aaattacaaa aactatacgg cgtatatgcg gaaggaagcg  420
cctgaagacg gaggcggtaa agacattgaa gcttctttcg acgatgaggc cgttatagag  480
actgaagcaa agccgacgga tatccgccac gtaaaagaaa tcggacacat cgatttggtc  540
tcccatatta ttggcgggcg ttccgtggac ggcagacctg caggcggtat tgcgcccgat  600
```

gcgacgctac acataatgaa tacgaatgat gaaaccaaga acgaaatgat ggttgcagcc 660
atccgcaatg catgggtcaa gctgggcgaa cgtggcgtgc gcatcgtcaa taacagtttt 720
ggaacaacat cgagggcagg cactgccgac cttttccaaa tagccaattc ggaggagcag 780
taccgccaag cgttgctcga ctattccggc ggtgataaaa cagacgaggg tatccgcctg 840
atgcaacaga gcgattacgg caacctgtcc taccacatcc gtaataaaaa catgcttttc 900
atcttttcga caggcaatga cgcacaagct cagcccaaca catatgccct attgccattt 960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgag ggatccggag gggtggtgt cgccgccgac 3180
atcggtgcgg ggcttgccga tgcactaacc gcaccgctcg accataaaga caaaggtttg 3240
cagtctttga cgctggatca gtccgtcagg aaaaacgaga aactgaagct ggcggcacaa 3300
ggtgcggaaa aaacttatgg aaacggtgac agcctcaata cgggcaaatt gaagaacgac 3360
aaggtcagcc gtttcgactt tatccgccaa atcgaagtgg acgggcagct cattaccttg 3420
gagagtggag agttccaagt atacaaacaa agccattccg ccttaaccgc ctttcagacc 3480
gagcaaatac aagattcgga gcattccggg aagatggttg cgaaacgcca gttcagaatc 3540
ggcgacatag cgggcgaaca tacatctttt gacaagcttc ccgaaggcgg cagggcgaca 3600
tatcgcggga cggcgttcgg ttcagacgat gccggcggaa aactgaccta caccatagat 3660
ttcgccgcca agcagggaaa cggcaaaatc gaacatttga aatcgccaga actcaatgtc 3720
gacctggccg ccgccgatat caagccggat ggaaaacgcc atgccgtcat cagcggttcc 3780
gtcctttaca accaagccga gaaaggcagt tactccctcg gtatctttgg cggaaaagcc 3840
caggaagttg ccggcagcgc ggaagtgaaa accgtaaacg gcatacgcca tatcggcctt 3900
gccgccaagc aactcgagca ccaccaccac caccactga 3939

<210> 103
<211> 1312

<212> PRT
<213> Artificial Sequence

<220>
<223> deltaG983-741

<400> 1.03

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255
```

Ser Glu Glu Gln Tyr Arg Gln Ala Leu Leu Asp Tyr Ser Gly Gly Asp
260 265 270

Lys Thr Asp Glu Gly Ile Arg Leu Met Gln Gln Ser Asp Tyr Gly Asn
275 280 285

Leu Ser Tyr His Ile Arg Asn Lys Asn Met Leu Phe Ile Phe Ser Thr
290 295 300

Gly Asn Asp Ala Gln Ala Gln Pro Asn Thr Tyr Ala Leu Leu Pro Phe
305 310 315 320

Tyr Glu Lys Asp Ala Gln Lys Gly Ile Ile Thr Val Ala Gly Val Asp
325 330 335

Arg Ser Gly Glu Lys Phe Lys Arg Glu Met Tyr Gly Glu Pro Gly Thr
340 345 350

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Ser Gly Gly Gly Gly
1045 1050 1055

Val Ala Ala Asp Ile Gly Ala Gly Leu Ala Asp Ala Leu Thr Ala Pro
1060 1065 1070

Leu Asp His Lys Asp Lys Gly Leu Gln Ser Leu Thr Leu Asp Gln Ser
1075 1080 1085

Val Arg Lys Asn Glu Lys Leu Lys Leu Ala Ala Gln Gly Ala Glu Lys
1090 1095 1100

Thr Tyr Gly Asn Gly Asp Ser Leu Asn Thr Gly Lys Leu Lys Asn Asp
1105 1110 1115 1120

Lys Val Ser Arg Phe Asp Phe Ile Arg Gln Ile Glu Val Asp Gly Gln
1125 1130 1135

Leu Ile Thr Leu Glu Ser Gly Glu Phe Gln Val Tyr Lys Gln Ser His
1140 1145 1150

Ser Ala Leu Thr Ala Phe Gln Thr Glu Gln Ile Gln Asp Ser Glu His
1155 1160 1165

Ser Gly Lys Met Val Ala Lys Arg Gln Phe Arg Ile Gly Asp Ile Ala
1170 1175 1180

Gly Glu His Thr Ser Phe Asp Lys Leu Pro Glu Gly Gly Arg Ala Thr
1185 1190 1195 1200

Tyr Arg Gly Thr Ala Phe Gly Ser Asp Asp Ala Gly Gly Lys Leu Thr
1205 1210 1215

Tyr Thr Ile Asp Phe Ala Ala Lys Gln Gly Asn Gly Lys Ile Glu His
1220 1225 1230

Leu Lys Ser Pro Glu Leu Asn Val Asp Leu Ala Ala Ala Asp Ile Lys
1235 1240 1245

Pro Asp Gly Lys Arg His Ala Val Ile Ser Gly Ser Val Leu Tyr Asn
1250 1255 1260

Gln Ala Glu Lys Gly Ser Tyr Ser Leu Gly Ile Phe Gly Gly Lys Ala
1265 1270 1275 1280

Gln Glu Val Ala Gly Ser Ala Glu Val Lys Thr Val Asn Gly Ile Arg
1285 1290 1295

His Ile Gly Leu Ala Ala Lys Gln Leu Glu His His His His His His
1300 1305 1310

<210> 104
<211> 4344
<212> DNA
<213> Artificial Sequence

<220>
<223> deltaG983-961

<400> 104

atgacttctg cgcccgactt caatgcaggc ggtaccggta tcggcagcaa cagcagagca 60
acaacagcga aatcagcagc agtatcttac gccggtatca agaacgaaat gtgcaaagac 120
agaagcatgc tctgtgccgg tcgggatgac gttgcggtta cagacaggga tgccaaaatc 180
aatgcccccc ccccgaatct gcataccgga gactttccaa acccaaatga cgcatacaag 240
aatttgatca acctcaaacc tgcaattgaa gcaggctata caggacgcgg ggtagaggta 300
ggtatcgtcg acacaggcga atccgtcggc agcatatcct ttcccgaact gtatggcaga 360
aaagaacacg gctataacga aaattacaaa aactatacgg cgtatatgcg gaaggaagcg 420
cctgaagacg gaggcggtaa agacattgaa gcttctttcg acgatgaggc cgttatagag 480
actgaagcaa agccgacgga tatccgccac gtaaaagaaa tcggacacat cgatttggtc 540
tcccatatta ttggcgggcg ttccgtggac ggcagacctg caggcggtat tgcgcccgat 600
gcgacgctac acataatgaa tacgaatgat gaaaccaaga acgaaatgat ggttgcagcc 660
atccgcaatg catgggtcaa gctgggcgaa cgtggcgtgc gcatcgtcaa taacagtttt 720
ggaacaacat cgagggcagg cactgccgac cttttccaaa tagccaattc ggaggagcag 780
taccgccaag cgttgctcga ctattccggc ggtgataaaa cagacgaggg tatccgcctg 840
atgcaacaga gcgattacgg caacctgtcc taccacatcc gtaataaaaa catgcttttc 900
atcttttcga caggcaatga cgcacaagct cagcccaaca catatgccct attgccattt 960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200

acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgag ggtggcggag gcactggatc cgccacaaac 3180
gacgacgatg ttaaaaaagc tgccactgtg gccattgctg ctgcctacaa caatggccaa 3240
gaaatcaacg gtttcaaagc tggagagacc atctacgaca ttgatgaaga cggcacaatt 3300
accaaaaaag acgcaactgc agccgatgtt gaagccgacg actttaaagg tctgggtctg 3360
aaaaaagtcg tgactaacct gaccaaaacc gtcaatgaaa acaaacaaaa cgtcgatgcc 3420
aaagtaaaag ctgcagaatc tgaaatagaa aagttaacaa ccaagttagc agacactgat 3480
gccgctttag cagatactga tgccgctctg gatgcaacca ccaacgcctt gaataaattg 3540
ggagaaaata taacgacatt tgctgaagag actaagacaa atatcgtaaa aattgatgaa 3600
aaattagaag ccgtggctga taccgtcgac aagcatgccg aagcattcaa cgatatcgcc 3660
gattcattgg atgaaaccaa cactaaggca gacgaagccg tcaaaaccgc caatgaagcc 3720
aaacagacgg ccgaagaaac caaacaaaac gtcgatgcca aagtaaaagc tgcagaaact 3780
gcagcaggca aagccgaagc tgccgctggc acagctaata ctgcagccga caaggccgaa 3840
gctgtcgctg caaaagttac cgacatcaaa gctgatatcg ctacgaacaa agataatatt 3900
gctaaaaaag caaacagtgc cgacgtgtac accagagaag agtctgacag caaatttgtc 3960
agaattgatg gtctgaacgc tactaccgaa aaattggaca cacgcttggc ttctgctgaa 4020
aaatccattg ccgatcacga tactcgcctg aacggtttgg ataaaacagt gtcagacctg 4080
cgcaaagaaa cccgccaagg ccttgcagaa caagccgcgc tctccggtct gttccaacct 4140
tacaacgtgg gtcggttcaa tgtaacggct gcagtcggcg gctacaaatc cgaatcggca 4200
gtcgccatcg gtaccggctt ccgctttacc gaaaactttg ccgccaaagc aggcgtggca 4260
gtcggcactt cgtccggttc ttccgcagcc taccatgtcg gcgtcaatta cgagtggctc 4320
gagcaccacc accaccacca ctga 4344

<210> 105
<211> 1447
<212> PRT

<213> Artificial Sequence

<220>
<223> deltaG983-961

<400> 105

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270
```

Lys Thr Asp Glu Gly Ile Arg Leu Met Gln Gln Ser Asp Tyr Gly Asn
275 280 285

Leu Ser Tyr His Ile Arg Asn Lys Asn Met Leu Phe Ile Phe Ser Thr
290 295 300

Gly Asn Asp Ala Gln Ala Gln Pro Asn Thr Tyr Ala Leu Leu Pro Phe
305 310 315 320

Tyr Glu Lys Asp Ala Gln Lys Gly Ile Ile Thr Val Ala Gly Val Asp
325 330 335

Arg Ser Gly Glu Lys Phe Lys Arg Glu Met Tyr Gly Glu Pro Gly Thr
340 345 350

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Gly Gly Gly Thr Gly
1045 1050 1055

Ser Ala Thr Asn Asp Asp Asp Val Lys Lys Ala Ala Thr Val Ala Ile
1060 1065 1070

Ala Ala Ala Tyr Asn Asn Gly Gln Glu Ile Asn Gly Phe Lys Ala Gly
1075 1080 1085

Glu Thr Ile Tyr Asp Ile Asp Glu Asp Gly Thr Ile Thr Lys Lys Asp
1090 1095 1100

Ala Thr Ala Ala Asp Val Glu Ala Asp Asp Phe Lys Gly Leu Gly Leu
1105 1110 1115 1120

Lys Lys Val Val Thr Asn Leu Thr Lys Thr Val Asn Glu Asn Lys Gln
1125 1130 1135

Asn Val Asp Ala Lys Val Lys Ala Ala Glu Ser Glu Ile Glu Lys Leu
1140 1145 1150

Thr Thr Lys Leu Ala Asp Thr Asp Ala Ala Leu Ala Asp Thr Asp Ala
1155 1160 1165

Ala Leu Asp Ala Thr Thr Asn Ala Leu Asn Lys Leu Gly Glu Asn Ile
1170 1175 1180

Thr Thr Phe Ala Glu Glu Thr Lys Thr Asn Ile Val Lys Ile Asp Glu
1185 1190 1195 1200

Lys Leu Glu Ala Val Ala Asp Thr Val Asp Lys His Ala Glu Ala Phe
1205 1210 1215

Asn Asp Ile Ala Asp Ser Leu Asp Glu Thr Asn Thr Lys Ala Asp Glu
1220 1225 1230

Ala Val Lys Thr Ala Asn Glu Ala Lys Gln Thr Ala Glu Glu Thr Lys
1235 1240 1245

Gln Asn Val Asp Ala Lys Val Lys Ala Ala Glu Thr Ala Ala Gly Lys
1250 1255 1260

Ala Glu Ala Ala Ala Gly Thr Ala Asn Thr Ala Ala Asp Lys Ala Glu
1265 1270 1275 1280

Ala Val Ala Ala Lys Val Thr Asp Ile Lys Ala Asp Ile Ala Thr Asn
1285 1290 1295

Lys Asp Asn Ile Ala Lys Lys Ala Asn Ser Ala Asp Val Tyr Thr Arg
1300 1305 1310

Glu Glu Ser Asp Ser Lys Phe Val Arg Ile Asp Gly Leu Asn Ala Thr
1315 1320 1325

Thr Glu Lys Leu Asp Thr Arg Leu Ala Ser Ala Glu Lys Ser Ile Ala
1330 1335 1340

Asp His Asp Thr Arg Leu Asn Gly Leu Asp Lys Thr Val Ser Asp Leu
1345 1350 1355 1360

Arg Lys Glu Thr Arg Gln Gly Leu Ala Glu Gln Ala Ala Leu Ser Gly
1365 1370 1375

Leu Phe Gln Pro Tyr Asn Val Gly Arg Phe Asn Val Thr Ala Ala Val
1380 1385 1390

Gly Gly Tyr Lys Ser Glu Ser Ala Val Ala Ile Gly Thr Gly Phe Arg
1395 1400 1405

Phe Thr Glu Asn Phe Ala Ala Lys Ala Gly Val Ala Val Gly Thr Ser
1410 1415 1420

Ser Gly Ser Ser Ala Ala Tyr His Val Gly Val Asn Tyr Glu Trp Leu
1425 1430 1435 1440

Glu His His His His His His
1445

<210> 106
<211> 4179
<212> DNA
<213> Artificial Sequence

<220>

<223> deltaG983-961c

<400> 106

```
atgacttctg cgcccgactt caatgcaggc ggtaccggta tcggcagcaa cagcagagca    60
acaacagcga aatcagcagc agtatcttac gccggtatca agaacgaaat gtgcaaagac   120
agaagcatgc tctgtgccgg tcgggatgac gttgcggtta cagacaggga tgccaaaatc   180
aatgcccccc ccccgaatct gcataccgga gactttccaa acccaaatga cgcatacaag   240
aatttgatca acctcaaacc tgcaattgaa gcaggctata caggacgcgg ggtagaggta   300
ggtatcgtcg acacaggcga atccgtcggc agcatatcct ttcccgaact gtatggcaga   360
aaagaacacg gctataacga aaattacaaa aactatacgg cgtatatgcg gaaggaagcg   420
cctgaagacg gaggcggtaa agacattgaa gcttctttcg acgatgaggc cgttatagag   480
actgaagcaa agccgacgga tatccgccac gtaaaagaaa tcggacacat cgatttggtc   540
tcccatatta ttggcgggcg ttccgtggac ggcagacctg caggcggtat tgcgcccgat   600
gcgacgctac acataatgaa tacgaatgat gaaaccaaga acgaaatgat ggttgcagcc   660
atccgcaatg catgggtcaa gctgggcgaa cgtggcgtgc gcatcgtcaa taacagtttt   720
ggaacaacat cgagggcagg cactgccgac cttttccaaa tagccaattc ggaggagcag   780
taccgccaag cgttgctcga ctattccggc ggtgataaaa cagacgaggg tatccgcctg   840
atgcaacaga gcgattacgg caacctgtcc taccacatcc gtaataaaaa catgcttttc   900
atcttttcga caggcaatga cgcacaagct cagcccaaca catatgccct attgccattt   960
tatgaaaaag acgctcaaaa aggcattatc acagtcgcag gcgtagaccg cagtggagaa  1020
aagttcaaac gggaaatgta tggagaaccg ggtacagaac cgcttgagta tggctccaac  1080
cattgcggaa ttactgccat gtggtgcctg tcggcaccct atgaagcaag cgtccgtttc  1140
acccgtacaa acccgattca aattgccgga acatcctttt ccgcacccat cgtaaccggc  1200
acggcggctc tgctgctgca gaaatacccg tggatgagca acgacaacct gcgtaccacg  1260
ttgctgacga cggctcagga catcggtgca gtcggcgtgg acagcaagtt cggctgggga  1320
ctgctggatg cgggtaaggc catgaacgga cccgcgtcct ttccgttcgg cgactttacc  1380
gccgatacga aaggtacatc cgatattgcc tactccttcc gtaacgacat ttcaggcacg  1440
ggcggcctga tcaaaaaagg cggcagccaa ctgcaactgc acggcaacaa cacctatacg  1500
ggcaaaacca ttatcgaagg cggttcgctg gtgttgtacg gcaacaacaa atcggatatg  1560
cgcgtcgaaa ccaaaggtgc gctgatttat aacggggcgg catccggcgg cagcctgaac  1620
agcgacggca ttgtctatct ggcagatacc gaccaatccg gcgcaaacga aaccgtacac  1680
atcaaaggca gtctgcagct ggacggcaaa ggtacgctgt acacacgttt gggcaaactg  1740
ctgaaagtgg acggtacggc gattatcggc ggcaagctgt acatgtcggc acgcggcaag  1800
ggggcaggct atctcaacag taccggacga cgtgttccct tcctgagtgc cgccaaaatc  1860
gggcaggatt attctttctt cacaaacatc gaaaccgacg gcggcctgct ggcttccctc  1920
gacagcgtcg aaaaaacagc gggcagtgaa ggcgacacgc tgtcctatta tgtccgtcgc  1980
ggcaatgcgg cacggactgc ttcggcagcg gcacattccg cgcccgccgg tctgaaacac  2040
gccgtagaac agggcggcag caatctggaa aacctgatgg tcgaactgga tgcctccgaa  2100
tcatccgcaa cacccgagac ggttgaaact gcggcagccg accgcacaga tatgccgggc  2160
atccgcccct acggcgcaac tttccgcgca gcggcagccg tacagcatgc gaatgccgcc  2220
gacggtgtac gcatcttcaa cagtctcgcc gctaccgtct atgccgacag taccgccgcc  2280
catgccgata tgcagggacg ccgcctgaaa gccgtatcgg acgggttgga ccacaacggc  2340
acgggtctgc gcgtcatcgc gcaaacccaa caggacggtg gaacgtggga acagggcggt  2400
gttgaaggca aaatgcgcgg cagtacccaa accgtcggca ttgccgcgaa aaccggcgaa  2460
aatacgacag cagccgccac actgggcatg ggacgcagca catggagcga aaacagtgca  2520
aatgcaaaaa ccgacagcat tagtctgttt gcaggcatac ggcacgatgc gggcgatatc  2580
ggctatctca aaggcctgtt ctcctacgga cgctacaaaa acagcatcag ccgcagcacc  2640
ggtgcggacg aacatgcgga aggcagcgtc aacggcacgc tgatgcagct gggcgcactg  2700
ggcggtgtca acgttccgtt tgccgcaacg ggagatttga cggtcgaagg cggtctgcgc  2760
tacgacctgc tcaaacagga tgcattcgcc gaaaaaggca gtgctttggg ctggagcggc  2820
aacagcctca ctgaaggcac gctggtcgga ctcgcgggtc tgaagctgtc gcaacccttg  2880
agcgataaag ccgtcctgtt tgcaacggcg ggcgtggaac gcgacctgaa cggacgcgac  2940
tacacggtaa cgggcggctt taccggcgcg actgcagcaa ccggcaagac gggggcacgc  3000
aatatgccgc acacccgtct ggttgccggc ctgggcgcgg atgtcgaatt cggcaacggc  3060
tggaacggct tggcacgtta cagctacgcc ggttccaaac agtacggcaa ccacagcgga  3120
cgagtcggcg taggctaccg gttcctcgag ggtggcggag gcactggatc cgccacaaac  3180
gacgacgatg ttaaaaaagc tgccactgtg gccattgctg ctgcctacaa caatggccaa  3240
```

```
gaaatcaacg gtttcaaagc tggagagacc atctacgaca ttgatgaaga cggcacaatt   3300
accaaaaaag acgcaactgc agccgatgtt gaagccgacg actttaaagg tctgggtctg   3360
aaaaaagtcg tgactaacct gaccaaaacc gtcaatgaaa acaaacaaaa cgtcgatgcc   3420
aaagtaaaag ctgcagaatc tgaaatagaa aagttaacaa ccaagttagc agacactgat   3480
gccgctttag cagatactga tgccgctctg gatgcaacca ccaacgcctt gaataaattg   3540
ggagaaaata taacgacatt tgctgaagag actaagacaa atatcgtaaa aattgatgaa   3600
aaattagaag ccgtggctga taccgtcgac aagcatgccg aagcattcaa cgatatcgcc   3660
gattcattgg atgaaaccaa cactaaggca gacgaagccg tcaaaaccgc caatgaagcc   3720
aaacagacgg ccgaagaaac caaacaaaac gtcgatgcca aagtaaaagc tgcagaaact   3780
gcagcaggca aagccgaagc tgccgctggc acagctaata ctgcagccga caaggccgaa   3840
gctgtcgctg caaaagttac cgacatcaaa gctgatatcg ctacgaacaa agataatatt   3900
gctaaaaaag caaacagtgc cgacgtgtac accagagaag agtctgacag caaatttgtc   3960
agaattgatg gtctgaacgc tactaccgaa aaattggaca cacgcttggc ttctgctgaa   4020
aaatccattg ccgatcacga tactcgcctg aacggtttgg ataaaacagt gtcagacctg   4080
cgcaaagaaa cccgccaagg ccttgcagaa caagccgcgc tctccggtct gttccaacct   4140
tacaacgtgg gtctcgagca ccaccaccac caccactga                          4179
```

<210> 107
<211> 1392
<212> PRT
<213> Artificial Sequence

<220>
<223> deltaG983-961c

<400> 107

Met Thr Ser Ala Pro Asp Phe Asn Ala Gly Gly Thr Gly Ile Gly Ser
1 5 10 15

Asn Ser Arg Ala Thr Thr Ala Lys Ser Ala Ala Val Ser Tyr Ala Gly
20 25 30

Ile Lys Asn Glu Met Cys Lys Asp Arg Ser Met Leu Cys Ala Gly Arg
35 40 45

Asp Asp Val Ala Val Thr Asp Arg Asp Ala Lys Ile Asn Ala Pro Pro
50 55 60

Pro Asn Leu His Thr Gly Asp Phe Pro Asn Pro Asn Asp Ala Tyr Lys
65 70 75 80

Asn Leu Ile Asn Leu Lys Pro Ala Ile Glu Ala Gly Tyr Thr Gly Arg
85 90 95

Gly Val Glu Val Gly Ile Val Asp Thr Gly Glu Ser Val Gly Ser Ile
100 105 110

Ser Phe Pro Glu Leu Tyr Gly Arg Lys Glu His Gly Tyr Asn Glu Asn
115 120 125

Tyr Lys Asn Tyr Thr Ala Tyr Met Arg Lys Glu Ala Pro Glu Asp Gly
130 135 140

Gly Gly Lys Asp Ile Glu Ala Ser Phe Asp Asp Glu Ala Val Ile Glu
145 150 155 160

Thr Glu Ala Lys Pro Thr Asp Ile Arg His Val Lys Glu Ile Gly His

165 170 175

Ile Asp Leu Val Ser His Ile Ile Gly Gly Arg Ser Val Asp Gly Arg
180 185 190

Pro Ala Gly Gly Ile Ala Pro Asp Ala Thr Leu His Ile Met Asn Thr
195 200 205

Asn Asp Glu Thr Lys Asn Glu Met Met Val Ala Ala Ile Arg Asn Ala
210 215 220

Trp Val Lys Leu Gly Glu Arg Gly Val Arg Ile Val Asn Asn Ser Phe
225 230 235 240

Gly Thr Thr Ser Arg Ala Gly Thr Ala Asp Leu Phe Gln Ile Ala Asn
245 250 255

Ser Glu Glu Gln Tyr Arg Gln Ala Leu Leu Asp Tyr Ser Gly Gly Asp
260 265 270

Lys Thr Asp Glu Gly Ile Arg Leu Met Gln Gln Ser Asp Tyr Gly Asn
275 280 285

Leu Ser Tyr His Ile Arg Asn Lys Asn Met Leu Phe Ile Phe Ser Thr
290 295 300

Gly Asn Asp Ala Gln Ala Gln Pro Asn Thr Tyr Ala Leu Leu Pro Phe
305 310 315 320

Tyr Glu Lys Asp Ala Gln Lys Gly Ile Ile Thr Val Ala Gly Val Asp
325 330 335

Arg Ser Gly Glu Lys Phe Lys Arg Glu Met Tyr Gly Glu Pro Gly Thr
340 345 350

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr

465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg

770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Gly Gly Gly Thr Gly
1045 1050 1055

Ser Ala Thr Asn Asp Asp Asp Val Lys Lys Ala Ala Thr Val Ala Ile
1060 1065 1070

Ala Ala Ala Tyr Asn Asn Gly Gln Glu Ile Asn Gly Phe Lys Ala Gly

1075 1080 1085

Glu Thr Ile Tyr Asp Ile Asp Glu Asp Gly Thr Ile Thr Lys Lys Asp
1090 1095 1100

Ala Thr Ala Ala Asp Val Glu Ala Asp Asp Phe Lys Gly Leu Gly Leu
1105 1110 1115 1120

Lys Lys Val Val Thr Asn Leu Thr Lys Thr Val Asn Glu Asn Lys Gln
1125 1130 1135

Asn Val Asp Ala Lys Val Lys Ala Ala Glu Ser Glu Ile Glu Lys Leu
1140 1145 1150

Thr Thr Lys Leu Ala Asp Thr Asp Ala Ala Leu Ala Asp Thr Asp Ala
1155 1160 1165

Ala Leu Asp Ala Thr Thr Asn Ala Leu Asn Lys Leu Gly Glu Asn Ile
1170 1175 1180

Thr Thr Phe Ala Glu Glu Thr Lys Thr Asn Ile Val Lys Ile Asp Glu
1185 1190 1195 1200

Lys Leu Glu Ala Val Ala Asp Thr Val Asp Lys His Ala Glu Ala Phe
1205 1210 1215

Asn Asp Ile Ala Asp Ser Leu Asp Glu Thr Asn Thr Lys Ala Asp Glu
1220 1225 1230

Ala Val Lys Thr Ala Asn Glu Ala Lys Gln Thr Ala Glu Glu Thr Lys
1235 1240 1245

Gln Asn Val Asp Ala Lys Val Lys Ala Ala Glu Thr Ala Ala Gly Lys
1250 1255 1260

Ala Glu Ala Ala Ala Gly Thr Ala Asn Thr Ala Ala Asp Lys Ala Glu
1265 1270 1275 1280

Ala Val Ala Ala Lys Val Thr Asp Ile Lys Ala Asp Ile Ala Thr Asn
1285 1290 1295

Lys Asp Asn Ile Ala Lys Lys Ala Asn Ser Ala Asp Val Tyr Thr Arg
1300 1305 1310

Glu Glu Ser Asp Ser Lys Phe Val Arg Ile Asp Gly Leu Asn Ala Thr
1315 1320 1325

Thr Glu Lys Leu Asp Thr Arg Leu Ala Ser Ala Glu Lys Ser Ile Ala
1330 1335 1340

Asp His Asp Thr Arg Leu Asn Gly Leu Asp Lys Thr Val Ser Asp Leu
1345 1350 1355 1360

Arg Lys Glu Thr Arg Gln Gly Leu Ala Glu Gln Ala Ala Leu Ser Gly
1365 1370 1375

Leu Phe Gln Pro Tyr Asn Val Gly Leu Glu His His His His His His

1380 1385 1390

<210> 108
<211> 1947
<212> DNA
<213> Artificial Sequence

<220>
<223> deltaG741-961

<400> 108

```
atggtcgccg ccgacatcgg tgcggggctt gccgatgcac taaccgcacc gctcgaccat    60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gagggtggcg gaggcactgg atccgccaca   780
aacgacgacg atgttaaaaa agctgccact gtggccattg ctgctgccta caacaatggc   840
caagaaatca acggtttcaa agctggagag accatctacg acattgatga agacggcaca   900
attaccaaaa aagacgcaac tgcagccgat gttgaagccg acgactttaa aggtctgggt   960
ctgaaaaaag tcgtgactaa cctgaccaaa accgtcaatg aaaacaaaca aaacgtcgat  1020
gccaaagtaa aagctgcaga atctgaaata gaaaagttaa caaccaagtt agcagacact  1080
gatgccgctt tagcagatac tgatgccgct ctggatgcaa ccaccaacgc cttgaataaa  1140
ttgggagaaa atataacgac atttgctgaa gagactaaga caaatatcgt aaaaattgat  1200
gaaaaattag aagccgtggc tgataccgtc gacaagcatg ccgaagcatt caacgatatc  1260
gccgattcat tggatgaaac caacactaag gcagacgaag ccgtcaaaac cgccaatgaa  1320
gccaaacaga cggccgaaga aaccaaacaa aacgtcgatg ccaaagtaaa agctgcagaa  1380
actgcagcag gcaaagccga agctgccgct ggcacagcta atactgcagc cgacaaggcc  1440
gaagctgtcg ctgcaaaagt taccgacatc aaagctgata tcgctacgaa caaagataat  1500
attgctaaaa aagcaaacag tgccgacgtg tacaccagag aagagtctga cagcaaattt  1560
gtcagaattg atggtctgaa cgctactacc gaaaaattgg acacacgctt ggcttctgct  1620
gaaaaatcca ttgccgatca cgatactcgc ctgaacggtt tggataaaac agtgtcagac  1680
ctgcgcaaag aaacccgcca aggccttgca gaacaagccg cgctctccgg tctgttccaa  1740
ccttacaacg tgggtcggtt caatgtaacg gctgcagtcg gcggctacaa atccgaatcg  1800
gcagtcgcca tcggtaccgg cttccgcttt accgaaaact ttgccgccaa agcaggcgtg  1860
gcagtcggca cttcgtccgg ttcttccgca gcctaccatg tcggcgtcaa ttacgagtgg  1920
ctcgagcacc accaccacca ccactga                                      1947
```

<210> 109
<211> 648
<212> PRT
<213> Artificial Sequence

<220>
<223> deltaG741-961

<400> 109

```
Met Val Ala Ala Asp Ile Gly Ala Gly Leu Ala Asp Ala Leu Thr Ala
1               5                   10                  15
```

```
Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60

Asp Lys Val Ser Arg Phe Asp Phe Ile Arg Gln Ile Glu Val Asp Gly
65                  70                  75                  80

Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
                165                 170                 175

His Leu Lys Ser Pro Glu Leu Asn Val Asp Leu Ala Ala Ala Asp Ile
            180                 185                 190

Lys Pro Asp Gly Lys Arg His Ala Val Ile Ser Gly Ser Val Leu Tyr
        195                 200                 205

Asn Gln Ala Glu Lys Gly Ser Tyr Ser Leu Gly Ile Phe Gly Gly Lys
    210                 215                 220

Ala Gln Glu Val Ala Gly Ser Ala Glu Val Lys Thr Val Asn Gly Ile
225                 230                 235                 240

Arg His Ile Gly Leu Ala Ala Lys Gln Leu Glu Gly Gly Gly Gly Thr
                245                 250                 255

Gly Ser Ala Thr Asn Asp Asp Asp Val Lys Lys Ala Ala Thr Val Ala
            260                 265                 270

Ile Ala Ala Ala Tyr Asn Asn Gly Gln Glu Ile Asn Gly Phe Lys Ala
        275                 280                 285

Gly Glu Thr Ile Tyr Asp Ile Asp Glu Asp Gly Thr Ile Thr Lys Lys
    290                 295                 300

Asp Ala Thr Ala Ala Asp Val Glu Ala Asp Asp Phe Lys Gly Leu Gly
305                 310                 315                 320
```

Leu Lys Lys Val Val Thr Asn Leu Thr Lys Thr Val Asn Glu Asn Lys
325 330 335

Gln Asn Val Asp Ala Lys Val Lys Ala Ala Glu Ser Glu Ile Glu Lys
340 345 350

Leu Thr Thr Lys Leu Ala Asp Thr Asp Ala Ala Leu Ala Asp Thr Asp
355 360 365

Ala Ala Leu Asp Ala Thr Thr Asn Ala Leu Asn Lys Leu Gly Glu Asn
370 375 380

Ile Thr Thr Phe Ala Glu Glu Thr Lys Thr Asn Ile Val Lys Ile Asp
385 390 395 400

Glu Lys Leu Glu Ala Val Ala Asp Thr Val Asp Lys His Ala Glu Ala
405 410 415

Phe Asn Asp Ile Ala Asp Ser Leu Asp Glu Thr Asn Thr Lys Ala Asp
420 425 430

Glu Ala Val Lys Thr Ala Asn Glu Ala Lys Gln Thr Ala Glu Glu Thr
435 440 445

Lys Gln Asn Val Asp Ala Lys Val Lys Ala Ala Glu Thr Ala Ala Gly
450 455 460

Lys Ala Glu Ala Ala Ala Gly Thr Ala Asn Thr Ala Ala Asp Lys Ala
465 470 475 480

Glu Ala Val Ala Ala Lys Val Thr Asp Ile Lys Ala Asp Ile Ala Thr
485 490 495

Asn Lys Asp Asn Ile Ala Lys Lys Ala Asn Ser Ala Asp Val Tyr Thr
500 505 510

Arg Glu Glu Ser Asp Ser Lys Phe Val Arg Ile Asp Gly Leu Asn Ala
515 520 525

Thr Thr Glu Lys Leu Asp Thr Arg Leu Ala Ser Ala Glu Lys Ser Ile
530 535 540

Ala Asp His Asp Thr Arg Leu Asn Gly Leu Asp Lys Thr Val Ser Asp
545 550 555 560

Leu Arg Lys Glu Thr Arg Gln Gly Leu Ala Glu Gln Ala Ala Leu Ser
565 570 575

Gly Leu Phe Gln Pro Tyr Asn Val Gly Arg Phe Asn Val Thr Ala Ala
580 585 590

Val Gly Gly Tyr Lys Ser Glu Ser Ala Val Ala Ile Gly Thr Gly Phe
595 600 605

Arg Phe Thr Glu Asn Phe Ala Ala Lys Ala Gly Val Ala Val Gly Thr
610 615 620

Ser Ser Gly Ser Ser Ala Ala Tyr His Val Gly Val Asn Tyr Glu Trp
625 630 635 640

Leu Glu His His His His His His
645

<210> 110
<211> 1782
<212> DNA
<213> Artificial Sequence

<220>
<223> deltaG741-961c

<400> 110

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg  120
aagctggcgg cacaaggtgc ggaaaaaact tatggaaacg gtgacagcct caatacgggc  180
aaattgaaga acgacaaggt cagccgtttc gactttatcc gccaaatcga agtggacggg  240
cagctcatta ccttggagag tggagagttc caagtataca aacaaagcca ttccgcctta  300
accgcctttc agaccgagca aatacaagat tcggagcatt ccgggaagat ggttgcgaaa  360
cgccagttca gaatcggcga catagcgggc gaacatacat cttttgacaa gcttcccgaa  420
ggcggcaggg cgacatatcg cgggacggcg ttcggttcag acgatgccgg cggaaaactg  480
acctacacca tagatttcgc cgccaagcag ggaaacggca aaatcgaaca tttgaaatcg  540
ccagaactca atgtcgacct ggccgccgcc gatatcaagc cggatggaaa acgccatgcc  600
gtcatcagcg gttccgtcct ttacaaccaa gccgagaaag gcagttactc cctcggtatc  660
tttggcggaa aagcccagga agttgccggc agcgcggaag tgaaaaccgt aaacggcata  720
cgccatatcg gccttgccgc caagcaactc gagggtggcg gaggcactgg atccgccaca  780
aacgacgacg atgttaaaaa agctgccact gtggccattg ctgctgccta caacaatggc  840
caagaaatca acggtttcaa agctggagag accatctacg acattgatga agacggcaca  900
attaccaaaa aagacgcaac tgcagccgat gttgaagccg acgactttaa aggtctgggt  960
ctgaaaaaag tcgtgactaa cctgaccaaa accgtcaatg aaaacaaaca aaacgtcgat 1020
gccaaagtaa aagctgcaga atctgaaata gaaaagttaa caaccaagtt agcagacact 1080
gatgccgctt tagcagatac tgatgccgct ctggatgcaa ccaccaacgc cttgaataaa 1140
ttgggagaaa atataacgac atttgctgaa gagactaaga caaatatcgt aaaaattgat 1200
gaaaaattag aagccgtggc tgataccgtc gacaagcatg ccgaagcatt caacgatatc 1260
gccgattcat tggatgaaac caacactaag gcagacgaag ccgtcaaaac cgccaatgaa 1320
gccaaacaga cggccgaaga aaccaaacaa aacgtcgatg ccaaagtaaa agctgcagaa 1380
actgcagcag gcaaagccga agctgccgct ggcacagcta atactgcagc cgacaaggcc 1440
gaagctgtcg ctgcaaaagt taccgacatc aaagctgata tcgctacgaa caaagataat 1500
attgctaaaa aagcaaacag tgccgacgtg tacaccagag aagagtctga cagcaaattt 1560
gtcagaattg atggtctgaa cgctactacc gaaaaattgg acacacgctt ggcttctgct 1620
gaaaaatcca ttgccgatca cgatactcgc ctgaacggtt tggataaaac agtgtcagac 1680
ctgcgcaaag aaacccgcca aggccttgca gaacaagccg cgctctccgg tctgttccaa 1740
ccttacaacg tgggtctcga gcaccaccac caccaccact ga                     1782
```

<210> 111
<211> 593
<212> PRT
<213> Artificial Sequence

<220>
<223> deltaG741-961c

<400> 111

Met Val Ala Ala Asp Ile Gly Ala Gly Leu Ala Asp Ala Leu Thr Ala

```
1               5                   10                  15
Pro Leu Asp His Lys Asp Lys Gly Leu Gln Ser Leu Thr Leu Asp Gln
            20                  25                  30
Ser Val Arg Lys Asn Glu Lys Leu Lys Leu Ala Ala Gln Gly Ala Glu
        35                  40                  45
Lys Thr Tyr Gly Asn Gly Asp Ser Leu Asn Thr Gly Lys Leu Lys Asn
    50                  55                  60
Asp Lys Val Ser Arg Phe Asp Phe Ile Arg Gln Ile Glu Val Asp Gly
65                  70                  75                  80
Gln Leu Ile Thr Leu Glu Ser Gly Glu Phe Gln Val Tyr Lys Gln Ser
                85                  90                  95
His Ser Ala Leu Thr Ala Phe Gln Thr Glu Gln Ile Gln Asp Ser Glu
            100                 105                 110
His Ser Gly Lys Met Val Ala Lys Arg Gln Phe Arg Ile Gly Asp Ile
        115                 120                 125
Ala Gly Glu His Thr Ser Phe Asp Lys Leu Pro Glu Gly Gly Arg Ala
    130                 135                 140
Thr Tyr Arg Gly Thr Ala Phe Gly Ser Asp Asp Ala Gly Gly Lys Leu
145                 150                 155                 160
Thr Tyr Thr Ile Asp Phe Ala Ala Lys Gln Gly Asn Gly Lys Ile Glu
                165                 170                 175
His Leu Lys Ser Pro Glu Leu Asn Val Asp Leu Ala Ala Ala Asp Ile
            180                 185                 190
Lys Pro Asp Gly Lys Arg His Ala Val Ile Ser Gly Ser Val Leu Tyr
        195                 200                 205
Asn Gln Ala Glu Lys Gly Ser Tyr Ser Leu Gly Ile Phe Gly Gly Lys
    210                 215                 220
Ala Gln Glu Val Ala Gly Ser Ala Glu Val Lys Thr Val Asn Gly Ile
225                 230                 235                 240
Arg His Ile Gly Leu Ala Ala Lys Gln Leu Glu Gly Gly Gly Gly Thr
                245                 250                 255
Gly Ser Ala Thr Asn Asp Asp Asp Val Lys Lys Ala Ala Thr Val Ala
            260                 265                 270
Ile Ala Ala Ala Tyr Asn Asn Gly Gln Glu Ile Asn Gly Phe Lys Ala
        275                 280                 285
Gly Glu Thr Ile Tyr Asp Ile Asp Glu Asp Gly Thr Ile Thr Lys Lys
    290                 295                 300
Asp Ala Thr Ala Ala Asp Val Glu Ala Asp Asp Phe Lys Gly Leu Gly
```

305 310 315 320

Leu Lys Lys Val Val Thr Asn Leu Thr Lys Thr Val Asn Glu Asn Lys
325 330 335

Gln Asn Val Asp Ala Lys Val Lys Ala Ala Glu Ser Glu Ile Glu Lys
340 345 350

Leu Thr Thr Lys Leu Ala Asp Thr Asp Ala Ala Leu Ala Asp Thr Asp
355 360 365

Ala Ala Leu Asp Ala Thr Thr Asn Ala Leu Asn Lys Leu Gly Glu Asn
370 375 380

Ile Thr Thr Phe Ala Glu Glu Thr Lys Thr Asn Ile Val Lys Ile Asp
385 390 395 400

Glu Lys Leu Glu Ala Val Ala Asp Thr Val Asp Lys His Ala Glu Ala
405 410 415

Phe Asn Asp Ile Ala Asp Ser Leu Asp Glu Thr Asn Thr Lys Ala Asp
420 425 430

Glu Ala Val Lys Thr Ala Asn Glu Ala Lys Gln Thr Ala Glu Glu Thr
435 440 445

Lys Gln Asn Val Asp Ala Lys Val Lys Ala Ala Glu Thr Ala Ala Gly
450 455 460

Lys Ala Glu Ala Ala Ala Gly Thr Ala Asn Thr Ala Ala Asp Lys Ala
465 470 475 480

Glu Ala Val Ala Ala Lys Val Thr Asp Ile Lys Ala Asp Ile Ala Thr
485 490 495

Asn Lys Asp Asn Ile Ala Lys Lys Ala Asn Ser Ala Asp Val Tyr Thr
500 505 510

Arg Glu Glu Ser Asp Ser Lys Phe Val Arg Ile Asp Gly Leu Asn Ala
515 520 525

Thr Thr Glu Lys Leu Asp Thr Arg Leu Ala Ser Ala Glu Lys Ser Ile
530 535 540

Ala Asp His Asp Thr Arg Leu Asn Gly Leu Asp Lys Thr Val Ser Asp
545 550 555 560

Leu Arg Lys Glu Thr Arg Gln Gly Leu Ala Glu Gln Ala Ala Leu Ser
565 570 575

Gly Leu Phe Gln Pro Tyr Asn Val Gly Leu Glu His His His His His
580 585 590

His

<210> 112
<211> 3939

<212> DNA
<213> Artificial Sequence

<220>
<223> deltaG741-983

<400> 112

```
atggtcgccg ccgacatcgg tgcggggctt gccgatgcac taaccgcacc gctcgaccat 60
aaagacaaag gtttgcagtc tttgacgctg gatcagtccg tcaggaaaaa cgagaaactg 120
aagctggcgg cacaaggtgc ggaaaaaact tatggaaacg gtgacagcct caatacgggc 180
aaattgaaga acgacaaggt cagccgtttc gactttatcc gccaaatcga agtggacggg 240
cagctcatta ccttggagag tggagagttc caagtataca aacaaagcca ttccgcctta 300
accgcctttc agaccgagca aatacaagat tcggagcatt ccgggaagat ggttgcgaaa 360
cgccagttca gaatcggcga catagcgggc gaacatacat cttttgacaa gcttcccgaa 420
ggcggcaggg cgacatatcg cgggacggcg ttcggttcag acgatgccgg cggaaaactg 480
acctacacca tagatttcgc cgccaagcag ggaaacggca aaatcgaaca tttgaaatcg 540
ccagaactca atgtcgacct ggccgccgcc gatatcaagc cggatggaaa acgccatgcc 600
gtcatcagcg gttccgtcct ttacaaccaa gccgagaaag gcagttactc cctcggtatc 660
tttggcggaa aagcccagga agttgccggc agcgcggaag tgaaaaccgt aaacggcata 720
cgccatatcg gccttgccgc caagcaactc gagggatccg gcggaggcgg cacttctgcg 780
cccgacttca atgcaggcgg taccggtatc ggcagcaaca gcagagcaac aacagcgaaa 840
tcagcagcag tatcttacgc cggtatcaag aacgaaatgt gcaaagacag aagcatgctc 900
tgtgccggtc gggatgacgt tgcggttaca gacagggatg ccaaaatcaa tgcccccccc 960
ccgaatctgc ataccggaga ctttccaaac ccaaatgacg catacaagaa tttgatcaac 1020
ctcaaacctg caattgaagc aggctataca ggacgcgggg tagaggtagg tatcgtcgac 1080
acaggcgaat ccgtcggcag catatccttt cccgaactgt atggcagaaa agaacacggc 1140
tataacgaaa attacaaaaa ctatacggcg tatatgcgga aggaagcgcc tgaagacgga 1200
ggcggtaaag acattgaagc ttctttcgac gatgaggccg ttatagagac tgaagcaaag 1260
ccgacggata tccgccacgt aaaagaaatc ggacacatcg atttggtctc ccatattatt 1320
ggcgggcgtt ccgtggacgg cagacctgca ggcggtattg cgcccgatgc gacgctacac 1380
ataatgaata cgaatgatga aaccaagaac gaaatgatgg ttgcagccat ccgcaatgca 1440
tgggtcaagc tgggcgaacg tggcgtgcgc atcgtcaata acagttttgg aacaacatcg 1500
agggcaggca ctgccgacct tttccaaata gccaattcgg aggagcagta ccgccaagcg 1560
ttgctcgact attccggcgg tgataaaaca gacgagggta tccgcctgat gcaacagagc 1620
gattacggca acctgtccta ccacatccgt aataaaaaca tgcttttcat cttttcgaca 1680
ggcaatgacg cacaagctca gcccaacaca tatgccctat tgccatttta tgaaaaagac 1740
gctcaaaaag gcattatcac agtcgcaggc gtagaccgca gtggagaaaa gttcaaacgg 1800
gaaatgtatg gagaaccggg tacagaaccg cttgagtatg gctccaacca ttgcggaatt 1860
actgccatgt ggtgcctgtc ggcaccctat gaagcaagcg tccgtttcac ccgtacaaac 1920
ccgattcaaa ttgccggaac atccttttcc gcacccatcg taaccggcac ggcggctctg 1980
ctgctgcaga aatacccgtg gatgagcaac gacaacctgc gtaccacgtt gctgacgacg 2040
gctcaggaca tcggtgcagt cggcgtggac agcaagttcg gctggggact gctggatgcg 2100
ggtaaggcca tgaacggacc cgcgtccttt ccgttcggcg actttaccgc cgatacgaaa 2160
ggtacatccg atattgccta ctccttccgt aacgacattt caggcacggg cggcctgatc 2220
aaaaaaggcg gcagccaact gcaactgcac ggcaacaaca cctatacggg caaaaccatt 2280
atcgaaggcg gttcgctggt gttgtacggc aacaacaaat cggatatgcg cgtcgaaacc 2340
aaaggtgcgc tgatttataa cggggcggca tccggcggca gcctgaacag cgacggcatt 2400
gtctatctgg cagataccga ccaatccggc gcaaacgaaa ccgtacacat caaaggcagt 2460
ctgcagctgg acggcaaagg tacgctgtac acacgtttgg gcaaactgct gaaagtggac 2520
ggtacggcga ttatcggcgg caagctgtac atgtcggcac gcggcaaggg ggcaggctat 2580
ctcaacagta ccggacgacg tgttcccttc ctgagtgccg ccaaaatcgg gcaggattat 2640
tctttcttca caaacatcga aaccgacggc ggcctgctgg cttccctcga cagcgtcgaa 2700
aaaacagcgg gcagtgaagg cgacacgctg tcctattatg tccgtcgcgg caatgcggca 2760
cggactgctt cggcagcggc acattccgcg cccgccggtc tgaaacacgc cgtagaacag 2820
ggcggcagca atctggaaaa cctgatggtc gaactggatg cctccgaatc atccgcaaca 2880
cccgagacgg ttgaaactgc ggcagccgac cgcacagata tgccgggcat ccgcccctac 2940
```

```
ggcgcaactt tccgcgcagc ggcagccgta cagcatgcga atgccgccga cggtgtacgc     3000
atcttcaaca gtctcgccgc taccgtctat gccgacagta ccgccgccca tgccgatatg     3060
cagggacgcc gcctgaaagc cgtatcggac gggttggacc acaacggcac gggtctgcgc     3120
gtcatcgcgc aaacccaaca ggacggtgga acgtgggaac agggcggtgt tgaaggcaaa     3180
atgcgcggca gtacccaaac cgtcggcatt gccgcgaaaa ccggcgaaaa tacgacagca     3240
gccgccacac tgggcatggg acgcagcaca tggagcgaaa acagtgcaaa tgcaaaaacc     3300
gacagcatta gtctgtttgc aggcatacgg cacgatgcgg gcgatatcgg ctatctcaaa     3360
ggcctgttct cctacggacg ctacaaaaac agcatcagcc gcagcaccgg tgcggacgaa     3420
catgcggaag gcagcgtcaa cggcacgctg atgcagctgg gcgcactggg cggtgtcaac     3480
gttccgtttg ccgcaacggg agatttgacg gtcgaaggcg gtctgcgcta cgacctgctc     3540
aaacaggatg cattcgccga aaaaggcagt gctttgggct ggagcggcaa cagcctcact     3600
gaaggcacgc tggtcggact cgcgggtctg aagctgtcgc aacccttgag cgataaagcc     3660
gtcctgtttg caacggcggg cgtggaacgc gacctgaacg gacgcgacta cacggtaacg     3720
ggcggcttta ccggcgcgac tgcagcaacc ggcaagacgg gggcacgcaa tatgccgcac     3780
acccgtctgg ttgccggcct gggcgcggat gtcgaattcg gcaacggctg gaacggcttg     3840
gcacgttaca gctacgccgg ttccaaacag tacggcaacc acagcggacg agtcggcgta     3900
ggctaccggt tcctcgagca ccaccaccac caccactga                            3939
```

<210> 113
<211> 1312
<212> PRT
<213> Artificial Sequence

<220>
<223> deltaG741-983

<400> 113

Met Val Ala Ala Asp Ile Gly Ala Gly Leu Ala Asp Ala Leu Thr Ala
1               5                   10                  15

Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60

Asp Lys Val Ser Arg Phe Asp Phe Ile Arg Gln Ile Glu Val Asp Gly
65                  70                  75                  80

Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
165 170 175

His Leu Lys Ser Pro Glu Leu Asn Val Asp Leu Ala Ala Ala Asp Ile
180 185 190

Lys Pro Asp Gly Lys Arg His Ala Val Ile Ser Gly Ser Val Leu Tyr
195 200 205

Asn Gln Ala Glu Lys Gly Ser Tyr Ser Leu Gly Ile Phe Gly Gly Lys
210 215 220

Ala Gln Glu Val Ala Gly Ser Ala Glu Val Lys Thr Val Asn Gly Ile
225 230 235 240

Arg His Ile Gly Leu Ala Ala Lys Gln Leu Glu Gly Ser Gly Gly Gly
245 250 255

Gly Thr Ser Ala Pro Asp Phe Asn Ala Gly Gly Thr Gly Ile Gly Ser
260 265 270

Asn Ser Arg Ala Thr Thr Ala Lys Ser Ala Ala Val Ser Tyr Ala Gly
275 280 285

Ile Lys Asn Glu Met Cys Lys Asp Arg Ser Met Leu Cys Ala Gly Arg
290 295 300

Asp Asp Val Ala Val Thr Asp Arg Asp Ala Lys Ile Asn Ala Pro Pro
305 310 315 320

Pro Asn Leu His Thr Gly Asp Phe Pro Asn Pro Asn Asp Ala Tyr Lys
325 330 335

Asn Leu Ile Asn Leu Lys Pro Ala Ile Glu Ala Gly Tyr Thr Gly Arg
340 345 350

Gly Val Glu Val Gly Ile Val Asp Thr Gly Glu Ser Val Gly Ser Ile
355 360 365

Ser Phe Pro Glu Leu Tyr Gly Arg Lys Glu His Gly Tyr Asn Glu Asn
370 375 380

Tyr Lys Asn Tyr Thr Ala Tyr Met Arg Lys Glu Ala Pro Glu Asp Gly
385 390 395 400

Gly Gly Lys Asp Ile Glu Ala Ser Phe Asp Asp Glu Ala Val Ile Glu
405 410 415

Thr Glu Ala Lys Pro Thr Asp Ile Arg His Val Lys Glu Ile Gly His
420 425 430

Ile Asp Leu Val Ser His Ile Ile Gly Gly Arg Ser Val Asp Gly Arg
435 440 445

Pro Ala Gly Gly Ile Ala Pro Asp Ala Thr Leu His Ile Met Asn Thr
450 455 460

Asn Asp Glu Thr Lys Asn Glu Met Met Val Ala Ala Ile Arg Asn Ala
465 470 475 480

Trp Val Lys Leu Gly Glu Arg Gly Val Arg Ile Val Asn Asn Ser Phe
485 490 495

Gly Thr Thr Ser Arg Ala Gly Thr Ala Asp Leu Phe Gln Ile Ala Asn
500 505 510

Ser Glu Glu Gln Tyr Arg Gln Ala Leu Leu Asp Tyr Ser Gly Gly Asp
515 520 525

Lys Thr Asp Glu Gly Ile Arg Leu Met Gln Gln Ser Asp Tyr Gly Asn
530 535 540

Leu Ser Tyr His Ile Arg Asn Lys Asn Met Leu Phe Ile Phe Ser Thr
545 550 555 560

Gly Asn Asp Ala Gln Ala Gln Pro Asn Thr Tyr Ala Leu Leu Pro Phe
565 570 575

Tyr Glu Lys Asp Ala Gln Lys Gly Ile Ile Thr Val Ala Gly Val Asp
580 585 590

Arg Ser Gly Glu Lys Phe Lys Arg Glu Met Tyr Gly Glu Pro Gly Thr
595 600 605

Glu Pro Leu Glu Tyr Gly Ser Asn His Cys Gly Ile Thr Ala Met Trp
610 615 620

Cys Leu Ser Ala Pro Tyr Glu Ala Ser Val Arg Phe Thr Arg Thr Asn
625 630 635 640

Pro Ile Gln Ile Ala Gly Thr Ser Phe Ser Ala Pro Ile Val Thr Gly
645 650 655

Thr Ala Ala Leu Leu Leu Gln Lys Tyr Pro Trp Met Ser Asn Asp Asn
660 665 670

Leu Arg Thr Thr Leu Leu Thr Thr Ala Gln Asp Ile Gly Ala Val Gly
675 680 685

Val Asp Ser Lys Phe Gly Trp Gly Leu Leu Asp Ala Gly Lys Ala Met
690 695 700

Asn Gly Pro Ala Ser Phe Pro Phe Gly Asp Phe Thr Ala Asp Thr Lys
705 710 715 720

Gly Thr Ser Asp Ile Ala Tyr Ser Phe Arg Asn Asp Ile Ser Gly Thr
725 730 735

Gly Gly Leu Ile Lys Lys Gly Gly Ser Gln Leu Gln Leu His Gly Asn
740 745 750

Asn Thr Tyr Thr Gly Lys Thr Ile Ile Glu Gly Gly Ser Leu Val Leu
755 760 765

Tyr Gly Asn Asn Lys Ser Asp Met Arg Val Glu Thr Lys Gly Ala Leu
770 775 780

Ile Tyr Asn Gly Ala Ala Ser Gly Gly Ser Leu Asn Ser Asp Gly Ile
785 790 795 800

Val Tyr Leu Ala Asp Thr Asp Gln Ser Gly Ala Asn Glu Thr Val His
805 810 815

Ile Lys Gly Ser Leu Gln Leu Asp Gly Lys Gly Thr Leu Tyr Thr Arg
820 825 830

Leu Gly Lys Leu Leu Lys Val Asp Gly Thr Ala Ile Ile Gly Gly Lys
835 840 845

Leu Tyr Met Ser Ala Arg Gly Lys Gly Ala Gly Tyr Leu Asn Ser Thr
850 855 860

Gly Arg Arg Val Pro Phe Leu Ser Ala Ala Lys Ile Gly Gln Asp Tyr
865 870 875 880

Ser Phe Phe Thr Asn Ile Glu Thr Asp Gly Gly Leu Leu Ala Ser Leu
885 890 895

Asp Ser Val Glu Lys Thr Ala Gly Ser Glu Gly Asp Thr Leu Ser Tyr
900 905 910

Tyr Val Arg Arg Gly Asn Ala Ala Arg Thr Ala Ser Ala Ala Ala His
915 920 925

Ser Ala Pro Ala Gly Leu Lys His Ala Val Glu Gln Gly Gly Ser Asn
930 935 940

Leu Glu Asn Leu Met Val Glu Leu Asp Ala Ser Glu Ser Ser Ala Thr
945 950 955 960

Pro Glu Thr Val Glu Thr Ala Ala Ala Asp Arg Thr Asp Met Pro Gly
965 970 975

Ile Arg Pro Tyr Gly Ala Thr Phe Arg Ala Ala Ala Ala Val Gln His
980 985 990

Ala Asn Ala Ala Asp Gly Val Arg Ile Phe Asn Ser Leu Ala Ala Thr
995 1000 1005

Val Tyr Ala Asp Ser Thr Ala Ala His Ala Asp Met Gln Gly Arg Arg
1010 1015 1020

Leu Lys Ala Val Ser Asp Gly Leu Asp His Asn Gly Thr Gly Leu Arg
1025 1030 1035 1040

Val Ile Ala Gln Thr Gln Gln Asp Gly Gly Thr Trp Glu Gln Gly Gly
1045 1050 1055

Val Glu Gly Lys Met Arg Gly Ser Thr Gln Thr Val Gly Ile Ala Ala
1060 1065 1070

Lys Thr Gly Glu Asn Thr Thr Ala Ala Ala Thr Leu Gly Met Gly Arg
1075 1080 1085

Ser Thr Trp Ser Glu Asn Ser Ala Asn Ala Lys Thr Asp Ser Ile Ser
1090 1095 1100

Leu Phe Ala Gly Ile Arg His Asp Ala Gly Asp Ile Gly Tyr Leu Lys
1105 1110 1115 1120

Gly Leu Phe Ser Tyr Gly Arg Tyr Lys Asn Ser Ile Ser Arg Ser Thr
1125 1130 1135

Gly Ala Asp Glu His Ala Glu Gly Ser Val Asn Gly Thr Leu Met Gln
1140 1145 1150

Leu Gly Ala Leu Gly Gly Val Asn Val Pro Phe Ala Ala Thr Gly Asp
1155 1160 1165

Leu Thr Val Glu Gly Gly Leu Arg Tyr Asp Leu Leu Lys Gln Asp Ala
1170 1175 1180

Phe Ala Glu Lys Gly Ser Ala Leu Gly Trp Ser Gly Asn Ser Leu Thr
1185 1190 1195 1200

Glu Gly Thr Leu Val Gly Leu Ala Gly Leu Lys Leu Ser Gln Pro Leu
1205 1210 1215

Ser Asp Lys Ala Val Leu Phe Ala Thr Ala Gly Val Glu Arg Asp Leu
1220 1225 1230

Asn Gly Arg Asp Tyr Thr Val Thr Gly Gly Phe Thr Gly Ala Thr Ala
1235 1240 1245

Ala Thr Gly Lys Thr Gly Ala Arg Asn Met Pro His Thr Arg Leu Val
1250 1255 1260

Ala Gly Leu Gly Ala Asp Val Glu Phe Gly Asn Gly Trp Asn Gly Leu
1265 1270 1275 1280

Ala Arg Tyr Ser Tyr Ala Gly Ser Lys Gln Tyr Gly Asn His Ser Gly
1285 1290 1295

Arg Val Gly Val Gly Tyr Arg Phe Leu Glu His His His His His His
1300 1305 1310

<210> 114
<211> 2028
<212> DNA
<213> Artificial Sequence

<220>
<223> deltaG741-ORF46.1

<400> 114

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg  120
aagctggcgg cacaaggtgc ggaaaaaact tatggaaacg gtgacagcct caatacgggc  180
aaattgaaga acgacaaggt cagccgtttc gactttatcc gccaaatcga agtggacggg  240
cagctcatta ccttggagag tggagagttc caagtataca aacaaagcca ttccgcctta  300
accgcctttc agaccgagca aatacaagat tcggagcatt ccgggaagat ggttgcgaaa  360
cgccagttca gaatcggcga catagcgggc gaacatacat cttttgacaa gcttcccgaa  420
ggcggcaggg cgacatatcg cgggacggcg ttcggttcag acgatgccgg cggaaaactg  480
acctacacca tagatttcgc cgccaagcag ggaaacggca aaatcgaaca tttgaaatcg  540
ccagaactca atgtcgacct ggccgccgcc gatatcaagc cggatggaaa acgccatgcc  600
gtcatcagcg gttccgtcct ttacaaccaa gccgagaaag gcagttactc cctcggtatc  660
tttggcggaa aagcccagga agttgccggc agcgcggaag tgaaaaccgt aaacggcata  720
cgccatatcg gccttgccgc caagcaactc gacggtggcg gaggcactgg atcctcagat  780
ttggcaaacg attcttttat ccggcaggtt ctcgaccgtc agcatttcga acccgacggg  840
aaataccacc tattcggcag caggggggaa cttgccgagc gcagcggcca tatcggattg  900
ggaaaaatac aaagccatca gttgggcaac ctgatgattc aacaggcggc cattaaagga  960
aatatcggct acattgtccg cttttccgat cacgggcacg aagtccattc cccttcgac  1020
aaccatgcct cacattccga ttctgatgaa gccggtagtc ccgttgacgg atttagcctt  1080
taccgcatcc attgggacgg atacgaacac catcccgccg acggctatga cgggccacag  1140
ggcggcggct atcccgctcc caaaggcgcg agggatatat acagctacga cataaaaggc  1200
gttgcccaaa atatccgcct caacctgacc gacaaccgca gcaccggaca acggcttgcc  1260
gaccgtttcc acaatgccgg tagtatgctg acgcaaggag taggcgacgg attcaaacgc  1320
gccacccgat acagccccga gctggacaga tcgggcaatg ccgccgaagc cttcaacggc  1380
actgcagata tcgttaaaaa catcatcggc gcggcaggag aaattgtcgg cgcaggcgat  1440
gccgtgcagg gcataagcga aggctcaaac attgctgtca tgcacggctt gggtctgctt  1500
tccaccgaaa acaagatggc gcgcatcaac gatttggcag atatggcgca actcaaagac  1560
tatgccgcag cagccatccg cgattgggca gtccaaaacc ccaatgccgc acaaggcata  1620
gaagccgtca gcaatatctt tatggcagcc atccccatca aagggattgg agctgttcgg  1680
ggaaaatacg gcttgggcgg catcacggca catcctatca agcggtcgca gatggcgcg  1740
atcgcattgc cgaaagggaa atccgccgtc agcgacaatt ttgccgatgc ggcatacgcc  1800
aaatacccgt ccccttacca ttcccgaaat atccgttcaa acttggagca gcgttacggc  1860
aaagaaaaca tcacctcctc aaccgtgccg ccgtcaaacg gcaaaaatgt caaactggca  1920
gaccaacgcc acccgaagac aggcgtaccg tttgacggta aagggtttcc gaattttgag  1980
aagcacgtga aatatgatac gctcgagcac caccaccacc accactga               2028
```

<210> 115
<211> 675
<212> PRT
<213> Artificial Sequence

<220>
<223> deltaG741-ORF46.1

<400> 115

Met Val Ala Ala Asp Ile Gly Ala Gly Leu Ala Asp Ala Leu Thr Ala
1 5 10 15

Pro Leu Asp His Lys Asp Lys Gly Leu Gln Ser Leu Thr Leu Asp Gln
20 25 30

Ser Val Arg Lys Asn Glu Lys Leu Lys Leu Ala Ala Gln Gly Ala Glu
35 40 45

Lys Thr Tyr Gly Asn Gly Asp Ser Leu Asn Thr Gly Lys Leu Lys Asn
50 55 60

Asp Lys Val Ser Arg Phe Asp Phe Ile Arg Gln Ile Glu Val Asp Gly
65 70 75 80

```
Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
                165                 170                 175

His Leu Lys Ser Pro Glu Leu Asn Val Asp Leu Ala Ala Ala Asp Ile
            180                 185                 190

Lys Pro Asp Gly Lys Arg His Ala Val Ile Ser Gly Ser Val Leu Tyr
        195                 200                 205

Asn Gln Ala Glu Lys Gly Ser Tyr Ser Leu Gly Ile Phe Gly Gly Lys
    210                 215                 220

Ala Gln Glu Val Ala Gly Ser Ala Glu Val Lys Thr Val Asn Gly Ile
225                 230                 235                 240

Arg His Ile Gly Leu Ala Ala Lys Gln Leu Asp Gly Gly Gly Gly Thr
                245                 250                 255

Gly Ser Ser Asp Leu Ala Asn Asp Ser Phe Ile Arg Gln Val Leu Asp
            260                 265                 270

Arg Gln His Phe Glu Pro Asp Gly Lys Tyr His Leu Phe Gly Ser Arg
        275                 280                 285

Gly Glu Leu Ala Glu Arg Ser Gly His Ile Gly Leu Gly Lys Ile Gln
    290                 295                 300

Ser His Gln Leu Gly Asn Leu Met Ile Gln Gln Ala Ala Ile Lys Gly
305                 310                 315                 320

Asn Ile Gly Tyr Ile Val Arg Phe Ser Asp His Gly His Glu Val His
                325                 330                 335

Ser Pro Phe Asp Asn His Ala Ser His Ser Asp Ser Asp Glu Ala Gly
            340                 345                 350

Ser Pro Val Asp Gly Phe Ser Leu Tyr Arg Ile His Trp Asp Gly Tyr
        355                 360                 365

Glu His His Pro Ala Asp Gly Tyr Asp Gly Pro Gln Gly Gly Gly Tyr
    370                 375                 380
```

```
Pro Ala Pro Lys Gly Ala Arg Asp Ile Tyr Ser Tyr Asp Ile Lys Gly
385                 390                 395                 400

Val Ala Gln Asn Ile Arg Leu Asn Leu Thr Asp Asn Arg Ser Thr Gly
                405                 410                 415

Gln Arg Leu Ala Asp Arg Phe His Asn Ala Gly Ser Met Leu Thr Gln
            420                 425                 430

Gly Val Gly Asp Gly Phe Lys Arg Ala Thr Arg Tyr Ser Pro Glu Leu
        435                 440                 445

Asp Arg Ser Gly Asn Ala Ala Glu Ala Phe Asn Gly Thr Ala Asp Ile
    450                 455                 460

Val Lys Asn Ile Ile Gly Ala Ala Gly Glu Ile Val Gly Ala Gly Asp
465                 470                 475                 480

Ala Val Gln Gly Ile Ser Glu Gly Ser Asn Ile Ala Val Met His Gly
                485                 490                 495

Leu Gly Leu Leu Ser Thr Glu Asn Lys Met Ala Arg Ile Asn Asp Leu
            500                 505                 510

Ala Asp Met Ala Gln Leu Lys Asp Tyr Ala Ala Ala Ala Ile Arg Asp
        515                 520                 525

Trp Ala Val Gln Asn Pro Asn Ala Ala Gln Gly Ile Glu Ala Val Ser
    530                 535                 540

Asn Ile Phe Met Ala Ala Ile Pro Ile Lys Gly Ile Gly Ala Val Arg
545                 550                 555                 560

Gly Lys Tyr Gly Leu Gly Gly Ile Thr Ala His Pro Ile Lys Arg Ser
                565                 570                 575

Gln Met Gly Ala Ile Ala Leu Pro Lys Gly Lys Ser Ala Val Ser Asp
            580                 585                 590

Asn Phe Ala Asp Ala Ala Tyr Ala Lys Tyr Pro Ser Pro Tyr His Ser
        595                 600                 605

Arg Asn Ile Arg Ser Asn Leu Glu Gln Arg Tyr Gly Lys Glu Asn Ile
    610                 615                 620

Thr Ser Ser Thr Val Pro Pro Ser Asn Gly Lys Asn Val Lys Leu Ala
625                 630                 635                 640

Asp Gln Arg His Pro Lys Thr Gly Val Pro Phe Asp Gly Lys Gly Phe
                645                 650                 655

Pro Asn Phe Glu Lys His Val Lys Tyr Asp Thr Leu Glu His His His
            660                 665                 670

His His His
        675
```

<210> 116
<211> 249
<212> PRT
<213> Artificial Sequence

<220>
<223> Novel protein

<400> 116

```
Met Lys Lys Tyr Leu Phe Arg Ala Ala Leu Tyr Gly Ile Ala Ala Ala
1               5                   10                  15

Ile Leu Ala Ala Ala Ile Pro Ala Gly Asn Asp Ala Thr Thr Lys Pro
            20                  25                  30

Asp Leu Tyr Tyr Leu Lys Asn Glu Gln Ala Ile Asp Ser Leu Lys Leu
        35                  40                  45

Leu Pro Pro Pro Pro Glu Val Gly Ser Ile Gln Phe Leu Asn Asp Gln
    50                  55                  60

Ala Met Tyr Glu Lys Gly Arg Met Leu Arg Asn Thr Glu Arg Gly Lys
65                  70                  75                  80

Gln Ala Gln Ala Asp Ala Asp Leu Ala Ala Gly Gly Val Ala Thr Ala
                85                  90                  95

Phe Ser Gly Ala Phe Gly Tyr Pro Ile Thr Glu Lys Asp Ser Pro Glu
            100                 105                 110

Leu Tyr Lys Leu Leu Thr Asn Met Ile Glu Asp Ala Gly Asp Leu Ala
        115                 120                 125

Thr Arg Ser Ala Lys Glu His Tyr Met Arg Ile Arg Pro Phe Ala Phe
    130                 135                 140

Tyr Gly Thr Glu Thr Cys Asn Thr Lys Asp Gln Lys Lys Leu Ser Thr
145                 150                 155                 160

Asn Gly Ser Tyr Pro Ser Gly His Thr Ser Ile Gly Trp Ala Thr Ala
                165                 170                 175

Leu Val Leu Ala Glu Val Asn Pro Ala Asn Gln Asp Ala Ile Leu Glu
            180                 185                 190

Arg Gly Tyr Gln Leu Gly Gln Ser Arg Val Ile Cys Gly Tyr His Trp
        195                 200                 205

Gln Ser Asp Val Asp Ala Ala Arg Ile Val Gly Ser Ala Ala Val Ala
    210                 215                 220

Thr Leu His Ser Asp Pro Ala Phe Gln Ala Gln Leu Ala Lys Ala Lys
225                 230                 235                 240

Gln Glu Phe Ala Gln Lys Ser Gln Lys
                245
```

<210> 117
<211> 66
<212> DNA
<213> Artificial Sequence

<220>
<223> L1 linker

<220>
<221> N
<222> 13
<223> A, T/U, G or C

<400> 117

tatgaartay ytnttymgcg ccgccctgta cggcatcgcc gccgccatcc tcgccgccgc 60
gatccc 66

<210> 118
<211> 69
<212> DNA
<213> Artificial Sequence

<220>
<223> S1 linker

<220>
<221> N
<222> 25, 28
<223> A, T/U, G or C

<400> 118

tatgaaaaaa tacctattcc grgcngcnyt rtayggsatc gccgccgcca tcctcgccgc 60
cgcgatccc 69

<210> 119
<211> 27
<212> DNA
<213> Artificial Sequence

<220>
<223> 9Ll-a

<400> 119
atgaagaagt accttttcag cgccgcc 27

<210> 120
<211> 27
<212> DNA
<213> Artificial Sequence

<220>
<223> 9L1-e

<400> 120

atgaaaaaat actttttccg cgccgcc 27

<210> 121
<211> 27
<212> DNA
<213> Artificial Sequence

<220>
<223> 9Ll-d

<400> 121
atgaaaaaat actttttccg cgccgcc 27

<210> 122
<211> 60
<212> DNA
<213> Artificial Sequence

<220>
<223> 9L1-f

<400> 122
atgaaaaaat atctctttag cgccgccctg tacggcatcg ccgccgccat cctcgccgcc 60

<210> 123
<211> 60
<212> DNA
<213> Artificial Sequence

<220>
<223> 919sp

<400> 123
atgaaaaaat acctattccg cgccgccctg tacggcatcg ccgccgccat cctcgccgcc 60

<210> 124
<211> 9
<212> PRT
<213> Artificial Sequence

<220>
<223> 9Lla

<400> 124

Met Lys Lys Tyr Leu Phe Ser Ala Ala
1               5

<210> 125
<211> 9
<212> PRT
<213> Artificial Sequence

<220>
<223> 9L1e

<400> 125

Met Lys Lys Tyr Phe Phe Arg Ala Ala
1 5

<210> 126
<211> 9
<212> PRT
<213> Artificial Sequence

<220>
<223> 9Lld

<400> 126

Met Lys Lys Tyr Phe Phe Arg Ala Ala
1 5

<210> 127
<211> 20
<212> PRT
<213> Artificial Sequence

<220>
<223> 9L1f

<400> 127

Met Lys Lys Tyr Leu Phe Ser Ala Ala Leu Tyr Gly Ile Ala Ala Ala
1 5 10 15

Ile Leu Ala Ala
20

<210> 128
<211> 20
<212> PRT
<213> Artificial Sequence

<220>
<223> 9Llsp

<400> 128

Met Lys Lys Tyr Leu Phe Arg Ala Ala Leu Tyr Gly Ile Ala Ala Ala
1 5 10 15

Ile Leu Ala Ala
20

<210> 129
<211> 42
<212> DNA
<213> Artificial Sequence

<220>

<223> 9S1-e

<400> 129
atgaaaaaat acctattcat cgccgccgcc atcctcgccg cc 42

<210> 130
<211> 60
<212> DNA
<213> Artificial Sequence

<220>
<223> 9S1-c

<400> 130
atgaaaaaat acctattccg agctgcccaa tacggcatcg ccgccgccat cctcgccgcc 60

<210> 131
<211> 60
<212> DNA
<213> Artificial Sequence

<220>
<223> 9S1-b

<400> 131
atgaaaaaat acctattccg ggccgcccaa tacggcatcg ccgccgccat cctcgccgcc 60

<210> 132
<211> 60
<212> DNA
<213> Artificial Sequence

<220>
<223> 9S1-i

<400> 132
atgaaaaaat acctattccg ggcggctttg tacgggatcg ccgccgccat cctcgccgcc 60

<210> 133
<211> 14
<212> PRT
<213> Artificial Sequence

<220>
<223> 9S1e

<400> 133

Met Lys Lys Tyr Leu Phe Ile Ala Ala Ala Ile Leu Ala Ala
1               5                   10

<210> 134
<211> 20
<212> PRT
<213> Artificial Sequence

<220>

<223> 9S1c

<400> 134

Met Lys Lys Tyr Leu Phe Arg Ala Ala Gln Tyr Gly Ile Ala Ala Ala
1 5 10 15

Ile Leu Ala Ala
20

<210> 135
<211> 20
<212> PRT
<213> Artificial Sequence

<220>
<223> 9Slb

<400> 135

Met Lys Lys Tyr Leu Phe Arg Ala Ala Gln Tyr Gly Ile Ala Ala Ala
1 5 10 15

Ile Leu Ala Ala
20

<21.0> 136
<211> 20
<212> PRT
<213> Artificial Sequence

<220>
<223> 9Sli

<400> 136

Met Lys Lys Tyr Leu Phe Arg Ala Ala Leu Tyr Gly Ile Ala Ala Ala
1 5 10 15

Ile Leu Ala Ala
20

<210> 137
<211> 467
<212> PRT
<213> Artificial Sequence

<220>
<223> 730

<400> 137

Val Lys Pro Leu Arg Arg Leu Thr Asn Leu Leu Ala Ala Cys Ala Val
1 5 10 15

Ala Ala Ala Ala Leu Ile Gln Pro Ala Leu Ala Ala Asp Leu Ala Gln
20 25 30

Asp Pro Phe Ile Thr Asp Asn Ala Gln Arg Gln His Tyr Glu Pro Gly
35 40 45

Gly Lys Tyr His Leu Phe Gly Asp Pro Arg Gly Ser Val Ser Asp Arg
50 55 60

Thr Gly Lys Ile Asn Val Ile Gln Asp Tyr Thr His Gln Met Gly Asn
65 70 75 80

Leu Leu Ile Gln Gln Ala Asn Ile Asn Gly Thr Ile Gly Tyr His Thr
85 90 95

Arg Phe Ser Gly His Gly His Glu Glu His Ala Pro Phe Asp Asn His
100 105 110

Ala Ala Asp Ser Ala Ser Glu Glu Lys Gly Asn Val Asp Glu Gly Phe
115 120 125

Thr Val Tyr Arg Leu Asn Trp Glu Gly His Glu His His Pro Ala Asp
130 135 140

Ala Tyr Asp Gly Pro Lys Gly Gly Asn Tyr Pro Lys Pro Thr Gly Ala
145 150 155 160

Arg Asp Glu Tyr Thr Tyr His Val Asn Gly Thr Ala Arg Ser Ile Lys
165 170 175

Leu Asn Pro Thr Asp Thr Arg Ser Ile Arg Gln Arg Ile Ser Asp Asn
180 185 190

Tyr Ser Asn Leu Gly Ser Asn Phe Ser Asp Arg Ala Asp Glu Ala Asn
195 200 205

Arg Lys Met Phe Glu His Asn Ala Lys Leu Asp Arg Trp Gly Asn Ser
210 215 220

Met Glu Phe Ile Asn Gly Val Ala Ala Gly Ala Leu Asn Pro Phe Ile
225 230 235 240

Ser Ala Gly Glu Ala Leu Gly Ile Gly Asp Ile Leu Tyr Gly Thr Arg
245 250 255

Tyr Ala Ile Asp Lys Ala Ala Met Arg Asn Ile Ala Pro Leu Pro Ala
260 265 270

Glu Gly Lys Phe Ala Val Ile Gly Gly Leu Gly Ser Val Ala Gly Phe
275 280 285

Glu Lys Asn Thr Arg Glu Ala Val Asp Arg Trp Ile Gln Glu Asn Pro
290 295 300

Asn Ala Ala Glu Thr Val Glu Ala Val Phe Asn Val Ala Ala Ala Ala
305 310 315 320

Lys Val Ala Lys Leu Ala Lys Ala Ala Lys Pro Gly Lys Ala Ala Val
325 330 335

Ser Gly Asp Phe Ala Asp Ser Tyr Lys Lys Lys Leu Ala Leu Ser Asp
340 345 350

Ser Ala Arg Gln Leu Tyr Gln Asn Ala Lys Tyr Arg Glu Ala Leu Asp
355 360 365

Ile His Tyr Glu Asp Leu Ile Arg Arg Lys Thr Asp Gly Ser Ser Lys
370 375 380

Phe Ile Asn Gly Arg Glu Ile Asp Ala Val Thr Asn Asp Ala Leu Ile
385 390 395 400

Gln Ala Lys Arg Thr Ile Ser Ala Ile Asp Lys Pro Lys Asn Phe Leu
405 410 415

Asn Gln Lys Asn Arg Lys Gln Ile Lys Ala Thr Ile Glu Ala Ala Asn
420 425 430

Gln Gln Gly Lys Arg Ala Glu Phe Trp Phe Lys Tyr Gly Val His Ser
435 440 445

Gln Val Lys Ser Tyr Ile Glu Ser Lys Gly Gly Ile Val Lys Thr Gly
450 455 460

Leu Gly Asp
465

<210> 138
<211> 377
<212> PRT
<213> Artificial Sequence

<220>
<223> 730-C1

<400> 138

Met Ala Asp Leu Ala Gln Asp Pro Phe Ile Thr Asp Asn Ala Gln Arg
1 5 10 15

Gln His Tyr Glu Pro Gly Gly Lys Tyr His Leu Phe Gly Asp Pro Arg
20 25 30

Gly Ser Val Ser Asp Arg Thr Gly Lys Ile Asn Val Ile Gln Asp Tyr
35 40 45

Thr His Gln Met Gly Asn Leu Leu Ile Gln Gln Ala Asn Ile Asn Gly
50 55 60

Thr Ile Gly Tyr His Thr Arg Phe Ser Gly His Gly His Glu Glu His
65 70 75 80

Ala Pro Phe Asp Asn His Ala Ala Asp Ser Ala Ser Glu Glu Lys Gly
85 90 95

Asn Val Asp Glu Gly Phe Thr Val Tyr Arg Leu Asn Trp Glu Gly His
100 105 110

Glu His His Pro Ala Asp Ala Tyr Asp Gly Pro Lys Gly Gly Asn Tyr
115 120 125

Pro Lys Pro Thr Gly Ala Arg Asp Glu Tyr Thr Tyr His Val Asn Gly
130 135 140

Thr Ala Arg Ser Ile Lys Leu Asn Pro Thr Asp Thr Arg Ser Ile Arg
145 150 155 160

Gln Arg Ile Ser Asp Asn Tyr Ser Asn Leu Gly Ser Asn Phe Ser Asp
165 170 175

Arg Ala Asp Glu Ala Asn Arg Lys Met Phe Glu His Asn Ala Lys Leu
180 185 190

Asp Arg Trp Gly Asn Ser Met Glu Phe Ile Asn Gly Val Ala Ala Gly
195 200 205

Ala Leu Asn Pro Phe Ile Ser Ala Gly Glu Ala Leu Gly Ile Gly Asp
210 215 220

Ile Leu Tyr Gly Thr Arg Tyr Ala Ile Asp Lys Ala Ala Met Arg Asn
225 230 235 240

Ile Ala Pro Leu Pro Ala Glu Gly Lys Phe Ala Val Ile Gly Gly Leu
245 250 255

Gly Ser Val Ala Gly Phe Glu Lys Asn Thr Arg Glu Ala Val Asp Arg
260 265 270

Trp Ile Gln Glu Asn Pro Asn Ala Ala Glu Thr Val Glu Ala Val Phe
275 280 285

Asn Val Ala Ala Ala Ala Lys Val Ala Lys Leu Ala Lys Ala Ala Lys
290 295 300

Pro Gly Lys Ala Ala Val Ser Gly Asp Phe Ala Asp Ser Tyr Lys Lys
305 310 315 320

Lys Leu Ala Leu Ser Asp Ser Ala Arg Gln Leu Tyr Gln Asn Ala Lys
325 330 335

Tyr Arg Glu Ala Leu Asp Ile His Tyr Glu Asp Leu Ile Arg Arg Lys
340 345 350

Thr Asp Gly Ser Ser Lys Phe Ile Asn Gly Arg Glu Ile Asp Ala Val
355 360 365

Thr Asn Asp Ala Leu Ile Gln Ala Arg
370 375

<210> 139
<211> 353
<212> PRT
<213> Artificial Sequence

<220>
<223> 730-C2

<400> 139

```
Met Ala Asp Leu Ala Gln Asp Pro Phe Ile Thr Asp Asn Ala Gln Arg
1               5                   10                  15

Gln His Tyr Glu Pro Gly Gly Lys Tyr His Leu Phe Gly Asp Pro Arg
            20                  25                  30

Gly Ser Val Ser Asp Arg Thr Gly Lys Ile Asn Val Ile Gln Asp Tyr
        35                  40                  45

Thr His Gln Met Gly Asn Leu Leu Ile Gln Gln Ala Asn Ile Asn Gly
    50                  55                  60
```

```
Thr Ile Gly Tyr His Thr Arg Phe Ser Gly His Gly His Glu Glu His
65                  70                  75                  80

Ala Pro Phe Asp Asn His Ala Ala Asp Ser Ala Ser Glu Glu Lys Gly
                85                  90                  95

Asn Val Asp Glu Gly Phe Thr Val Tyr Arg Leu Asn Trp Glu Gly His
            100                 105                 110

Glu His His Pro Ala Asp Ala Tyr Asp Gly Pro Lys Gly Gly Asn Tyr
        115                 120                 125

Pro Lys Pro Thr Gly Ala Arg Asp Glu Tyr Thr Tyr His Val Asn Gly
    130                 135                 140

Thr Ala Arg Ser Ile Lys Leu Asn Pro Thr Asp Thr Arg Ser Ile Arg
145                 150                 155                 160

Gln Arg Ile Ser Asp Asn Tyr Ser Asn Leu Gly Ser Asn Phe Ser Asp
                165                 170                 175

Arg Ala Asp Glu Ala Asn Arg Lys Met Phe Glu His Asn Ala Lys Leu
            180                 185                 190

Asp Arg Trp Gly Asn Ser Met Glu Phe Ile Asn Gly Val Ala Ala Gly
        195                 200                 205

Ala Leu Asn Pro Phe Ile Ser Ala Gly Glu Ala Leu Gly Ile Gly Asp
    210                 215                 220

Ile Leu Tyr Gly Thr Arg Tyr Ala Ile Asp Lys Ala Ala Met Arg Asn
225                 230                 235                 240

Ile Ala Pro Leu Pro Ala Glu Gly Lys Phe Ala Val Ile Gly Gly Leu
                245                 250                 255

Gly Ser Val Ala Gly Phe Glu Lys Asn Thr Arg Glu Ala Val Asp Arg
            260                 265                 270

Trp Ile Gln Glu Asn Pro Asn Ala Ala Glu Thr Val Glu Ala Val Phe
        275                 280                 285

Asn Val Ala Ala Ala Ala Lys Val Ala Lys Leu Ala Lys Ala Ala Lys
    290                 295                 300

Pro Gly Lys Ala Ala Val Ser Gly Asp Phe Ala Asp Ser Tyr Lys Lys
305                 310                 315                 320

Lys Leu Ala Leu Ser Asp Ser Ala Arg Gln Leu Tyr Gln Asn Ala Lys
                325                 330                 335

Tyr Arg Glu Ala Leu Gly Lys Val Arg Ile Ser Gly Glu Ile Leu Leu
            340                 345                 350

Gly
```

<210> 140
<211> 2019
<212> DNA
<213> Artificial Sequence

<220>
<223> ORF46.1-741

<400> 140

```
atgtcagatt tggcaaacga ttcttttatc cggcaggttc tcgaccgtca gcatttcgaa    60
cccgacggga aataccacct attcggcagc aggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140
aaactggcag accaacgcca cccgaagaca ggcgtaccgt ttgacggtaa agggtttccg  1200
aattttgaga agcacgtgaa atatgatacg ggatccggag gggtggtgt cgccgccgac   1260
atcggtgcgg ggcttgccga tgcactaacc gcaccgctcg accataaaga caaaggtttg  1320
cagtctttga cgctggatca gtccgtcagg aaaaacgaga aactgaagct ggcggcacaa  1380
ggtgcggaaa aaacttatgg aaacggtgac agcctcaata cgggcaaatt gaagaacgac  1440
aaggtcagcc gtttcgactt tatccgccaa atcgaagtgg acgggcagct cattaccttg  1500
gagagtggag agttccaagt atacaaacaa agccattccg ccttaaccgc ctttcagacc  1560
gagcaaatac aagattcgga gcattccggg aagatggttg cgaaacgcca gttcagaatc  1620
ggcgacatag cgggcgaaca tacatctttt gacaagcttc ccgaaggcgg cagggcgaca  1680
tatcgcggga cggcgttcgg ttcagacgat gccggcggaa aactgaccta caccatagat  1740
ttcgccgcca agcagggaaa cggcaaaatc gaacatttga aatcgccaga actcaatgtc  1800
gacctggccg ccgccgatat caagccggat ggaaaacgcc atgccgtcat cagcggttcc  1860
gtcctttaca accaagccga gaaaggcagt tactccctcg gtatctttgg cggaaaagcc  1920
caggaagttg ccggcagcgc ggaagtgaaa accgtaaacg gcatacgcca tatcggcctt  1980
gccgccaagc aactcgagca ccaccaccac caccactga                         2019
```

<210> 141
<211> 672
<212> PRT
<213> Artificial Sequence

<220>
<223> ORF46.1-741

<400> 141

```
Met Ser Asp Leu Ala Asn Asp Ser Phe Ile Arg Gln Val Leu Asp Arg
1               5                   10                  15
```

Gln His Phe Glu Pro Asp Gly Lys Tyr His Leu Phe Gly Ser Arg Gly
20 25 30

Glu Leu Ala Glu Arg Ser Gly His Ile Gly Leu Gly Lys Ile Gln Ser
35 40 45

His Gln Leu Gly Asn Leu Met Ile Gln Gln Ala Ala Ile Lys Gly Asn
50 55 60

Ile Gly Tyr Ile Val Arg Phe Ser Asp His Gly His Glu Val His Ser
65 70 75 80

Pro Phe Asp Asn His Ala Ser His Ser Asp Ser Asp Glu Ala Gly Ser
85 90 95

Pro Val Asp Gly Phe Ser Leu Tyr Arg Ile His Trp Asp Gly Tyr Glu
100 105 110

His His Pro Ala Asp Gly Tyr Asp Gly Pro Gln Gly Gly Gly Tyr Pro
115 120 125

Ala Pro Lys Gly Ala Arg Asp Ile Tyr Ser Tyr Asp Ile Lys Gly Val
130 135 140

Ala Gln Asn Ile Arg Leu Asn Leu Thr Asp Asn Arg Ser Thr Gly Gln
145 150 155 160

Arg Leu Ala Asp Arg Phe His Asn Ala Gly Ser Met Leu Thr Gln Gly
165 170 175

Val Gly Asp Gly Phe Lys Arg Ala Thr Arg Tyr Ser Pro Glu Leu Asp
180 185 190

Arg Ser Gly Asn Ala Ala Glu Ala Phe Asn Gly Thr Ala Asp Ile Val
195 200 205

Lys Asn Ile Ile Gly Ala Ala Gly Glu Ile Val Gly Ala Gly Asp Ala
210 215 220

Val Gln Gly Ile Ser Glu Gly Ser Asn Ile Ala Val Met His Gly Leu
225 230 235 240

Gly Leu Leu Ser Thr Glu Asn Lys Met Ala Arg Ile Asn Asp Leu Ala
245 250 255

Asp Met Ala Gln Leu Lys Asp Tyr Ala Ala Ala Ala Ile Arg Asp Trp
260 265 270

Ala Val Gln Asn Pro Asn Ala Ala Gln Gly Ile Glu Ala Val Ser Asn
275 280 285

Ile Phe Met Ala Ala Ile Pro Ile Lys Gly Ile Gly Ala Val Arg Gly
290 295 300

Lys Tyr Gly Leu Gly Gly Ile Thr Ala His Pro Ile Lys Arg Ser Gln
305 310 315 320

Met Gly Ala Ile Ala Leu Pro Lys Gly Lys Ser Ala Val Ser Asp Asn
325 330 335

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Val Ala Ala Asp Ile Gly Ala Gly Leu Ala Asp Ala Leu Thr Ala Pro
420 425 430

Leu Asp His Lys Asp Lys Gly Leu Gln Ser Leu Thr Leu Asp Gln Ser
435 440 445

Val Arg Lys Asn Glu Lys Leu Lys Leu Ala Ala Gln Gly Ala Glu Lys
450 455 460

Thr Tyr Gly Asn Gly Asp Ser Leu Asn Thr Gly Lys Leu Lys Asn Asp
465 470 475 480

Lys Val Ser Arg Phe Asp Phe Ile Arg Gln Ile Glu Val Asp Gly Gln
485 490 495

Leu Ile Thr Leu Glu Ser Gly Glu Phe Gln Val Tyr Lys Gln Ser His
500 505 510

Ser Ala Leu Thr Ala Phe Gln Thr Glu Gln Ile Gln Asp Ser Glu His
515 520 525

Ser Gly Lys Met Val Ala Lys Arg Gln Phe Arg Ile Gly Asp Ile Ala
530 535 540

Gly Glu His Thr Ser Phe Asp Lys Leu Pro Glu Gly Gly Arg Ala Thr
545 550 555 560

Tyr Arg Gly Thr Ala Phe Gly Ser Asp Asp Ala Gly Gly Lys Leu Thr
565 570 575

Tyr Thr Ile Asp Phe Ala Ala Lys Gln Gly Asn Gly Lys Ile Glu His
580 585 590

Leu Lys Ser Pro Glu Leu Asn Val Asp Leu Ala Ala Ala Asp Ile Lys
595 600 605

Pro Asp Gly Lys Arg His Ala Val Ile Ser Gly Ser Val Leu Tyr Asn
610 615 620

Gln Ala Glu Lys Gly Ser Tyr Ser Leu Gly Ile Phe Gly Gly Lys Ala
625 630 635 640

Gln Glu Val Ala Gly Ser Ala Glu Val Lys Thr Val Asn Gly Ile Arg
645 650 655

His Ile Gly Leu Ala Ala Lys Gln Leu Glu His His His His His His
660 665 670

<210> 142
<211> 2421
<212> DNA
<213> Artificial Sequence

<220>
<223> ORF46.1-961

<400> 142

```
atgtcagatt tggcaaacga ttcttttatc cggcaggttc tcgaccgtca gcatttcgaa 60
cccgacggga aataccacct attcggcagc agggggggaac ttgccgagcg cagcggccat 120
atcggattgg gaaaaataca aagccatcag ttgggcaacc tgatgattca acaggcggcc 180
attaaaggaa atatcggcta cattgtccgc ttttccgatc acgggcacga agtccattcc 240
cccttcgaca accatgcctc acattccgat tctgatgaag ccggtagtcc cgttgacgga 300
tttagccttt accgcatcca ttgggacgga tacgaacacc atcccgccga cggctatgac 360
gggccacagg gcggcggcta tcccgctccc aaaggcgcga gggatatata cagctacgac 420
ataaaaggcg ttgcccaaaa tatccgcctc aacctgaccg acaaccgcag caccggacaa 480
cggcttgccg accgtttcca caatgccggt agtatgctga cgcaaggagt aggcgacgga 540
ttcaaacgcg ccacccgata cagccccgag ctggacagat cgggcaatgc cgccgaagcc 600
ttcaacggca ctgcagatat cgttaaaaac atcatcggcg cggcaggaga aattgtcggc 660
gcaggcgatg ccgtgcaggg cataagcgaa ggctcaaaca ttgctgtcat gcacggcttg 720
ggtctgcttt ccaccgaaaa caagatggcg cgcatcaacg atttggcaga tatggcgcaa 780
ctcaaagact atgccgcagc agccatccgc gattgggcag tccaaaaccc caatgccgca 840
caaggcatag aagccgtcag caatatcttt atggcagcca tccccatcaa agggattgga 900
gctgttcggg gaaaatacgg cttgggcggc atcacggcac atcctatcaa gcggtcgcag 960
atgggcgcga tcgcattgcc gaaagggaaa tccgccgtca gcgacaattt tgccgatgcg 1020
gcatacgcca aatacccgtc cccttaccat tcccgaaata tccgttcaaa cttggagcag 1080
cgttacggca aagaaaacat cacctcctca accgtgccgc cgtcaaacgg caaaaatgtc 1140
aaactggcag accaacgcca cccgaagaca ggcgtaccgt ttgacggtaa agggtttccg 1200
aattttgaga agcacgtgaa atatgatacg ggatccggag gaggaggagc cacaaacgac 1260
gacgatgtta aaaaagctgc cactgtggcc attgctgctg cctacaacaa tggccaagaa 1320
atcaacggtt tcaaagctgg agagaccatc tacgacattg atgaagacgg cacaattacc 1380
aaaaaagacg caactgcagc cgatgttgaa gccgacgact ttaaaggtct gggtctgaaa 1440
aaagtcgtga ctaacctgac caaaaccgtc aatgaaaaca aacaaaacgt cgatgccaaa 1500
gtaaaagctg cagaatctga aatagaaaag ttaacaacca agttagcaga cactgatgcc 1560
gctttagcag atactgatgc cgctctggat gcaaccacca acgccttgaa taaattggga 1620
gaaaatataa cgacatttgc tgaagagact aagacaaata tcgtaaaaat tgatgaaaaa 1680
ttagaagccg tggctgatac cgtcgacaag catgccgaag cattcaacga tatcgccgat 1740
tcattggatg aaaccaacac taaggcagac gaagccgtca aaaccgccaa tgaagccaaa 1800
cagacggccg aagaaaccaa acaaaacgtc gatgccaaag taaaagctgc agaaactgca 1860
gcaggcaaag ccgaagctgc cgctggcaca gctaatactg cagccgacaa ggccgaagct 1920
gtcgctgcaa aagttaccga catcaaagct gatatcgcta cgaacaaaga taatattgct 1980
aaaaaagcaa acagtgccga cgtgtacacc agagaagagt ctgacagcaa atttgtcaga 2040
attgatggtc tgaacgctac taccgaaaaa ttggacacac gcttggcttc tgctgaaaaa 2100
tccattgccg atcacgatac tcgcctgaac ggtttggata aaacagtgtc agacctgcgc 2160
aaagaaaccc gccaaggcct tgcagaacaa gccgcgctct ccggtctgtt ccaaccttac 2220
aacgtgggtc ggttcaatgt aacggctgca gtcggcggct acaaatccga atcggcagtc 2280
```

gccatcggta ccggcttccg ctttaccgaa aactttgccg ccaaagcagg cgtggcagtc 2340
ggcacttcgt ccggttcttc cgcagcctac catgtcggcg tcaattacga gtggctcgag 2400
caccaccacc accaccactg a 2421

<210> 143
<211> 806
<212> PRT
<213> Artificial Sequence

<220>
<223> ORF46.1-961

<400> 143

Met Ser Asp Leu Ala Asn Asp Ser Phe Ile Arg Gln Val Leu Asp Arg
1 5 10 15

Gln His Phe Glu Pro Asp Gly Lys Tyr His Leu Phe Gly Ser Arg Gly
20 25 30

Glu Leu Ala Glu Arg Ser Gly His Ile Gly Leu Gly Lys Ile Gln Ser
35 40 45

His Gln Leu Gly Asn Leu Met Ile Gln Gln Ala Ala Ile Lys Gly Asn
50 55 60

Ile Gly Tyr Ile Val Arg Phe Ser Asp His Gly His Glu Val His Ser
65 70 75 80

Pro Phe Asp Asn His Ala Ser His Ser Asp Ser Asp Glu Ala Gly Ser
85 90 95

Pro Val Asp Gly Phe Ser Leu Tyr Arg Ile His Trp Asp Gly Tyr Glu
100 105 110

His His Pro Ala Asp Gly Tyr Asp Gly Pro Gln Gly Gly Gly Tyr Pro
115 120 125

Ala Pro Lys Gly Ala Arg Asp Ile Tyr Ser Tyr Asp Ile Lys Gly Val
130 135 140

Ala Gln Asn Ile Arg Leu Asn Leu Thr Asp Asn Arg Ser Thr Gly Gln
145 150 155 160

Arg Leu Ala Asp Arg Phe His Asn Ala Gly Ser Met Leu Thr Gln Gly
165 170 175

Val Gly Asp Gly Phe Lys Arg Ala Thr Arg Tyr Ser Pro Glu Leu Asp
180 185 190

Arg Ser Gly Asn Ala Ala Glu Ala Phe Asn Gly Thr Ala Asp Ile Val
195 200 205

Lys Asn Ile Ile Gly Ala Ala Gly Glu Ile Val Gly Ala Gly Asp Ala
210 215 220

Val Gln Gly Ile Ser Glu Gly Ser Asn Ile Ala Val Met His Gly Leu
225 230 235 240

Gly Leu Leu Ser Thr Glu Asn Lys Met Ala Arg Ile Asn Asp Leu Ala
245 250 255

Asp Met Ala Gln Leu Lys Asp Tyr Ala Ala Ala Ala Ile Arg Asp Trp
260 265 270

Ala Val Gln Asn Pro Asn Ala Ala Gln Gly Ile Glu Ala Val Ser Asn
275 280 285

Ile Phe Met Ala Ala Ile Pro Ile Lys Gly Ile Gly Ala Val Arg Gly
290 295 300

Lys Tyr Gly Leu Gly Gly Ile Thr Ala His Pro Ile Lys Arg Ser Gln
305 310 315 320

Met Gly Ala Ile Ala Leu Pro Lys Gly Lys Ser Ala Val Ser Asp Asn
325 330 335

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Ala Thr Asn Asp Asp Asp Val Lys Lys Ala Ala Thr Val Ala Ile Ala
420 425 430

Ala Ala Tyr Asn Asn Gly Gln Glu Ile Asn Gly Phe Lys Ala Gly Glu
435 440 445

Thr Ile Tyr Asp Ile Asp Glu Asp Gly Thr Ile Thr Lys Lys Asp Ala
450 455 460

Thr Ala Ala Asp Val Glu Ala Asp Asp Phe Lys Gly Leu Gly Leu Lys
465 470 475 480

Lys Val Val Thr Asn Leu Thr Lys Thr Val Asn Glu Asn Lys Gln Asn
485 490 495

Val Asp Ala Lys Val Lys Ala Ala Glu Ser Glu Ile Glu Lys Leu Thr
500 505 510

Thr Lys Leu Ala Asp Thr Asp Ala Ala Leu Ala Asp Thr Asp Ala Ala
515 520 525

Leu Asp Ala Thr Thr Asn Ala Leu Asn Lys Leu Gly Glu Asn Ile Thr
530 535 540

Thr Phe Ala Glu Glu Thr Lys Thr Asn Ile Val Lys Ile Asp Glu Lys
545 550 555 560

Leu Glu Ala Val Ala Asp Thr Val Asp Lys His Ala Glu Ala Phe Asn
565 570 575

Asp Ile Ala Asp Ser Leu Asp Glu Thr Asn Thr Lys Ala Asp Glu Ala
580 585 590

Val Lys Thr Ala Asn Glu Ala Lys Gln Thr Ala Glu Glu Thr Lys Gln
595 600 605

Asn Val Asp Ala Lys Val Lys Ala Ala Glu Thr Ala Ala Gly Lys Ala
610 615 620

Glu Ala Ala Ala Gly Thr Ala Asn Thr Ala Ala Asp Lys Ala Glu Ala
625 630 635 640

Val Ala Ala Lys Val Thr Asp Ile Lys Ala Asp Ile Ala Thr Asn Lys
645 650 655

Asp Asn Ile Ala Lys Lys Ala Asn Ser Ala Asp Val Tyr Thr Arg Glu
660 665 670

Glu Ser Asp Ser Lys Phe Val Arg Ile Asp Gly Leu Asn Ala Thr Thr
675 680 685

Glu Lys Leu Asp Thr Arg Leu Ala Ser Ala Glu Lys Ser Ile Ala Asp
690 695 700

His Asp Thr Arg Leu Asn Gly Leu Asp Lys Thr Val Ser Asp Leu Arg
705 710 715 720

Lys Glu Thr Arg Gln Gly Leu Ala Glu Gln Ala Ala Leu Ser Gly Leu
725 730 735

Phe Gln Pro Tyr Asn Val Gly Arg Phe Asn Val Thr Ala Ala Val Gly
740 745 750

Gly Tyr Lys Ser Glu Ser Ala Val Ala Ile Gly Thr Gly Phe Arg Phe
755 760 765

Thr Glu Asn Phe Ala Ala Lys Ala Gly Val Ala Val Gly Thr Ser Ser
770 775 780

Gly Ser Ser Ala Ala Tyr His Val Gly Val Asn Tyr Glu Trp Leu Glu
785 790 795 800

His His His His His His
805

<210> 144
<211> 2256
<212> DNA
<213> Artificial Sequence

<220>
<223> ORF46.1-961c

<400> 144

```
atgtcagatt tggcaaacga ttcttttatc cggcaggttc tcgaccgtca gcatttcgaa    60
cccgacggga aataccacct attcggcagc aggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140
aaactggcag accaacgcca cccgaagaca ggcgtaccgt ttgacggtaa agggtttccg  1200
aattttgaga agcacgtgaa atatgatacg ggatccggag gaggaggagc cacaaacgac  1260
gacgatgtta aaaaagctgc cactgtggcc attgctgctg cctacaacaa tggccaagaa  1320
atcaacggtt tcaaagctgg agagaccatc tacgacattg atgaagacgg cacaattacc  1380
aaaaaagacg caactgcagc cgatgttgaa gccgacgact ttaaaggtct gggtctgaaa  1440
aaagtcgtga ctaacctgac caaaaccgtc aatgaaaaca aacaaaacgt cgatgccaaa  1500
gtaaaagctg cagaatctga aatagaaaag ttaacaacca agttagcaga cactgatgcc  1560
gctttagcag atactgatgc cgctctggat gcaaccacca acgccttgaa taaattggga  1620
gaaaatataa cgacatttgc tgaagagact aagacaaata tcgtaaaaat tgatgaaaaa  1680
ttagaagccg tggctgatac cgtcgacaag catgccgaag cattcaacga tatcgccgat  1740
tcattggatg aaaccaacac taaggcagac gaagccgtca aaaccgccaa tgaagccaaa  1800
cagacggccg aagaaaccaa acaaaacgtc gatgccaaag taaaagctgc agaaactgca  1860
gcaggcaaag ccgaagctgc cgctggcaca gctaatactg cagccgacaa ggccgaagct  1920
gtcgctgcaa aagttaccga catcaaagct gatatcgcta cgaacaaaga taatattgct  1980
aaaaaagcaa acagtgccga cgtgtacacc agagaagagt ctgacagcaa atttgtcaga  2040
attgatggtc tgaacgctac taccgaaaaa ttggacacac gcttggcttc tgctgaaaaa  2100
tccattgccg atcacgatac tcgcctgaac ggtttggata aaacagtgtc agacctgcgc  2160
aaagaaaccc gccaaggcct tgcagaacaa gccgcgctct ccggtctgtt ccaaccttac  2220
aacgtgggtc tcgagcacca ccaccaccac cactga                            2256
```

<210> 145
<211> 751
<212> PRT
<213> Artificial Sequence

<220>
<223> ORF46.1-961c

<400> 145

```
Met Ser Asp Leu Ala Asn Asp Ser Phe Ile Arg Gln Val Leu Asp Arg
1               5                   10                  15

Gln His Phe Glu Pro Asp Gly Lys Tyr His Leu Phe Gly Ser Arg Gly
            20                  25                  30
```

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175

Val Gly Asp Gly Phe Lys Arg Ala Thr Arg Tyr Ser Pro Glu Leu Asp
            180                 185                 190

Arg Ser Gly Asn Ala Ala Glu Ala Phe Asn Gly Thr Ala Asp Ile Val
        195                 200                 205

Lys Asn Ile Ile Gly Ala Ala Gly Glu Ile Val Gly Ala Gly Asp Ala
    210                 215                 220

Val Gln Gly Ile Ser Glu Gly Ser Asn Ile Ala Val Met His Gly Leu
225                 230                 235                 240

Gly Leu Leu Ser Thr Glu Asn Lys Met Ala Arg Ile Asn Asp Leu Ala
                245                 250                 255

Asp Met Ala Gln Leu Lys Asp Tyr Ala Ala Ala Ala Ile Arg Asp Trp
            260                 265                 270

Ala Val Gln Asn Pro Asn Ala Ala Gln Gly Ile Glu Ala Val Ser Asn
        275                 280                 285

Ile Phe Met Ala Ala Ile Pro Ile Lys Gly Ile Gly Ala Val Arg Gly
    290                 295                 300

Lys Tyr Gly Leu Gly Gly Ile Thr Ala His Pro Ile Lys Arg Ser Gln
305                 310                 315                 320

Met Gly Ala Ile Ala Leu Pro Lys Gly Lys Ser Ala Val Ser Asp Asn
                325                 330                 335

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Ala Thr Asn Asp Asp Asp Val Lys Lys Ala Ala Thr Val Ala Ile Ala
420 425 430

Ala Ala Tyr Asn Asn Gly Gln Glu Ile Asn Gly Phe Lys Ala Gly Glu
435 440 445

Thr Ile Tyr Asp Ile Asp Glu Asp Gly Thr Ile Thr Lys Lys Asp Ala
450 455 460

Thr Ala Ala Asp Val Glu Ala Asp Asp Phe Lys Gly Leu Gly Leu Lys
465 470 475 480

Lys Val Val Thr Asn Leu Thr Lys Thr Val Asn Glu Asn Lys Gln Asn
485 490 495

Val Asp Ala Lys Val Lys Ala Ala Glu Ser Glu Ile Glu Lys Leu Thr
500 505 510

Thr Lys Leu Ala Asp Thr Asp Ala Ala Leu Ala Asp Thr Asp Ala Ala
515 520 525

Leu Asp Ala Thr Thr Asn Ala Leu Asn Lys Leu Gly Glu Asn Ile Thr
530 535 540

Thr Phe Ala Glu Glu Thr Lys Thr Asn Ile Val Lys Ile Asp Glu Lys
545 550 555 560

Leu Glu Ala Val Ala Asp Thr Val Asp Lys His Ala Glu Ala Phe Asn
565 570 575

Asp Ile Ala Asp Ser Leu Asp Glu Thr Asn Thr Lys Ala Asp Glu Ala
580 585 590

Val Lys Thr Ala Asn Glu Ala Lys Gln Thr Ala Glu Glu Thr Lys Gln
595 600 605

Asn Val Asp Ala Lys Val Lys Ala Ala Glu Thr Ala Ala Gly Lys Ala
610 615 620

Glu Ala Ala Ala Gly Thr Ala Asn Thr Ala Ala Asp Lys Ala Glu Ala
625 630 635 640

Val Ala Ala Lys Val Thr Asp Ile Lys Ala Asp Ile Ala Thr Asn Lys
645 650 655

Asp Asn Ile Ala Lys Lys Ala Asn Ser Ala Asp Val Tyr Thr Arg Glu
660 665 670

Glu Ser Asp Ser Lys Phe Val Arg Ile Asp Gly Leu Asn Ala Thr Thr
675 680 685

Glu Lys Leu Asp Thr Arg Leu Ala Ser Ala Glu Lys Ser Ile Ala Asp
690 695 700

His Asp Thr Arg Leu Asn Gly Leu Asp Lys Thr Val Ser Asp Leu Arg
705 710 715 720

Lys Glu Thr Arg Gln Gly Leu Ala Glu Gln Ala Ala Leu Ser Gly Leu
725 730 735

Phe Gln Pro Tyr Asn Val Gly Leu Glu His His His His His His
740 745 750

<210> 146
<211> 2421
<212> DNA
<213> Artificial Sequence

<220>
<223> 961-ORF46.1

<400> 146

```
atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggagg aggaggatca gatttggcaa acgattcttt tatccggcag 1200
gttctcgacc gtcagcattt cgaacccgac gggaaatacc acctattcgg cagcaggggg 1260
gaacttgccg agcgcagcgg ccatatcgga ttgggaaaaa tacaaagcca tcagttgggc 1320
aacctgatga ttcaacaggc ggccattaaa ggaaatatcg gctacattgt ccgcttttcc 1380
gatcacgggc acgaagtcca ttcccccttc gacaaccatg cctcacattc cgattctgat 1440
gaagccggta gtcccgttga cggatttagc ctttaccgca tccattggga cggatacgaa 1500
caccatcccg ccgacggcta tgacgggcca cagggcggcg gctatcccgc tcccaaaggc 1560
```

```
gcgagggata tatacagcta cgacataaaa ggcgttgccc aaaatatccg cctcaacctg 1620
accgacaacc gcagcaccgg acaacggctt gccgaccgtt tccacaatgc cggtagtatg 1680
ctgacgcaag gagtaggcga cggattcaaa cgcgccaccc gatacagccc cgagctggac 1740
agatcgggca atgccgccga agccttcaac ggcactgcag atatcgttaa aaacatcatc 1800
ggcgcggcag gagaaattgt cggcgcaggc gatgccgtgc agggcataag cgaaggctca 1860
aacattgctg tcatgcacgg cttgggtctg ctttccaccg aaaacaagat ggcgcgcatc 1920
aacgatttgg cagatatggc gcaactcaaa gactatgccg cagcagccat ccgcgattgg 1980
gcagtccaaa accccaatgc cgcacaaggc atagaagccg tcagcaatat ctttatggca 2040
gccatcccca tcaaagggat tggagctgtt cggggaaaat acggcttggg cggcatcacg 2100
gcacatccta tcaagcggtc gcagatgggc gcgatcgcat tgccgaaagg gaaatccgcc 2160
gtcagcgaca attttgccga tgcggcatac gccaaatacc cgtcccctta ccattcccga 2220
aatatccgtt caaacttgga gcagcgttac ggcaaagaaa acatcacctc ctcaaccgtg 2280
ccgccgtcaa acggcaaaaa tgtcaaactg gcagaccaac gccacccgaa gacaggcgta 2340
ccgtttgacg gtaaagggtt tccgaatttt gagaagcacg tgaaatatga tacgctcgag 2400
caccaccacc accaccactg a 2421
```

<210> 147
<211> 806
<212> PRT
<213> Artificial Sequence

<220>
<223> 961-ORF46.1

<400> 147

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175
```

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Ser Asp Leu Ala Asn Asp Ser Phe Ile Arg Gln
385 390 395 400

Val Leu Asp Arg Gln His Phe Glu Pro Asp Gly Lys Tyr His Leu Phe
405 410 415

Gly Ser Arg Gly Glu Leu Ala Glu Arg Ser Gly His Ile Gly Leu Gly
420 425 430

Lys Ile Gln Ser His Gln Leu Gly Asn Leu Met Ile Gln Gln Ala Ala
435 440 445

Ile Lys Gly Asn Ile Gly Tyr Ile Val Arg Phe Ser Asp His Gly His
450 455 460

Glu Val His Ser Pro Phe Asp Asn His Ala Ser His Ser Asp Ser Asp
465 470 475 480

Glu Ala Gly Ser Pro Val Asp Gly Phe Ser Leu Tyr Arg Ile His Trp
485 490 495

Asp Gly Tyr Glu His His Pro Ala Asp Gly Tyr Asp Gly Pro Gln Gly
500 505 510

Gly Gly Tyr Pro Ala Pro Lys Gly Ala Arg Asp Ile Tyr Ser Tyr Asp
515 520 525

Ile Lys Gly Val Ala Gln Asn Ile Arg Leu Asn Leu Thr Asp Asn Arg
530 535 540

Ser Thr Gly Gln Arg Leu Ala Asp Arg Phe His Asn Ala Gly Ser Met
545 550 555 560

Leu Thr Gln Gly Val Gly Asp Gly Phe Lys Arg Ala Thr Arg Tyr Ser
565 570 575

Pro Glu Leu Asp Arg Ser Gly Asn Ala Ala Glu Ala Phe Asn Gly Thr
580 585 590

Ala Asp Ile Val Lys Asn Ile Ile Gly Ala Ala Gly Glu Ile Val Gly
595 600 605

Ala Gly Asp Ala Val Gln Gly Ile Ser Glu Gly Ser Asn Ile Ala Val
610 615 620

Met His Gly Leu Gly Leu Leu Ser Thr Glu Asn Lys Met Ala Arg Ile
625 630 635 640

Asn Asp Leu Ala Asp Met Ala Gln Leu Lys Asp Tyr Ala Ala Ala Ala
645 650 655

Ile Arg Asp Trp Ala Val Gln Asn Pro Asn Ala Ala Gln Gly Ile Glu
660 665 670

Ala Val Ser Asn Ile Phe Met Ala Ala Ile Pro Ile Lys Gly Ile Gly
675 680 685

Ala Val Arg Gly Lys Tyr Gly Leu Gly Gly Ile Thr Ala His Pro Ile
690 695 700

Lys Arg Ser Gln Met Gly Ala Ile Ala Leu Pro Lys Gly Lys Ser Ala
705 710 715 720

Val Ser Asp Asn Phe Ala Asp Ala Ala Tyr Ala Lys Tyr Pro Ser Pro
725 730 735

Tyr His Ser Arg Asn Ile Arg Ser Asn Leu Glu Gln Arg Tyr Gly Lys
740 745 750

Glu Asn Ile Thr Ser Ser Thr Val Pro Pro Ser Asn Gly Lys Asn Val
755 760 765

Lys Leu Ala Asp Gln Arg His Pro Lys Thr Gly Val Pro Phe Asp Gly
770 775 780

Lys Gly Phe Pro Asn Phe Glu Lys His Val Lys Tyr Asp Thr Leu Glu
785 790 795 800

His His His His His His
805

<210> 148
<211> 1938
<212> DNA
<213> Artificial Sequence

<220>
<223> 961-741

<400> 148

```
atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggagg gggtggtgtc gccgccgaca tcggtgcggg gcttgccgat 1200
gcactaaccg caccgctcga ccataaagac aaaggtttgc agtctttgac gctggatcag 1260
tccgtcagga aaaacgagaa actgaagctg gcggcacaag gtgcggaaaa aacttatgga 1320
aacggtgaca gcctcaatac gggcaaattg aagaacgaca aggtcagccg tttcgacttt 1380
atccgccaaa tcgaagtgga cgggcagctc attaccttgg agagtggaga gttccaagta 1440
tacaaacaaa gccattccgc cttaaccgcc tttcagaccg agcaaataca agattcggag 1500
cattccggga agatggttgc gaaacgccag ttcagaatcg gcgacatagc gggcgaacat 1560
acatcttttg acaagcttcc cgaaggcggc agggcgacat atcgcgggac ggcgttcggt 1620
tcagacgatg ccggcggaaa actgacctac accatagatt tcgccgccaa gcagggaaac 1680
ggcaaaatcg aacatttgaa atcgccagaa ctcaatgtcg acctggccgc cgccgatatc 1740
aagccggatg gaaaacgcca tgccgtcatc agcggttccg tcctttacaa ccaagccgag 1800
aaaggcagtt actccctcgg tatctttggc ggaaaagccc aggaagttgc cggcagcgcg 1860
gaagtgaaaa ccgtaaacgg catacgccat atcggccttg ccgccaagca actcgagcac 1920
caccaccacc accactga 1938
```

<210> 149
<211> 645
<212> PRT
<213> Artificial Sequence

<220>
<223> 961-741

<400> 149

Met Ala Thr Asn Asp Asp Asp Val Lys Lys Ala Ala Thr Val Ala Ile
1 5 10 15

Ala Ala Ala Tyr Asn Asn Gly Gln Glu Ile Asn Gly Phe Lys Ala Gly
20 25 30

Glu Thr Ile Tyr Asp Ile Asp Glu Asp Gly Thr Ile Thr Lys Lys Asp
35 40 45

Ala Thr Ala Ala Asp Val Glu Ala Asp Asp Phe Lys Gly Leu Gly Leu
50 55 60

Lys Lys Val Val Thr Asn Leu Thr Lys Thr Val Asn Glu Asn Lys Gln
65 70 75 80

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu

290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Val Ala Ala Asp Ile Gly Ala Gly Leu Ala Asp
385 390 395 400

Ala Leu Thr Ala Pro Leu Asp His Lys Asp Lys Gly Leu Gln Ser Leu
405 410 415

Thr Leu Asp Gln Ser Val Arg Lys Asn Glu Lys Leu Lys Leu Ala Ala
420 425 430

Gln Gly Ala Glu Lys Thr Tyr Gly Asn Gly Asp Ser Leu Asn Thr Gly
435 440 445

Lys Leu Lys Asn Asp Lys Val Ser Arg Phe Asp Phe Ile Arg Gln Ile
450 455 460

Glu Val Asp Gly Gln Leu Ile Thr Leu Glu Ser Gly Glu Phe Gln Val
465 470 475 480

Tyr Lys Gln Ser His Ser Ala Leu Thr Ala Phe Gln Thr Glu Gln Ile
485 490 495

Gln Asp Ser Glu His Ser Gly Lys Met Val Ala Lys Arg Gln Phe Arg
500 505 510

Ile Gly Asp Ile Ala Gly Glu His Thr Ser Phe Asp Lys Leu Pro Glu
515 520 525

Gly Gly Arg Ala Thr Tyr Arg Gly Thr Ala Phe Gly Ser Asp Asp Ala
530 535 540

Gly Gly Lys Leu Thr Tyr Thr Ile Asp Phe Ala Ala Lys Gln Gly Asn
545 550 555 560

Gly Lys Ile Glu His Leu Lys Ser Pro Glu Leu Asn Val Asp Leu Ala
565 570 575

Ala Ala Asp Ile Lys Pro Asp Gly Lys Arg His Ala Val Ile Ser Gly
580 585 590

Ser Val Leu Tyr Asn Gln Ala Glu Lys Gly Ser Tyr Ser Leu Gly Ile

595 600 605

Phe Gly Gly Lys Ala Gln Glu Val Ala Gly Ser Ala Glu Val Lys Thr
610 615 620

Val Asn Gly Ile Arg His Ile Gly Leu Ala Ala Lys Gln Leu Glu His
625 630 635 640

His His His His His
645

<210> 150
<211> 4335
<212> DNA
<213> Artificial Sequence

<220>
<223> 961-983

<400> 150

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggcgg aggcggcact tctgcgcccg acttcaatgc aggcggtacc 1200
ggtatcggca gcaacagcag agcaacaaca gcgaaatcag cagcagtatc ttacgccggt 1260
atcaagaacg aaatgtgcaa agacagaagc atgctctgtg ccggtcggga tgacgttgcg 1320
gttacagaca gggatgccaa aatcaatgcc ccccccccga atctgcatac cggagacttt 1380
ccaaacccaa atgacgcata caagaatttg atcaacctca aacctgcaat tgaagcaggc 1440
tatacaggac gcggggtaga ggtaggtatc gtcgacacag gcgaatccgt cggcagcata 1500
tcctttcccg aactgtatgg cagaaaagaa cacggctata acgaaaatta caaaaactat 1560
acggcgtata tgcggaagga agcgcctgaa gacggaggcg gtaaagacat tgaagcttct 1620
ttcgacgatg aggccgttat agagactgaa gcaaagccga cggatatccg ccacgtaaaa 1680
gaaatcggac acatcgattt ggtctcccat attattggcg ggcgttccgt ggacggcaga 1740
cctgcaggcg gtattgcgcc cgatgcgacg ctacacataa tgaatacgaa tgatgaaacc 1800
aagaacgaaa tgatggttgc agccatccgc aatgcatggg tcaagctggg cgaacgtggc 1860
gtgcgcatcg tcaataacag ttttggaaca acatcgaggg caggcactgc cgaccttttc 1920
caaatagcca attcggagga gcagtaccgc caagcgttgc tcgactattc cggcggtgat 1980
aaaacagacg agggtatccg cctgatgcaa cagagcgatt acggcaacct gtcctaccac 2040
atccgtaata aaaacatgct tttcatcttt tcgacaggca atgacgcaca agctcagccc 2100
aacacatatg ccctattgcc attttatgaa aaagacgctc aaaaaggcat tatcacagtc 2160
gcaggcgtag accgcagtgg agaaaagttc aaacgggaaa tgtatggaga accgggtaca 2220
```

```
gaaccgcttg agtatggctc caaccattgc ggaattactg ccatgtggtg cctgtcggca   2280
ccctatgaag caagcgtccg tttcacccgt acaaacccga ttcaaattgc cggaacatcc   2340
ttttccgcac ccatcgtaac cggcacggcg gctctgctgc tgcagaaata cccgtggatg   2400
agcaacgaca acctgcgtac cacgttgctg acgacggctc aggacatcgg tgcagtcggc   2460
gtggacagca agttcggctg gggactgctg gatgcgggta aggccatgaa cggacccgcg   2520
tcctttccgt tcggcgactt taccgccgat acgaaaggta catccgatat tgcctactcc   2580
ttccgtaacg acatttcagg cacgggcggc ctgatcaaaa aaggcggcag ccaactgcaa   2640
ctgcacggca acaacaccta tacgggcaaa accattatcg aaggcggttc gctggtgttg   2700
tacggcaaca acaaatcgga tatgcgcgtc gaaaccaaag gtgcgctgat ttataacggg   2760
gcggcatccg gcggcagcct gaacagcgac ggcattgtct atctggcaga taccgaccaa   2820
tccggcgcaa acgaaaccgt acacatcaaa ggcagtctgc agctggacgg caaaggtacg   2880
ctgtacacac gtttgggcaa actgctgaaa gtggacggta cggcgattat cggcggcaag   2940
ctgtacatgt cggcacgcgg caagggggca ggctatctca acagtaccgg acgacgtgtt   3000
cccttcctga gtgccgccaa aatcgggcag gattattctt tcttcacaaa catcgaaacc   3060
gacggcggcc tgctggcttc cctcgacagc gtcgaaaaaa cagcgggcag tgaaggcgac   3120
acgctgtcct attatgtccg tcgcggcaat gcggcacgga ctgcttcggc agcggcacat   3180
tccgcgcccg ccggtctgaa acacgccgta gaacagggcg gcagcaatct ggaaaacctg   3240
atggtcgaac tggatgcctc cgaatcatcc gcaacacccg agacggttga aactgcggca   3300
gccgaccgca cagatatgcc gggcatccgc ccctacggcg caactttccg cgcagcggca   3360
gccgtacagc atgcgaatgc cgccgacggt gtacgcatct tcaacagtct cgccgctacc   3420
gtctatgccg acagtaccgc cgcccatgcc gatatgcagg gacgccgcct gaaagccgta   3480
tcggacgggt tggaccacaa cggcacgggt ctgcgcgtca tcgcgcaaac ccaacaggac   3540
ggtggaacgt gggaacaggg cggtgttgaa ggcaaaatgc gcggcagtac ccaaaccgtc   3600
ggcattgccg cgaaaaccgg cgaaaatacg acagcagccg ccacactggg catgggacgc   3660
agcacatgga gcgaaaacag tgcaaatgca aaaaccgaca gcattagtct gtttgcaggc   3720
atacggcacg atgcgggcga tatcggctat ctcaaaggcc tgttctccta cggacgctac   3780
aaaaacagca tcagccgcag caccggtgcg gacgaacatg cggaaggcag cgtcaacggc   3840
acgctgatgc agctgggcgc actgggcggt gtcaacgttc cgtttgccgc aacgggagat   3900
ttgacggtcg aaggcggtct gcgctacgac ctgctcaaac aggatgcatt cgccgaaaaa   3960
ggcagtgctt tgggctggag cggcaacagc ctcactgaag gcacgctggt cggactcgcg   4020
ggtctgaagc tgtcgcaacc cttgagcgat aaagccgtcc tgtttgcaac ggcgggcgtg   4080
gaacgcgacc tgaacggacg cgactacacg gtaacgggcg gctttaccgg cgcgactgca   4140
gcaaccggca agacgggggc acgcaatatg ccgcacaccc gtctggttgc cggcctgggc   4200
gcggatgtcg aattcggcaa cggctggaac ggcttggcac gttacagcta cgccggttcc   4260
aaacagtacg gcaaccacag cggacgagtc ggcgtaggct accggttcct cgagcaccac   4320
caccaccacc actga                                                    4335
```

<210> 151
<211> 1444
<212> PRT
<213> Artificial Sequence

<220>
<223> 961-983

<400> 151

Met Ala Thr Asn Asp Asp Asp Val Lys Lys Ala Ala Thr Val Ala Ile
1 5 10 15

Ala Ala Ala Tyr Asn Asn Gly Gln Glu Ile Asn Gly Phe Lys Ala Gly
20 25 30

Glu Thr Ile Tyr Asp Ile Asp Glu Asp Gly Thr Ile Thr Lys Lys Asp
35 40 45

Ala Thr Ala Ala Asp Val Glu Ala Asp Asp Phe Lys Gly Leu Gly Leu
50 55 60

Lys Lys Val Val Thr Asn Leu Thr Lys Thr Val Asn Glu Asn Lys Gln
65 70 75 80

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Thr Ser Ala Pro Asp Phe Asn Ala Gly Gly Thr
385 390 395 400

Gly Ile Gly Ser Asn Ser Arg Ala Thr Thr Ala Lys Ser Ala Ala Val
405 410 415

Ser Tyr Ala Gly Ile Lys Asn Glu Met Cys Lys Asp Arg Ser Met Leu
420 425 430

Cys Ala Gly Arg Asp Asp Val Ala Val Thr Asp Arg Asp Ala Lys Ile
435 440 445

Asn Ala Pro Pro Pro Asn Leu His Thr Gly Asp Phe Pro Asn Pro Asn
450 455 460

Asp Ala Tyr Lys Asn Leu Ile Asn Leu Lys Pro Ala Ile Glu Ala Gly
465 470 475 480

Tyr Thr Gly Arg Gly Val Glu Val Gly Ile Val Asp Thr Gly Glu Ser
485 490 495

Val Gly Ser Ile Ser Phe Pro Glu Leu Tyr Gly Arg Lys Glu His Gly
500 505 510

Tyr Asn Glu Asn Tyr Lys Asn Tyr Thr Ala Tyr Met Arg Lys Glu Ala
515 520 525

Pro Glu Asp Gly Gly Gly Lys Asp Ile Glu Ala Ser Phe Asp Asp Glu
530 535 540

Ala Val Ile Glu Thr Glu Ala Lys Pro Thr Asp Ile Arg His Val Lys
545 550 555 560

Glu Ile Gly His Ile Asp Leu Val Ser His Ile Ile Gly Gly Arg Ser
565 570 575

Val Asp Gly Arg Pro Ala Gly Gly Ile Ala Pro Asp Ala Thr Leu His
580 585 590

Ile Met Asn Thr Asn Asp Glu Thr Lys Asn Glu Met Met Val Ala Ala
595 600 605

Ile Arg Asn Ala Trp Val Lys Leu Gly Glu Arg Gly Val Arg Ile Val
610 615 620

Asn Asn Ser Phe Gly Thr Thr Ser Arg Ala Gly Thr Ala Asp Leu Phe
625 630 635 640

Gln Ile Ala Asn Ser Glu Glu Gln Tyr Arg Gln Ala Leu Leu Asp Tyr
645 650 655

Ser Gly Gly Asp Lys Thr Asp Glu Gly Ile Arg Leu Met Gln Gln Ser
660 665 670

```
Asp Tyr Gly Asn Leu Ser Tyr His Ile Arg Asn Lys Asn Met Leu Phe
        675                 680                 685

Ile Phe Ser Thr Gly Asn Asp Ala Gln Ala Gln Pro Asn Thr Tyr Ala
    690                 695                 700

Leu Leu Pro Phe Tyr Glu Lys Asp Ala Gln Lys Gly Ile Ile Thr Val
705                 710                 715                 720

Ala Gly Val Asp Arg Ser Gly Glu Lys Phe Lys Arg Glu Met Tyr Gly
                725                 730                 735

Glu Pro Gly Thr Glu Pro Leu Glu Tyr Gly Ser Asn His Cys Gly Ile
            740                 745                 750

Thr Ala Met Trp Cys Leu Ser Ala Pro Tyr Glu Ala Ser Val Arg Phe
        755                 760                 765

Thr Arg Thr Asn Pro Ile Gln Ile Ala Gly Thr Ser Phe Ser Ala Pro
    770                 775                 780

Ile Val Thr Gly Thr Ala Ala Leu Leu Leu Gln Lys Tyr Pro Trp Met
785                 790                 795                 800

Ser Asn Asp Asn Leu Arg Thr Thr Leu Leu Thr Thr Ala Gln Asp Ile
                805                 810                 815

Gly Ala Val Gly Val Asp Ser Lys Phe Gly Trp Gly Leu Leu Asp Ala
            820                 825                 830

Gly Lys Ala Met Asn Gly Pro Ala Ser Phe Pro Phe Gly Asp Phe Thr
        835                 840                 845

Ala Asp Thr Lys Gly Thr Ser Asp Ile Ala Tyr Ser Phe Arg Asn Asp
    850                 855                 860

Ile Ser Gly Thr Gly Gly Leu Ile Lys Lys Gly Gly Ser Gln Leu Gln
865                 870                 875                 880

Leu His Gly Asn Asn Thr Tyr Thr Gly Lys Thr Ile Ile Glu Gly Gly
                885                 890                 895

Ser Leu Val Leu Tyr Gly Asn Asn Lys Ser Asp Met Arg Val Glu Thr
            900                 905                 910

Lys Gly Ala Leu Ile Tyr Asn Gly Ala Ala Ser Gly Gly Ser Leu Asn
        915                 920                 925

Ser Asp Gly Ile Val Tyr Leu Ala Asp Thr Asp Gln Ser Gly Ala Asn
    930                 935                 940

Glu Thr Val His Ile Lys Gly Ser Leu Gln Leu Asp Gly Lys Gly Thr
945                 950                 955                 960

Leu Tyr Thr Arg Leu Gly Lys Leu Leu Lys Val Asp Gly Thr Ala Ile
                965                 970                 975
```

Ile Gly Gly Lys Leu Tyr Met Ser Ala Arg Gly Lys Gly Ala Gly Tyr
980 985 990

Leu Asn Ser Thr Gly Arg Arg Val Pro Phe Leu Ser Ala Ala Lys Ile
995 1000 1005

Gly Gln Asp Tyr Ser Phe Phe Thr Asn Ile Glu Thr Asp Gly Gly Leu
1010 1015 1020

Leu Ala Ser Leu Asp Ser Val Glu Lys Thr Ala Gly Ser Glu Gly Asp
1025 1030 1035 1040

Thr Leu Ser Tyr Tyr Val Arg Arg Gly Asn Ala Ala Arg Thr Ala Ser
1045 1050 1055

Ala Ala Ala His Ser Ala Pro Ala Gly Leu Lys His Ala Val Glu Gln
1060 1065 1070

Gly Gly Ser Asn Leu Glu Asn Leu Met Val Glu Leu Asp Ala Ser Glu
1075 1080 1085

Ser Ser Ala Thr Pro Glu Thr Val Glu Thr Ala Ala Ala Asp Arg Thr
1090 1095 1100

Asp Met Pro Gly Ile Arg Pro Tyr Gly Ala Thr Phe Arg Ala Ala Ala
1105 1110 1115 1120

Ala Val Gln His Ala Asn Ala Ala Asp Gly Val Arg Ile Phe Asn Ser
1125 1130 1135

Leu Ala Ala Thr Val Tyr Ala Asp Ser Thr Ala Ala His Ala Asp Met
1140 1145 1150

Gln Gly Arg Arg Leu Lys Ala Val Ser Asp Gly Leu Asp His Asn Gly
1155 1160 1165

Thr Gly Leu Arg Val Ile Ala Gln Thr Gln Gln Asp Gly Gly Thr Trp
1170 1175 1180

Glu Gln Gly Gly Val Glu Gly Lys Met Arg Gly Ser Thr Gln Thr Val
1185 1190 1195 1200

Gly Ile Ala Ala Lys Thr Gly Glu Asn Thr Thr Ala Ala Ala Thr Leu
1205 1210 1215

Gly Met Gly Arg Ser Thr Trp Ser Glu Asn Ser Ala Asn Ala Lys Thr
1220 1225 1230

Asp Ser Ile Ser Leu Phe Ala Gly Ile Arg His Asp Ala Gly Asp Ile
1235 1240 1245

Gly Tyr Leu Lys Gly Leu Phe Ser Tyr Gly Arg Tyr Lys Asn Ser Ile
1250 1255 1260

Ser Arg Ser Thr Gly Ala Asp Glu His Ala Glu Gly Ser Val Asn Gly
1265 1270 1275 1280

Thr Leu Met Gln Leu Gly Ala Leu Gly Gly Val Asn Val Pro Phe Ala
1285 1290 1295

Ala Thr Gly Asp Leu Thr Val Glu Gly Gly Leu Arg Tyr Asp Leu Leu
1300 1305 1310

Lys Gln Asp Ala Phe Ala Glu Lys Gly Ser Ala Leu Gly Trp Ser Gly
1315 1320 1325

Asn Ser Leu Thr Glu Gly Thr Leu Val Gly Leu Ala Gly Leu Lys Leu
1330 1335 1340

Ser Gln Pro Leu Ser Asp Lys Ala Val Leu Phe Ala Thr Ala Gly Val
1345 1350 1355 1360

Glu Arg Asp Leu Asn Gly Arg Asp Tyr Thr Val Thr Gly Gly Phe Thr
1365 1370 1375

Gly Ala Thr Ala Ala Thr Gly Lys Thr Gly Ala Arg Asn Met Pro His
1380 1385 1390

Thr Arg Leu Val Ala Gly Leu Gly Ala Asp Val Glu Phe Gly Asn Gly
1395 1400 1405

Trp Asn Gly Leu Ala Arg Tyr Ser Tyr Ala Gly Ser Lys Gln Tyr Gly
1410 1415 1420

Asn His Ser Gly Arg Val Gly Val Gly Tyr Arg Phe Leu Glu His His
1425 1430 1435 1440

His His His His

<210> 152
<211> 2256
<212> DNA
<213> Artificial Sequence

<220>
<223> 961c-ORF46.1

<400> 152

```
atggccacaa acgacgacga tgttaaaaaa gctgccactg tggccattgc tgctgcctac     60
aacaatggcc aagaaatcaa cggtttcaaa gctggagaga ccatctacga cattgatgaa    120
gacggcacaa ttaccaaaaa agacgcaact gcagccgatg ttgaagccga cgactttaaa    180
ggtctgggtc tgaaaaaagt cgtgactaac ctgaccaaaa ccgtcaatga aaacaaacaa    240
aacgtcgatg ccaaagtaaa agctgcagaa tctgaaatag aaaagttaac aaccaagtta    300
gcagacactg atgccgcttt agcagatact gatgccgctc tggatgcaac caccaacgcc    360
ttgaataaat tgggagaaaa tataacgaca tttgctgaag agactaagac aaatatcgta    420
aaaattgatg aaaaattaga agccgtggct gataccgtcg acaagcatgc cgaagcattc    480
aacgatatcg ccgattcatt ggatgaaacc aacactaagg cagacgaagc cgtcaaaacc    540
gccaatgaag ccaaacagac ggccgaagaa accaaacaaa acgtcgatgc caaagtaaaa    600
gctgcagaaa ctgcagcagg caaagccgaa gctgccgctg gcacagctaa tactgcagcc    660
gacaaggccg aagctgtcgc tgcaaaagtt accgacatca aagctgatat cgctacgaac    720
aaagataata ttgctaaaaa agcaaacagt gccgacgtgt acaccagaga agagtctgac    780
agcaaatttg tcagaattga tggtctgaac gctactaccg aaaaattgga cacacgcttg    840
```

```
gcttctgctg aaaaatccat tgccgatcac gatactcgcc tgaacggttt ggataaaaca    900
gtgtcagacc tgcgcaaaga aacccgccaa ggccttgcag aacaagccgc gctctccggt    960
ctgttccaac cttacaacgt gggtggatcc ggaggaggag gatcagattt ggcaaacgat   1020
tcttttatcc ggcaggttct cgaccgtcag catttcgaac ccgacgggaa ataccaccta   1080
ttcggcagca ggggggaact tgccgagcgc agcggccata tcggattggg aaaaatacaa   1140
agccatcagt tgggcaacct gatgattcaa caggcggcca ttaaaggaaa tatcggctac   1200
attgtccgct tttccgatca cgggcacgaa gtccattccc ccttcgacaa ccatgcctca   1260
cattccgatt ctgatgaagc cggtagtccc gttgacggat ttagccttta ccgcatccat   1320
tgggacggat acgaacacca tcccgccgac ggctatgacg ggccacaggg cggcggctat   1380
cccgctccca aaggcgcgag ggatatatac agctacgaca taaaaggcgt tgcccaaaat   1440
atccgcctca acctgaccga caaccgcagc accggacaac ggcttgccga ccgtttccac   1500
aatgccggta gtatgctgac gcaaggagta ggcgacggat tcaaacgcgc cacccgatac   1560
agccccgagc tggacagatc gggcaatgcc gccgaagcct tcaacggcac tgcagatatc   1620
gttaaaaaca tcatcggcgc ggcaggagaa attgtcggcg caggcgatgc cgtgcagggc   1680
ataagcgaag gctcaaacat tgctgtcatg cacggcttgg gtctgctttc caccgaaaac   1740
aagatggcgc gcatcaacga tttggcagat atggcgcaac tcaaagacta tgccgcagca   1800
gccatccgcg attgggcagt ccaaaacccc aatgccgcac aaggcataga agccgtcagc   1860
aatatcttta tggcagccat ccccatcaaa gggattggag ctgttcgggg aaaatacggc   1920
ttgggcggca tcacggcaca tcctatcaag cggtcgcaga tgggcgcgat cgcattgccg   1980
aaagggaaat ccgccgtcag cgacaatttt gccgatgcgg catacgccaa atacccgtcc   2040
ccttaccatt cccgaaatat ccgttcaaac ttggagcagc gttacggcaa agaaaacatc   2100
acctcctcaa ccgtgccgcc gtcaaacggc aaaaatgtca aactggcaga ccaacgccac   2160
ccgaagacag gcgtaccgtt tgacggtaaa gggtttccga attttgagaa gcacgtgaaa   2220
tatgatacgc tcgagcacca ccaccaccac cactga                             2256
```

<210> 153
<211> 751
<212> PRT
<213> Artificial Sequence

<220>
<223> 961c-ORF46.1

<400> 153

Met Ala Thr Asn Asp Asp Asp Val Lys Lys Ala Ala Thr Val Ala Ile
1 5 10 15

Ala Ala Ala Tyr Asn Asn Gly Gln Glu Ile Asn Gly Phe Lys Ala Gly
20 25 30

Glu Thr Ile Tyr Asp Ile Asp Glu Asp Gly Thr Ile Thr Lys Lys Asp
35 40 45

Ala Thr Ala Ala Asp Val Glu Ala Asp Asp Phe Lys Gly Leu Gly Leu
50 55 60

Lys Lys Val Val Thr Asn Leu Thr Lys Thr Val Asn Glu Asn Lys Gln
65 70 75 80

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Gly Ser Gly Gly Gly Gly Ser Asp
325 330 335

Leu Ala Asn Asp Ser Phe Ile Arg Gln Val Leu Asp Arg Gln His Phe
340 345 350

Glu Pro Asp Gly Lys Tyr His Leu Phe Gly Ser Arg Gly Glu Leu Ala
355 360 365

Glu Arg Ser Gly His Ile Gly Leu Gly Lys Ile Gln Ser His Gln Leu
370 375 380

Gly Asn Leu Met Ile Gln Gln Ala Ala Ile Lys Gly Asn Ile Gly Tyr
385 390 395 400

Ile Val Arg Phe Ser Asp His Gly His Glu Val His Ser Pro Phe Asp
405 410 415

Asn His Ala Ser His Ser Asp Ser Asp Glu Ala Gly Ser Pro Val Asp
420 425 430

Gly Phe Ser Leu Tyr Arg Ile His Trp Asp Gly Tyr Glu His His Pro
435 440 445

Ala Asp Gly Tyr Asp Gly Pro Gln Gly Gly Gly Tyr Pro Ala Pro Lys
450 455 460

Gly Ala Arg Asp Ile Tyr Ser Tyr Asp Ile Lys Gly Val Ala Gln Asn
465 470 475 480

Ile Arg Leu Asn Leu Thr Asp Asn Arg Ser Thr Gly Gln Arg Leu Ala
485 490 495

Asp Arg Phe His Asn Ala Gly Ser Met Leu Thr Gln Gly Val Gly Asp
500 505 510

Gly Phe Lys Arg Ala Thr Arg Tyr Ser Pro Glu Leu Asp Arg Ser Gly
515 520 525

Asn Ala Ala Glu Ala Phe Asn Gly Thr Ala Asp Ile Val Lys Asn Ile
530 535 540

Ile Gly Ala Ala Gly Glu Ile Val Gly Ala Gly Asp Ala Val Gln Gly
545 550 555 560

Ile Ser Glu Gly Ser Asn Ile Ala Val Met His Gly Leu Gly Leu Leu
565 570 575

Ser Thr Glu Asn Lys Met Ala Arg Ile Asn Asp Leu Ala Asp Met Ala
580 585 590

Gln Leu Lys Asp Tyr Ala Ala Ala Ala Ile Arg Asp Trp Ala Val Gln
595 600 605

Asn Pro Asn Ala Ala Gln Gly Ile Glu Ala Val Ser Asn Ile Phe Met
610 615 620

Ala Ala Ile Pro Ile Lys Gly Ile Gly Ala Val Arg Gly Lys Tyr Gly
625 630 635 640

Leu Gly Gly Ile Thr Ala His Pro Ile Lys Arg Ser Gln Met Gly Ala
645 650 655

Ile Ala Leu Pro Lys Gly Lys Ser Ala Val Ser Asp Asn Phe Ala Asp
660 665 670

Ala Ala Tyr Ala Lys Tyr Pro Ser Pro Tyr His Ser Arg Asn Ile Arg
675 680 685

Ser Asn Leu Glu Gln Arg Tyr Gly Lys Glu Asn Ile Thr Ser Ser Thr
690 695 700

Val Pro Pro Ser Asn Gly Lys Asn Val Lys Leu Ala Asp Gln Arg His
705 710 715 720

Pro Lys Thr Gly Val Pro Phe Asp Gly Lys Gly Phe Pro Asn Phe Glu
725 730 735

Lys His Val Lys Tyr Asp Thr Leu Glu His His His His His His
740 745 750

<210> 154
<211> 1773
<212> DNA
<213> Artificial Sequence

<220>
<223> 961c-741

<400> 154

```
atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtggatcc ggagggggtg gtgtcgccgc cgacatcggt 1020
gcggggcttg ccgatgcact aaccgcaccg ctcgaccata aagacaaagg tttgcagtct 1080
ttgacgctgg atcagtccgt caggaaaaac gagaaactga agctggcggc acaaggtgcg 1140
gaaaaaactt atggaaacgg tgacagcctc aatacgggca aattgaagaa cgacaaggtc 1200
agccgtttcg actttatccg ccaaatcgaa gtggacgggc agctcattac cttggagagt 1260
ggagagttcc aagtatacaa acaaagccat tccgccttaa ccgcctttca gaccgagcaa 1320
atacaagatt cggagcattc cgggaagatg gttgcgaaac gccagttcag aatcggcgac 1380
atagcgggcg aacatacatc ttttgacaag cttcccgaag gcggcagggc gacatatcgc 1440
gggacggcgt tcggttcaga cgatgccggc ggaaaactga cctacaccat agatttcgcc 1500
gccaagcagg gaaacggcaa aatcgaacat ttgaaatcgc cagaactcaa tgtcgacctg 1560
gccgccgccg atatcaagcc ggatggaaaa cgccatgccg tcatcagcgg ttccgtcctt 1620
tacaaccaag ccgagaaagg cagttactcc ctcggtatct ttggcggaaa agcccaggaa 1680
gttgccggca gcgcggaagt gaaaaccgta aacggcatac gccatatcgg ccttgccgcc 1740
aagcaactcg agcaccacca ccaccaccac tga 1773
```

<210> 155
<211> 590
<212> PRT
<213> Artificial Sequence

<220>
<223> 961c-741

<400> 155

Met Ala Thr Asn Asp Asp Asp Val Lys Lys Ala Ala Thr Val Ala Ile
1 5 10 15

Ala Ala Ala Tyr Asn Asn Gly Gln Glu Ile Asn Gly Phe Lys Ala Gly

20 25 30

Glu Thr Ile Tyr Asp Ile Asp Glu Asp Gly Thr Ile Thr Lys Lys Asp
35 40 45

Ala Thr Ala Ala Asp Val Glu Ala Asp Asp Phe Lys Gly Leu Gly Leu
50 55 60

Lys Lys Val Val Thr Asn Leu Thr Lys Thr Val Asn Glu Asn Lys Gln
65 70 75 80

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Gly Ser Gly Gly Gly Gly Val Ala

325 330 335

Ala Asp Ile Gly Ala Gly Leu Ala Asp Ala Leu Thr Ala Pro Leu Asp
340 345 350

His Lys Asp Lys Gly Leu Gln Ser Leu Thr Leu Asp Gln Ser Val Arg
355 360 365

Lys Asn Glu Lys Leu Lys Leu Ala Ala Gln Gly Ala Glu Lys Thr Tyr
370 375 380

Gly Asn Gly Asp Ser Leu Asn Thr Gly Lys Leu Lys Asn Asp Lys Val
385 390 395 400

Ser Arg Phe Asp Phe Ile Arg Gln Ile Glu Val Asp Gly Gln Leu Ile
405 410 415

Thr Leu Glu Ser Gly Glu Phe Gln Val Tyr Lys Gln Ser His Ser Ala
420 425 430

Leu Thr Ala Phe Gln Thr Glu Gln Ile Gln Asp Ser Glu His Ser Gly
435 440 445

Lys Met Val Ala Lys Arg Gln Phe Arg Ile Gly Asp Ile Ala Gly Glu
450 455 460

His Thr Ser Phe Asp Lys Leu Pro Glu Gly Gly Arg Ala Thr Tyr Arg
465 470 475 480

Gly Thr Ala Phe Gly Ser Asp Asp Ala Gly Gly Lys Leu Thr Tyr Thr
485 490 495

Ile Asp Phe Ala Ala Lys Gln Gly Asn Gly Lys Ile Glu His Leu Lys
500 505 510

Ser Pro Glu Leu Asn Val Asp Leu Ala Ala Ala Asp Ile Lys Pro Asp
515 520 525

Gly Lys Arg His Ala Val Ile Ser Gly Ser Val Leu Tyr Asn Gln Ala
530 535 540

Glu Lys Gly Ser Tyr Ser Leu Gly Ile Phe Gly Gly Lys Ala Gln Glu
545 550 555 560

Val Ala Gly Ser Ala Glu Val Lys Thr Val Asn Gly Ile Arg His Ile
565 570 575

Gly Leu Ala Ala Lys Gln Leu Glu His His His His His His
580 585 590

<210> 156
<211> 4170
<212> DNA
<213> Artificial Sequence

<220>

<223> 961c-983

<400> 156

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtggatcc ggcggaggcg gcacttctgc gcccgacttc 1020
aatgcaggcg gtaccggtat cggcagcaac agcagagcaa caacagcgaa atcagcagca 1080
gtatcttacg ccggtatcaa gaacgaaatg tgcaaagaca gaagcatgct ctgtgccggt 1140
cgggatgacg ttgcggttac agacagggat gccaaaatca atgccccccc cccgaatctg 1200
cataccggag actttccaaa cccaaatgac gcatacaaga atttgatcaa cctcaaacct 1260
gcaattgaag caggctatac aggacgcggg gtagaggtag gtatcgtcga cacaggcgaa 1320
tccgtcggca gcatatcctt tcccgaactg tatggcagaa aagaacacgg ctataacgaa 1380
aattacaaaa actatacggc gtatatgcgg aaggaagcgc ctgaagacgg aggcggtaaa 1440
gacattgaag cttctttcga cgatgaggcc gttatagaga ctgaagcaaa gccgacggat 1500
atccgccacg taaaagaaat cggacacatc gatttggtct cccatattat tggcgggcgt 1560
tccgtggacg gcagacctgc aggcggtatt gcgcccgatg cgacgctaca cataatgaat 1620
acgaatgatg aaaccaagaa cgaaatgatg gttgcagcca tccgcaatgc atgggtcaag 1680
ctgggcgaac gtggcgtgcg catcgtcaat aacagttttg gaacaacatc gagggcaggc 1740
actgccgacc ttttccaaat agccaattcg gaggagcagt accgccaagc gttgctcgac 1800
tattccggcg gtgataaaac agacgagggt atccgcctga tgcaacagag cgattacggc 1860
aacctgtcct accacatccg taataaaaac atgcttttca tcttttcgac aggcaatgac 1920
gcacaagctc agcccaacac atatgcccta ttgccatttt atgaaaaaga cgctcaaaaa 1980
ggcattatca cagtcgcagg cgtagaccgc agtggagaaa agttcaaacg ggaaatgtat 2040
ggagaaccgg gtacagaacc gcttgagtat ggctccaacc attgcggaat tactgccatg 2100
tggtgcctgt cggcacccta tgaagcaagc gtccgtttca cccgtacaaa cccgattcaa 2160
attgccggaa catccttttc cgcacccatc gtaaccggca cggcggctct gctgctgcag 2220
aaatacccgt ggatgagcaa cgacaacctg cgtaccacgt tgctgacgac ggctcaggac 2280
atcggtgcag tcggcgtgga cagcaagttc ggctggggac tgctggatgc gggtaaggcc 2340
atgaacggac ccgcgtcctt tccgttcggc gactttaccg ccgatacgaa aggtacatcc 2400
gatattgcct actccttccg taacgacatt tcaggcacgg gcggcctgat caaaaaaggc 2460
ggcagccaac tgcaactgca cggcaacaac acctatacgg gcaaaaccat tatcgaaggc 2520
ggttcgctgg tgttgtacgg caacaacaaa tcggatatgc gcgtcgaaac caaaggtgcg 2580
ctgatttata acggggcggc atccggcggc agcctgaaca gcgacggcat tgtctatctg 2640
gcagataccg accaatccgg cgcaaacgaa accgtacaca tcaaaggcag tctgcagctg 2700
gacggcaaag gtacgctgta cacacgtttg ggcaaactgc tgaaagtgga cggtacggcg 2760
attatcggcg gcaagctgta catgtcggca cgcggcaagg ggcaggcta tctcaacagt 2820
accggacgac gtgttcccttt cctgagtgcc gccaaaatcg ggcaggatta ttctttcttc 2880
acaaacatcg aaaccgacgg cggcctgctg gcttccctcg acagcgtcga aaaaacagcg 2940
ggcagtgaag gcgacacgct gtcctattat gtccgtcgcg gcaatgcggc acggactgct 3000
tcggcagcgg cacattccgc gcccgccggt ctgaaacacg ccgtagaaca gggcggcagc 3060
aatctggaaa acctgatggt cgaactggat gcctccgaat catccgcaac acccgagacg 3120
gttgaaactg cggcagccga ccgcacagat atgccgggca tccgccccta cggcgcaact 3180
ttccgcgcag cggcagccgt acagcatgcg aatgccgccg acggtgtacg catcttcaac 3240
agtctcgccg ctaccgtcta tgccgacagt accgccgccc atgccgatat gcagggacgc 3300
```

```
cgcctgaaag ccgtatcgga cgggttggac cacaacggca cgggtctgcg cgtcatcgcg   3360
caaacccaac aggacggtgg aacgtgggaa cagggcggtg ttgaaggcaa aatgcgcggc   3420
agtacccaaa ccgtcggcat tgccgcgaaa accggcgaaa atacgacagc agccgccaca   3480
ctgggcatgg gacgcagcac atggagcgaa aacagtgcaa atgcaaaaac cgacagcatt   3540
agtctgtttg caggcatacg gcacgatgcg ggcgatatcg gctatctcaa aggcctgttc   3600
tcctacggac gctacaaaaa cagcatcagc cgcagcaccg gtgcggacga acatgcggaa   3660
ggcagcgtca acggcacgct gatgcagctg ggcgcactgg gcggtgtcaa cgttccgttt   3720
gccgcaacgg gagatttgac ggtcgaaggc ggtctgcgct acgacctgct caaacaggat   3780
gcattcgccg aaaaaggcag tgctttgggc tggagcggca acagcctcac tgaaggcacg   3840
ctggtcggac tcgcgggtct gaagctgtcg caacccttga gcgataaagc cgtcctgttt   3900
gcaacggcgg gcgtggaacg cgacctgaac ggacgcgact acacggtaac gggcggcttt   3960
accggcgcga ctgcagcaac cggcaagacg gggcacgca atatgccgca cacccgtctg   4020
gttgccggcc tgggcgcgga tgtcgaattc ggcaacggct ggaacggctt ggcacgttac   4080
agctacgccg gttccaaaca gtacggcaac cacagcggac gagtcggcgt aggctaccgg   4140
ttcctcgagc accaccacca ccaccactga                                    4170
```

<210> 157
<211> 1389
<212> PRT
<213> Artificial Sequence

<220>
<223> 961c-983

<400> 157

Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Gly Ser Gly Gly Gly Gly Thr Ser
325 330 335

Ala Pro Asp Phe Asn Ala Gly Gly Thr Gly Ile Gly Ser Asn Ser Arg
340 345 350

Ala Thr Thr Ala Lys Ser Ala Ala Val Ser Tyr Ala Gly Ile Lys Asn
355 360 365

Glu Met Cys Lys Asp Arg Ser Met Leu Cys Ala Gly Arg Asp Asp Val
370 375 380

Ala Val Thr Asp Arg Asp Ala Lys Ile Asn Ala Pro Pro Pro Asn Leu
385 390 395 400

His Thr Gly Asp Phe Pro Asn Pro Asn Asp Ala Tyr Lys Asn Leu Ile
405 410 415

Asn Leu Lys Pro Ala Ile Glu Ala Gly Tyr Thr Gly Arg Gly Val Glu
420 425 430

Val Gly Ile Val Asp Thr Gly Glu Ser Val Gly Ser Ile Ser Phe Pro
435 440 445

Glu Leu Tyr Gly Arg Lys Glu His Gly Tyr Asn Glu Asn Tyr Lys Asn
450 455 460

Tyr Thr Ala Tyr Met Arg Lys Glu Ala Pro Glu Asp Gly Gly Gly Lys
465 470 475 480

Asp Ile Glu Ala Ser Phe Asp Asp Glu Ala Val Ile Glu Thr Glu Ala
485 490 495

Lys Pro Thr Asp Ile Arg His Val Lys Glu Ile Gly His Ile Asp Leu
500 505 510

Val Ser His Ile Ile Gly Gly Arg Ser Val Asp Gly Arg Pro Ala Gly
515 520 525

Gly Ile Ala Pro Asp Ala Thr Leu His Ile Met Asn Thr Asn Asp Glu
530 535 540

Thr Lys Asn Glu Met Met Val Ala Ala Ile Arg Asn Ala Trp Val Lys
545 550 555 560

Leu Gly Glu Arg Gly Val Arg Ile Val Asn Asn Ser Phe Gly Thr Thr
565 570 575

Ser Arg Ala Gly Thr Ala Asp Leu Phe Gln Ile Ala Asn Ser Glu Glu
580 585 590

Gln Tyr Arg Gln Ala Leu Leu Asp Tyr Ser Gly Gly Asp Lys Thr Asp
595 600 605

Glu Gly Ile Arg Leu Met Gln Gln Ser Asp Tyr Gly Asn Leu Ser Tyr
610 615 620

His Ile Arg Asn Lys Asn Met Leu Phe Ile Phe Ser Thr Gly Asn Asp
625 630 635 640

Ala Gln Ala Gln Pro Asn Thr Tyr Ala Leu Leu Pro Phe Tyr Glu Lys
645 650 655

Asp Ala Gln Lys Gly Ile Ile Thr Val Ala Gly Val Asp Arg Ser Gly
660 665 670

Glu Lys Phe Lys Arg Glu Met Tyr Gly Glu Pro Gly Thr Glu Pro Leu
675 680 685

Glu Tyr Gly Ser Asn His Cys Gly Ile Thr Ala Met Trp Cys Leu Ser
690 695 700

Ala Pro Tyr Glu Ala Ser Val Arg Phe Thr Arg Thr Asn Pro Ile Gln
705 710 715 720

Ile Ala Gly Thr Ser Phe Ser Ala Pro Ile Val Thr Gly Thr Ala Ala
725 730 735

Leu Leu Leu Gln Lys Tyr Pro Trp Met Ser Asn Asp Asn Leu Arg Thr
740 745 750

Thr Leu Leu Thr Thr Ala Gln Asp Ile Gly Ala Val Gly Val Asp Ser
755 760 765

Lys Phe Gly Trp Gly Leu Leu Asp Ala Gly Lys Ala Met Asn Gly Pro
770 775 780

Ala Ser Phe Pro Phe Gly Asp Phe Thr Ala Asp Thr Lys Gly Thr Ser
785 790 795 800

Asp Ile Ala Tyr Ser Phe Arg Asn Asp Ile Ser Gly Thr Gly Gly Leu
805 810 815

Ile Lys Lys Gly Gly Ser Gln Leu Gln Leu His Gly Asn Asn Thr Tyr
820 825 830

Thr Gly Lys Thr Ile Ile Glu Gly Gly Ser Leu Val Leu Tyr Gly Asn
835 840 845

Asn Lys Ser Asp Met Arg Val Glu Thr Lys Gly Ala Leu Ile Tyr Asn
850 855 860

Gly Ala Ala Ser Gly Gly Ser Leu Asn Ser Asp Gly Ile Val Tyr Leu
865 870 875 880

Ala Asp Thr Asp Gln Ser Gly Ala Asn Glu Thr Val His Ile Lys Gly
885 890 895

Ser Leu Gln Leu Asp Gly Lys Gly Thr Leu Tyr Thr Arg Leu Gly Lys
900 905 910

Leu Leu Lys Val Asp Gly Thr Ala Ile Ile Gly Gly Lys Leu Tyr Met
915 920 925

Ser Ala Arg Gly Lys Gly Ala Gly Tyr Leu Asn Ser Thr Gly Arg Arg
930 935 940

Val Pro Phe Leu Ser Ala Ala Lys Ile Gly Gln Asp Tyr Ser Phe Phe
945 950 955 960

Thr Asn Ile Glu Thr Asp Gly Gly Leu Leu Ala Ser Leu Asp Ser Val
965 970 975

Glu Lys Thr Ala Gly Ser Glu Gly Asp Thr Leu Ser Tyr Tyr Val Arg
980 985 990

Arg Gly Asn Ala Ala Arg Thr Ala Ser Ala Ala Ala His Ser Ala Pro
995 1000 1005

Ala Gly Leu Lys His Ala Val Glu Gln Gly Gly Ser Asn Leu Glu Asn
1010 1015 1020

Leu Met Val Glu Leu Asp Ala Ser Glu Ser Ser Ala Thr Pro Glu Thr
1025 1030 1035 1040

Val Glu Thr Ala Ala Ala Asp Arg Thr Asp Met Pro Gly Ile Arg Pro
1045 1050 1055

Tyr Gly Ala Thr Phe Arg Ala Ala Ala Ala Val Gln His Ala Asn Ala
1060 1065 1070

Ala Asp Gly Val Arg Ile Phe Asn Ser Leu Ala Ala Thr Val Tyr Ala
1075 1080 1085

Asp Ser Thr Ala Ala His Ala Asp Met Gln Gly Arg Arg Leu Lys Ala
1090 1095 1100

Val Ser Asp Gly Leu Asp His Asn Gly Thr Gly Leu Arg Val Ile Ala
1105 1110 1115 1120

Gln Thr Gln Gln Asp Gly Gly Thr Trp Glu Gln Gly Gly Val Glu Gly
1125 1130 1135

Lys Met Arg Gly Ser Thr Gln Thr Val Gly Ile Ala Ala Lys Thr Gly
1140 1145 1150

Glu Asn Thr Thr Ala Ala Ala Thr Leu Gly Met Gly Arg Ser Thr Trp
1155 1160 1165

Ser Glu Asn Ser Ala Asn Ala Lys Thr Asp Ser Ile Ser Leu Phe Ala
1170 1175 1180

Gly Ile Arg His Asp Ala Gly Asp Ile Gly Tyr Leu Lys Gly Leu Phe
1185 1190 1195 1200

Ser Tyr Gly Arg Tyr Lys Asn Ser Ile Ser Arg Ser Thr Gly Ala Asp
1205 1210 1215

Glu His Ala Glu Gly Ser Val Asn Gly Thr Leu Met Gln Leu Gly Ala
1220 1225 1230

Leu Gly Gly Val Asn Val Pro Phe Ala Ala Thr Gly Asp Leu Thr Val
1235 1240 1245

Glu Gly Gly Leu Arg Tyr Asp Leu Leu Lys Gln Asp Ala Phe Ala Glu
1250 1255 1260

Lys Gly Ser Ala Leu Gly Trp Ser Gly Asn Ser Leu Thr Glu Gly Thr
1265 1270 1275 1280

Leu Val Gly Leu Ala Gly Leu Lys Leu Ser Gln Pro Leu Ser Asp Lys
1285 1290 1295

Ala Val Leu Phe Ala Thr Ala Gly Val Glu Arg Asp Leu Asn Gly Arg
1300 1305 1310

Asp Tyr Thr Val Thr Gly Gly Phe Thr Gly Ala Thr Ala Ala Thr Gly
1315 1320 1325

Lys Thr Gly Ala Arg Asn Met Pro His Thr Arg Leu Val Ala Gly Leu
1330 1335 1340

Gly Ala Asp Val Glu Phe Gly Asn Gly Trp Asn Gly Leu Ala Arg Tyr
1345 1350 1355 1360

Ser Tyr Ala Gly Ser Lys Gln Tyr Gly Asn His Ser Gly Arg Val Gly
1365 1370 1375

Val Gly Tyr Arg Phe Leu Glu His His His His His His
1380 1385

<210> 158
<211> 2304
<212> DNA
<213> Artificial Sequence

<220>
<223> 961cL-ORF46.1

<400> 158

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420
aacgccttga ataaattggg agaaaatata acgacatttg ctgaagagac taagacaaat  480
atcgtaaaaa ttgatgaaaa attagaagcc gtggctgata ccgtcgacaa gcatgccgaa  540
gcattcaacg atatcgccga ttcattggat gaaaccaaca ctaaggcaga cgaagccgtc  600
aaaaccgcca atgaagccaa acagacggcc gaagaaacca aacaaaacgt cgatgccaaa  660
gtaaaagctg cagaaactgc agcaggcaaa gccgaagctg ccgctggcac agctaatact  720
gcagccgaca aggccgaagc tgtcgctgca aaagttaccg acatcaaagc tgatatcgct  780
acgaacaaag ataatattgc taaaaaagca aacagtgccg acgtgtacac cagagaagag  840
tctgacagca aatttgtcag aattgatggt ctgaacgcta ctaccgaaaa attggacaca  900
cgcttggctt ctgctgaaaa atccattgcc gatcacgata ctcgcctgaa cggtttggat  960
aaaacagtgt cagacctgcg caaagaaacc cgccaaggcc ttgcagaaca agccgcgctc 1020
tccggtctgt tccaacctta caacgtgggt ggatccggag gaggaggatc agatttggca 1080
aacgattctt ttatccggca ggttctcgac cgtcagcatt tcgaacccga cgggaaatac 1140
cacctattcg gcagcagggg ggaacttgcc gagcgcagcg gccatatcgg attgggaaaa 1200
atacaaagcc atcagttggg caacctgatg attcaacagg cggccattaa aggaaatatc 1260
ggctacattg tccgcttttc cgatcacggg cacgaagtcc attcccctt cgacaaccat 1320
gcctcacatt ccgattctga tgaagccggt agtcccgttg acggatttag cctttaccgc 1380
atccattggg acggatacga acaccatccc gccgacggct atgacgggcc acagggcggc 1440
ggctatcccg ctcccaaagg cgcgagggat atatacagct acgacataaa aggcgttgcc 1500
caaaatatcc gcctcaacct gaccgacaac cgcagcaccg gacaacggct tgccgaccgt 1560
ttccacaatg ccggtagtat gctgacgcaa ggagtaggcg acggattcaa acgcgccacc 1620
cgatacagcc ccgagctgga cagatcgggc aatgccgccg aagccttcaa cggcactgca 1680
gatatcgtta aaaacatcat cggcgcggca ggagaaattg tcggcgcagg cgatgccgtg 1740
cagggcataa gcgaaggctc aaacattgct gtcatgcacg gcttgggtct gctttccacc 1800
gaaaacaaga tggcgcgcat caacgatttg gcagatatgg cgcaactcaa agactatgcc 1860
gcagcagcca tccgcgattg ggcagtccaa aaccccaatg ccgcacaagg catagaagcc 1920
gtcagcaata tctttatggc agccatcccc atcaaaggga ttggagctgt tcggggaaaa 1980
tacggcttgg gcggcatcac ggcacatcct atcaagcggt cgcagatggg cgcgatcgca 2040
ttgccgaaag ggaaatccgc cgtcagcgac aattttgccg atgcggcata cgccaaatac 2100
ccgtcccctt accattcccg aaatatccgt tcaaacttgg agcagcgtta cggcaaagaa 2160
aacatcacct cctcaaccgt gccgccgtca aacggcaaaa atgtcaaact ggcagaccaa 2220
cgccacccga agacaggcgt accgtttgac ggtaaagggt ttccgaattt tgagaagcac 2280
gtgaaatatg atacgtaact cgag                                        2304
```

<210> 159
<211> 765
<212> PRT
<213> Artificial Sequence

<220>
<223> 961cL-ORF46.1

<400> 159

Met Lys His Phe Pro Ser Lys Val Leu Thr Thr Ala Ile Leu Ala Thr
1 5 10 15

Phe Cys Ser Gly Ala Leu Ala Ala Thr Asn Asp Asp Asp Val Lys Lys
20 25 30

Ala Ala Thr Val Ala Ile Ala Ala Ala Tyr Asn Asn Gly Gln Glu Ile
35 40 45

Asn Gly Phe Lys Ala Gly Glu Thr Ile Tyr Asp Ile Asp Glu Asp Gly
50 55 60

Thr Ile Thr Lys Lys Asp Ala Thr Ala Ala Asp Val Glu Ala Asp Asp
65 70 75 80

Phe Lys Gly Leu Gly Leu Lys Lys Val Val Thr Asn Leu Thr Lys Thr
85 90 95

Val Asn Glu Asn Lys Gln Asn Val Asp Ala Lys Val Lys Ala Ala Glu
100 105 110

Ser Glu Ile Glu Lys Leu Thr Thr Lys Leu Ala Asp Thr Asp Ala Ala
115 120 125

Leu Ala Asp Thr Asp Ala Ala Leu Asp Ala Thr Thr Asn Ala Leu Asn
130 135 140

Lys Leu Gly Glu Asn Ile Thr Thr Phe Ala Glu Glu Thr Lys Thr Asn
145 150 155 160

Ile Val Lys Ile Asp Glu Lys Leu Glu Ala Val Ala Asp Thr Val Asp
165 170 175

Lys His Ala Glu Ala Phe Asn Asp Ile Ala Asp Ser Leu Asp Glu Thr
180 185 190

Asn Thr Lys Ala Asp Glu Ala Val Lys Thr Ala Asn Glu Ala Lys Gln
195 200 205

Thr Ala Glu Glu Thr Lys Gln Asn Val Asp Ala Lys Val Lys Ala Ala
210 215 220

Glu Thr Ala Ala Gly Lys Ala Glu Ala Ala Ala Gly Thr Ala Asn Thr
225 230 235 240

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser

290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Ser Asp Leu Ala Asn Asp Ser Phe Ile Arg Gln Val
355 360 365

Leu Asp Arg Gln His Phe Glu Pro Asp Gly Lys Tyr His Leu Phe Gly
370 375 380

Ser Arg Gly Glu Leu Ala Glu Arg Ser Gly His Ile Gly Leu Gly Lys
385 390 395 400

Ile Gln Ser His Gln Leu Gly Asn Leu Met Ile Gln Gln Ala Ala Ile
405 410 415

Lys Gly Asn Ile Gly Tyr Ile Val Arg Phe Ser Asp His Gly His Glu
420 425 430

Val His Ser Pro Phe Asp Asn His Ala Ser His Ser Asp Ser Asp Glu
435 440 445

Ala Gly Ser Pro Val Asp Gly Phe Ser Leu Tyr Arg Ile His Trp Asp
450 455 460

Gly Tyr Glu His His Pro Ala Asp Gly Tyr Asp Gly Pro Gln Gly Gly
465 470 475 480

Gly Tyr Pro Ala Pro Lys Gly Ala Arg Asp Ile Tyr Ser Tyr Asp Ile
485 490 495

Lys Gly Val Ala Gln Asn Ile Arg Leu Asn Leu Thr Asp Asn Arg Ser
500 505 510

Thr Gly Gln Arg Leu Ala Asp Arg Phe His Asn Ala Gly Ser Met Leu
515 520 525

Thr Gln Gly Val Gly Asp Gly Phe Lys Arg Ala Thr Arg Tyr Ser Pro
530 535 540

Glu Leu Asp Arg Ser Gly Asn Ala Ala Glu Ala Phe Asn Gly Thr Ala
545 550 555 560

Asp Ile Val Lys Asn Ile Ile Gly Ala Ala Gly Glu Ile Val Gly Ala
565 570 575

Gly Asp Ala Val Gln Gly Ile Ser Glu Gly Ser Asn Ile Ala Val Met
580 585 590

His Gly Leu Gly Leu Leu Ser Thr Glu Asn Lys Met Ala Arg Ile Asn

595 600 605

Asp Leu Ala Asp Met Ala Gln Leu Lys Asp Tyr Ala Ala Ala Ala Ile
610 615 620

Arg Asp Trp Ala Val Gln Asn Pro Asn Ala Ala Gln Gly Ile Glu Ala
625 630 635 640

Val Ser Asn Ile Phe Met Ala Ala Ile Pro Ile Lys Gly Ile Gly Ala
645 650 655

Val Arg Gly Lys Tyr Gly Leu Gly Gly Ile Thr Ala His Pro Ile Lys
660 665 670

Arg Ser Gln Met Gly Ala Ile Ala Leu Pro Lys Gly Lys Ser Ala Val
675 680 685

Ser Asp Asn Phe Ala Asp Ala Ala Tyr Ala Lys Tyr Pro Ser Pro Tyr
690 695 700

His Ser Arg Asn Ile Arg Ser Asn Leu Glu Gln Arg Tyr Gly Lys Glu
705 710 715 720

Asn Ile Thr Ser Ser Thr Val Pro Pro Ser Asn Gly Lys Asn Val Lys
725 730 735

Leu Ala Asp Gln Arg His Pro Lys Thr Gly Val Pro Phe Asp Gly Lys
740 745 750

Gly Phe Pro Asn Phe Glu Lys His Val Lys Tyr Asp Thr
755 760 765

<210> 160
<211> 1839
<212> DNA
<213> Artificial Sequence

<220>
<223> 961cL-741

<400> 160

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420
aacgccttga ataaattggg agaaaatata acgacatttg ctgaagagac taagacaaat  480
atcgtaaaaa ttgatgaaaa attagaagcc gtggctgata ccgtcgacaa gcatgccgaa  540
gcattcaacg atatcgccga ttcattggat gaaaccaaca ctaaggcaga cgaagccgtc  600
aaaaccgcca atgaagccaa acagacggcc gaagaaacca aacaaaacgt cgatgccaaa  660
gtaaaagctg cagaaactgc agcaggcaaa gccgaagctg ccgctggcac agctaatact  720
gcagccgaca aggccgaagc tgtcgctgca aaagttaccg acatcaaagc tgatatcgct  780
acgaacaaag ataatattgc taaaaaagca aacagtgccg acgtgtacac cagagaagag  840
tctgacagca aatttgtcag aattgatggt ctgaacgcta ctaccgaaaa attggacaca  900
cgcttggctt ctgctgaaaa atccattgcc gatcacgata ctcgcctgaa cggtttggat  960

aaaacagtgt cagacctgcg caaagaaacc cgccaaggcc ttgcagaaca agccgcgctc 1020
tccggtctgt tccaacctta caacgtgggt ggatccggag ggggtggtgt cgccgccgac 1080
atcggtgcgg ggcttgccga tgcactaacc gcaccgctcg accataaaga caaaggtttg 1140
cagtctttga cgctggatca gtccgtcagg aaaaacgaga aactgaagct ggcggcacaa 1200
ggtgcggaaa aaacttatgg aaacggtgac agcctcaata cgggcaaatt gaagaacgac 1260
aaggtcagcc gtttcgactt tatccgccaa atcgaagtgg acgggcagct cattaccttg 1320
gagagtggag agttccaagt atacaaacaa agccattccg ccttaaccgc ctttcagacc 1380
gagcaaatac aagattcgga gcattccggg aagatggttg cgaaacgcca gttcagaatc 1440
ggcgacatag cgggcgaaca tacatctttt gacaagcttc ccgaaggcgg cagggcgaca 1500
tatcgcggga cggcgttcgg ttcagacgat gccggcggaa aactgaccta caccatagat 1560
ttcgccgcca agcagggaaa cggcaaaatc gaacatttga aatcgccaga actcaatgtc 1620
gacctggccg ccgccgatat caagccggat ggaaaacgcc atgccgtcat cagcggttcc 1680
gtcctttaca accaagccga gaaaggcagt tactccctcg gtatctttgg cggaaaagcc 1740
caggaagttg ccggcagcgc ggaagtgaaa accgtaaacg gcatacgcca tatcggcctt 1800
gccgccaagc aactcgagca ccaccaccac caccactga                        1839
```

<210> 161
<211> 612
<212> PRT
<213> Artificial Sequence

<220>
<223> 961cL-741

<400> 161

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45

Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Ala Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175
```

Lys His Ala Glu Ala Phe Asn Asp Ile Ala Asp Ser Leu Asp Glu Thr
180 185 190

Asn Thr Lys Ala Asp Glu Ala Val Lys Thr Ala Asn Glu Ala Lys Gln
195 200 205

Thr Ala Glu Glu Thr Lys Gln Asn Val Asp Ala Lys Val Lys Ala Ala
210 215 220

Glu Thr Ala Ala Gly Lys Ala Glu Ala Ala Ala Gly Thr Ala Asn Thr
225 230 235 240

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Val Ala Ala Asp Ile Gly Ala Gly Leu Ala Asp Ala
355 360 365

Leu Thr Ala Pro Leu Asp His Lys Asp Lys Gly Leu Gln Ser Leu Thr
370 375 380

Leu Asp Gln Ser Val Arg Lys Asn Glu Lys Leu Lys Leu Ala Ala Gln
385 390 395 400

Gly Ala Glu Lys Thr Tyr Gly Asn Gly Asp Ser Leu Asn Thr Gly Lys
405 410 415

Leu Lys Asn Asp Lys Val Ser Arg Phe Asp Phe Ile Arg Gln Ile Glu
420 425 430

Val Asp Gly Gln Leu Ile Thr Leu Glu Ser Gly Glu Phe Gln Val Tyr
435 440 445

Lys Gln Ser His Ser Ala Leu Thr Ala Phe Gln Thr Glu Gln Ile Gln
450 455 460

Asp Ser Glu His Ser Gly Lys Met Val Ala Lys Arg Gln Phe Arg Ile
465 470 475 480

Gly Asp Ile Ala Gly Glu His Thr Ser Phe Asp Lys Leu Pro Glu Gly
485 490 495

Gly Arg Ala Thr Tyr Arg Gly Thr Ala Phe Gly Ser Asp Asp Ala Gly
500 505 510

Gly Lys Leu Thr Tyr Thr Ile Asp Phe Ala Ala Lys Gln Gly Asn Gly
515 520 525

Lys Ile Glu His Leu Lys Ser Pro Glu Leu Asn Val Asp Leu Ala Ala
530 535 540

Ala Asp Ile Lys Pro Asp Gly Lys Arg His Ala Val Ile Ser Gly Ser
545 550 555 560

Val Leu Tyr Asn Gln Ala Glu Lys Gly Ser Tyr Ser Leu Gly Ile Phe
565 570 575

Gly Gly Lys Ala Gln Glu Val Ala Gly Ser Ala Glu Val Lys Thr Val
580 585 590

Asn Gly Ile Arg His Ile Gly Leu Ala Ala Lys Gln Leu Glu His His
595 600 605

His His His His
610

<210> 162
<211> 4218
<212> DNA
<213> Artificial Sequence

<220>
<223> 961cL-983

<400> 162

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420
aacgccttga ataaattggg agaaaatata acgacatttg ctgaagagac taagacaaat  480
atcgtaaaaa ttgatgaaaa attagaagcc gtggctgata ccgtcgacaa gcatgccgaa  540
gcattcaacg atatcgccga ttcattggat gaaaccaaca ctaaggcaga cgaagccgtc  600
aaaaccgcca atgaagccaa acagacggcc gaagaaacca aacaaaacgt cgatgccaaa  660
gtaaaagctg cagaaactgc agcaggcaaa gccgaagctg ccgctggcac agctaatact  720
gcagccgaca aggccgaagc tgtcgctgca aaagttaccg acatcaaagc tgatatcgct  780
acgaacaaag ataatattgc taaaaaagca aacagtgccg acgtgtacac cagagaagag  840
tctgacagca aatttgtcag aattgatggt ctgaacgcta ctaccgaaaa attggacaca  900
cgcttggctt ctgctgaaaa atccattgcc gatcacgata ctcgcctgaa cggtttggat  960
aaaacagtgt cagacctgcg caaagaaacc cgccaaggcc ttgcagaaca agccgcgctc 1020
tccggtctgt tccaacctta caacgtgggt ggatccggcg gaggcggcac ttctgcgccc 1080
gacttcaatg caggcggtac cggtatcggc agcaacagca gagcaacaac agcgaaatca 1140
gcagcagtat cttacgccgg tatcaagaac gaaatgtgca aagacagaag catgctctgt 1200
```

```
gccggtcggg atgacgttgc ggttacagac agggatgcca aaatcaatgc cccccccccg   1260
aatctgcata ccggagactt tccaaaccca aatgacgcat acaagaattt gatcaacctc   1320
aaacctgcaa ttgaagcagg ctatacagga cgcggggtag aggtaggtat cgtcgacaca   1380
ggcgaatccg tcggcagcat atcctttccc gaactgtatg gcagaaaaga acacggctat   1440
aacgaaaatt acaaaaacta tacggcgtat atgcggaagg aagcgcctga agacggaggc   1500
ggtaaagaca ttgaagcttc tttcgacgat gaggccgtta tagagactga agcaaagccg   1560
acggatatcc gccacgtaaa agaaatcgga cacatcgatt tggtctccca tattattggc   1620
gggcgttccg tggacggcag acctgcaggc ggtattgcgc ccgatgcgac gctacacata   1680
atgaatacga atgatgaaac caagaacgaa atgatggttg cagccatccg caatgcatgg   1740
gtcaagctgg gcgaacgtgg cgtgcgcatc gtcaataaca gttttggaac aacatcgagg   1800
gcaggcactg ccgacctttt ccaaatagcc aattcggagg agcagtaccg ccaagcgttg   1860
ctcgactatt ccggcggtga taaaacagac gagggtatcc gcctgatgca acagagcgat   1920
tacggcaacc tgtcctacca catccgtaat aaaaacatgc ttttcatctt ttcgacaggc   1980
aatgacgcac aagctcagcc caacacatat gccctattgc cattttatga aaaagacgct   2040
caaaaaggca ttatcacagt cgcaggcgta gaccgcagtg gagaaaagtt caaacgggaa   2100
atgtatggag aaccgggtac agaaccgctt gagtatggct ccaaccattg cggaattact   2160
gccatgtggt gcctgtcggc accctatgaa gcaagcgtcc gtttcacccg tacaaacccg   2220
attcaaattg ccggaacatc cttttccgca cccatcgtaa ccggcacggc ggctctgctg   2280
ctgcagaaat acccgtggat gagcaacgac aacctgcgta ccacgttgct gacgacggct   2340
caggacatcg gtgcagtcgg cgtggacagc aagttcggct ggggactgct ggatgcgggt   2400
aaggccatga acggacccgc gtcctttccg ttcggcgact ttaccgccga tacgaaaggt   2460
acatccgata ttgcctactc cttccgtaac gacatttcag gcacgggcgg cctgatcaaa   2520
aaaggcggca gccaactgca actgcacggc aacaacacct atacgggcaa aaccattatc   2580
gaaggcggtt cgctggtgtt gtacggcaac aacaaatcgg atatgcgcgt cgaaaccaaa   2640
ggtgcgctga tttataacgg ggcggcatcc ggcggcagcc tgaacagcga cggcattgtc   2700
tatctggcag ataccgacca atccggcgca aacgaaaccg tacacatcaa aggcagtctg   2760
cagctggacg gcaaaggtac gctgtacaca cgtttgggca aactgctgaa agtggacggt   2820
acggcgatta tcggcggcaa gctgtacatg tcggcacgcg gcaagggggc aggctatctc   2880
aacagtaccg gacgacgtgt tcccttcctg agtgccgcca aaatcgggca ggattattct   2940
ttcttcacaa acatcgaaac cgacggcggc ctgctggctt ccctcgacag cgtcgaaaaa   3000
acagcgggca gtgaaggcga cacgctgtcc tattatgtcc gtcgcggcaa tgcggcacgg   3060
actgcttcgg cagcggcaca ttccgcgccc gccggtctga aacacgccgt agaacagggc   3120
ggcagcaatc tggaaaacct gatggtcgaa ctggatgcct ccgaatcatc cgcaacaccc   3180
gagacggttg aaactgcggc agccgaccgc acagatatgc cgggcatccg cccctacggc   3240
gcaactttcc gcgcagcggc agccgtacag catgcgaatg ccgccgacgg tgtacgcatc   3300
ttcaacagtc tcgccgctac cgtctatgcc gacagtaccg ccgcccatgc cgatatgcag   3360
ggacgccgcc tgaaagccgt atcggacggg ttggaccaca acggcacggg tctgcgcgtc   3420
atcgcgcaaa cccaacagga cggtggaacg tgggaacagg gcggtgttga aggcaaaatg   3480
cgcggcagta cccaaaccgt cggcattgcc gcgaaaaccg gcgaaaatac gacagcagcc   3540
gccacactgg gcatgggacg cagcacatgg agcgaaaaca gtgcaaatgc aaaaaccgac   3600
agcattagtc tgtttgcagg catacggcac gatgcgggcg atatcggcta tctcaaaggc   3660
ctgttctcct acggacgcta caaaaacagc atcagccgca gcaccggtgc ggacgaacat   3720
gcggaaggca gcgtcaacgg cacgctgatg cagctgggcg cactgggcgg tgtcaacgtt   3780
ccgtttgccg caacgggaga tttgacggtc gaaggcggtc tgcgctacga cctgctcaaa   3840
caggatgcat tcgccgaaaa aggcagtgct ttgggctgga gcggcaacag cctcactgaa   3900
ggcacgctgg tcggactcgc gggtctgaag ctgtcgcaac ccttgagcga taaagccgtc   3960
ctgtttgcaa cggcgggcgt ggaacgcgac ctgaacggac gcgactacac ggtaacgggc   4020
ggctttaccg gcgcgactgc agcaaccggc aagacggggg cacgcaatat gccgcacacc   4080
cgtctggttg ccggcctggg cgcggatgtc gaattcggca acggctggaa cggcttggca   4140
cgttacagct acgccggttc caaacagtac ggcaaccaca gcggacgagt cggcgtaggc   4200
taccggttct gactcgag                                                 4218
```

<210> 163
<211> 1403
<212> PRT
<213> Artificial Sequence

<220>
<223> 961cL-983

<400> 163

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45

Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Ala Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175

Lys His Ala Glu Ala Phe Asn Asp Ile Ala Asp Ser Leu Asp Glu Thr
            180                 185                 190

Asn Thr Lys Ala Asp Glu Ala Val Lys Thr Ala Asn Glu Ala Lys Gln
        195                 200                 205

Thr Ala Glu Glu Thr Lys Gln Asn Val Asp Ala Lys Val Lys Ala Ala
    210                 215                 220

Glu Thr Ala Ala Gly Lys Ala Glu Ala Ala Ala Gly Thr Ala Asn Thr
225                 230                 235                 240

Ala Ala Asp Lys Ala Glu Ala Val Ala Ala Lys Val Thr Asp Ile Lys
                245                 250                 255

Ala Asp Ile Ala Thr Asn Lys Asp Asn Ile Ala Lys Lys Ala Asn Ser
            260                 265                 270

Ala Asp Val Tyr Thr Arg Glu Glu Ser Asp Ser Lys Phe Val Arg Ile
        275                 280                 285
```

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Thr Ser Ala Pro Asp Phe Asn Ala Gly Gly Thr Gly
355 360 365

Ile Gly Ser Asn Ser Arg Ala Thr Thr Ala Lys Ser Ala Ala Val Ser
370 375 380

Tyr Ala Gly Ile Lys Asn Glu Met Cys Lys Asp Arg Ser Met Leu Cys
385 390 395 400

Ala Gly Arg Asp Asp Val Ala Val Thr Asp Arg Asp Ala Lys Ile Asn
405 410 415

Ala Pro Pro Pro Asn Leu His Thr Gly Asp Phe Pro Asn Pro Asn Asp
420 425 430

Ala Tyr Lys Asn Leu Ile Asn Leu Lys Pro Ala Ile Glu Ala Gly Tyr
435 440 445

Thr Gly Arg Gly Val Glu Val Gly Ile Val Asp Thr Gly Glu Ser Val
450 455 460

Gly Ser Ile Ser Phe Pro Glu Leu Tyr Gly Arg Lys Glu His Gly Tyr
465 470 475 480

Asn Glu Asn Tyr Lys Asn Tyr Thr Ala Tyr Met Arg Lys Glu Ala Pro
485 490 495

Glu Asp Gly Gly Gly Lys Asp Ile Glu Ala Ser Phe Asp Asp Glu Ala
500 505 510

Val Ile Glu Thr Glu Ala Lys Pro Thr Asp Ile Arg His Val Lys Glu
515 520 525

Ile Gly His Ile Asp Leu Val Ser His Ile Ile Gly Gly Arg Ser Val
530 535 540

Asp Gly Arg Pro Ala Gly Gly Ile Ala Pro Asp Ala Thr Leu His Ile
545 550 555 560

Met Asn Thr Asn Asp Glu Thr Lys Asn Glu Met Met Val Ala Ala Ile
565 570 575

Arg Asn Ala Trp Val Lys Leu Gly Glu Arg Gly Val Arg Ile Val Asn
580 585 590

Asn Ser Phe Gly Thr Thr Ser Arg Ala Gly Thr Ala Asp Leu Phe Gln
595 600 605

Ile Ala Asn Ser Glu Glu Gln Tyr Arg Gln Ala Leu Leu Asp Tyr Ser
610 615 620

Gly Gly Asp Lys Thr Asp Glu Gly Ile Arg Leu Met Gln Gln Ser Asp
625 630 635 640

Tyr Gly Asn Leu Ser Tyr His Ile Arg Asn Lys Asn Met Leu Phe Ile
645 650 655

Phe Ser Thr Gly Asn Asp Ala Gln Ala Gln Pro Asn Thr Tyr Ala Leu
660 665 670

Leu Pro Phe Tyr Glu Lys Asp Ala Gln Lys Gly Ile Ile Thr Val Ala
675 680 685

Gly Val Asp Arg Ser Gly Glu Lys Phe Lys Arg Glu Met Tyr Gly Glu
690 695 700

Pro Gly Thr Glu Pro Leu Glu Tyr Gly Ser Asn His Cys Gly Ile Thr
705 710 715 720

Ala Met Trp Cys Leu Ser Ala Pro Tyr Glu Ala Ser Val Arg Phe Thr
725 730 735

Arg Thr Asn Pro Ile Gln Ile Ala Gly Thr Ser Phe Ser Ala Pro Ile
740 745 750

Val Thr Gly Thr Ala Ala Leu Leu Leu Gln Lys Tyr Pro Trp Met Ser
755 760 765

Asn Asp Asn Leu Arg Thr Thr Leu Leu Thr Thr Ala Gln Asp Ile Gly
770 775 780

Ala Val Gly Val Asp Ser Lys Phe Gly Trp Gly Leu Leu Asp Ala Gly
785 790 795 800

Lys Ala Met Asn Gly Pro Ala Ser Phe Pro Phe Gly Asp Phe Thr Ala
805 810 815

Asp Thr Lys Gly Thr Ser Asp Ile Ala Tyr Ser Phe Arg Asn Asp Ile
820 825 830

Ser Gly Thr Gly Gly Leu Ile Lys Lys Gly Gly Ser Gln Leu Gln Leu
835 840 845

His Gly Asn Asn Thr Tyr Thr Gly Lys Thr Ile Ile Glu Gly Gly Ser
850 855 860

Leu Val Leu Tyr Gly Asn Asn Lys Ser Asp Met Arg Val Glu Thr Lys
865 870 875 880

Gly Ala Leu Ile Tyr Asn Gly Ala Ala Ser Gly Gly Ser Leu Asn Ser
885 890 895

Asp Gly Ile Val Tyr Leu Ala Asp Thr Asp Gln Ser Gly Ala Asn Glu
900 905 910

Thr Val His Ile Lys Gly Ser Leu Gln Leu Asp Gly Lys Gly Thr Leu
915 920 925

Tyr Thr Arg Leu Gly Lys Leu Leu Lys Val Asp Gly Thr Ala Ile Ile
930 935 940

Gly Gly Lys Leu Tyr Met Ser Ala Arg Gly Lys Gly Ala Gly Tyr Leu
945 950 955 960

Asn Ser Thr Gly Arg Arg Val Pro Phe Leu Ser Ala Ala Lys Ile Gly
965 970 975

Gln Asp Tyr Ser Phe Phe Thr Asn Ile Glu Thr Asp Gly Gly Leu Leu
980 985 990

Ala Ser Leu Asp Ser Val Glu Lys Thr Ala Gly Ser Glu Gly Asp Thr
995 1000 1005

Leu Ser Tyr Tyr Val Arg Arg Gly Asn Ala Ala Arg Thr Ala Ser Ala
1010 1015 1020

Ala Ala His Ser Ala Pro Ala Gly Leu Lys His Ala Val Glu Gln Gly
1025 1030 1035 1040

Gly Ser Asn Leu Glu Asn Leu Met Val Glu Leu Asp Ala Ser Glu Ser
1045 1050 1055

Ser Ala Thr Pro Glu Thr Val Glu Thr Ala Ala Ala Asp Arg Thr Asp
1060 1065 1070

Met Pro Gly Ile Arg Pro Tyr Gly Ala Thr Phe Arg Ala Ala Ala Ala
1075 1080 1085

Val Gln His Ala Asn Ala Ala Asp Gly Val Arg Ile Phe Asn Ser Leu
1090 1095 1100

Ala Ala Thr Val Tyr Ala Asp Ser Thr Ala Ala His Ala Asp Met Gln
1105 1110 1115 1120

Gly Arg Arg Leu Lys Ala Val Ser Asp Gly Leu Asp His Asn Gly Thr
1125 1130 1135

Gly Leu Arg Val Ile Ala Gln Thr Gln Gln Asp Gly Gly Thr Trp Glu
1140 1145 1150

Gln Gly Gly Val Glu Gly Lys Met Arg Gly Ser Thr Gln Thr Val Gly
1155 1160 1165

Ile Ala Ala Lys Thr Gly Glu Asn Thr Thr Ala Ala Ala Thr Leu Gly
1170 1175 1180

Met Gly Arg Ser Thr Trp Ser Glu Asn Ser Ala Asn Ala Lys Thr Asp
1185 1190 1195 1200

Ser Ile Ser Leu Phe Ala Gly Ile Arg His Asp Ala Gly Asp Ile Gly
1205 1210 1215

Tyr Leu Lys Gly Leu Phe Ser Tyr Gly Arg Tyr Lys Asn Ser Ile Ser
1220 1225 1230

Arg Ser Thr Gly Ala Asp Glu His Ala Glu Gly Ser Val Asn Gly Thr
1235 1240 1245

Leu Met Gln Leu Gly Ala Leu Gly Gly Val Asn Val Pro Phe Ala Ala
1250 1255 1260

Thr Gly Asp Leu Thr Val Glu Gly Gly Leu Arg Tyr Asp Leu Leu Lys
1265 1270 1275 1280

Gln Asp Ala Phe Ala Glu Lys Gly Ser Ala Leu Gly Trp Ser Gly Asn
1285 1290 1295

Ser Leu Thr Glu Gly Thr Leu Val Gly Leu Ala Gly Leu Lys Leu Ser
1300 1305 1310

Gln Pro Leu Ser Asp Lys Ala Val Leu Phe Ala Thr Ala Gly Val Glu
1315 1320 1325

Arg Asp Leu Asn Gly Arg Asp Tyr Thr Val Thr Gly Gly Phe Thr Gly
1330 1335 1340

Ala Thr Ala Ala Thr Gly Lys Thr Gly Ala Arg Asn Met Pro His Thr
1345 1350 1355 1360

Arg Leu Val Ala Gly Leu Gly Ala Asp Val Glu Phe Gly Asn Gly Trp
1365 1370 1375

Asn Gly Leu Ala Arg Tyr Ser Tyr Ala Gly Ser Lys Gln Tyr Gly Asn
1380 1385 1390

His Ser Gly Arg Val Gly Val Gly Tyr Arg Phe
1395 1400

<210> 164
<211> 33
<212> DNA
<213> Artificial Sequence

<220>
<223> Oligonucleotide

<400> 164
cgcggatccg ctagcaaaac aaccgacaaa cgg 33

<210> 165
<211> 27
<212> DNA
<2.13> Artificial Sequence

<220>

<223> Oligonucleotide

<400> 165
cccgctcgag ttaccagcgg tagccta 27

<210> 166
<211> 30
<212> DNA
<213> Artificial Sequence

<220>
<223> Oligonucleotide

<400> 166
ctagctagcg gacacactta tttcggcatc 30

<210> 167
<211> 32
<212> DNA
<213> Artificial Sequence

<220>
<223> Oligonucleotide

<400> 167
cccgctcgag ttaccagcgg tagcctaatt tg 32

<210> 168
<211> 10
<212> DNA
<213> Artificial Sequence

<220>
<223> Oligonucleotide

<400> 168
cccgctcgag 10

<210> 169
<211> 33
<212> DNA
<213> Artificial Sequence

<220>
<223> Oligonucleotide

<400> 169
cgcggatccc atatgaaaac cttcttcaaa acc 33

<210> 170
<211> 28
<212> DNA
<213> Artificial Sequence

<220>
<223> Oligonucleotide

<400> 170

cccgctcgag ttatttggct gcgccttc 28

<210> 171
<211> 35
<212> DNA
<213> Artificial Sequence

<220>
<223> Oligonucleotide

<400> 171
gcggcattaa tatgttgaga aaattgttga aatgg 35

<210> 172
<211> 34
<212> DNA
<213> Artificial Sequence

<220>
<223> Oligonucleotide

<400> 172
gcggcctcga gttatttttt caaaatatat ttgc 34

<210> 173
<211> 33
<212> DNA
<213> Artificial Sequence

<220>
<223> Oligonucleotide

<400> 173
gcggccatat gttacctaac cgtttcaaaa tgt 33

<210> 174
<211> 31
<212> DNA
<213> Artificial Sequence

<220>
<223> Oligonucleotide

<400> 174
gcggcctcga gttatttccg aggttttcgg g 31

<210> 175
<211> 32
<212> DNA
<213> Artificial Sequence

<220>
<223> Oligonucleotide

<400> 175
cgcggatccc atatgacacg cttcaaatat tc 32

<210> 176

<211> 31
<212> DNA
<213> Artificial Sequence

<220>
<223> Oligonucleotide

<400> 176
cccgctcgag ttatttaaac cgataggtaa a 31

<210> 177
<211> 31
<212> DNA
<213> Artificial Sequence

<220>
<223> Oligonucleotide

<400> 177
cgcggatccc atatgggcag ggaagaaccg c 31

<210> 178
<211> 28
<212> DNA
<213> Artificial Sequence

<220>
<223> Oligonucleotide

<400> 178
gcccaagctt atcgatggaa tagccgcg 28

<210> 179
<211> 32
<212> DNA
<213> Artificial Sequence

<220>
<223> Oligonucleotide

<400> 179
cgcggatccg ctagcaacgg tttggatgcc cg 32

<210> 180
<211> 30
<212> DNA
<213> Artificial Sequence

<220>
<223> Oligonucleotide

<400> 180
cccgctcgag tttgtctaag ttcctgatat 30

<210> 181
<211> 26
<212> DNA
<213> Artificial Sequence

<220>
<223> Oligonucleotide

<400> 181
cccgctcgag attcccacct gccatc 26

<210> 182
<211> 37
<212> DNA
<213> Artificial Sequence

<220>
<223> Oligonucleotide

<400> 182
cgcggatccg ctagcatgaa tttgcctatt caaaaat 37

<210> 183
<211> 29
<212> DNA
<213> Artificial Sequence

<220>
<223> Oligonucleotide

<400> 183
cccgctcgag ttaattccca cctgccatc 29

<210> 184
<211> 37
<212> DNA
<213> Artificial Sequence

<220>
<223> Oligonucleotide

<400> 184
cgcggatccg ctagcatgaa tttgcctatt caaaaat 37

<210> 185
<211> 27
<212> DNA
<213> Artificial Sequence

<220>
<223> Oligonucleotide

<400> 185
cccgctcgag ttggacgatg cccgcga 27

<210> 186
<211> 37
<212> DNA
<213> Artificial Sequence

<220>
<223> Oligonucleotide

<400> 186
cgcggatccg ctagcatgaa tttgcctatt caaaaat 37

<210> 187
<211> 28
<212> DNA
<213> Artificial Sequence

<220>
<223> Oligonucleotide

<400> 187
cccgctcgag ttattggacg atgcccgc 28

<210> 188
<211> 32
<212> DNA
<213> Artificial Sequence

<220>
<223> Oligonucleotide

<400> 188
cgcggatccc atatgtatcg caaactgatt gc 32

<210> 189
<211> 28
<212> DNA
<213> Artificial Sequence

<220>
<223> Oligonucleotide

<400> 189
cccgctcgag ctaatcgatg gaatagcc 28

<210> 190
<211> 32
<212> DNA
<213> Artificial Sequence

<220>
<223> Oligonucleotide

<400> 190
cgcggatccc atatgaaaca gacagtcaaa tg 32

<210> 191
<211> 28
<212> DNA
<213> Artificial Sequence

<220>
<223> Oligonucleotide

<400> 191
cccgctcgag tcaataaccc gccttcag 28

<210> 192
<211> 45
<212> DNA
<213> Artificial Sequence

<220>
<223> Oligonucleotide

<400> 192
cgcggatccc atatgttacg tttgactgct ttagccgtat gcacc 45

<210> 193
<211> 40
<212> DNA
<213> Artificial Sequence

<220>
<223> Oligonucleotide

<400> 193
cccgctcgag ttattttgcc gcgttaaaag cgtcggcaac 40

<210> 194
<211> 32
<212> DNA
<213> Artificial Sequence

<220>
<223> Oligonucleotide

<400> 194
cgcggatccc atatgaacaa aatataccgc at 32

<210> 195
<211> 28
<212> DNA
<213> Artificial Sequence

<220>
<223> Oligonucleotide

<400> 195
cccgctcgag ttaccactga taaccgac 28

<210> 196
<211> 34
<212> DNA
<213> Artificial Sequence

<220>
<223> Oligonucleotide

<400> 196
cgcggatccc atatgaccga tgacgacgat ttat 34

<210> 197
<211> 28
<212> DNA

<213> Artificial Sequence

<220>
<223> Oligonucleotide

<400> 197
gcccaagctt ccactgataa ccgacaga 28

<210> 198
<211> 32
<212> DNA
<213> Artificial Sequence

<220>
<223> Oligonucleotide

<400> 198
cgcggatccc atatgaacaa aatataccgc at 32

<210> 199
<211> 28
<212> DNA
<213> Artificial Sequence

<220>
<223> Oligonucleotide

<400> 199
gcccaagctt ttaccactga taaccgac 28

<210> 200
<211> 34
<212> DNA
<213> Artificial Sequence

<220>
<223> Oligonucleotide

<400> 200
gggaattcca tatgggcatt tcccgcaaaa tatc 34

<210> 201
<211> 40
<212> DNA
<213> Artificial Sequence

<220>
<223> Oligonucleotide

<400> 201
cccgctcgag ttatttactc ctataacgag gtctcttaac 40

<210> 202
<211> 36
<212> DNA
<213> Artificial Sequence

<220>

<223> Oligonucleotide

<400> 202
gggaattcca tatgtcagat ttggcaaacg attctt 36

<210> 203
<211> 40
<212> DNA
<213> Artificial Sequence

<220>
<223> Oligonucleotide

<400> 203
cccgctcgag ttatttactc ctataacgag gtctcttaac 40

<210> 204
<211> 34
<212> DNA
<213> Artificial Sequence

<220>
<223> Oligonucleotide

<400> 204
gggaattcca tatgggcatt tcccgcaaaa tatc 34

<210> 205
<211> 32
<212> DNA
<213> Artificial Sequence

<220>
<223> Oligonucleotide

<400> 205
cccgctcgag ttacgtatca tatttcacgt gc 32

<210> 206
<211> 35
<212> DNA
<213> Artificial Sequence

<220>
<223> Oligonucleotide

<400> 206
gggaattcca tatgcacgtg aaatatgata cgaag 35

<210> 207
<211> 37
<212> DNA
<213> Artificial Sequence

<220>
<223> Oligonucleotide

<400> 207

cccgctcgag tttactccta taacgaggtc tcttaac 37

<210> 208
<211> 36
<212> DNA
<213> Artificial Sequence

<220>
<223> Oligonucleotide

<400> 208
gggaattcca tatgtcagat ttggcaaacg attctt 36

<210> 209
<211> 29
<212> DNA
<213> Artificial Sequence

<220>
<223> Oligonucleotide

<400> 209
cccgctcgag cgtatcatat ttcacgtgc 29

<210> 210
<211> 36
<212> DNA
<213> Artificial Sequence

<220>
<223> Oligonucleotide

<400> 210
gggaattcca tatgtcagat ttggcaaacg attctt 36

<210> 211
<211> 37
<212> DNA
<213> Artificial Sequence

<220>
<223> Oligonucleotide

<400> 211
cccgctcgag tttactccta taacgaggtc tcttaac 37

<210> 212
<211> 35
<212> DNA
<213> Artificial Sequence

<22.0>
<223> Oligonucleotide

<400> 212
cgcggatccc atatgcaaaa tgcgttcaaa atccc 35

<210> 213

<211> 32
<212> DNA
<213> Artificial Sequence

<220>
<223> Oligonucleotide

<400> 213
cgcggatccc atatgaacaa aatataccgc at 32

<210> 214
<211> 29
<212> DNA
<213> Artificial Sequence

<220>
<223> Oligonucleotide

<400> 214
cccgctcgag tttgctttcg atagaacgg 29

<210> 215
<211> 34
<212> DNA
<213> Artificial Sequence

<220>
<223> Oligonucleotide

<400> 215
gcggccatat ggtcataaaa tatacaaatt tgaa 34

<210> 216
<211> 34
<212> DNA
<213> Artificial Sequence

<220>
<223> Oligonucleotide

<400> 216
gcggcctcga gttagcctga gacctttgca aatt 34

<210> 217
<211> 30
<212> DNA
<213> Artificial Sequence

<220>
<223> Oligonucleotide

<400> 217
gcggccatat gaaacagaaa aaaaccgctg 30

<210> 218
<211> 32
<212> DNA
<213> Artificial Sequence

<220>
<223> Oligonucleotide

<400> 218
gcggcctcga gttacggttt gacaccgttt tc 32

<210> 219
<211> 30
<212> DNA
<213> Artificial Sequence

<220>
<223> Oligonucleotide

<400> 219
cgcggatccc atatgaaaac cctgctcctc 30

<210> 220
<211> 27
<212> DNA
<213> Artificial Sequence

<220>
<223> Oligonucleotide

<400> 220
cccgctcgag ttatcctcct ttgcggc 27

<210> 221
<211> 30
<212> DNA
<213> Artificial Sequence

<220>
<223> Oligonucleotide

<400> 221
gcggccatat ggcaaaaatg atgaaatggg 30

<210> 222
<211> 29
<212> DNA
<213> Artificial Sequence

<220>
<223> Oligonucleotide

<400> 222
gcggcctcga gttatcggcg cggcgggcc 29

<210> 223
<211> 30
<212> DNA
<213> Artificial Sequence

<220>
<223> Oligonucleotide

<400> 223
gcggccatat gaaaaaatcc tccctcatca 30

<210> 224
<211> 32
<212> DNA
<213> Artificial Sequence

<220>
<223> Oligonucleotide

<400> 224
gcggcctcga gttatttgcc gccgtttttg gc 32

<210> 225
<211> 31
<212> DNA
<213> Artificial Sequence

<220>
<223> Oligonucleotide

<400> 225
gcggccatat ggcccctgcc gacgcggtaa g 31

<210> 226
<211> 33
<212> DNA
<213> Artificial Sequence

<220>
<223> Oligonucleotide

<400> 226
gcggcctcga gtttgccgcc gtttttggct ttc 33

<210> 227
<211> 30
<212> DNA
<213> Artificial Sequence

<220>
<223> Oligonucleotide

<400> 227
gcggccatat gaaacacata ctccccctga 30

<210> 228
<211> 32
<212> DNA
<213> Artificial Sequence

<220>
<223> Oligonucleotide

<400> 228
gcggcctcga gttattcgcc tacggttttt tg 32

<210> 229
<211> 30
<212> DNA
<213> Artificial Sequence

<220>
<223> Oligonucleotide

<400> 229
gcggccatat gatttacatc gtactgtttc 30

<210> 230
<211> 32
<212> DNA
<213> Artificial Sequence

<220>
<223> Oligonucleotide

<400> 230
gcggcctcga gttaggagaa caggcgcaat gc 32

<210> 231
<211> 32
<212> DNA
<213> Artificial Sequence

<220>
<223> Oligonucleotide

<400> 231
gcggccatat gtacaacatg tatcaggaaa ac 32

<210> 232
<211> 31
<212> DNA
<213> Artificial Sequence

<220>
<223> Oligonucleotide

<400> 232
gcggcctcga gggagaacag gcgcaatgcg g 31

<210> 233
<211> 29
<212> DNA
<213> Artificial Sequence

<220>
<223> Oligonucleotide

<400> 233
cgcggatccg ctagctgcgg cacggcggg 29

<210> 234
<211> 28
<212> DNA

<213> Artificial Sequence

<220>
<223> Oligonucleotide

<400> 234
cccgctcgag ataacggtat gccgccag 28

<210> 235
<211> 31
<212> DNA
<213> Artificial Sequence

<220>
<223> Oligonucleotide

<400> 235
cgcggatccc atatggaatc aacactttca c 31

<210> 236
<211> 27
<212> DNA
<213> Artificial Sequence

<220>
<223> Oligonucleotide

<400> 236
cccgctcgag ttacacgcgg ttgctgt 27

<210> 237
<211> 31
<212> DNA
<213> Artificial Sequence

<220>
<223> Oligonucleotide

<400> 237
cgcggatccc atatgaacaa cagacatttt g 31

<210> 238
<211> 28
<212> DNA
<213> Artificial Sequence

<220>
<223> Oligonucleotide

<400> 238
cccgctcgag ttacctgtcc ggtaaaag 28

<210> 239
<211> 33
<212> DNA
<213> Artificial Sequence

<220>

<223> Oligonucleotide

<400> 239
cgcggatccg ctagcaccgt catcaaacag gaa 33

<210> 240
<211> 27
<212> DNA
<213> Artificial Sequence

<220>
<223> Oligonucleotide

<400> 240
cccgctcgag tcaagattcg acgggga 27

<210> 241
<211> 31
<212> DNA
<213> Artificial Sequence

<220>
<223> Oligonucleotide

<400> 241
cgcggatccc atatgtccgc aaacgaatac g 31

<210> 242
<211> 28
<212> DNA
<213> Artificial Sequence

<220>
<223> Oligonucleotide

<400> 242
cccgctcgag tcagtgttct gccagttt 28

<210> 243
<211> 29
<212> DNA
<213> Artificial Sequence

<220>
<223> Oligonucleotide

<400> 243
cgcggatccc atatgccgtc tgaaacacg 29

<210> 244
<211> 28
<212> DNA
<213> Artificial Sequence

<220>
<223> Oligonucleotide

<400> 244

cccgctcgag ttagcggagc agtttttc 28

<210> 245
<211> 28
<212> DNA
<213> Artificial Sequence

<220>
<223> Oligonucleotide

<400> 245
cgcggatccc atatgaccgc catcagcc 28

<210> 246
<211> 27
<212> DNA
<213> Artificial Sequence

<220>
<223> Oligonucleotide

<400> 246
cccgctcgag ttaaagccgg gtaacgc 27

<210> 247
<211> 31
<212> DNA
<213> Artificial Sequence

<220>
<223> Oligonucleotide

<400> 247
gcggccatat ggaaacacag ctttacatcg g 31

<210> 248
<211> 30
<212> DNA
<213> Artificial Sequence

<220>
<223> Oligonucleotide

<400> 248
gcggcctcga gtcaataata atatcccgcg 30

<210> 249
<211> 30
<212> DNA
<213> Artificial Sequence

<220>
<223> Oligonucleotide

<400> 249
gcggccatat gattaaaatc cgcaatatcc 30

<210> 250

<211> 36
<212> DNA
<213> Artificial Sequence

<220>
<223> Oligonucleotide

<400> 250
gcggcctcga gttaaatctt ggtagattgg atttgg 36

<210> 251
<211> 30
<212> DNA
<213> Artificial Sequence

<220>
<223> Oligonucleotide

<400> 251
gcggccatat gactgacaac gcactgctcc 30

<210> 252
<211> 31
<212> DNA
<213> Artificial Sequence

<220>
<223> Oligonucleotide

<400> 252
gcggcctcga gtcagaccgc gttgtcgaaa c 31

<210> 253
<211> 32
<212> DNA
<213> Artificial Sequence

<220>
<223> Oligonucleotide

<400> 253
cgcggatccc atatggcgtt aaaaacatca aa 32

<210> 254
<211> 27
<212> DNA
<213> Artificial Sequence

<220>
<223> Oligonucleotide

<400> 254
cccgctcgag tcagcccttc atacagc 27

<210> 255
<211> 32
<212> DNA
<213> Artificial Sequence

<220>
<223> Oligonucleotide

<400> 255
gcggcattaa tggcacaaac tacactcaaa cc 32

<210> 256
<211> 33
<212> DNA
<213> Artificial Sequence

<220>
<223> Oligonucleotide

<400> 256
gcggcctcga gttaaaactt cacgttcacg ccg 33

<210> 257
<211> 34
<212> DNA
<213> Artificial Sequence

<220>
<223> Oligonucleotide

<400> 257
gcggcattaa tgcatgaaac tgagcaatcg gtgg 34

<210> 258
<211> 38
<212> DNA
<213> Artificial Sequence

<220>
<223> Oligonucleotide

<400> 258
gcggcctcga gaaacttcac gttcacgccg ccggtaaa 38

<210> 259
<211> 33
<212> DNA
<213> Artificial Sequence

<220>
<223> Oligonucleotide

<400> 259
cgcggatccc atatgggcaa atccgaaaat acg 33

<210> 260
<211> 26
<212> DNA
<213> Artificial Sequence

<220>
<223> Oligonucleotide

<400> 260
cccgctcgag ataatggcgg cggcgg 26

<210> 261
<211> 29
<212> DNA
<213> Artificial Sequence

<220>
<223> Oligonucleotide

<400> 261
cgcggatccc atatgtttcc ccccgacaa 29

<210> 262
<211> 31
<212> DNA
<213> Artificial Sequence

<220>
<223> Oligonucleotide

<400> 262
cccgctcgag tcattctgta aaaaaagtat g 31

<210> 263
<211> 32
<212> DNA
<213> Artificial Sequence

<220>
<223> Oligonucleotide

<400> 263
cgcggatccc atatgcttca aagcgacagc ag 32

<210> 264
<211> 29
<212> DNA
<213> Artificial Sequence

<220>
<223> Oligonucleotide

<400> 264
cccgctcgag ttcggatttt tgcgtactc 29

<210> 265
<211> 31
<212> DNA
<213> Artificial Sequence

<220>
<223> Oligonucleotide

<400> 265
cgcggatccc atatggcaat ggcagaaaac g 31

<210> 266
<211> 27
<212> DNA
<213> Artificial Sequence

<220>
<223> Oligonucleotide

<400> 266
cccgctcgag ctatacaatc cgtgccg 27

<210> 267
<211> 32
<212> DNA
<213> Artificial Sequence

<220>
<223> Oligonucleotide

<400> 267
cgcggatccc atatggattc ttttttcaaa cc 32

<210> 268
<211> 27
<212> DNA
<213> Artificial Sequence

<220>
<223> Oligonucleotide

<400> 268
cccgctcgag tcagttcaga aagcggg 27

<210> 269
<211> 32
<212> DNA
<213> Artificial Sequence

<220>
<223> Oligonucleotide

<400> 269
cgcggatccc atatgaaacc tttgatttta gg 32

<210> 270
<211> 28
<212> DNA
<213> Artificial Sequence

<220>
<223> Oligonucleotide

<400> 270
cccgctcgag ttatttgggc tgctcttc 28

<210> 271
<211> 30
<212> DNA

<213> Artificial Sequence

<220>
<223> Oligonucleotide

<400> 271
cgcggatccc atatggtaat cgtctggttg 30

<210> 272
<211> 27
<212> DNA
<213> Artificial Sequence

<220>
<223> Oligonucleotide

<400> 272
cccgctcgag ctacgacttg gttaccg 27

<210> 273
<211> 33
<212> DNA
<213> Artificial Sequence

<220>
<223> Oligonucleotide

<400> 273
gcggccatat gagacgtaaa atgctaaagc tac 33

<210> 274
<211> 31
<212> DNA
<213> Artificial Sequence

<220>
<223> Oligonucleotide

<400> 274
gcggcctcga gtcaaagtgt tctgtttgcg c 31

<210> 275
<211> 30
<212> DNA
<213> Artificial Sequence

<220>
<223> Oligonucleotide

<400> 275
gccgccatat gttgacttta acccgaaaaa 30

<210> 276
<211> 34
<212> DNA
<213> Artificial Sequence

<220>

<223> Oligonucleotide

<400> 276
gccgcctcga ggccggcggt caataccgcc cgaa 34

<210> 277
<211> 32
<212> DNA
<213> Artificial Sequence

<220>
<223> Oligonucleotide

<400> 277
cgcggatccc atatggcgca atgcgatttg ac 32

<210> 278
<211> 27
<212> DNA
<213> Artificial Sequence

<220>
<223> Oligonucleotide

<400> 278
cccgctcgag ttcggcggta aatgccg 27

<210> 279
<211> 28
<212> DNA
<213> Artificial Sequence

<220>
<223> Oligonucleotide

<400> 279
gcggccatat ggcggggccg atttttgt 28

<210> 280
<211> 33
<212> DNA
<213> Artificial Sequence

<220>
<223> Oligonucleotide

<400> 280
gcggcctcga gttatttgct ttcagtatta ttg 33

<210> 281
<211> 30
<212> DNA
<213> Artificial Sequence

<220>
<223> Oligonucleotide

<400> 281

gcggccatat gaactttgct ttatccgtca 30

<210> 282
<211> 32
<212> DNA
<213> Artificial Sequence

<220>
<223> Oligonucleotide

<400> 282
gcggcctcga gttaacggca gtatttgttt ac 32

<210> 283
<211> 31
<212> DNA
<213> Artificial Sequence

<220>
<223> Oligonucleotide

<400> 283
cgcggatccc atatgggttt gcgcttcggg c 31

<210> 284
<211> 29
<212> DNA
<213> Artificial Sequence

<220>
<223> Oligonucleotide

<400> 284
gcccaagctt ttttcctttg ccgtttccg 29

<210> 285
<211> 32
<212> DNA
<213> Artificial Sequence

<220>
<223> Oligonucleotide

<400> 285
cgcggatccc atatggccga cctttccgaa aa 32

<210> 286
<211> 27
<212> DNA
<213> Artificial Sequence

<220>
<223> Oligonucleotide

<400> 286
cccgctcgag gaagcgcgtt cccaagc 27

<210> 287

<211> 29
<212> DNA
<213> Artificial Sequence

<220>
<223> Oligonucleotide

<400> 287
cgcggatccc atatgcacga cacccgtac 29

<210> 288
<211> 28
<212> DNA
<213> Artificial Sequence

<220>
<223> Oligonucleotide

<400> 288
cccgctcgag ttagaagcgc gttcccaa 28

<210> 289
<211> 34
<212> DNA
<213> Artificial Sequence

<220>
<223> Oligonucleotide

<400> 289
ctagctagct ttaaacgcag cgtaatcgca atgg 34

<210> 290
<211> 31
<212> DNA
<213> Artificial Sequence

<220>
<223> Oligonucleotide

<400> 290
cccgctcgag tcaatcctgc tcttttttgc c 31

<210> 291
<211> 25
<212> DNA
<213> Artificial Sequence

<220>
<223> Oligonucleotide

<400> 291
ctagctagcg ggggcggcgg tggcg 25

<210> 292
<211> 31
<212> DNA
<213> Artificial Sequence

<220>
<223> Oligonucleotide

<400> 292
cccgctcgag tcaatcctgc tcttttttgc c 31

<210> 293
<211> 40
<212> DNA
<213> Artificial Sequence

<220>
<223> Oligonucleotide

<400> 293
ctagctagcg ctcatcctcg ccgcctgcgg gggcggcggt 40

<210> 294
<211> 31
<212> DNA
<213> Artificial Sequence

<220>
<223> Oligonucleotide

<400> 294
cccgctcgag tcaatcctgc tcttttttgc c 31

<210> 295
<211> 25
<212> DNA
<213> Artificial Sequence

<220>
<223> Oligonucleotide

<400> 295
cggggatccg ggggcggcgg tggcg 25

<210> 296
<211> 31
<212> DNA
<213> Artificial Sequence

<220>
<223> Oligonucleotide

<400> 296
cccgctcgag tcaatcctgc tcttttttgc c 31

<210> 297
<211> 25
<212> DNA
<213> Artificial Sequence

<220>
<223> Oligonucleotide

<400> 297
ctagctagcg ggggcggcgg tggcg 25

<210> 298
<211> 28
<212> DNA
<213> Artificial Sequence

<220>
<223> Oligonucleotide

<400> 298
cccgctcgag atcctgctct tttttgcc 28

<210> 299
<211> 28
<212> DNA
<213> Artificial Sequence

<220>
<223> Oligonucleotide

<400> 299
ctagctagct gcgggggcgg cggtggcg 28

<210> 300
<211> 28
<212> DNA
<213> Artificial Sequence

<220>
<223> Oligonucleotide

<400> 300
cccgctcgag atcctgctct tttttgcc 28

<210> 301
<211> 32
<212> DNA
<213> Artificial Sequence

<220>
<223> Oligonucleotide

<400> 301
cgcggatccg ctagccccga tgttaaatcg gc 32

<210> 302
<211> 32
<212> DNA
<213> Artificial Sequence

<220>
<223> Oligonucleotide

<400> 302
cgcggatccg ctagccaaga tatggcggca gt 32

<210> 303
<211> 32
<212> DNA
<213> Artificial Sequence

<220>
<223> Oligonucleotide

<400> 303
cgcggatccg ctagcgccga atccgcaaat ca 32

<210> 304
<211> 32
<212> DNA
<213> Artificial Sequence

<220>
<223> Oligonucleotide

<400> 304
cgcgctagcg gaagggttga tttggctaat gg 32

<210> 305
<211> 32
<212> DNA
<213> Artificial Sequence

<220>
<223> Oligonucleotide

<400> 305
cgcgctagcg gaagggttga tttggctaat gg 32

<210> 306
<211> 29
<212> DNA
<213> Artificial Sequence

<220>
<223> Oligonucleotide

<400> 306
cgccatatgt ttaaacgcag cgtaatcgc 29

<210> 307
<211> 34
<212> DNA
<213> Artificial Sequence

<220>
<223> Oligonucleotide

<400> 307
cccgctcgag aaaattgcta ccgccattcg cagg 34

<210> 308
<211> 32
<212> DNA

<213> Artificial Sequence

<220>
<223> Oligonucleotide

<400> 308
cgccatatgg gaagggttga tttggctaat gg 32

<210> 309
<211> 38
<212> DNA
<213> Artificial Sequence

<220>
<223> Oligonucleotide

<400> 309
cccgctcgag cttgtcttta taaatgatga catatttg 38

<210> 310
<211> 40
<212> DNA
<213> Artificial Sequence

<220>
<223> Oligonucleotide

<400> 310
cccgctcgag tttataaaag ataatatatt gattgattcc 40

<210> 311
<211> 31
<212> DNA
<213> Artificial Sequence

<220>
<223> Oligonucleotide

<400> 311
cgcgctagca tgccgctgat tcccgtcaat c 31

<210> 312
<211> 25
<212> DNA
<213> Artificial Sequence

<220>
<223> Oligonucleotide

<400> 312
ctagctagcg ggggcggcgg tggcg 25

<210> 313
<211> 31
<212> DNA
<213> Artificial Sequence

<220>

<223> Oligonucleotide

<400> 313
cccgctcgag tcaatcctgc tcttttttgc c 31

<210> 314
<211> 32
<212> DNA
<213> Artificial Sequence

<220>
<223> Oligonucleotide

<400> 314
cgcggatccg ctagccccga tgttaaatcg gc 32

<210> 315
<211> 28
<212> DNA
<213> Artificial Sequence

<220>
<223> Oligonucleotide

<400> 315
cccgctcgag atcctgctct tttttgcc 28

<210> 316
<211> 32
<212> DNA
<213> Artificial Sequence

<220>
<223> Oligonucleotide

<400> 316
cgcggatccg ctagccccga tgttaaatcg gc 32

<210> 317
<211> 31
<212> DNA
<213> Artificial Sequence

<220>
<223> Oligonucleotide

<400> 317
cccgctcgag tcaatcctgc tcttttttgc c 31

<210> 318
<211> 87
<212> DNA
<213> Artificial Sequence

<220>
<223> Oligonucleotide

<400> 318

cgcggatccg ctagctttga acgcagtgtg attgcaatgg cttgtatttt tgccctttca 60
gcctgttcgc ccgatgttaa atcggcg 87

<210> 319
<211> 31
<212> DNA
<213> Artificial Sequence

<220>
<223> Oligonucleotide

<400> 319
cccgctcgag tcaatcctgc tcttttttgc c 31

<210> 320
<211> 90
<212> DNA
<213> Artificial Sequence

<220>
<223> Oligonucleotide

<400> 320

cgcggatccg ctagcaaaac cttcttcaaa accctttccg ccgccgcact cgcgctcatc 60

ctcgccgcct gctcgcccga tgttaaatcg 90

<210> 321
<211> 31
<212> DNA
<213> Artificial Sequence

<220>
<223> Oligonucleotide

<400> 321
cccgctcgag tcaatcctgc tcttttttgc c 31

<210> 322
<211> 33
<212> DNA
<213> Artificial Sequence

<220>
<223> Oligonucleotide

<400> 322
cgcggatccc atatgaaaac caagttaatc aaa 33

<210> 323
<211> 30
<212> DNA
<213> Artificial Sequence

<220>
<223> Oligonucleotide

<400> 323
cccgctcgag ttattgattt ttgcggatga 30

<210> 324
<211> 34
<212> DNA
<213> Artificial Sequence

<220>
<223> Oligonucleotide

<400> 324
cgcggatccc atatgttaaa tcgggtattt tatc 34

<210> 325
<211> 28
<212> DNA
<213> Artificial Sequence

<220>
<223> Oligonucleotide

<400> 325
cccgctcgag ttaatccgcc attccctg 28

<210> 326
<211> 30
<212> DNA
<213> Artificial Sequence

<220>
<223> Oligonucleotide

<400> 326
gcggccatat gaaattacaa caattggctg 30

<210> 327
<211> 31
<212> DNA
<213> Artificial Sequence

<220>
<223> Oligonucleotide

<400> 327
gcggcctcga gttaccttac gtttttcaaa g 31

<210> 328
<211> 29
<212> DNA
<213> Artificial Sequence

<220>
<223> Oligonucleotide

<400> 328
cgcggatccc atatgcaagc acggctgct 29

<210> 329
<211> 29
<212> DNA
<213> Artificial Sequence

<220>
<223> Oligonucleotide

<400> 329
cccgctcgag tcaaggttgt ccttgtcta 29

<210> 330
<211> 30
<212> DNA
<213> Artificial Sequence

<220>
<223> Oligonucleotide

<400> 330
cgcggatccc atatgatgaa accgcacaac 30

<210> 331
<211> 28
<212> DNA
<213> Artificial Sequence

<220>
<223> Oligonucleotide

<400> 331 28
cccgctcgag tcagttgctc aacacgtc 28

<210> 332
<211> 32
<212> DNA
<213> Artificial Sequence

<220>
<223> Oligonucleotide

<400> 332
cgcggatccc atatggtaga cgcgcttaag ca 32

<210> 333
<211> 25
<212> DNA
<213> Artificial Sequence

<220>
<223> Oligonucleotide

<400> 333 25
cccgctcgag agctgcatgg cggcg 25

<210> 334
<211> 30
<212> DNA
<213> Artificial Sequence

<220>
<223> Oligonucleotide

<400> 334 30
cgcggatccc atatggcacg gtcgttatac 30

<210> 335
<211> 26
<212> DNA
<213> Artificial Sequence

<220>
<223> Oligonucleotide

<400> 335 26
cccgctcgag ctaccgcgca ttcctg 26

<210> 336
<211> 31
<212> DNA
<213> Artificial Sequence

<220>
<223> Oligonucleotide

<400> 336
gcggccatat ggaatttttc attatcttgt t 31

<210> 337
<211> 31
<212> DNA
<213> Artificial Sequence

<220>
<223> Oligonucleotide

<400> 337
gcggcctcga gttatttggc ggttttgctg c 31

<210> 338
<211> 32
<212> DNA
<213> Artificial Sequence

<220>
<223> Oligonucleotide

<400> 338
gcggccatat gaagtatgtc cggttatttt tc 32

<210> 339
<211> 30
<212> DNA

<213> Artificial Sequence

<220>
<223> Oligonucleotide

<400> 339
gcggcctcga gttatcggct tgtgcaacgg 30

<210> 340
<211> 32
<212> DNA
<213> Artificial Sequence

<220>
<223> Oligonucleotide

<400> 340
cgcggatccg ctagctccgg cagcaaaacc ga 32

<210> 341
<211> 28
<212> DNA
<213> Artificial Sequence

<220>
<223> Oligonucleotide

<400> 341
gcccaagctt acgcagttcg gaatggag 28

<210> 342
<211> 35
<212> DNA
<213> Artificial Sequence

<220>
<223> Oligonucleotide

<400> 342
gccgccatat gttgaatatt aaactgaaaa ccttg 35

<210> 343
<211> 32
<212> DNA
<213> Artificial Sequence

<220>
<223> Oligonucleotide

<400> 343
gccgcctcga gttatttctg atgccttttc cc 32

<210> 344
<211> 29
<212> DNA
<213> Artificial Sequence

<220>

<223> Oligonucleotide

<400> 344
gccgccatat ggacaataag accaaactg 29

<210> 345
<211> 30
<212> DNA
<213> Artificial Sequence

<220>
<223> Oligonucleotide

<400> 345
gccgcctcga gttaacggtg cggacgtttc 30

<210> 346
<211> 32
<212> DNA
<213> Artificial Sequence

<220>
<223> Oligonucleotide

<400> 346
cgcggatccc atatgaacaa actgtttctt ac 32

<210> 347
<211> 28
<212> DNA
<213> Artificial Sequence

<220>
<223> Oligonucleotide

<400> 347
cccgctcgag tcattccgcc ttcagaaa 28

<210> 348
<211> 45
<212> DNA
<213> Artificial Sequence

<220>
<223> Oligonucleotide

<400> 348
cgcggatccc atatgcaagg tatcgttgcc gacaaatccg cacct 45

<210> 349
<211> 42
<212> DNA
<213> Artificial Sequence

<220>
<223> Oligonucleotide

<400> 349

cccgctcgag agctaattgt gcttggtttg cagataggag tt 42

<210> 350
<211> 52
<212> DNA
<213> Artificial Sequence

<220>
<223> Oligonucleotide

<400> 350
cgcggatccc atatgaaccg caccctgtac aaagttgtat ttaacaaaca tc 52

<210> 351
<211> 45
<212> DNA
<213> Artificial Sequence

<220>
<223> Oligonucleotide

<400> 351
cccgctcgag ttaagctaat tgtgcttggt ttgcagatag gagtt 45

<210> 352
<211> 46
<212> DNA
<213> Artificial Sequence

<220>
<223> Oligonucleotide

<400> 352
cgcggatccc atatgacggg agaaaatcat gcggtttcac ttcatg 46

<210> 353
<211> 42
<212> DNA
<213> Artificial Sequence

<220>
<223> Oligonucleotide

<400> 353
cccgctcgag agctaattgt gcttggtttg cagataggag tt 42

<210> 354
<211> 51
<212> DNA
<213> Artificial Sequence

<220>
<223> Oligonucleotide

<400> 354
cgcggatccc atatggtttc agacggccta tacaaccaac atggtgaaat t 51

<210> 355

<211> 41
<212> DNA
<213> Artificial Sequence

<220>
<223> Oligonucleotide

<400> 355
cccgctcgag gcggtaactg ccgcttgcac tgaatccgta a 41

<210> 356
<211> 46
<212> DNA
<213> Artificial Sequence

<220>
<223> Oligonucleotide

<400> 356
cgcggatccc atatgacggg agaaaatcat gcggtttcac ttcatg 46

<210> 357
<211> 41
<212> DNA
<213> Artificial Sequence

<220>
<223> Oligonucleotide

<400> 357
cccgctcgag gcggtaactg ccgcttgcac tgaatccgta a 41

<210> 358
<211> 49
<212> DNA
<213> Artificial Sequence

<220>
<223> Oligonucleotide

<400> 358
cgcggatccc atatgcaaag caaagtcaaa gcagaccatg cctccgtaa 49

<210> 359
<211> 56
<212> DNA
<213> Artificial Sequence

<220>
<223> Oligonucleotide

<400> 359
cccgctcgag tcttttcctt tcaattataa ctttagtagg ttcaattttg gtcccc 56

<210> 360
<211> 51
<212> DNA
<213> Artificial Sequence

<220>
<223> Oligonucleotide

<400> 360
cgcggatccc atatggtttc agacggccta tacaaccaac atggtgaaat t 51

<210> 361
<211> 56
<212> DNA
<213> Artificial Sequence
<220>
<223> Oligonucleotide

<400> 361
cccgctcgag tcttttcctt tcaattataa ctttagtagg ttcaattttg gtcccc 56

<210> 362
<211> 27
<212> DNA
<213> Artificial Sequence

<220>
<223> Oligonucleotide

<400> 362
gcggccatat gacccgtttg acccgcg 27

<210> 363
<211> 31
<212> DNA
<213> Artificial Sequence

<220>
<223> Oligonucleotide

<400> 363
gcggcctcga gtcagcgggc gttcatttct t 31

<210> 364
<211> 33
<212> DNA
<213> Artificial Sequence

<220>
<223> Oligonucleotide

<400> 364
cgcggatccc atatgaacac cattttcaaa atc 33

<210> 365
<211> 32
<212> DNA
<213> Artificial Sequence

<220>
<223> Oligonucleotide

<400> 365

cccgctcgag ttaatttact tttttgatgt cg 32

<210> 366
<211> 28
<212> DNA
<213> Artificial Sequence

<220>
<223> Oligonucleotide

<400> 366
gcggccatat ggattcgccc aaggtcgg 28

<210> 367
<211> 31
<212> DNA
<213> Artificial Sequence

<220>
<223> Oligonucleotide

<400> 367
gcggcctcga gctacacttc ccccgaagtg g 31

<210> 368
<211> 31
<212> DNA
<213> Artificial Sequence

<220>
<223> Oligonucleotide

<400> 368
cgcggatccc atatgatagt tgaccaaagc c 31

<210> 369
<211> 30
<212> DNA
<213> Artificial Sequence

<220>
<223> Oligonucleotide

<400> 369
cccgctcgag ttatttttcc gatttttcgg 30

<210> 370
<211> 28
<212> DNA
<213> Artificial Sequence

<220>
<223> Oligonucleotide

<400> 370
gcggccatat gcttgaactg aacggact 28

<210> 371

<211> 30
<212> DNA
<213> Artificial Sequence

<220>
<223> Oligonucleotide

<400> 371
gcggcctcga gtcagcggaa gcggacgatt 30

<210> 372
<211> 34
<212> DNA
<213> Artificial Sequence

<220>
<223> Oligonucleotide

<400> 372
cgcggatccc atatgtccaa actcaaaacc atcg 34

<210> 373
<211> 29
<212> DNA
<213> Artificial Sequence

<220>
<223> Oligonucleotide

<400> 373
cccgctcgag gcttccaatc agtttgacc 29

<210> 374
<211> 32
<212> DNA
<213> Artificial Sequence

<220>
<223> Oligonucleotide

<400> 374
gcggccatat gagcgcaatc gttgatattt tc 32

<210> 375
<211> 34
<212> DNA
<213> Artificial Sequence

<220>
<223> Oligonucleotide

<400> 375
gcggcctcga gttatttgcc cagttggtag aatg 34

<210> 376
<211> 32
<212> DNA
<213> Artificial Sequence

<220>
<223> Oligonucleotide

<400> 376
gcggccatat ggtgatacat ccgcactact tc 32

<210> 377
<211> 32
<212> DNA
<213> Artificial Sequence

<220>
<223> Oligonucleotide

<400> 377
gcggcctcga gtcaaaatcg agttttacac ca 32

<210> 378
<211> 31
<212> DNA
<213> Artificial Sequence

<220>
<223> Oligonucleotide

<400> 378
gcggccatat gaccatctat ttcaaaaacg g 31

<210> 379
<211> 34
<212> DNA
<213> Artificial Sequence

<220>
<223> Oligonucleotide

<400> 379
gcggcctcga gtcagccgat gtttagcgtc catt 34

<210> 380
<211> 31
<212> DNA
<213> Artificial Sequence

<220>
<223> Oligonucleotide

<400> 380
cgcggatccc atatgagcag cggagggggt g 31

<210> 381
<211> 27
<212> DNA
<213> Artificial Sequence

<220>
<223> Oligonucleotide

<400> 381
cccgctcgag ttgcttggcg gcaaggc 27

<210> 382
<211> 31
<212> DNA
<213> Artificial Sequence

<220>
<223> Oligonucleotide

<400> 382
cgcggatccc atatggtcgc cgccgacatc g 31

<210> 383
<211> 27
<212> DNA
<213> Artificial Sequence

<220>
<223> Oligonucleotide

<400> 383
cccgctcgag ttgcttggcg gcaaggc 27

<210> 384
<211> 31
<212> DNA
<213> Artificial Sequence

<220>
<223> Oligonucleotide

<400> 384
cgcggatccc atatgggcgg ttcggaaggc g 31

<210> 385
<211> 33
<212> DNA
<213> Artificial Sequence

<220>
<223> Oligonucleotide

<400> 385
cccgctcgag ttgaacactg atgtcttttc cga 33

<210> 386
<211> 35
<212> DNA
<213> Artificial Sequence

<220>
<223> Oligonucleotide

<400> 386
cgcggatccg ctagcaaact gtcgttggtg ttaac 35

<210> 387
<211> 26
<212> DNA
<213> Artificial Sequence

<220>
<223> Oligonucleotide

<400> 387
cccgctcgag ttgacccgct ccacgg 26

<210> 388
<211> 31
<212> DNA
<213> Artificial Sequence

<220>
<223> Oligonucleotide

<400> 388
gccgccatat ggcggacttg gcgcaagacc c 31

<210> 389
<211> 39
<212> DNA
<213> Artificial Sequence

<220>
<223> Oligonucleotide

<400> 389
gccgcctcga gatctcctaa acctgtttta acaatgccg 39

<210> 390
<211> 31
<212> DNA
<213> Artificial Sequence

<220>
<223> Oligonucleotide

<400> 390
gccgccatat ggcggacttg gcgcaagacc c 31

<210> 391
<211> 31
<212> DNA
<213> Artificial Sequence

<220>
<223> Oligonucleotide

<400> 391
gcggcctcga gctccatgct gttgccccag c 31

<210> 392
<211> 31
<212> DNA

<213> Artificial Sequence

<220>
<223> Oligonucleotide

<400> 392
gccgccatat ggcggacttg gcgcaagacc c 31

<210> 393
<211> 31
<212> DNA
<213> Artificial Sequence

<220>
<223> Oligonucleotide

<400> 393
gcggcctcga gaaaatcccc gctaaccgca g 31

<210> 394
<211> 31
<212> DNA
<213> Artificial Sequence

<220>
<223> Oligonucleotide

<400> 394
cgcggatccc atatgagcag cggagggggt g 31

<210> 395
<211> 27
<212> DNA
<213> Artificial Sequence

<220>
<223> Oligonucleotide

<400> 395
cccgctcgag ttgcttggcg gcaaggc 27

<210> 396
<211> 31
<212> DNA
<213> Artificial Sequence

<220>
<223> Oligonucleotide

<400> 396
cgcggatccc atatggtcgc cgccgacatc g 31

<210> 397
<211> 27
<212> DNA
<213> Artificial Sequence

<220>

<223> Oligonucleotide

<400> 397
cccgctcgag ttgcttggcg gcaaggc 27

<210> 398
<211> 33
<212> DNA
<213> Artificial Sequence

<220>
<223> Oligonucleotide

<400> 398
cgcggatccc atatggacgg tgttgtgcct gtt 33

<210> 399
<211> 29
<212> DNA
<213> Artificial Sequence

<220>
<223> Oligonucleotide

<400> 399
cccgctcgag cttacggatc aaattgacg 29

<210> 400
<211> 33
<212> DNA
<213> Artificial Sequence

<220>
<223> Oligonucleotide

<400> 400
cgcggatccc atatgggcag ccaatctgaa gaa 33

<210> 401
<211> 28
<212> DNA
<213> Artificial Sequence

<220>
<223> Oligonucleotide

<400> 401
cccgctcgag ctcagctttt gccgtcaa 28

<210> 402
<211> 33
<212> DNA
<213> Artificial Sequence

<220>
<223> Oligonucleotide

<400> 402

cgcggatccg ctagctactc atccattgtc cgc 33

<210> 403
<211> 29
<212> DNA
<213> Artificial Sequence

<220>
<223> Oligonucleotide

<400> 403
cccgctcgag ccagttgtag cctattttg 29

<210> 404
<211> 32
<212> DNA
<213> Artificial Sequence

<220>
<223> Oligonucleotide

<400> 404
cgcggatccg ctagcatgcg cttcacacac ac 32

<210> 405
<211> 30
<212> DNA
<213> Artificial Sequence

<220>
<223> Oligonucleotide

<400> 405
cccgctcgag ttaccagttg tagcctattt 30

<210> 406
<211> 32
<212> DNA
<213> Artificial Sequence

<220>
<223> Oligonucleotide

<400> 406
gccgccatat ggcacaaacg gaaggtttgg aa 32

<210> 407
<211> 36
<212> DNA
<213> Artificial Sequence

<220>
<223> Oligonucleotide

<400> 407
gccgcctcga gaaaactgta acgcaggttt gccgtc 36

<210> 408

<211> 32
<212> DNA
<213> Artificial Sequence

<220>
<223> Oligonucleotide

<400> 408
gcggccatat ggaagaaaca ccgcgcgaac cg 32

<210> 409
<211> 32
<212> DNA
<213> Artificial Sequence

<220>
<223> Oligonucleotide

<400> 409
gcggcctcga ggaacgtttt attaaactcg ac 32

<210> 410
<211> 32
<212> DNA
<213> Artificial Sequence

<220>
<223> Oligonucleotide

<400> 410
gcggccatat gagaaaaccg accgataccc ta 32

<210> 411
<211> 33
<212> DNA
<213> Artificial Sequence

<220>
<223> Oligonucleotide

<400> 411
gcggcctcga gtcaacgcca ctgccagcgg ttg 33

<210> 412
<211> 48
<212> DNA
<213> Artificial Sequence

<220>
<223> Oligonucleotide

<400> 412
cgcggatccc atatgaagaa gaacatattg gaattttggg tcggactg 48

<210> 413
<211> 38
<212> DNA
<213> Artificial Sequence

<220>
<223> Oligonucleotide

<400> 413
cccgctcgag ttattcggcg gctttttccg cattgccg 38

<210> 414
<211> 103
<212> DNA
<213> Artificial Sequence

<220>
<223> Oligonucleotide

<400> 414

gggaattcca tatgaaaaag acagctatcg cgattgcagt ggcactggct ggtttcgcta 60
ccgtagcgca ggccgctagc gctttccgcg tggccggcgg tgc 103

<210> 415
<211> 38
<212> DNA
<213> Artificial Sequence

<220>
<223> oligonucleotide

<400> 415
cccgctcgag ttattcggcg gctttttccg cattgccg 38

<210> 416
<211> 32
<212> DNA
<213> Artificial Sequence

<220>
<223> Oligonucleotide

<400> 416
catgccatgg ctttccgcgt ggccggcggt gc 32

<210> 417
<211> 38
<212> DNA
<213> Artificial Sequence

<220>
<223> Oligonucleotide

<400> 417
cccgctcgag ttattcggcg gctttttccg cattgccg 38

<210> 418
<211> 31
<212> DNA
<213> Artificial Sequence

<220>
<223> oligonucleotide

<400> 418
cgcggatccc atatgtttgc cgaaacccgc c 31

<210> 419
<211> 28
<212> DNA
<213> Artificial Sequence

<220>
<223> Oligonucleotide

<400> 419
cccgctcgag aggttgtgtt ccaggttg 28

<210> 420
<211> 31
<212> DNA
<213> Artificial Sequence

<220>
<223> Oligonucleotide

<400> 420
cgcggatccc atatgaaaaa aaccgcctat g 31

<210> 421
<211> 28
<212> DNA
<213> Artificial Sequence

<220>
<223> Oligonucleotide

<400> 421
cccgctcgag ttaaggttgt gttccagg 28

<210> 422
<211> 33
<212> DNA
<213> Artificial Sequence

<220>
<223> Oligonucleotide

<400> 422
cgcggatccc atatgaaaaa atacctattc cgc 33

<210> 423
<211> 27
<212> DNA
<213> Artificial Sequence

<220>
<223> Oligonucleotide

<400> 423
cccgctcgag ttacgggcgg tattcgg 27

<210> 424
<211> 34
<212> DNA
<213> Artificial Sequence

<220>
<223> Oligonucleotide

<400> 424
cgcggatccc atatgcaaag caagagcatc caaa 34

<210> 425
<211> 27
<212> DNA
<213> Artificial Sequence

<220>
<223> Oligonucleotide

<400> 425
cccgctcgag ttacgggcgg tattcgg 27

<210> 426
<211> 86
<212> DNA
<213> Artificial Sequence

<220>
<223> Oligonucleotide

<400> 426

gggaattcca tatgaaaacc ttcttcaaaa ccctttccgc cgccgcgcta gcgctcatcc 60
tcgccgcctg ccaaagcaag agcatc 86

<210> 427
<211> 38
<212> DNA
<213> Artificial Sequence

<220>
<223> Oligonucleotide

<400> 427
cccgctcgag ttacgggcgg tattcgggct tcataccg 38

<210> 428
<211> 33
<212> DNA
<213> Artificial Sequence

<220>
<223> Oligonucleotide

<400> 428
cgcggatccg tcgactgtgg gggcggcggt ggc 33

<210> 429
<211> 31
<212> DNA
<213> Artificial Sequence

<220>
<223> Oligonucleotide

<400> 429
cccgctcgag tcaatcctgc tcttttttgc c 31

<210> 430
<211> 30
<212> DNA
<213> Artificial Sequence

<220>
<223> Oligonucleotide

<400> 430
gcggccatat gaagaaaaca ttgacactgc 30

<210> 431
<211> 32
<212> DNA
<213> Artificial Sequence

<220>
<223> Oligonucleotide

<400> 431
gcggcctcga gttaatggtg cgaatgaccg at 32

<210> 432
<211> 46
<212> DNA
<213> Artificial Sequence

<220>
<223> Oligonucleotide

<400> 432
ggggacaagt ttgtacaaaa aagcaggctt gcggcaagga tgccgg 46

<210> 433
<211> 47
<212> DNA
<213> Artificial Sequence

<220>
<223> Oligonucleotide

<400> 433
ggggaccact ttgtacaaga aagctgggtc taaagcaaca atgccgg 47

<210> 434
<211> 30
<212> DNA
<213> Artificial Sequence

<220>
<223> Oligonucleotide

<400> 434
cgcggatccc atatgaaaca caccgtatcc 30

<210> 435
<211> 26
<212> DNA
<213> Artificial Sequence

<220>
<223> Oligonucleotide

<400> 435
cccgctcgag ttatctcgtg cgcgcc 26

<210> 436
<211> 30
<212> DNA
<213> Artificial Sequence

<220>
<223> Oligonucleotide

<400> 436
cgcggatccc atatgagccc cgcgccgatt 30

<210> 437
<211> 28
<212> DNA
<213> Artificial Sequence

<220>
<223> oligonucleotide

<400> 437
cccgctcgag tttttgtgcg gtcaggcg 28

<210> 438
<211> 62
<212> DNA
<213> Artificial Sequence

<220>
<223> Oligonucleotide

<400> 438

ggggacaagt ttgtacaaaa aagcaggctt gttcgtttgg gggatttaaa ccaaaccaaa 60
tc 62

<210> 439
<211> 30
<212> DNA
<213> Artificial Sequence

<220>
<223> oligonucleotide

<400> 439
cgcggatccc atatggcgga tgcgcccgcg 30

<210> 440
<211> 26
<212> DNA
<213> Artificial Sequence

<220>
<223> Oligonucleotide

<400> 440
cccgctcgag aaaccgccaa tccgcc 26

<210> 441
<211> 61
<212> DNA
<213> Artificial Sequence

<220>
<223> Oligonucleotide

<400> 441

ggggaccact ttgtacaaga aagctgggtt cattttgttt ttccttcttc tcgaggccat 60
t 61

<210> 442
<211> 30
<212> DNA
<213> Artificial Sequence

<220>
<223> Oligonucleotide

<400> 442
cgcggatccc atatgaaacc caaaccgcac 30

<210> 443
<211> 27
<212> DNA
<213> Artificial Sequence

<220>
<223> Oligonucleotide

<400> 443
cccgctcgag tcagcgttgg acgtagt 27

<210> 444
<211> 33
<212> DNA
<213> Artificial Sequence

<220>
<223> Oligonucleotide

<400> 444
gggaattcca tatgaaaaaa atcatcttcg ccg 33

<210> 445
<211> 31
<212> DNA
<213> Artificial Sequence

<220>
<223> Oligonucleotide

<400> 445
cccgctcgag ttattgtttg gctgcctcga t 31

<210> 446
<211> 33
<212> DNA
<213> Artificial Sequence

<220>
<223> Oligonucleotide

<400> 446
gggaattcca tatggccacc tacaaagtgg acg 33

<210> 447
<211> 30
<212> DNA
<213> Artificial Sequence

<220>
<223> Oligonucleotide

<400> 447
cggggatcct tgtttggctg cctcgatttg 30

<210> 448
<211> 34
<212> DNA
<213> Artificial Sequence

<220>
<223> Oligonucleotide

<400> 448
cgcggatccc atatgcaaga acaatcgcag aaag 34

<210> 449
<211> 30
<212> DNA

<213> Artificial Sequence

<220>
<223> Oligonucleotide

<400> 449
cccgctcgag ttttttcggc aaattggctt 30

<210> 450
<211> 45
<212> DNA
<213> Artificial Sequence

<220>
<223> Oligonucleotide

<400> 450
ggggacaagt ttgtacaaaa aagcaggctg ccgatgccgt tgcgg 45

<210> 451
<211> 47
<212> DNA
<213> Artificial Sequence

<220>
<223> Oligonucleotide

<400> 451
ggggaccact ttgtacaaga aagctgggtt cagggtcgtt tgttgcg 47

<210> 452
<211> 30
<212> DNA
<213> Artificial Sequence

<220>
<223> Oligonucleotide

<400> 452
cgcggatccc atatgaaaca ctttccatcc 30

<210> 453
<211> 28
<212> DNA
<213> Artificial Sequence

<220>
<223> Oligonucleotide

<400> 453
cccgctcgag ttaccactcg taattgac 28

<210> 454
<211> 30
<212> DNA
<213> Artificial Sequence

<220>

<223> Oligonucleotide

<400> 454
cgcggatccc atatggccac aagcgacgac 30

<210> 455
<211> 28
<212> DNA
<213> Artificial Sequence

<220>
<223> Oligonucleotide

<400> 455
cccgctcgag ttaccactcg taattgac 28

<210> 456
<211> 28
<212> DNA
<213> Artificial Sequence

<220>
<223> Oligonucleotide

<400> 456
cgcggatccc atatggccac aaacgacg 28

<210> 457
<211> 28
<212> DNA
<213> Artificial Sequence

<220>
<223> Oligonucleotide

<400> 457
cccgctcgag acccacgttg taaggttg 28

<210> 458
<211> 32
<212> DNA
<213> Artificial Sequence

<220>
<223> Oligonucleotide

<400> 458
cgcggatccc atatggccac aagcgacgac ga 32

<210> 459
<211> 28
<212> DNA
<213> Artificial Sequence

<220>
<223> Oligonucleotide

<400> 459

cccgctcgag acccacgttg taaggttg 28

<210> 460
<211> 33
<212> DNA
<213> Artificial Sequence

<220>
<223> Oligonucleotide

<400> 460
cgcggatccc atatgatgaa acactttcca tcc 33

<210> 461
<211> 29
<212> DNA
<213> Artificial Sequence

<220>
<223> Oligonucleotide

<400> 461
cccgctcgag ttaacccacg ttgtaaggt 29

<210> 462
<211> 33
<212> DNA
<213> Artificial Sequence

<220>
<223> Oligonucleotide

<400> 462
cgcggatccc atatgatgaa acactttcca tcc 33

<210> 463
<211> 29
<212> DNA
<213> Artificial Sequence

<220>
<223> Oligonucleotide

<400> 463
cccgctcgag ttaacccacg ttgtaaggt 29

<210> 464
<211> 28
<212> DNA
<213> Artificial Sequence

<220>
<223> Oligonucleotide

<400> 464
cgcggatccc atatggccac aaacgacg 28

<210> 465

<211> 29
<212> DNA
<213> Artificial Sequence

<220>
<223> Oligonucleotide

<400> 465
cccgctcgag gtctgacact gttttatcc 29

<210> 466
<211> 33
<212> DNA
<213> Artificial Sequence

<220>
<223> Oligonucleotide

<400> 466
cgcggatccc atatgatgaa acactttcca tcc 33

<210> 467
<211> 29
<212> DNA
<213> Artificial Sequence

<220>
<223> Oligonucleotide

<400> 467
cccgctcgag ttatgctttg gcggcaaag 29

<210> 468
<211> 30
<212> DNA
<213> Artificial Sequence

<220>
<223> Oligonucleotide

<400> 468
cgcggatccc atatggccac aaacgacgac 30

<210> 469
<211> 27
<212> DNA
<213> Artificial Sequence

<220>
<223> Oligonucleotide

<400> 469
cgcggatccc cactcgtaat tgacgcc 27

<210> 470
<211> 30
<212> DNA
<213> Artificial Sequence

<220>
<223> Oligonucleotide

<400> 470
cgcggatccc atatggccac aagcgacgac 30

<210> 471
<211> 27
<212> DNA
<213> Artificial Sequence

<220>
<223> Oligonucleotide

<400> 471
cgcggatccc cactcgtaat tgacgcc 27

<210> 472
<211> 30
<212> DNA
<213> Artificial Sequence

<220>
<223> Oligonucleotide

<400> 472
cgcggatccc atatggccac aaacgacgac 30

<210> 473
<211> 27
<212> DNA
<213> Artificial Sequence

<220>
<223> Oligonucleotide

<400> 473
cgcggatcca cccacgttgt aaggttg 27

<210> 474
<211> 33
<212> DNA
<213> Artificial Sequence

<220>
<223> Oligonucleotide

<400> 474
cgcggatccc atatgatgaa acactttcca tcc 33

<210> 475
<211> 27
<212> DNA
<213> Artificial Sequence

<220>
<223> Oligonucleotide

<400> 475
cgcggatcca cccacgttgt aaggttg 27

<210> 476
<211> 25
<212> DNA
<213> Artificial Sequence

<220>
<223> Oligonucleotide

<400> 476
cgcggatccg gagggggtgg tgtcg 25

<210> 477
<211> 27
<212> DNA
<213> Artificial Sequence

<220>
<223> Oligonucleotide

<400> 477
cccgctcgag ttgcttggcg gcaaggc 27

<210> 478
<211> 25
<212> DNA
<213> Artificial Sequence

<220>
<223> Oligonucleotide

<400> 478
cgcggatccg gcggaggcgg cactt 25

<210> 479
<211> 26
<212> DNA
<213> Artificial Sequence

<220>
<223> Oligonucleotide

<400> 479
cccgctcgag gaaccggtag cctacg 26

<210> 480
<211> 41
<212> DNA
<213> Artificial Sequence

<220>
<223> Oligonucleotide

<400> 480
cgcggatccg gtggtggtgg ttcagatttg gcaaacgatt c 41

<210> 481
<211> 29
<212> DNA
<213> Artificial Sequence

<220>
<223> Oligonucleotide

<400> 481
cccgctcgag cgtatcatat ttcacgtgc 29

<210> 482
<211> 25
<212> DNA
<213> Artificial Sequence

<220>
<223> Oligonucleotide

<400> 482
cgcggatccg gagggggtgg tgtcg 25

<210> 483
<211> 28
<212> DNA
<213> Artificial Sequence

<220>
<223> Oligonucleotide

<400> 483
cccgctcgag ttattgcttg gcggcaag 28

<210> 484
<211> 25
<212> DNA
<213> Artificial Sequence

<220>
<223> Oligonucleotide

<400> 484
cgcggatccg gcggaggcgg cactt 25

<210> 485
<211> 28
<212> DNA
<213> Artificial Sequence

<220>
<223> Oligonucleotide

<400> 485
cccgctcgag tcagaaccgg tagcctac 28

<210> 486
<211> 41
<212> DNA

<213> Artificial Sequence

<220>
<223> Oligonucleotide

<400> 486
cgcggatccg gtggtggtgg ttcagatttg gcaaacgatt c 41

<210> 487
<211> 32
<212> DNA
<213> Artificial Sequence

<220>
<223> Oligonucleotide

<400> 487
cccgctcgag ttacgtatca tatttcacgt gc 32

<210> 488
<211> 31
<212> DNA
<213> Artificial Sequence

<220>
<223> Oligonucleotide

<400> 488
cgcggatccc atatggccac aagcgacgac g 31

<210> 489
<211> 28
<212> DNA
<213> Artificial Sequence

<220>
<223> Oligonucleotide

<400> 489
cccgctcgag ccactcgtaa ttgacgcc 28

<210> 490
<211> 30
<212> DNA
<213> Artificial Sequence

<220>
<223> Oligonucleotide

<400> 490
cgcggatccc atatggccac aaacgacgac 30

<210> 491
<211> 28
<212> DNA
<213> Artificial Sequence

<220>

<223> Oligonucleotide

<400> 491
cccgctcgag tgctttggcg gcaaagtt 28

<210> 492
<211> 30
<212> DNA
<213> Artificial Sequence

<220>
<223> Oligonucleotide

<400> 492
cgcggatccc atatggccac aaacgacgac 30

<210> 493
<211> 37
<212> DNA
<213> Artificial Sequence

<220>
<223> Oligonucleotide

<400> 493
cccgctcgag tttagcaata ttatctttgt tcgtagc 37

<210> 494
<211> 32
<212> DNA
<213> Artificial Sequence

<220>
<223> Oligonucleotide

<400> 494
cgcggatccc atatgaaagc aaaccgtgcc ga 32

<210> 495
<211> 28
<212> DNA
<213> Artificial Sequence

<220>
<223> Oligonucleotide

<400> 495
cccgctcgag ccactcgtaa ttgacgcc 28

<210> 496
<211> 61
<212> DNA
<213> Artificial Sequence

<220>
<223> Oligonucleotide

<400> 496

ggggacaagt ttgtacaaaa aagcaggctg cagccacaaa cgacgacgat gttaaaaaag 60
c 61

<210> 497
<211> 61
<212> DNA
<213> Artificial Sequence

<220>
<223> Oligonucleotide

<400> 497

ggggaccact ttgtacaaga aagctgggtt taccactcgt aattgacgcc gacatggtag 60
g 61

<210> 498
<211> 31
<212> DNA
<213> Artificial Sequence

<220>
<223> Oligonucleotide

<400> 498
gcggccatat ggcagcaaaa gacgtacagt t 31

<210> 499
<211> 33
<212> DNA
<213> Artificial Sequence

<220>
<223> Oligonucleotide

<400> 499
gcggcctcga gttacatcat gccgcccata cca 33

<210> 500
<211> 31
<212> DNA
<213> Artificial Sequence

<220>
<223> Oligonucleotide

<400> 500
cgcggatccg ctagcttagg cggcggcgga g 31

<210> 501
<211> 26
<212> DNA
<213> Artificial Sequence

<220>
<223> Oligonucleotide

<400> 501
cccgctcgag gaaccggtag cctacg 26

<210> 502
<211> 29
<212> DNA
<213> Artificial Sequence

<220>
<223> Oligonucleotide

<400> 502
cccctagcta gcacttctgc gcccgactt 29

<210> 503
<211> 26
<212> DNA
<213> Artificial Sequence

<220>
<223> Oligonucleotide

<400> 503
cccgctcgag gaaccggtag cctacg 26

<210> 504
<211> 31
<212> DNA
<213> Artificial Sequence

<220>
<223> Oligonucleotide

<400> 504
cgcggatccg ctagcttagg cggcggcgga g 31

<210> 505
<211> 26
<212> DNA
<213> Artificial Sequence

<220>
<223> Oligonucleotide

<400> 505
cccgctcgag gaaccggtag cctacg 26

<210> 506
<211> 32
<212> DNA
<213> Artificial Sequence

<220>
<223> Oligonucleotide

<400> 506
cgcggatccg ctagcacttc tgcgcccgac tt 32

<210> 507
<211> 26
<212> DNA
<213> Artificial Sequence

<220>
<223> Oligonucleotide

<400> 507
cccgctcgag gaaccggtag cctacg 26

<210> 508
<211> 50
<212> DNA
<213> Artificial Sequence

<220>
<223> Oligonucleotide

<400> 508
cgcggatccg ctagccgaac gaccccaacc ttccctacaa aaactttcaa 50

<210> 509
<211> 35
<212> DNA
<213> Artificial Sequence

<220>
<223> Oligonucleotide

<400> 509
cccgctcgag tcagaaccga cgtgccaagc cgttc 35

<210> 510
<211> 32
<212> DNA
<213> Artificial Sequence

<220>
<223> Oligonucleotide

<400> 510
gccgccatat gcccccactg gaagaacgga cg 32

<210> 511
<211> 35
<212> DNA
<213> Artificial Sequence

<220>
<223> Oligonucleotide

<400> 511
gccgcctcga gtaataaacc ttctatgggc agcag 35

<210> 512
<211> 31
<212> DNA

<213> Artificial Sequence

<220>
<223> Oligonucleotide

<400> 512
cgcggatccc atatgtccgt ccacgcatcc g 31

<210> 513
<211> 31
<212> DNA
<213> Artificial Sequence

<220>
<223> Oligonucleotide

<400> 513
cccgctcgag tttgaatttg taggtgtatt g 31

<210> 514
<211> 29
<212> DNA
<213> Artificial Sequence

<220>
<223> Oligonucleotide

<400> 514
cgcggatccc atatgacccc ttccgcact 29

<210> 515
<211> 32
<212> DNA
<213> Artificial Sequence

<220>
<223> Oligonucleotide

<400> 515
cccgctcgag ttatttgaat ttgtaggtgt at 32

<210> 516
<211> 33
<212> DNA
<213> Artificial Sequence

<220>
<223> Oligonucleotide

<400> 516
cgcggatccc atatgaaaac caattcagaa gaa 33

<210> 517
<211> 28
<212> DNA
<213> Artificial Sequence

<220>

<223> Oligonucleotide

<400> 517
cccgctcgag tccacagaga ttgtttcc 28

<210> 518
<211> 17
<212> DNA
<213> Artificial Sequence

<220>
<223> Oligonucleotide

<400> 518
gatgcccgaa gggcggg 17

<210> 519
<211> 29
<212> DNA
<213> Artificial Sequence

<220>
<223> Oligonucleotide

<400> 519
gcccaagctt tcagaagaag acttcacgc 29

<210> 520
<211> 36
<212> DNA
<213> Artificial Sequence

<220>
<223> Oligonucleotide

<400> 520
cgcggatccc atatgcaaac ccataaatac gctatt 36

<210> 521
<211> 29
<212> DNA
<213> Artificial Sequence

<220>
<223> Oligonucleotide

<400> 521
gcccaagctt gaagaagact tcacgccag 29

<210> 522
<211> 35
<212> DNA
<213> Artificial Sequence

<220>
<223> Oligonucleotide

<400> 522

cgcggatccc atatggtctt tttcgacaat accga 35

<210> 523
<211> 10
<212> DNA
<213> Artificial Sequence

<220>
<223> Oligonucleotide

<400> 523
gcccaagctt 10

<210> 524
<211> 36
<212> DNA
<213> Artificial Sequence

<220>
<223> Oligonucleotide

<400> 524
cgcggatccc atatgaataa aactttaaaa aggcgg 36

<210> 525
<211> 29
<212> DNA
<213> Artificial Sequence

<220>
<223> Oligonucleotide

<400> 525
gcccaagctt tcagaagaag acttcacgc 29

<210> 526
<211> 35
<212> DNA
<213> Artificial Sequence

<220>
<223> Oligonucleotide

<400> 526
cgcgaatccc atatgttcga tcttgattct gtcga 35

<210> 527
<211> 28
<212> DNA
<213> Artificial Sequence

<220>
<223> Oligonucleotide

<400> 527
cccgctcgag tcgcacaggc tgttggcg 28

<210> 528

<211> 32
<212> DNA
<213> Artificial Sequence

<220>
<223> Oligonucleotide

<400> 528
cgcgaatccc atatgttggg cggaggcggc ag 32

<210> 529
<211> 28
<212> DNA
<213> Artificial Sequence

<220>
<223> Oligonucleotide

<400> 529
cccgctcgag tcgcacaggc tgttggcg 28

<210> 530
<211> 32
<212> DNA
<213> Artificial Sequence

<220>
<223> Oligonucleotide

<400> 530
cgcgaatccc atatgttggg cggaggcggc ag 32

<210> 531
<211> 28
<212> DNA
<213> Artificial Sequence

<220>
<223> Oligonucleotide

<400> 531
cccgctcgag tcgcacaggc tgttggcg 28

<210> 532
<211> 33
<212> DNA
<213> Artificial Sequence

<220>
<223> Oligonucleotide

<400> 532
cgcggatccc atatggcaaa tttggaggtg cgc 33

<210> 533
<211> 27
<212> DNA
<213> Artificial Sequence

<220>
<223> Oligonucleotide

<400> 533
cccgctcgag ttcggagcgg ttgaagc 27

<210> 534
<211> 34
<212> DNA
<213> Artificial Sequence

<220>
<223> Oligonucleotide

<400> 534
cgcggatccc atatgcaacg tcgtattata accc 34

<210> 53.5
<211> 29
<212> DNA
<213> Artificial Sequence

<220>
<223> Oligonucleotide

<400> 535
cccgctcgag ttattcggag cggttgaag 29

<210> 536
<211> 42
<212> DNA
<213> Artificial Sequence

<220>
<223> Oligonucleotide

<400> 536
cgcggatccc atatgggcat caaagtcgcc atcaacggct ac 42

<210> 537
<211> 35
<212> DNA
<213> Artificial Sequence

<220>
<223> Oligonucleotide

<400> 537
cccgctcgag tttgagcggg cgcacttcaa gtccg 35

<210> 538
<211> 33
<212> DNA
<213> Artificial Sequence

<220>
<223> Oligonucleotide

<400> 538
cgcggatccc atatgggcgg cagcgaaaaa aac 33

<210> 539
<211> 28
<212> DNA
<213> Artificial Sequence

<220>
<223> Oligonucleotide

<400> 539
cccgctcgag gttggtgccg actttgat 28

<210> 540
<211> 31
<212> DNA
<213> Artificial Sequence

<220>
<223> Oligonucleotide

<400> 540
cgcggatccc atatgggcgg cggaagcgat a 31

<210> 541
<211> 27
<212> DNA
<213> Artificial Sequence

<220>
<223> Oligonucleotide

<400> 541
cccgctcgag tttgcccgct ttgagcc 27

<210> 542
<211> 33
<212> DNA
<213> Artificial Sequence

<220>
<223> Oligonucleotide

<400> 542
cgcggatccc atatgggcaa atccgaaaat acg 33

<210> 543
<211> 27
<212> DNA
<213> Artificial Sequence

<220>
<223> Oligonucleotide

<400> 543
cccgctcgag catcccgtac tgtttcg 27

<210> 544
<211> 62
<212> DNA
<213> Artificial Sequence

<220>
<223> Oligonucleotide

<400> 544

ggggacaagt ttgtacaaaa aagcaggctc cgacattacc gtgtacaacg gccaacaaag 60
aa 62

<210> 545
<211> 61
<212> DNA
<213> Artificial Sequence

<220>
<223> Oligonucleotide

<400> 545

ggggaccact ttgtacaaga aagctgggtc ttatttcata ccggcttgct caagcagccg 60
g 61

<210> 546
<211> 61
<212> DNA
<213> Artificial Sequence

<220>
<223> Oligonucleotide

<400> 546

ggggacaagt ttgtacaaaa aagcaggctg atacggtgtt ttcctgtaaa acggacaaca 60
a 61

<210> 547
<211> 60
<212> DNA
<213> Artificial Sequence

<220>
<223> Oligonucleotide

<400> 547
ggggaccact ttgtacaaga aagctgggtc taggaaaaat cgtcatcgtt gaaattcgcc 60

<210> 548
<211> 47
<212> DNA
<213> Artificial Sequence

<220>
<223> Oligonucleotide

<400> 548
ggggacaagt ttgtacaaaa aagcaggcta tgcaccccat cgaaacc 47

<210> 549
<211> 47
<212> DNA
<213> Artificial Sequence

<220>
<223> Oligonucleotide

<400> 549
ggggaccact ttgtacaaga aagctgggtc tagtcttgca gtgcctc 47

<210> 550
<211> 41
<212> DNA
<213> Artificial Sequence

<220>
<223> Oligonucleotide

<400> 550
cgcggatccc atatgggaaa tttcttatat agaggcatta g 41

<210> 551
<211> 40
<212> DNA
<213> Artificial Sequence

<220>
<223> Oligonucleotide

<400> 551
cccgctcgag gttaatttct atcaactctt tagcaataat 40

<210> 552
<211> 31
<212> DNA
<213> Artificial Sequence

<220>
<223> Oligonucleotide

<400> 552
cgcggatccc atatggcctg ccaagacgac a 31

<210> 553
<211> 26
<212> DNA
<213> Artificial Sequence

<220>
<223> Oligonucleotide

<400> 553
cccgctcgag ccgcctcctg ccgaaa 26

<210> 554
<211> 34
<212> DNA
<213> Artificial Sequence

<220>
<223> Oligonucleotide

<400> 554
cgcggatccc atatggcaga gatctgtttg ataa 34

<210> 555
<211> 27
<212> DNA
<213> Artificial Sequence

<220>
<223> Oligonucleotide

<400> 555
cccgctcgag cggttttccg cccaatg 27

<210> 556
<211> 30
<212> DNA
<213> Artificial Sequence

<220>
<223> Oligonucleotide

<400> 556
cgcggatccc atatgcagcc ggatacggtc 30

<210> 557
<211> 30
<212> DNA
<213> Artificial Sequence

<220>
<223> Oligonucleotide

<400> 557
cccgctcgag aatcacttcc aacacaaaat 30

<210> 558
<211> 33
<212> DNA
<213> Artificial Sequence

<220>
<223> Oligonucleotide

<400> 558
cgcggatccc atatgtggtt gctgatgaag ggc 33

<210> 559
<211> 28
<212> DNA
<213> Artificial Sequence

<220>
<223> Oligonucleotide

<400> 559
cccgctcgag gactgcttca tcttctgc 28

<210> 560
<211> 34
<212> DNA
<213> Artificial Sequence

<220>
<223> Oligonucleotide

<400> 560
cgcggatccc atatggaact gatgactgtt ttgc 34

<210> 561
<211> 29
<212> DNA
<213> Artificial Sequence

<220>
<223> Oligonucleotide

<400> 561
cccgctcgag tcagactgct tcatcttct 29

<210> 562
<211> 45
<212> DNA
<213> Artificial Sequence

<220>
<223> Oligonucleotide

<400> 562
cgcggatccc atatgagcat taaagtagcg attaacggtt tcggc 45

<210> 563
<211> 40
<212> DNA
<213> Artificial Sequence

<220>
<223> Oligonucleotide

<400> 563
cccgctcgag gattttgcct gcgaagtatt ccaaagtgcg 40

<210> 564
<211> 32
<212> DNA

<213> Artificial Sequence

<220>
<223> Oligonucleotide

<400> 564
cgcggatccg ctagccccga tgttaaatcg gc 32

<210> 565
<211> 29
<212> DNA
<213> Artificial Sequence

<220>
<223> Oligonucleotide

<400> 565
cggggatcca tcctgctctt ttttgccgg 29

<210> 566
<211> 42
<212> DNA
<213> Artificial Sequence

<220>
<223> Oligonucleotide

<400> 566
cgcggatccg gtggtggtgg tcaaagcaag agcatccaaa cc 42

<210> 567
<211> 30
<212> DNA
<213> Artificial Sequence

<220>
<223> Oligonucleotide

<400> 567
cccaagcttt tcgggcggta ttcgggcttc 30

<210> 568
<211> 39
<212> DNA
<213> Artificial Sequence

<220>
<223> Oligonucleotide

<400> 568
cgcggatccg gtggtggtgg tgccacctac aaagtggac 39

<210> 569
<211> 28
<212> DNA
<213> Artificial Sequence

<220>

<223> Oligonucleotide

<400> 569
gcccaagctt ttgtttggct gcctcgat 28

<210> 570
<211> 34
<212> DNA
<213> Artificial Sequence

<220>
<223> Oligonucleotide

<400> 570
cgcggatccg gtggtggtgg tacaagcgac gacg 34

<210> 571
<211> 28
<212> DNA
<213> Artificial Sequence

<220>
<223> Oligonucleotide

<400> 571
gcccaagctt ccactcgtaa ttgacgcc 28

<210> 572
<211> 41
<212> DNA
<213> Artificial Sequence

<220>
<223> Oligonucleotide

<400> 572
cgcggatccg gtggtggtgg ttcagatttg gcaaacgatt c 41

<210> 573
<211> 28
<212> DNA
<213> Artificial Sequence

<220>
<223> Oligonucleotide

<400> 573
cccaagcttc gtatcatatt tcacgtgc 28

<210> 574
<211> 44
<212> DNA
<213> Artificial Sequence

<220>
<223> Oligonucleotide

<400> 574

cccaagcttg gtggtggtgg tggttcagat ttggcaaacg attc 44

<210> 575
<211> 29
<212> DNA
<213> Artificial Sequence

<220>
<223> Oligonucleotide

<400> 575
cccgctcgag cgtatcatat ttcacgtgc 29

<210> 576
<211> 45
<212> DNA
<213> Artificial Sequence

<220>
<223> Oligonucleotide

<400> 576
cccaagcttg gtggtggtgg tggtcaaagc aagagcatcc aaacc 45

<210> 577
<211> 28
<212> DNA
<213> Artificial Sequence

<220>
<223> Oligonucleotide

<400> 577
cccgctcgag cgggcggtat tcgggctt 28

<210> 578
<211> 32
<212> DNA
<213> Artificial Sequence

<220>
<223> Oligonucleotide

<400> 578
cgcggatccg ctagccccga tgttaaatcg gc 32

<210> 579
<211> 29
<212> DNA
<213> Artificial Sequence

<220>
<223> Oligonucleotide

<400> 579
cggggatcca tcctgctctt ttttgccgg 29

<210> 580

<211> 36
<212> DNA
<213> Artificial Sequence

<220>
<223> Oligonucleotide

<400> 580
cgcggatccg ctagcggaca cacttatttc ggcatc 36

<210> 581
<211> 30
<212> DNA
<213> Artificial Sequence

<220>
<223> Oligonucleotide

<400> 581
cgcggatccc cagcggtagc ctaatttgat 30

<210> 582
<211> 41
<212> DNA
<213> Artificial Sequence

<220>
<223> Oligonucleotide

<400> 582
cgcggatccg gtggtggtgg ttcagatttg gcaaacgatt c 41

<210> 583
<211> 28
<212> DNA
<213> Artificial Sequence

<220>
<223> Oligonucleotide

<400> 583
cccaagcttc gtatcatatt tcacgtgc 28

<210> 584
<211> 36
<212> DNA
<213> Artificial Sequence

<220>
<223> Oligonucleotide

<400> 584
gcggcgtcga cggtggcgga ggcactggat cctcag 36

<210> 585
<211> 35
<212> DNA
<213> Artificial Sequence

<220>
<223> Oligonucleotide

<400> 585
ggaggcactg gatcctcaga tttggcaaac gattc 35

<210> 586
<211> 29
<212> DNA
<213> Artificial Sequence

<220>
<223> Oligonucleotide

<400> 586
cccgctcgag cgtatcatat ttcacgtgc 29

<210> 587
<211> 33
<212> DNA
<213> Artificial Sequence

<220>
<223> Oligonucleotide

<400> 587
ggaattccat atgtcagatt tggcaaacga ttc 33

<210> 588
<211> 28
<212> DNA
<213> Artificial Sequence

<220>
<223> Oligonucleotide

<400> 588
cgcggatccc gtatcatatt tcacgtgc 28

<210> 589
<211> 25
<212> DNA
<213> Artificial Sequence

<220>
<223> Oligonucleotide

<400> 589
cggggatccg gggcggcgg tggcg 25

<210> 590
<211> 30
<212> DNA
<213> Artificial Sequence

<220>
<223> Oligonucleotide

<400> 590
cccaagctta tcctgctctt ttttgccggc 30

<210> 591
<211> 42
<212> DNA
<213> Artificial Sequence

<220>
<223> Oligonucleotide

<400> 591
cgcggatccg gtggtggtgg tcaaagcaag agcatccaaa cc 42

<210> 592
<211> 28
<212> DNA
<213> Artificial Sequence

<220>
<223> Oligonucleotide

<400> 592
cccaagcttc gggcggtatt cgggcttc 28

<210> 593
<211> 26
<212> DNA
<213> Artificial Sequence

<220>
<223> Oligonucleotide

<400> 593
ccccaagctt gggggcggcg gtggcg 26

<210> 594
<211> 31
<212> DNA
<213> Artificial Sequence

<220>
<223> Oligonucleotide

<400> 594
cccgctcgag atcctgctct tttttgccgg c 31

<210> 595
<211> 45
<212> DNA
<213> Artificial Sequence

<220>
<223> Oligonucleotide

<400> 595
cccaagcttg gtggtggtgg tggtcaaagc aagagcatcc aaacc 45

<210> 596
<211> 28
<212> DNA
<213> Artificial Sequence

<220>
<223> Oligonucleotide

<400> 596
cccgctcgag cgggcggtat tcgggctt 28

<210> 597
<211> 35
<212> DNA
<213> Artificial Sequence

<220>
<223> Oligonucleotide

<400> 597
ggaggcactg gatccgcagc cacaaacgac gacga 35

<210> 598
<211> 36
<212> DNA
<213> Artificial Sequence

<220>
<223> Oligonucleotide

<400> 598
gcggcctcga gggtggcgga ggcactggat ccgcag 36

<210> 599
<211> 28
<212> DNA
<213> Artificial Sequence

<220>
<223> Oligonucleotide

<400> 599
cccgctcgag acccagcttg taaggttg 28

<210> 600
<211> 35
<212> DNA
<213> Artificial Sequence

<220>
<223> Oligonucleotide

<400> 600
ggaggcactg gatccgcagc cacaaacgac gacga 35

<210> 601
<211> 36
<212> DNA

<213> Artificial Sequence

<220>
<223> Oligonucleotide

<400> 601
gcggcctcga gggtggcgga ggcactggat ccgcag 36

<210> 602
<211> 28
<212> DNA
<213> Artificial Sequence

<220>
<223> Oligonucleotide

<400> 602
cccgctcgag ccactcgtaa ttgacgcc 28

<210> 603
<211> 38
<212> DNA
<213> Artificial Sequence

<220>
<223> Oligonucleotide

<400> 603
gcggcctcga gggatccggc ggaggcggca cttctgcg 38

<210> 604
<211> 26
<212> DNA
<213> Artificial Sequence

<220>
<223> Oligonucleotide

<400> 604
cccgctcgag gaaccggtag cctacg 26

<210> 605
<211> 35
<212> DNA
<213> Artificial Sequence

<220>
<223> Oligonucleotide

<400> 605
ggaggcactg gatcctcaga tttggcaaac gattc 35

<210> 606
<211> 37
<212> DNA
<213> Artificial Sequence

<220>

<223> Oligonucleotide

<400> 606
gcggcgtcga cggtggcgga ggcactggat cctcaga 37

<210> 607
<211> 29
<212> DNA
<213> Artificial Sequence

<220>
<223> Oligonucleotide

<400> 607
cccgctcgag cgtatcatat ttcacgtgc 29

<210> 608
<211> 35
<212> DNA
<213> Artificial Sequence

<220>
<223> Oligonucleotide

<400> 608
gcggcctcga gggatccgga gggggtggtg tcgcc 35

<210> 609
<211> 25
<212> DNA
<213> Artificial Sequence

<220>
<223> Oligonucleotide

<400> 609
cccgctcgag ttgcttggcg gcaag 25

<210> 610
<211> 35
<212> DNA
<213> Artificial Sequence

<220>
<223> Oligonucleotide

<400> 610
ggaggcactg gatccgcagc cacaaacgac gacga 35

<210> 611
<211> 36
<212> DNA
<213> Artificial Sequence

<220>
<223> Oligonucleotide

<400> 611

gcggcctcga gggtggcgga ggcactggat ccgcag 36

<210> 612
<211> 28
<212> DNA
<213> Artificial Sequence

<220>
<223> Oligonucleotide

<400> 612
cccgctcgag acccagcttg taaggttg 28

<210> 613
<211> 35
<212> DNA
<213> Artificial Sequence

<220>
<223> Oligonucleotide

<400> 613
ggaggcactg gatccgcagc cacaaacgac gacga 35

<210> 614
<211> 36
<212> DNA
<213> Artificial Sequence

<220>
<223> Oligonucleotide

<400> 614
gcggcctcga gggtggcgga ggcactggat ccgcag 36

<210> 615
<211> 28
<212> DNA
<213> Artificial Sequence

<220>
<223> Oligonucleotide

<400> 615
cccgctcgag ccactcgtaa ttgacgcc 28

<210> 616
<211> 35
<212> DNA
<213> Artificial Sequence

<220>
<223> Oligonucleotide

<400> 616
ggaggcactg gatcctcaga tttggcaaac gattc 35

<210> 617

<211> 37
<212> DNA
<213> Artificial Sequence

<220>
<223> Oligonucleotide

<400> 617
gcggcgtcga cggtggcgga ggcactggat cctcaga 37

<210> 618
<211> 29
<212> DNA
<213> Artificial Sequence

<220>
<223> Oligonucleotide

<400> 618
cccgctcgag cgtatcatat ttcacgtgc 29

<210> 619
<211> 488
<212> PRT
<213> Artificial Sequence

<220>
<223> MC58

<400> 619

Met Phe Lys Arg Ser Val Ile Ala Met Ala Cys Ile Phe Ala Leu Ser
1 5 10 15

Ala Cys Gly Gly Gly Gly Gly Gly Ser Pro Asp Val Lys Ser Ala Asp
20 25 30

Thr Leu Ser Lys Pro Ala Ala Pro Val Val Ser Glu Lys Glu Thr Glu
35 40 45

Ala Lys Glu Asp Ala Pro Gln Ala Gly Ser Gln Gly Gln Gly Ala Pro
50 55 60

Ser Ala Gln Gly Ser Gln Asp Met Ala Ala Val Ser Glu Glu Asn Thr
65 70 75 80

Gly Asn Gly Gly Ala Val Thr Ala Asp Asn Pro Lys Asn Glu Asp Glu
85 90 95

Val Ala Gln Asn Asp Met Pro Gln Asn Ala Ala Gly Thr Asp Ser Ser
100 105 110

Thr Pro Asn His Thr Pro Asp Pro Asn Met Leu Ala Gly Asn Met Glu
115 120 125

Asn Gln Ala Thr Asp Ala Gly Glu Ser Ser Gln Pro Ala Asn Gln Pro
130 135 140

Asp Met Ala Asn Ala Ala Asp Gly Met Gln Gly Asp Asp Pro Ser Ala
145 150 155 160

Gly Gly Gln Asn Ala Gly Asn Thr Ala Ala Gln Gly Ala Asn Gln Ala
165 170 175

Gly Asn Asn Gln Ala Ala Gly Ser Ser Asp Pro Ile Pro Ala Ser Asn
180 185 190

Pro Ala Pro Ala Asn Gly Gly Ser Asn Phe Gly Arg Val Asp Leu Ala
195 200 205

Asn Gly Val Leu Ile Asp Gly Pro Ser Gln Asn Ile Thr Leu Thr His
210 215 220

Cys Lys Gly Asp Ser Cys Ser Gly Asn Asn Phe Leu Asp Glu Glu Val
225 230 235 240

Gln Leu Lys Ser Glu Phe Glu Lys Leu Ser Asp Ala Asp Lys Ile Ser
245 250 255

Asn Tyr Lys Lys Asp Gly Lys Asn Asp Lys Phe Val Gly Leu Val Ala
260 265 270

Asp Ser Val Gln Met Lys Gly Ile Asn Gln Tyr Ile Ile Phe Tyr Lys
275 280 285

Pro Lys Pro Thr Ser Phe Ala Arg Phe Arg Arg Ser Ala Arg Ser Arg
290 295 300

Arg Ser Leu Pro Ala Glu Met Pro Leu Ile Pro Val Asn Gln Ala Asp
305 310 315 320

Thr Leu Ile Val Asp Gly Glu Ala Val Ser Leu Thr Gly His Ser Gly
325 330 335

Asn Ile Phe Ala Pro Glu Gly Asn Tyr Arg Tyr Leu Thr Tyr Gly Ala
340 345 350

Glu Lys Leu Pro Gly Gly Ser Tyr Ala Leu Arg Val Gln Gly Glu Pro
355 360 365

Ala Lys Gly Glu Met Leu Ala Gly Ala Ala Val Tyr Asn Gly Glu Val
370 375 380

Leu His Phe His Thr Glu Asn Gly Arg Pro Tyr Pro Thr Arg Gly Arg
385 390 395 400

Phe Ala Ala Lys Val Asp Phe Gly Ser Lys Ser Val Asp Gly Ile Ile
405 410 415

Asp Ser Gly Asp Asp Leu His Met Gly Thr Gln Lys Phe Lys Ala Ala
420 425 430

Ile Asp Gly Asn Gly Phe Lys Gly Thr Trp Thr Glu Asn Gly Ser Gly
435 440 445

Asp Val Ser Gly Lys Phe Tyr Gly Pro Ala Gly Glu Glu Val Ala Gly
450 455 460

Lys Tyr Ser Tyr Arg Pro Thr Asp Ala Glu Lys Gly Gly Phe Gly Val
465 470 475 480

Phe Ala Gly Lys Lys Glu Gln Asp
485

<210> 620
<211> 427
<212> PRT
<213> Artificial Sequence

<220>
<223> 2996

<400> 620

Met Phe Glu Arg Ser Val Ile Ala Met Ala Cys Ile Phe Ala Leu Ser
1               5                   10                  15

Ala Cys Gly Gly Gly Gly Gly Gly Ser Pro Asp Val Lys Ser Ala Asp
            20                  25                  30

Thr Leu Ser Lys Pro Ala Ala Pro Val Val Ala Glu Lys Glu Thr Glu
        35                  40                  45

Val Lys Glu Asp Ala Pro Gln Ala Gly Ser Gln Gly Gln Gly Ala Pro
    50                  55                  60

Ser Thr Gln Gly Ser Gln Asp Met Ala Ala Val Ser Ala Glu Asn Thr
65                  70                  75                  80

Gly Asn Gly Gly Ala Ala Thr Thr Asp Lys Pro Lys Asn Glu Asp Glu
                85                  90                  95

Gly Pro Gln Asn Asp Met Pro Gln Asn Ser Ala Glu Ser Ala Asn Gln
            100                 105                 110

Thr Gly Asn Asn Gln Pro Ala Asp Ser Ser Asp Ser Ala Pro Ala Ser
115 120 125

Asn Pro Ala Pro Ala Asn Gly Gly Ser Asn Phe Gly Arg Val Asp Leu
130 135 140

Ala Asn Gly Val Leu Ile Asp Gly Pro Ser Gln Asn Ile Thr Leu Thr
145 150 155 160

His Cys Lys Gly Asp Ser Cys Asn Gly Asp Asn Leu Leu Asp Glu Glu
165 170 175

Ala Pro Ser Lys Ser Glu Phe Glu Asn Leu Asn Glu Ser Glu Arg Ile
180 185 190

Glu Lys Tyr Lys Lys Asp Gly Lys Ser Asp Lys Phe Thr Asn Leu Val
195 200 205

Ala Thr Ala Val Gln Ala Asn Gly Thr Asn Lys Tyr Val Ile Ile Tyr
210 215 220

Lys Asp Lys Ser Ala Ser Ser Ser Ser Ala Arg Phe Arg Arg Ser Ala
225 230 235 240

Arg Ser Arg Arg Ser Leu Pro Ala Glu Met Pro Leu Ile Pro Val Asn
245 250 255

Gln Ala Asp Thr Leu Ile Val Asp Gly Glu Ala Val Ser Leu Thr Gly
260 265 270

His Ser Gly Asn Ile Phe Ala Pro Glu Gly Asn Tyr Arg Tyr Leu Thr
275 280 285

Tyr Gly Ala Glu Lys Leu Pro Gly Gly Ser Tyr Ala Leu Arg Val Gln
290 295 300

Gly Glu Pro Ala Lys Gly Glu Met Leu Ala Gly Thr Ala Val Tyr Asn
305 310 315 320

Gly Glu Val Leu His Phe His Thr Glu Asn Gly Arg Pro Tyr Pro Thr
325 330 335

Arg Gly Arg Phe Ala Ala Lys Val Asp Phe Gly Ser Lys Ser Val Asp
340 345 350

Gly Ile Ile Asp Ser Gly Asp Asp Leu His Met Gly Thr Gln Lys Phe
355 360 365

Lys Ala Ala Ile Asp Gly Asn Gly Phe Lys Gly Thr Trp Thr Glu Asn
370 375 380

Gly Gly Gly Asp Val Ser Gly Arg Phe Tyr Gly Pro Ala Gly Glu Glu
385 390 395 400

Val Ala Gly Lys Tyr Ser Tyr Arg Pro Thr Asp Ala Glu Lys Gly Gly
405 410 415

```
Phe Gly Val Phe Ala Gly Lys Lys Glu Gln Asp
            420                 425
```

## Description

### TECHNICAL FIELD

This invention is in the field of protein expression. In particular, it relates to the heterologous expression of proteins from *Neisseria* (*e.g. N.gonorrhoeae* or, preferably, *N.meningitidis*).

### BACKGROUND ART

International patent applications WO99/24578, WO99/36544, WO99/57280 and WO00/22430 disclose proteins from *Neisseria meningitidis* and *Neisseria gonorrhoeae.* These proteins are typically described as being expressed in *E.coli* (*i.e.* heterologous expression) as either N-terminal GST-fusions or C-terminal His-tag fusions, although other expression systems, including expression in native *Neisseria,* are also disclosed. FR-A-2720408 discloses the heterologous production of a N.meningitidis protein in which at least a domain has been deleted.
It is an object of the present invention to provide alternative and improved approaches for the heterologous expression of these proteins. These approaches will typically affect the level of expression, the ease of purification, the cellular localisation of expression, and/or the immunological properties of the expressed protein.

### DISCLOSURE OF THE INVENTION

The invention relates to protein ’961’ disclosed in WO99/57280.
The naming conventions used in WO99/24578, WO99/36544 and WO99/57280 are used herein (*e.g.* ’ORF4’, ’ORF40’, ’ORF40-I’ *etc.* as used in WO99/24578 and WO99/36544; ’m919’, ’g919’ and ’a919’ *etc.* as used in WO99/57280).
Preferred protein ’961’ of the invention is found in *N. meningitidis* serogroup B.
Preferred protein ’961’ for use according to the invention is from serogroup B *N. meningitidis* strain 2996 or strain 394/98 (a New Zealand strain). Unless otherwise stated, proteins mentioned herein are from *N. meningitidis* strain 2996. It will be appreciated, however, that the invention is not in general limited by strain. References to a particular protein (*e.g.* ’287’, ’919’ *etc.*) may be taken to include that protein from any strain.

#### *Domain-based expression*

In this approach to heterologous expression, the 961 protein is expressed as domains. This may be used in association with fusion systems (*e.g.* GST or His-tag fusions).
Thus the invention provides a method for the heterologous expression of *N.meningitidis* protein ’961’, wherein:

(a) protein 961 has the amino acid sequence ’961’ in strain MC58:

```
MSMKHFPAKVLTTAILATFCSGALAATSDDDVKKAATVAIVAAYNNGQEINGFKAGETIYDIGE
DGTITQKDATAADVEADDFKGLGLKKVVTNLTKTVNENKQNVDAKVKAAESEIEKLTTKLADTD
AALADTDAALDETTNALNKLGENITTFAEETKTNIVKIDEKLEAVADTVDKHAEAFNDIADSLD
ETNTKADEAVKTANEAKQTAEETKQNVDAKVKAAETAAGKAEAAAGTANTAADKAEAVAAKVTD
IKADIATNKADIAKNSARIDSLDKNVANLRKETRQGLAEQAALSGLFQPYNVGRFNVTAAVGGY
KSESAVAIGTGFRFTENFAAKAGVAVGTSSGSSAAYHVGVNYEW
```

and wherein

(b) at least one domain in the protein is deleted, wherein the domains of ’961’ in strain MC58 are as follows: (1) amino acids 1-23; (2) amino acids 24-268; (3) amino acids 269-307; and (4) amino acids 308-364,

and wherein the ’961’ protein is expressed in a host cell.
The method will typically involve the steps of: obtaining nucleic acid encoding a protein of the invention; manipulating said nucleic acid to remove at least one domain from within the protein. The resulting nucleic acid may be inserted into an expression vector, or may already be part of an expression vector. Where no fusion partners are used, the first amino acid of the expressed protein will be that of a domain of the protein.
A protein is typically divided into notional domains by aligning it with known sequences in databases and then determining regions of the protein which show different alignment patterns from each other.

Once a protein has been divided into domains, these can be (a) expressed singly (b) deleted from with the protein *e.g.* protein ABCD → ABD, ACD, BCD *etc.* or (c) rearranged *e.g.* protein ABC → ACB, CAB *etc.* These three strategies can be combined with fusion partners is desired.
The domains of protein 961 are illustrated in Figure 12.

### *Hybrid proteins*

The protein 961 may be part of a hybrid of two or more (*e.g.* 3, 4, 5, 6 or more) neisserial proteins expressed as a single hybrid protein. It is preferred that no non-Neisserial fusion partner (*e.g.* GST or poly-His) is used.
This offers two advantages. Firstly, a protein that may be unstable or poorly expressed on its own can be assisted by adding a suitable hybrid partner that overcomes the probtem-Secondly, commercial manufacture is simplified - only one expression and purification need be employed in order to produce two separately-useful proteins.
Thus the invention provides a method for the simultaneous heterologous expression of protein 961 with one or more neisserial proteins in which said proteins are fused (*i.e.* they are translated as a single polypeptide chain).
The method will typically involve the steps of: obtaining a first nucleic acid encoding a first protein of the invention; obtaining a second nucleic acid encoding a second protein of the invention; ligating the first and second nucleic acids. The resulting nucleic acid may be inserted into an expression vector, or may already be part of an expression vector.
Preferably, the constituent proteins in a hybrid protein according to the invention will be from the same strain.
The fused proteins in the hybrid may be joined directly, or may be joined via a linker peptide *e.g.* via a poly-glycine linker (*i.e.* $G_n$ where $n$ = 3, 4, 5, 6, 7, 8, 9, 10 or more) or via a short peptide sequence which facilitates cloning. It is evidently preferred not to join a ΔG protein to the C-terminus of a poly-glycine linker.
The fused proteins may lack native leader peptides or may include the leader peptide sequence of the N-tenninal fusion partner.
The hybrids indicated by 'X' in the following table of form $NH_2$-A-B-COOH are preferred:

| ↓A B→ | ORF46.1 | 287 | 741 | 919 | 953 | 961 | 983 |
|---|---|---|---|---|---|---|---|
| ORF46.1 | | | | | | X | |
| 287 | | | | | | X | |
| 741 | | | | | | X | |
| 919 | | | | | | X | |
| 953 | | | | | | X | |
| 961 | X | X | X | X | X | | X |
| 983 | | | | | | X | |

Preferred proteins to be expressed as hybrids with protein '961' are thus ORF46.1, 287, 741, 919, 953 and 983. These may be used in their essentially full-length form, or poly-glycine deletions (ΔG) forms may be used (*e.g.* ΔG-287, ΔGTbp2, ΔG741, ΔG983 *etc.*), or truncated forms may be used (*e.g.* Δ1-287, Δ2-287 *etc.*), or domain-deleted versions may be used (*e.g.* 287B, 287C, 287BC, $ORF46_{1-433}$, $ORF46_{433-608}$, ORF46,961c *etc.*).
Where 287 is used, it is preferably at the C-terminal end of a hybrid; if it is to be used at the N-terminus, if is preferred to use a ΔG form of 287 is used (*e.g.* as the N-terminus of a hybrid with 961).
Where 287 is used, this is preferably from strain 2996 or from strain 394/98.
961 is preferably at the N-terminus. Preferably Domain forms of 961 are used, in such hybrids.
Alignments of polymorphic forms of ORF46, 287, 919 and 953 are disclosed in WO00/66741. Any of these polymorphs can be used according to the present invention.

### *Heterologous host*

Whilst expression of the protein 961 or hybrid thereof may take place in the native host (*i.e.* the organism in which the protein is expressed in nature), the present invention utilises a heterologous host. The heterologous host may be prokaryotic or eukaryotic. It is preferably *E.coli,* but other suitable hosts include *Bacillus subtilis, Vibrio cholerae, Salmonella typhi, Salmonenna typhimurium, Neisseria meningitidis, Neisseria gonorrhoeae, Neisseria lactamica, Neisseria cinerea, Mycobateria* (*e.g. M.tuberculosis*), yeast *etc.*

### *Vectors* etc.

As well as the methods described above, the invention provides (a) nucleic acid and vectors useful in these methods (b) host cells containing said vectors (c) proteins expressed or expressable by the methods (d) compositions comprising these proteins, which may be suitable as vaccines, for instance, or as diagnostic reagents, or as immunogenic compositions (e) these compositions for use as medicaments (*e.g.* as vaccines) or as diagnostic reagents (f) the use of these compositions in the manufacture of (1) a medicament for treating or preventing infection due to Neisserial bacteria (2) a diagnostic reagent for detecting the presence of Neisserial bacteria or of antibodies raised against Neisserial bacteria, and/or (3) a reagent which can raise antibodies against Neisserial bacteria and (g) a method of treating a patient, comprising administering to the patient a therapeutically effective amount of these compositions.

### *Sequences*

The invention also provides a protein or a nucleic acid having any of the sequences set out in the following examples. It also provides proteins and nucleic acid having sequence identity to these. As described above, the degree of 'sequence identity' is preferably greater than 50% (eg. 60%, 70%, 80%, 90%, 95%, 99% or more).

Furthermore, the invention provides nucleic acid which can hybridise to the nucleic acid disclosed in the examples, preferably under "high stringency" conditions (*eg.* 65°C in a 0.1xSSC, 0.5% SDS solution).

The invention also uses nucleic acid encoding proteins used in the invention.

It should also be appreciated that the invention provides nucleic acid comprising sequences complementary to those described above (*eg.* for antisense or probing purposes).

Nucleic acid according to the invention can, of course, be prepared in many ways (*eg.* by chemical synthesis, from genomic or cDNA libraries, from the organism itself *etc.*) and can take various forms (*eg.* single stranded, double stranded, vectors, probes *etc.*).

In addition, the term "nucleic acid" includes DNA and RNA, and also their analogues, such as those containing modified backbones, and also peptide nucleic acids (PNA) *etc.*

## BRIEF DESCRIPTION OF DRAWINGS

Figure 4 shows expression data for protein 961.

Figure 12 shows domains of protein 961.

Figure 14 shows 26 hybrid proteins according to the invention.

## MODES FOR CARRYING OUT THE INVENTION

### *Example 5-pSM214 and pET-24b vectors*

953 protein with its native leader peptide and no fusion partners was expressed from the pET vector and also from pSM214 [Velati Bellini *et al.* (1991) *J. Biotechnol.* 18, 177-192].

The 953 sequence was cloned as a full-length gene into pSM214 using the *E. coli* MM294-1 strain as a host. To do this, the entire DNA sequence of the 953 gene (from ATG to the STOP codon) was amplified by PCR using the following primers:

953L for/2 CCGGAATTCTTATGAAAAAAATCATCTTCGCCGC Eco RI

953L rev/2 GCCCAAGCTTTTATTGTTTGGCTGCCTCGATT Hind III

which contain *Eco*RI and *Hind*III restriction sites, respectively. The amplified fragment was digested with *Eco*RI and *Hind*III and ligated with the pSM214 vector digested with the same two enzymes. The ligated plasmid was transformed into *E.coli* MM294-1 cells (by incubation in ice for 65 minutes at 37° C) and bacterial cells plated on LB agar containing 20μg/ml of chloramphenicol.

Recombinant colonies were grown over-night at 37°C in 4 ml of LB broth containing 20 μg/ml of chloramphenicol; bacterial cells were centrifuged and plasmid DNA extracted as and analysed by restriction with *Eco*RI and *Hind*III. To analyse the ability of the recombinant colonies to express the protein, they were inoculated in LB broth containing 20μg/ml of chloramphenicol and let to grown for 16 hours at 37°C. Bacterial cells were centrifuged and resuspended in PBS-Expression of the protein was analysed by SDS-PAGE and Coomassie Blue staining.

Expression levels were unexpectedly high from the pSM214 plasmid.

Oligos used to clone '961' sequences into pSM-214 vectors were as follows:

| | | | |
|---|---|---|---|
| **961L** | Fwd | CCGGAATTCATATG-AAACACTTTCCATCC | EcoRI |
| **(pSM-214)** | Rev | GCCCAAGCTT-TTACCACTCGTAATTGAC | HindIII |
| **961** | Fwd | CCGGAATTCATATG-GCCACAAGCGACGAC | EcoRI |
| **(pSM-214)** | Rev | GCCCAAGCTT-TTACCACTCCACTCGTA | HindIII |
| **961c L** | Fwd | CCGGAATTCTTATG-AAACACTTTCCATCC | EcoRI |
| **pSM-214** | Rev | GCCCAAGCTT-TCAACCCACGTTGTAAGGTTG | HindIII |
| **961c** | Fwd | CCGGAATTCTTATG-GCCACAAACCACGACG | EcoRI |
| **pSM-214** | Rev | GCCCAAGCTT-TCAACCCACGTTGTAAGGTTG | HindIII |

These sequences were manipulated, cloned and expressed as described for 953L.

For the pET-24 vector, sequences were cloned and the proteins expressed in pET-24 as described below for pET21. pET2 has the same sequence as pET-21, but with the kanamycin resistance cassette instead of ampicillin cassette.

Oligonucleotides used to clone sequences into pET-24b vector were:

| | | | |
|---|---|---|---|
| **961 K** | Fwd | CGCGGATCCCATATG-GCCACAAGCGACGACGA | NdeI |
| **(MC58)** | Rev | CCCGCTCGAG-TTACCACTCGTAATTGAC | XhoI |
| **961a K** | Fwd | CGCGGATCCCATATG-GCCACAAACGACG | NdeI |
| | Rev | CCCGCTCGAG-TCATTTAGCAATATTATCTTTGTTC | XhoI |
| **961b K** | Fwd | CGCGGATCCCATATG-AAAGCAAACAGTGCCGAC | NdeI |
| | Rev | CCCGCTCGAG-TTACCACTCGTAATTGAC | XhoI |
| **961c K** | Fwd | CGCGGATCCCATATG-GCCACAAACGACG | NdeI |
| | Rev | CCCGCTCGAG-TTAACCCACGTTGTAAGGT | XhoI |
| **961cL K** | Fwd | CGCGGATCCCATATG-ATGAAACACTTTCCATCC | NdeI |
| | Rev | CCCGCTCGAG-TTAACCCACGTTGTAAGGT | XhoI |
| **961d K** | Fwd | CGCGGATCCCATATG-GCCACAAACGACG | NdeI |
| | Rev | CCCGCTCGAG-TCAGTCTGACACTGTTTTATCC | XhoI |
| * This primer was used as a Reverse primer for all the 287 forms.<br>§ Forward primers used in combination with the ΔG278 K reverse primer. | | | |

***Example 9-protein 961***

The complete 961 protein from *N.meningitidis* (serogroup B, strain MC58) has the following sequence:

```
  1  MSMKHFPAKV LTTAILATFC SGALAATSDD DVKKAATVAI VAAYNNGQEI
 51  NGFKAGETIY DIGEDGTITQ KDATAADVEA DDFKGLGLKK VVTNLTKTVN
101  ENKQNVDAKV KAAESEIEKL TTKLADTDAA LADTDAALDE TTNALNKLGE
151  NITTFAEETK TNIVKIDEKL EAVADTVDKH AEAFNDIADS LDETNTKADE
201  AVKTANEAKQ TAEETKQNVD AKVKAAETAA GKAEAAAGTA NTAADKAEAV
251  AAKVTDIKAD IATNKADIAK NSARIDSLDK NVANLRKETR QGLAEQAALS
301  GLFQPYNVGR FNVTAAVGGY KSESAVAIGT GFRFTENFAA KAGVAVGTSS
351  GSSAAYHVGV NYEW*
```

The leader peptide is underlined.

Three approaches to 961 expression were used:

1) 961 using a GST fusion, following WO99/57280 ('GST961');

2) 961 with its own leader peptide but without any fusion partner ('96IL'); and
3) 961 without its leader peptide and without any fusion partner ('961$^{untagged}$') with the leader peptide omitted by designing the 5'-end PCR primer downstream from the predicted leader sequence.

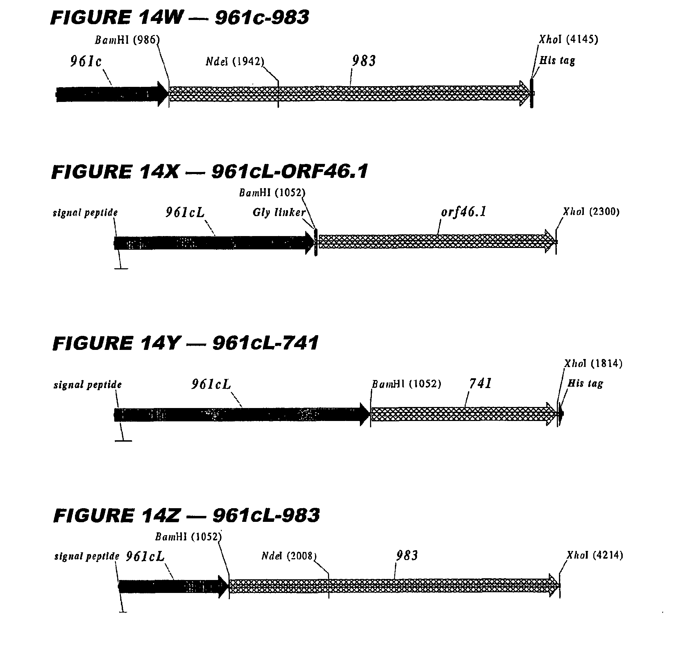

All three forms of the protein were expressed. The GST-fusion protein could be purified and antibodies against it confirmed that 961 is surface exposed (Figure 4). The protein was used to immunise mice, and the resulting sera gave excellent results in the bactericidal assay. 961L could also be purified and gave very high ELISA titres.
Protein 961 appears to be phase variable. Furthermore, it is not found in all strains of *N.meningitidis.*

***Example 22 - domains in 961***

As described in example 9 above, the GST-fusion of 961 was the best-expressed in *E.coli.* To improve expression, the protein was divided into domains (figure 12).
The domains of 961 were designed on the basis of YadA (an adhesin produced by *Yersinia* which has been demonstrated to be an adhesin localized on the bacterial surface that forms oligomers that generate surface projection [Hoiczyk *et al.* (2000) *EMBO J* 19:5989-99]) and are: leader peptide, head domain, coiled-coil region (stalk), and membrane anchor domain. These domains were expressed with or without the leader peptide, and optionally fused either to C-terminal His-tag or to N-terminal GST. *E.coli* clones expressing different domains of 961 were analyzed by SDS-PAGE and western blot for the production and localization of the expressed protein, from over-night (o/n) culture or after 3 hours induction with IPTG. The results were:

| | Total lysate (Western Blot) | Periplasm (Western Blot) | Supernatant (Western Blot) | OMV SDS-PAGE |
|---|---|---|---|---|
| 961 (o/n)<br>961 (IPTG) | -<br>+/- | -<br>- | -<br>- | |
| 961-L (o/n)<br>961-L (IPTG) | +<br>+ | -<br>- | -<br>- | +<br>+ |
| 961c-L (o/n)<br>961c-L (IPTG) | -<br>+ | -<br>+ | -<br>+ | |
| 961$\Delta_1$-L (o/n)<br>961$\Delta_1$-L(IPTG) | -<br>+ | -<br>- | -<br>- | <br>+ |

The results show that in *E.coli:*

■ 961-L is highly expressed and localized on the outer membrane. By western blot analysis two specific bands have been detected: one at ~45kDa (the predicted molecular weight) and one at ~180kDa, indicating that 961-L can form oligomers. Additionally, these aggregates are more expressed in the over-night culture (without IPTG induction). OMV preparations of this clone were used to immunize mice and serum was obtained. Using overnight culture (predominantly by oligomeric form) the serum was bactericidal; the IPTG-induced culture (predominantly monomeric) was not bactericidal.

■ 961$\Delta_1$-L (with a partial deletion in the anchor region) is highly expressed and localized on the outer membrane, but does not form oligomers;

■ the 961c-L (without the anchor region) is produced in soluble form and exported in the supernatant.

Titres in ELISA and in the serum bactericidal assay using His-fusions were as follows:

| | ELISA | Bactericidal |
|---|---|---|
| 961a (aa 24-268) | 24397 | 4096 |
| 961b (aa 269-405) | 7763 | 64 |
| 961c-L | 29770 | 8192 |

(continued)

| | ELISA | Bactericidal |
|---|---|---|
| 961c (2996) | 30774 | >65536 |
| 961c (MC58) | 33437 | 16384 |
| 961d | 26069 | >65536 |

*E.coli* clones expressing different forms of 961 (961, 961-L, 961 $\Delta_1$L and 961c-L) were used to investigate if the 961 is an adhesin (*c.f.* YadA). An adhesion assay was performed using (a) the human epithelial cells and (b) *E.coli* clones after either over-night culture or three hours IPTG induction. 961-L grown over-night (961$\Delta_1$-L) and IPTG-induced 961c-L (the clones expressing protein on surface) adhere to human epithelial cells.

961c was also used in hybrid proteins (see above). As 961 and its domain variants direct efficient expression, they are ideally suited as the N-terminal portion of a hybrid protein.

***Example 23 - further hybrids of protein 961***

Further hybrid proteins of the invention are shown below (see also Figure 14). These are advantageous when compared to the individual proteins:

<u>**961-ORF46.1**</u>

```
   1  ATGGCCACAA ACGACGACGA TGTTAAAAAA GCTGCCACTG TGGCCATTGC
  51  TGCTGCCTAC AACAATGGCC AAGAAATCAA CGGTTTCAAA GCTGGAGAGA
 101  CCATCTACGA CATTGATGAA GACGGCACAA TTACCAAAAA AGACGCAACT
 151  GCAGCCGATG TTGAAGCCGA CGACTTTAAA GGTCTGGGTC TGAAAAAAGT
 201  CGTGACTAAC CTGACCAAAA CCGTCAATGA AAACAAACAA AACGTCGATG
 251  CCAAAGTAAA AGCTGCAGAA TCTGAAATAG AAAAGTTAAC AACCAAGTTA
 301  GCAGACACTG ATGCCGCTTT AGCAGATACT GATGCCGCTC TGGATGCAAC
 351  CACCAACGCC TTGAATAAAT TGGGAGAAAA TATAACGACA TTTGCTGAAG
 401  AGACTAAGAC AAATATCGTA AAAATTGATG AAAAATTAGA AGCCGTGGCT
 451  GATACCGTCG ACAAGCATGC CGAAGCATTC AACGATATCG CCGATTCATT
 501  GGATGAAACC AACACTAAGG CAGACGAAGC CGTCAAAACC GCCAATGAAG
 551  CCAAACAGAC GGCCGAAGAA ACCAAACAAA ACGTCGATGC CAAAGTAAAA
 601  GCTGCAGAAA CTGCAGCAGG CAAAGCCGAA GCTGCCGCTG GCACAGCTAA
 651  TACTGCAGCC GACAAGGCCG AAGCTGTCGC TGCAAAAGTT ACCGACATCA
 701  AAGCTGATAT CGCTACGAAC AAAGATAATA TTGCTAAAAA AGCAAACAGT
 751  GCCGACGTGT ACACCAGAGA AGAGTCTGAC AGCAAATTTG TCAGAATTGA
 801  TGGTCTGAAC GCTACTACCG AAAAATTGGA CACACGCTTG GCTTCTGCTG
 851  AAAAATCCAT TGCCGATCAC GATACTCGCC TGAACGGTTT GGATAAAACA
 901  GTGTCAGACC TGCGCAAAGA AACCCGCCAA GGCCTTGCAG AACAAGCCGC
 951  GCTCTCCGGT CTGTTCCAAC CTTACAACGT GGGTCGGTTC AATGTAACGG
1001  CTGCAGTCGG CGGCTACAAA TCCGAATCGG CAGTCGCCAT CGGTACCGGC
1051  TTCCGCTTTA CCGAAAACTT TGCCGCCAAA GCAGGCGTGG CAGTCGGCAC
1101  TTCGTCCGGT TCTTCCGCAG CCTACCATGT CGGCGTCAAT TACGAGTGGG
1151  GATCCGGAGG AGGAGGATCA GATTTGGCAA ACGATTCTTT TATCCGGCAG
1201  GTTCTCGACC GTCAGCATTT CGAACCCGAC GGGAAATACC ACCTATTCGG
1251  CAGCAGGGGG GAACTTGCCG AGCGCAGCGG CCATATCGGA TTGGGAAAAA
1301  TACAAAGCCA TCAGTTGGGC AACCTGATGA TTCAACAGGC GGCCATTAAA
1351  GGAAATATCG GCTACATTGT CCGCTTTTCC GATCACGGGC ACGAAGTCCA
1401  TTCCCCCTTC GACAACCATG CCTCACATTC CGATTCTGAT GAAGCCGGTA
1451  GTCCCGTTGA CGGATTTAGC CTTTACCGCA TCCATTGGGA CGGATACGAA
1501  CACCATCCCG CCGACGGCTA TGACGGGCCA CAGGGCGGCG GCTATCCCGC
1551  TCCCAAAGGC GCGAGGGATA TATACAGCTA CGACATAAAA GGCGTTGCCC
1601  AAAATATCCG CCTCAACCTG ACCGACAACC GCAGCACCGG ACAACGGCTT
1651  GCCGACCGTT TCCACAATGC CGGTAGTATG CTGACGCAAG GAGTAGGCGA
1701  CGGATTCAAA CGCGCCACCC GATACAGCCC CGAGCTGGAC AGATCGGGCA
1751  ATGCCGCCGA AGCCTTCAAC GGCACTGCAG ATATCGTTAA AAACATCATC
1801  GGCGCGGCAG GAGAAATTGT CGGCGCAGGC GATGCCGTGC AGGGCATAAG
1851  CGAAGGCTCA AACATTGCTG TCATGCACGG CTTGGGTCTG CTTTCCACCG
1901  AAAACAAGAT GGCGCGCATC AACGATTTGG CAGATATGGC GCAACTCAAA
```

```
1951  GACTATGCCG CAGCAGCCAT CCGCGATTGG GCAGTCCAAA ACCCCAATGC
2001  CGCACAAGGC ATAGAAGCCG TCAGCAATAT CTTTATGGCA GCCATCCCCA
2051  TCAAAGGGAT TGGAGCTGTT CGGGGAAAAT ACGGCTTGGG CGGCATCACG
2101  GCACATCCTA TCAAGCGGTC GCAGATGGGC GCGATCGCAT TGCCGAAAGG
2151  GAAATCCGCC GTCAGCGACA ATTTTGCCGA TGCGGCATAC GCCAAATACC
2201  CGTCCCCTTA CCATTCCCGA AATATCCGTT CAAACTTGGA GCAGCGTTAC
2251  GGCAAAGAAA ACATCACCTC CTCAACCGTG CCGCCGTCAA ACGGCAAAAA
2301  TGTCAAACTG GCAGACCAAC GCCACCCGAA GACAGGCGTA CCGTTTGACG
2351  GTAAAGGGTT TCCGAATTTT GAGAAGCACG TGAAATATGA TACGCTCGAG
2401  CACCACCACC ACCACCACTG A

   1  MATNDDDVKK AATVAIAAAY NNGQEINGFK AGETIYDIDE DGTITKKDAT
  51  AADVEADDFK GLGLKKVVTN LTKTVNENKQ NVDAKVKAAE SEIEKLTTKL
 101  ADTDAALADT DAALDATTNA LNKLGENITT FAEETKTNIV KIDEKLEAVA
 151  DTVDKHAEAF NDIADSLDET NTKADEAVKT ANEAKQTAEE TKQNVDAKVK
 201  AAETAAGKAE AAAGTANTAA DKAEAVAAKV TDIKADIATN KDNIAKKANS
 251  ADVYTREESD SKFVRIDGLN ATTEKLDTRL ASAEKSIADH DTRLNGLDKT
 301  VSDLRKETRQ GLAEQAALSG LFQPYNVGRF NVTAAVGGYK SESAVAIGTG
 351  FRFTENFAAK AGVAVGTSSG SSAAYHVGVN YEWGSGGGGS DLANDSFIRQ
 401  VLDRQHFEPD GKYHLFGSRG ELAERSGHIG LGKIQSHQLG NLMIQQAAIK
 451  GNIGYIVRFS DHGHEVHSPF DNHASHSDSD EAGSPVDGFS LYRIHWDGYE
 501  HHPADGYDGP QGGGYPAPKG ARDIYSYDIK GVAQNIRLNL TDNRSTGQRL
 551  ADRFHNAGSM LTQGVGDGFK RATRYSPELD RSGNAAEAFN GTADIVKNII
 601  GAAGEIVGAG DAVQGISEGS NIAVMHGLGL LSTENKMARI NDLADMAQLK
 651  DYAAAAIRDW AVQNPNAAQG IEAVSNIFMA AIPIKGIGAV RGKYGLGGIT
 701  AHPIKRSQMG AIALPKGKSA VSDNFADAAY AKYPSPYHSR NIRSNLEQRY
 751  GKENITSSTV PPSNGKNVKL ADQRHPKTGV PFDGKGFPNF EKHVKYDTLE
 801  HHHHHH*
```

**961-741**

```
   1  ATGGCCACAA ACGACGACGA TGTTAAAAAA GCTGCCACTG TGGCCATTGC
  51  TGCTGCCTAC AACAATGGCC AAGAAATCAA CGGTTTCAAA GCTGGAGAGA
 101  CCATCTACGA CATTGATGAA GACGGCACAA TTACCAAAAA AGACGCAACT
 151  GCAGCCGATG TTGAAGCCGA CGACTTTAAA GGTCTGGGTC TGAAAAAAGT
 201  CGTGACTAAC CTGACCAAAA CCGTCAATGA AAACAAACAA AACGTCGATG
 251  CCAAAGTAAA AGCTGCAGAA TCTGAAATAG AAAAGTTAAC AACCAAGTTA
 301  GCAGACACTG ATGCCGCTTT AGCAGATACT GATGCCGCTC TGGATGCAAC
 351  CACCAACGCC TTGAATAAAT TGGGAGAAAA TATAACGACA TTTGCTGAAG
 401  AGACTAAGAC AAATATCGTA AAAATTGATG AAAAATTAGA AGCCGTGGCT
 451  GATACCGTCG ACAAGCATGC CGAAGCATTC AACGATATCG CCGATTCATT
 501  GGATGAAACC AACACTAAGG CAGACGAAGC CGTCAAAACC GCCAATGAAG
 551  CCAAACAGAC GGCCGAAGAA ACCAAACAAA ACGTCGATGC CAAAGTAAAA
 601  GCTGCAGAAA CTGCAGCAGG CAAAGCCGAA GCTGCCGCTG GCACAGCTAA
 651  TACTGCAGCC GACAAGGCCG AAGCTGTCGC TGCAAAAGTT ACCGACATCA
 701  AAGCTGATAT CGCTACGAAC AAAGATAATA TTGCTAAAAA AGCAAACAGT
 751  GCCGACGTGT ACACCAGAGA AGAGTCTGAC AGCAAATTTG TCAGAATTGA
 801  TGGTCTGAAC GCTACTACCG AAAAATTGGA CACACGCTTG GCTTCTGCTG
 851  AAAAATCCAT TGCCGATCAC GATACTCGCC TGAACGGTTT GGATAAAACA
 901  GTGTCAGACC TGCGCAAAGA AACCCGCCAA GGCCTTGCAG AACAAGCCGC
 951  GCTCTCCGGT CTGTTCCAAC CTTACAACGT GGGTCGGTTC AATGTAACGG
1001  CTGCAGTCGG CGGCTACAAA TCCGAATCGG CAGTCGCCAT CGGTACCGGC
1051  TTCCGCTTTA CCGAAAACTT TGCCGCCAAA GCAGGCGTGG CAGTCGGCAC
1101  TTCGTCCGGT TCTTCCGCAG CCTACCATGT CGGCGTCAAT TACGAGTGGG
1151  GATCCGGAGG GGGTGGTGTC GCCGCCGACA TCGGTGCGGG GCTTGCCGAT
1201  GCACTAACCG CACCGCTCGA CCATAAAGAC AAAGGTTTGC AGTCTTTGAC
1251  GCTGGATCAG TCCGTCAGGA AAAACGAGAA ACTGAAGCTG GCGGCACAAG
1301  GTGCGGAAAA AACTTATGGA AACGGTGACA GCCTCAATAC GGGCAAATTG
1351  AAGAACGACA AGGTCAGCCG TTTCGACTTT ATCCGCCAAA TCGAAGTGGA
1401  CGGGCAGCTC ATTACCTTGG AGAGTGGAGA GTTCCAAGTA TACAAACAAA
1451  GCCATTCCGC CTTAACCGCC TTTCAGACCG AGCAAATACA AGATTCGGAG
1501  CATTCCGGGA AGATGGTTGC GAAACGCCAG TTCAGAATCG GCGACATAGC
1551  GGGCGAACAT ACATCTTTTG ACAAGCTTCC CGAAGGCGGC AGGGCGACAT
1601  ATCGCGGGAC GGCGTTCGGT TCAGACGATG CCGGCGGAAA ACTGACCTAC
1651  ACCATAGATT TCGCCGCCAA GCAGGGAAAC GGCAAAATCG AACATTTGAA
1701  ATCGCCAGAA CTCAATGTCG ACCTGGCCGC CGCCGATATC AAGCCGGATG
1751  GAAAACGCCA TGCCGTCATC AGCGGTTCCG TCCTTTACAA CCAAGCCGAG
1801  AAAGGCAGTT ACTCCCTCGG TATCTTTGGC GGAAAAGCCC AGGAAGTTGC
```

1851 CGGCAGCGCG GAAGTGAAAA CCGTAAACGG CATACGCCAT ATCGGCCTTG
1901 CCGCCAAGCA ACTCGAGCAC CACCACCACC ACCACTGA

1 MATNDDDVKK AATVAIAAAY NNGQEINGFK AGETIYDIDE DGTITKKDAT
51 AADVEADDFK GLGLKKVVTN LTKTVNENKQ NVDAKVKAAE SEIEKLTTKL
101 ADTDAALADT DAALDATTNA LNKLGENITT FAEETKTNIV KIDEKLEAVA
151 DTVDKHAEAF NDIADSLDET NTKADEAVKT ANEAKQTAEE TKQNVDAKVK
201 AAETAAGKAE AAAGTANTAA DKAEAVAAKV TDIKADIATN KDNIAKKANS
251 ADVYTREESD SKFVRIDGLN ATTEKLDTRL ASAEKSIADH DTRLNGLDKT
301 VSDLRKETRQ GLAEQAALSG LFQPYNVGRF NVTAAVGGYK SESAVAIGTG
351 FRFTENFAAK AGVAVGTSSG SSAAYHVGVN YEWGSGGGGV AADIGAGLAD
401 ALTAPLDHKD KGLQSLTLDQ SVRKNEKLKL AAQGAEKTYG NGDSLNTGKL
451 KNDKVSRFDF IRQIEVDGQL ITLESGEFQV YKQSHSALTA FQTEQIQDSE
501 HSGKMVAKRQ FRIGDIAGEH TSFDKLPEGG RATYRGTAFG SDDAGGKLTY
551 TIDFAAKQGN GKIEHLKSPE LNVDLAAADI KPDGKRHAVI SGSVLYNQAE
601 KGSYSLGIFG GKAQEVAGSA EVKTVNGIRH IGLAAKQLEH HHHHH*

**961-983**

1 ATGGCCACAA ACGACGACGA TGTTAAAAAA GCTGCCACTG TGGCCATTGC
51 TGCTGCCTAC AACAATGGCC AAGAAATCAA CGGTTTCAAA GCTGGAGAGA
101 CCATCTACGA CATTGATGAA GACGGCACAA TTACCAAAAA AGACGCAACT
151 GCAGCCGATG TTGAAGCCGA CGACTTTAAA GGTCTGGGTC TGAAAAAAGT
201 CGTGACTAAC CTGACCAAAA CCGTCAATGA AAACAAACAA AACGTCGATG
251 CCAAAGTAAA AGCTGCAGAA TCTGAAATAG AAAAGTTAAC AACCAAGTTA
301 GCAGACACTG ATGCCGCTTT AGCAGATACT GATGCCGCTC TGGATGCAAC
351 CACCAACGCC TTGAATAAAT TGGGAGAAAA TATAACGACA TTTGCTGAAG
401 AGACTAAGAC AAATATCGTA AAAATTGATG AAAAATTAGA AGCCGTGGCT
451 GATACCGTCG ACAAGCATGC CGAAGCATTC AACGATATCG CCGATTCATT
501 GGATGAAACC AACACTAAGG CAGACGAAGC CGTCAAAACC GCCAATGAAG
551 CCAAACAGAC GGCCGAAGAA ACCAAACAAA ACGTCGATGC CAAAGTAAAA
601 GCTGCAGAAA CTGCAGCAGG CAAAGCCGAA GCTGCCGCTG GCACAGCTAA
651 TACTGCAGCC GACAAGGCCG AAGCTGTCGC TGCAAAAGTT ACCGACATCA
701 AAGCTGATAT CGCTACGAAC AAAGATAATA TTGCTAAAAA AGCAAACAGT
751 GCCGACGTGT ACACCAGAGA AGAGTCTGAC AGCAAATTTG TCAGAATTGA
801 TGGTCTGAAC GCTACTACCG AAAAATTGGA CACACGCTTG GCTTCTGCTG
851 AAAAATCCAT TGCCGATCAC GATACTCGCC TGAACGGTTT GGATAAAACA
901 GTGTCAGACC TGCGCAAAGA AACCCGCCAA GGCCTTGCAG AACAAGCCGC
951 GCTCTCCGGT CTGTTCCAAC CTTACAACGT GGGTCGGTTC AATGTAACGG
1001 CTGCAGTCGG CGGCTACAAA TCCGAATCGG CAGTCGCCAT CGGTACCGGC
1051 TTCCGCTTTA CCGAAAACTT TGCCGCCAAA GCAGGCGTGG CAGTCGGCAC
1101 TTCGTCCGGT TCTTCCGCAG CCTACCATGT CGGCGTCAAT TACGAGTGGG
1151 GATCCGGCGG AGGCGGCACT TCTGCGCCCG ACTTCAATGC AGGCGGTACC
1201 GGTATCGGCA GCAACAGCAG AGCAACAACA GCGAAATCAG CAGCAGTATC
1251 TTACGCCGGT ATCAAGAACG AAATGTGCAA AGACAGAAGC ATGCTCTGTG
1301 CCGGTCGGGA TGACGTTGCG GTTACAGACA GGGATGCCAA AATCAATGCC
1351 CCCCCCCCGA ATCTGCATAC CGGAGACTTT CCAAACCCAA ATGACGCATA
1401 CAAGAATTTG ATCAACCTCA AACCTGCAAT TGAAGCAGGC TATACAGGAC
1451 GCGGGGTAGA GGTAGGTATC GTCGACACAG GCGAATCCGT CGGCAGCATA
1501 TCCTTTCCCG AACTGTATGG CAGAAAAGAA CACGGCTATA ACGAAAATTA
1551 CAAAAACTAT ACGGCGTATA TGCGGAAGGA AGCGCCTGAA GACGGAGGCG
1601 GTAAAGACAT TGAAGCTTCT TTCGACGATG AGGCCGTTAT AGAGACTGAA
1651 GCAAAGCCGA CGGATATCCG CCACGTAAAA GAAATCGGAC ACATCGATTT
1701 GGTCTCCCAT ATTATTGGCG GGCGTTCCGT GGACGGCAGA CCTGCAGGCG
1751 GTATTGCGCC CGATGCGACG CTACACATAA TGAATACGAA TGATGAAACC
1801 AAGAACGAAA TGATGGTTGC AGCCATCCGC AATGCATGGG TCAAGCTGGG
1851 CGAACGTGGC GTGCGCATCG TCAATAACAG TTTTGGAACA ACATCGAGGG
1901 CAGGCACTGC CGACCTTTTC CAAATAGCCA ATTCGGAGGA GCAGTACCGC
1951 CAAGCGTTGC TCGACTATTC CGGCGGTGAT AAAACAGACG AGGGTATCCG
2001 CCTGATGCAA CAGAGCGATT ACGGCAACCT GTCCTACCAC ATCCGTAATA
2051 AAAACATGCT TTTCATCTTT TCGACAGGCA ATGACGCACA AGCTCAGCCC
2101 AACACATATG CCCTATTGCC ATTTTATGAA AAAGACGCTC AAAAAGGCAT
2151 TATCACAGTC GCAGGCGTAG ACCGCAGTGG AGAAAAGTTC AAACGGGAAA
2201 TGTATGGAGA ACCGGGTACA GAACCGCTTG AGTATGGCTC CAACCATTGC
2251 GGAATTACTG CCATGTGGTG CCTGTCGGCA CCCTATGAAG CAAGCGTCCG
2301 TTTCACCCGT ACAAACCCGA TTCAAATTGC CGGAACATCC TTTTCCGCAC
2351 CCATCGTAAC CGGCACGGCG GCTCTGCTGC TGCAGAAATA CCCGTGGATG

```
2401  AGCAACGACA ACCTGCGTAC CACGTTGCTG ACGACGGCTC AGGACATCGG
2451  TGCAGTCGGC GTGGACAGCA AGTTCGGCTG GGGACTGCTG GATGCGGGTA
2501  AGGCCATGAA CGGACCCGCG TCCTTTCCGT TCGGCGACTT TACCGCCGAT
2551  ACGAAAGGTA CATCCGATAT TGCCTACTCC TTCCGTAACG ACATTTCAGG
2601  CACGGGCGGC CTGATCAAAA AAGGCGGCAG CCAACTGCAA CTGCACGGCA
2651  ACAACACCTA TACGGGCAAA ACCATTATCG AAGGCGGTTC GCTGGTGTTG
2701  TACGGCAACA ACAAATCGGA TATGCGCGTC GAAACCAAAG GTGCGCTGAT
2751  TTATAACGGG GCGGCATCCG GCGGCAGCCT GAACAGCGAC GGCATTGTCT
2801  ATCTGGCAGA TACCGACCAA TCCGGCGCAA ACGAAACCGT ACACATCAAA
2851  GGCAGTCTGC AGCTGGACGG CAAAGGTACG CTGTACACAC GTTTGGGCAA
2901  ACTGCTGAAA GTGGACGGTA CGGCGATTAT CGGCGGCAAG CTGTACATGT
2951  CGGCACGCGG CAAGGGGGCA GGCTATCTCA ACAGTACCGG ACGACGTGTT
3001  CCCTTCCTGA GTGCCGCCAA AATCGGGCAG GATTATTCTT TCTTCACAAA
3051  CATCGAAACC GACGGCGGCC TGCTGGCTTC CCTCGACAGC GTCGAAAAAA
3101  CAGCGGGCAG TGAAGGCGAC ACGCTGTCCT ATTATGTCCG TCGCGGCAAT
3151  GCGGCACGGA CTGCTTCGGC AGCGGCACAT TCCGCGCCCG CCGGTCTGAA
3201  ACACGCCGTA GAACAGGGCG GCAGCAATCT GGAAAACCTG ATGGTCGAAC
3251  TGGATGCCTC CGAATCATCC GCAACACCCG AGACGGTTGA AACTGCGGCA
3301  GCCGACCGCA CAGATATGCC GGGCATCCGC CCCTACGGCG CAACTTTCCG
3351  CGCAGCGGCA GCCGTACAGC ATGCGAATGC CGCCGACGGT GTACGCATCT
3401  TCAACAGTCT CGCCGCTACC GTCTATGCCG ACAGTACCGC CGCCCATGCC
3451  GATATGCAGG GACGCCGCCT GAAAGCCGTA TCGGACGGGT TGGACCACAA
3501  CGGCACGGGT CTGCGCGTCA TCGCGCAAAC CCAACAGGAC GGTGGAACGT
3551  GGGAACAGGG CGGTGTTGAA GGCAAAATGC GCGGCAGTAC CCAAACCGTC
3601  GGCATTGCCG CGAAAACCGG CGAAAATACG ACAGCAGCCG CCACACTGGG
3651  CATGGGACGC AGCACATGGA GCGAAAACAG TGCAAATGCA AAAACCGACA
3701  GCATTAGTCT GTTTGCAGGC ATACGGCACG ATGCGGGCGA TATCGGCTAT
3751  CTCAAAGGCC TGTTCTCCTA CGGACGCTAC AAAAACAGCA TCAGCCGCAG
3801  CACCGGTGCG GACGAACATG CGGAAGGCAG CGTCAACGGC ACGCTGATGC
3851  AGCTGGGCGC ACTGGGCGGT GTCAACGTTC CGTTTGCCGC AACGGGAGAT
3901  TTGACGGTCG AAGGCGGTCT GCGCTACGAC CTGCTCAAAC AGGATGCATT
3951  CGCCGAAAAA GGCAGTGCTT TGGGCTGGAG CGGCAACAGC CTCACTGAAG
4001  GCACGCTGGT CGGACTCGCG GGTCTGAAGC TGTCGCAACC CTTGAGCGAT
4051  AAAGCCGTCC TGTTTGCAAC GGCGGGCGTG GAACGCGACC TGAACGGACG
4101  CGACTACACG GTAACGGGCG GCTTTACCGG CGCGACTGCA GCAACCGGCA
4151  AGACGGGGGC ACGCAATATG CCGCACACCC GTCTGGTTGC CGGCCTGGGC
4201  GCGGATGTCG AATTCGGCAA CGGCTGGAAC GGCTTGGCAC GTTACAGCTA
4251  CGCCGGTTCC AAACAGTACG GCAACCACAG CGGACGAGTC GGCGTAGGCT
4301  ACCGGTTCCT CGAGCACCAC CACCACCACC ACTGA
```

```
   1  MATNDDDVKK AATVAIAAAY NNGQEINGFK AGETIYDIDE DGTITKKDAT
  51  AADVEADDFK GLGLKKVVTN LTKTVNENKQ NVDAKVKAAE SEIEKLTTKL
 101  ADTDAALADT DAALDATTNA LNKLGENITT FAEETKTNIV KIDEKLEAVA
 151  DTVDKHAEAF NDIADSLDET NTKADEAVKT ANEAKQTAEE TKQNVDAKVK
 201  AAETAAGKAE AAAGTANTAA DKAEAVAAKV TDIKADIATN KDNIAKKANS
 251  ADVYTREESD SKFVRIDGLN ATTEKLDTRL ASAEKSIADH DTRLNGLDKT
 301  VSDLRKETRQ GLAEQAALSG LFQPYNVGRF NVTAAVGGYK SESAVAIGTG
 351  FRFTENFAAK AGVAVGTSSG SSAAYHVGVN YEWGSGGGT SAPDFNAGGT
 401  GIGSNSRATT AKSAAVSYAG IKNEMCKDRS MLCAGRDDVA VTDRDAKINA
 451  PPPNLHTGDF PNPNDAYKNL INLKPAIEAG YTGRGVEVGI VDTGESVGSI
 501  SFPELYGRKE HGYNENYKNY TAYMRKEAPE DGGGKDIEAS FDDEAVIETE
 551  AKPTDIRHVK EIGHIDLVSH IIGGRSVDGR PAGGIAPDAT LHIMNTNDET
 601  KNEMMVAAIR NAWVKLGERG VRIVNNSFGT TSRAGTADLF QIANSEEQYR
 651  QALLDYSGGD KTDEGIRLMQ QSDYGNLSYH IRNKNMLFIF STGNDAQAQP
 701  NTYALLPFYE KDAQKGIITV AGVDRSGEKF KREMYGEPGT EPLEYGSNHC
 751  GITAMWCLSA PYEASVRFTR TNPIQIAGTS FSAPIVTGTA ALLLQKYPWM
 801  SNDNLRTTLL TTAQDIGAVG VDSKFGWGLL DAGKAMNGPA SFPFGDFTAD
 851  TKGTSDIAYS FRNDISGTGG LIKKGGSQLQ LHGNNTYTGK TIIEGGSLVL
 901  YGNNKSDMRV ETKGALIYNG AASGGSLNSD GIVYLADTDQ SGANETVHIK
 951  GSLQLDGKGT LYTRLGKLLK VDGTAIIGGK LYMSARGKGA GYLNSTGRRV
1001  PFLSAAKIGQ DYSFFTNIET DGGLLASLDS VEKTAGSEGD TLSYYVRRGN
1051  AARTASAAAH SAPAGLKHAV EQGGSNLENL MVELDASESS ATPETVETAA
1101  ADRTDMPGIR PYGATFRAAA AVQHANAADG VRIFNSLAAT VYADSTAAHA
1151  DMQGRRLKAV SDGLDHNGTG LRVIAQTQQD GGTWEQGGVE GKMRGSTQTV
1201  GIAAKTGENT TAAATLGMGR STWSENSANA KTDSISLFAG IRHDAGDIGY
1251  LKGLFSYGRY KNSISRSTGA DEHAEGSVNG TLMQLGALGG VNVPFAATGD
1301  LTVEGGLRYD LLKQDAFAEK GSALGWSGNS LTEGTLVGLA GLKLSQPLSD
```

1351 KAVLFATAGV ERDLNGRDYT VTGGFTGATA ATGKTGARNM PHTRLVAGLG
1401 ADVEFGNGWN GLARYSYAGS KQYGNHSGRV GVGYRFLEHH HHHH*

**961c-ORF46.1**

1 ATGGCCACAA ACGACGACGA TGTTAAAAAA GCTGCCACTG TGGCCATTGC
51 TGCTGCCTAC AACAATGGCC AAGAAATCAA CGGTTTCAAA GCTGGAGAGA
101 CCATCTACGA CATTGATGAA GACGGCACAA TTACCAAAAA AGACGCAACT
151 GCAGCCGATG TTGAAGCCGA CGACTTTAAA GGTCTGGGTC TGAAAAAAGT
201 CGTGACTAAC CTGACCAAAA CCGTCAATGA AAACAAACAA AACGTCGATG
251 CCAAAGTAAA AGCTGCAGAA TCTGAAATAG AAAAGTTAAC AACCAAGTTA
301 GCAGACACTG ATGCCGCTTT AGCAGATACT GATGCCGCTC TGGATGCAAC
351 CACCAACGCC TTGAATAAAT TGGGAGAAAA TATAACGACA TTTGCTGAAG
401 AGACTAAGAC AAATATCGTA AAAATTGATG AAAAATTAGA AGCCGTGGCT
451 GATACCGTCG ACAAGCATGC CGAAGCATTC AACGATATCG CCGATTCATT
501 GGATGAAACC AACACTAAGG CAGACGAAGC CGTCAAAACC GCCAATGAAG
551 CCAAACAGAC GGCCGAAGAA ACCAAACAAA ACGTCGATGC CAAAGTAAAA
601 GCTGCAGAAA CTGCAGCAGG CAAAGCCGAA GCTGCCGCTG GCACAGCTAA
651 TACTGCAGCC GACAAGGCCG AAGCTGTCGC TGCAAAAGTT ACCGACATCA
701 AAGCTGATAT CGCTACGAAC AAAGATAATA TTGCTAAAAA AGCAAACAGT
751 GCCGACGTGT ACACCAGAGA AGAGTCTGAC AGCAAATTTG TCAGAATTGA
801 TGGTCTGAAC GCTACTACCG AAAAATTGGA CACACGCTTG GCTTCTGCTG
851 AAAAATCCAT TGCCGATCAC GATACTCGCC TGAACGGTTT GGATAAAACA
901 GTGTCAGACC TGCGCAAAGA AACCCGCCAA GGCCTTGCAG AACAAGCCGC
951 GCTCTCCGGT CTGTTCCAAC CTTACAACGT GGGTGGATCC GGAGGAGGAG
1001 GATCAGATTT GGCAAACGAT TCTTTTATCC GGCAGGTTCT CGACCGTCAG
1051 CATTTCGAAC CCGACGGGAA ATACCACCTA TTCGGCAGCA GGGGGGAACT
1101 TGCCGAGCGC AGCGGCCATA TCGGATTGGG AAAAATACAA AGCCATCAGT
1151 TGGGCAACCT GATGATTCAA CAGGCGGCCA TTAAAGGAAA TATCGGCTAC
1201 ATTGTCCGCT TTTCCGATCA CGGGCACGAA GTCCATTCCC CCTTCGACAA
1251 CCATGCCTCA CATTCCGATT CTGATGAAGC CGGTAGTCCC GTTGACGGAT
1301 TTAGCCTTTA CCGCATCCAT TGGGACGGAT ACGAACACCA TCCCGCCGAC
1351 GGCTATGACG GGCCACAGGG CGGCGGCTAT CCCGCTCCCA AAGGCGCGAG
1401 GGATATATAC AGCTACGACA TAAAAGGCGT TGCCCAAAAT ATCCGCCTCA
1451 ACCTGACCGA CAACCGCAGC ACCGGACAAC GGCTTGCCGA CCGTTTCCAC
1501 AATGCCGGTA GTATGCTGAC GCAAGGAGTA GGCGACGGAT TCAAACGCGC
1551 CACCCGATAC AGCCCCGAGC TGGACAGATC GGGCAATGCC GCCGAAGCCT
1601 TCAACGGCAC TGCAGATATC GTTAAAAACA TCATCGGCGC GGCAGGAGAA
1651 ATTGTCGGCG CAGGCGATGC CGTGCAGGGC ATAAGCGAAG GCTCAAACAT
1701 TGCTGTCATG CACGGCTTGG GTCTGCTTTC CACCGAAAAC AAGATGGCGC
1751 GCATCAACGA TTTGGCAGAT ATGGCGCAAC TCAAAGACTA TGCCGCAGCA
1801 GCCATCCGCG ATTGGGCAGT CCAAAACCCC AATGCCGCAC AAGGCATAGA
1851 AGCCGTCAGC AATATCTTTA TGGCAGCCAT CCCCATCAAA GGGATTGGAG
1901 CTGTTCGGGG AAAATACGGC TTGGGCGGCA TCACGGCACA TCCTATCAAG
1951 CGGTCGCAGA TGGGCGCGAT CGCATTGCCG AAAGGGAAAT CCGCCGTCAG
2001 CGACAATTTT GCCGATGCGG CATACGCCAA ATACCCGTCC CCTTACCATT
2051 CCCGAAATAT CCGTTCAAAC TTGGAGCAGC GTTACGGCAA AGAAAACATC
2101 ACCTCCTCAA CCGTGCCGCC GTCAAACGGC AAAAATGTCA AACTGGCAGA
2151 CCAACGCCAC CCGAAGACAG GCGTACCGTT TGACGGTAAA GGGTTTCCGA
2201 ATTTTGAGAA GCACGTGAAA TATGATACGC TCGAGCACCA CCACCACCAC
2251 CACTGA

1 MATNDDDVKK AATVAIAAAY NNGQEINGFK AGETIYDIDE DGTITKKDAT
51 AADVEADDFK GLGLKKVVTN LTKTVNENKQ NVDAKVKAAE SEIEKLTTKL
101 ADTDAALADT DAALDATTNA LNKLGENITT FAEETKTNIV KIDEKLEAVA
151 DTVDKHAEAF NDIADSLDET NTKADEAVKT ANEAKQTAEE TKQNVDAKVK
201 AAETAAGKAE AAAGTANTAA DKAEAVAAKV TDIKADIATN KDNIAKKANS
251 ADVYTREESD SKFVRIDGLN ATTEKLDTRL ASAEKSIADH DTRLNGLDKT
301 VSDLRKETRQ GLAEQAALSG LFQPYNVGGS GGGGSDLAND SFIRQVLDRQ
351 HFEPDGKYHL FGSRGELAER SGHIGLGKIQ SHQLGNLMIQ QAAIKGNIGY
401 IVRFSDHGHE VHSPFDNHAS HSDSDEAGSP VDGFSLYRIH WDGYEHHPAD
451 GYDGPQGGGY PAPKGARDIY SYDIKGVAQN IRLNLTDNRS TGQRLADRFH
501 NAGSMLTQGV GDGFKRATRY SPELDRSGNA AEAFNGTADI VKNIIGAAGE
551 IVGAGDAVQG ISEGSNIAVM HGLGLLSTEN KMARINDLAD MAQLKDYAAA
601 AIRDWAVQNP NAAQGIEAVS NIFMAAIPIK GIGAVRGKYG LGGITAHPIK
651 RSQMGAIALP KGKSAVSDNF ADAAYAKYPS PYHSRNIRSN LEQRYGKENI
701 TSSTVPPSNG KNVKLADQRH PKTGVPFDGK GFPNFEKHVK YDTLEHHHHH

751 H*

**961c-741**

1 ATGGCCACAA ACGACGACGA TGTTAAAAAA GCTGCCACTG TGGCCATTGC
51 TGCTGCCTAC AACAATGGCC AAGAAATCAA CGGTTTCAAA GCTGGAGAGA
101 CCATCTACGA CATTGATGAA GACGGCACAA TTACCAAAAA AGACGCAACT
151 GCAGCCGATG TTGAAGCCGA CGACTTTAAA GGTCTGGGTC TGAAAAAAGT
201 CGTGACTAAC CTGACCAAAA CCGTCAATGA AAACAAACAA AACGTCGATG
251 CCAAAGTAAA AGCTGCAGAA TCTGAAATAG AAAAGTTAAC AACCAAGTTA
301 GCAGACACTG ATGCCGCTTT AGCAGATACT GATGCCGCTC TGGATGCAAC
351 CACCAACGCC TTGAATAAAT TGGGAGAAAA TATAACGACA TTTGCTGAAG
401 AGACTAAGAC AAATATCGTA AAAATTGATG AAAAATTAGA AGCCGTGGCT
451 GATACCGTCG ACAAGCATGC CGAAGCATTC AACGATATCG CCGATTCATT
501 GGATGAAACC AACACTAAGG CAGACGAAGC CGTCAAAACC GCCAATGAAG
551 CCAAACAGAC GGCCGAAGAA ACCAAACAAA ACGTCGATGC CAAAGTAAAA
601 GCTGCAGAAA CTGCAGCAGG CAAAGCCGAA GCTGCCGCTG GCACAGCTAA
651 TACTGCAGCC GACAAGGCCG AAGCTGTCGC TGCAAAAGTT ACCGACATCA
701 AAGCTGATAT CGCTACGAAC AAAGATAATA TTGCTAAAAA AGCAAACAGT
751 GCCGACGTGT ACACCAGAGA AGAGTCTGAC AGCAAATTTG TCAGAATTGA
801 TGGTCTGAAC GCTACTACCG AAAAATTGGA CACACGCTTG GCTTCTGCTG
851 AAAAATCCAT TGCCGATCAC GATACTCGCC TGAACGGTTT GGATAAAACA
901 GTGTCAGACC TGCGCAAAGA AACCCGCCAA GGCCTTGCAG AACAAGCCGC
951 GCTCTCCGGT CTGTTCCAAC CTTACAACGT GGGTGGATCC GGAGGGGGTG
1001 GTGTCGCCGC CGACATCGGT GCGGGGCTTG CCGATGCACT AACCGCACCG
1051 CTCGACCATA AAGACAAAGG TTTGCAGTCT TTGACGCTGG ATCAGTCCGT
1101 CAGGAAAAAC GAGAAACTGA AGCTGGCGGC ACAAGGTGCG GAAAAAACTT
1151 ATGGAAACGG TGACAGCCTC AATACGGGCA AATTGAAGAA CGACAAGGTC
1201 AGCCGTTTCG ACTTTATCCG CCAAATCGAA GTGGACGGGC AGCTCATTAC
1251 CTTGGAGAGT GGAGAGTTCC AAGTATACAA ACAAAGCCAT TCCGCCTTAA
1301 CCGCCTTTCA GACCGAGCAA ATACAAGATT CGGAGCATTC CGGGAAGATG
1351 GTTGCGAAAC GCCAGTTCAG AATCGGCGAC ATAGCGGGCG AACATACATC
1401 TTTTGACAAG CTTCCCGAAG GCGGCAGGGC GACATATCGC GGGACGGCGT
1451 TCGGTTCAGA CGATGCCGGC GGAAAACTGA CCTACACCAT AGATTTCGCC
1501 GCCAAGCAGG GAAACGGCAA AATCGAACAT TTGAAATCGC CAGAACTCAA
1551 TGTCGACCTG GCCGCCGCCG ATATCAAGCC GGATGGAAAA CGCCATGCCG
1601 TCATCAGCGG TTCCGTCCTT TACAACCAAG CCGAGAAAGG CAGTTACTCC
1651 CTCGGTATCT TTGGCGGAAA AGCCCAGGAA GTTGCCGGCA GCGCGGAAGT
1701 GAAAACCGTA AACGGCATAC GCCATATCGG CCTTGCCGCC AAGCAACTCG
1751 AGCACCACCA CCACCACCAC TGA

1 MATNDDDVKK AATVAIAAAY NNGQEINGFK AGETIYDIDE DGTITKKDAT
51 AADVEADDFK GLGLKKVVTN LTKTVNENKQ NVDAKVKAAE SEIEKLTTKL
101 ADTDAALADT DAALDATTNA LNKLGENITT FAEETKTNIV KIDEKLEAVA
151 DTVDKHAEAF NDIADSLDET NTKADEAVKT ANEAKQTAEE TKQNVDAKVK
201 AAETAAGKAE AAAGTANTAA DKAEAVAAKV TDIKADIATN KDNIAKKANS
251 ADVYTREESD SKFVRIDGLN ATTEKLDTRL ASAEKSIADH DTRLNGLDKT
301 VSDLRKETRQ GLAEQAALSG LFQPYNVGGS GGGGVAADIG AGLADALTAP
351 LDHKDKGLQS LTLDQSVRKN EKLKLAAQGA EKTYGNGDSL NTGKLKNDKV
401 SRFDFIRQIE VDGQLITLES GEFQVYKQSH SALTAFQTEQ IQDSEHSGKM
451 VAKRQFRIGD IAGEHTSFDK LPEGGRATYR GTAFGSDDAG GKLTYTIDFA
501 AKQGNGKIEH LKSPELNVDL AAADIKPDGK RHAVISGSVL YNQAEKGSYS
551 LGIFGGKAQE VAGSAEVKTV NGIRHIGLAA KQLEHHHHHH *

**961c-983**

1 ATGGCCACAA ACGACGACGA TGTTAAAAAA GCTGCCACTG TGGCCATTGC
51 TGCTGCCTAC AACAATGGCC AAGAAATCAA CGGTTTCAAA GCTGGAGAGA
101 CCATCTACGA CATTGATGAA GACGGCACAA TTACCAAAAA AGACGCAACT
151 GCAGCCGATG TTGAAGCCGA CGACTTTAAA GGTCTGGGTC TGAAAAAAGT
201 CGTGACTAAC CTGACCAAAA CCGTCAATGA AAACAAACAA AACGTCGATG
251 CCAAAGTAAA AGCTGCAGAA TCTGAAATAG AAAAGTTAAC AACCAAGTTA
301 GCAGACACTG ATGCCGCTTT AGCAGATACT GATGCCGCTC TGGATGCAAC
351 CACCAACGCC TTGAATAAAT TGGGAGAAAA TATAACGACA TTTGCTGAAG
401 AGACTAAGAC AAATATCGTA AAAATTGATG AAAAATTAGA AGCCGTGGCT
451 GATACCGTCG ACAAGCATGC CGAAGCATTC AACGATATCG CCGATTCATT
501 GGATGAAACC AACACTAAGG CAGACGAAGC CGTCAAAACC GCCAATGAAG

551 CCAAACAGAC GGCCGAAGAA ACCAAACAAA ACGTCGATGC CAAAGTAAAA
601 GCTGCAGAAA CTGCAGCAGG CAAAGCCGAA GCTGCCGCTG GCACAGCTAA
651 TACTGCAGCC GACAAGGCCG AAGCTGTCGC TGCAAAAGTT ACCGACATCA
701 AAGCTGATAT CGCTACGAAC AAAGATAATA TTGCTAAAAA AGCAAACAGT
751 GCCGACGTGT ACACCAGAGA AGAGTCTGAC AGCAAATTTG TCAGAATTGA
801 TGGTCTGAAC GCTACTACCG AAAAATTGGA CACACGCTTG GCTTCTGCTG
851 AAAAATCCAT TGCCGATCAC GATACTCGCC TGAACGGTTT GGATAAAACA
901 GTGTCAGACC TGCGCAAAGA AACCCGCCAA GGCCTTGCAG AACAAGCCGC
951 GCTCTCCGGT CTGTTCCAAC CTTACAACGT GGGTGGATCC GGCGGAGGCG
1001 GCACTTCTGC GCCCGACTTC AATGCAGGCG GTACCGGTAT CGGCAGCAAC
1051 AGCAGAGCAA CAACAGCGAA ATCAGCAGCA GTATCTTACG CCGGTATCAA
1101 GAACGAAATG TGCAAAGACA GAAGCATGCT CTGTGCCGGT CGGGATGACG
1151 TTGCGGTTAC AGACAGGGAT GCCAAAATCA ATGCCCCCCC CCCGAATCTG
1201 CATACCGGAG ACTTTCCAAA CCCAAATGAC GCATACAAGA ATTTGATCAA
1251 CCTCAAACCT GCAATTGAAG CAGGCTATAC AGGACGCGGG GTAGAGGTAG
1301 GTATCGTCGA CACAGGCGAA TCCGTCGGCA GCATATCCTT TCCCGAACTG
1351 TATGGCAGAA AAGAACACGG CTATAACGAA AATTACAAAA ACTATACGGC
1401 GTATATGCGG AAGGAAGCGC CTGAAGACGG AGGCGGTAAA GACATTGAAG
1451 CTTCTTTCGA CGATGAGGCC GTTATAGAGA CTGAAGCAAA GCCGACGGAT
1501 ATCCGCCACG TAAAAGAAAT CGGACACATC GATTTGGTCT CCCATATTAT
1551 TGGCGGGCGT TCCGTGGACG GCAGACCTGC AGGCGGTATT GCGCCCGATG
1601 CGACGCTACA CATAATGAAT ACGAATGATG AAACCAAGAA CGAAATGATG
1651 GTTGCAGCCA TCCGCAATGC ATGGGTCAAG CTGGGCGAAC GTGGCGTGCG
1701 CATCGTCAAT AACAGTTTTG GAACAACATC GAGGGCAGGC ACTGCCGACC
1751 TTTTCCAAAT AGCCAATTCG GAGGAGCAGT ACCGCCAAGC GTTGCTCGAC
1801 TATTCCGGCG GTGATAAAAC AGACGAGGGT ATCCGCCTGA TGCAACAGAG
1851 CGATTACGGC AACCTGTCCT ACCACATCCG TAATAAAAAC ATGCTTTTCA
1901 TCTTTTCGAC AGGCAATGAC GCACAAGCTC AGCCCAACAC ATATGCCCTA
1951 TTGCCATTTT ATGAAAAAGA CGCTCAAAAA GGCATTATCA CAGTCGCAGG
2001 CGTAGACCGC AGTGGAGAAA AGTTCAAACG GGAAATGTAT GGAGAACCGG
2051 GTACAGAACC GCTTGAGTAT GGCTCCAACC ATTGCGGAAT TACTGCCATG
2101 TGGTGCCTGT CGGCACCCTA TGAAGCAAGC GTCCGTTTCA CCCGTACAAA
2151 CCCGATTCAA ATTGCCGGAA CATCCTTTTC CGCACCCATC GTAACCGGCA
2201 CGGCGGCTCT GCTGCTGCAG AAATACCCGT GGATGAGCAA CGACAACCTG
2251 CGTACCACGT TGCTGACGAC GGCTCAGGAC ATCGGTGCAG TCGGCGTGGA
2301 CAGCAAGTTC GGCTGGGGAC TGCTGGATGC GGGTAAGGCC ATGAACGGAC
2351 CCGCGTCCTT TCCGTTCGGC GACTTTACCG CCGATACGAA AGGTACATCC
2401 GATATTGCCT ACTCCTTCCG TAACGACATT TCAGGCACGG GCGGCCTGAT
2451 CAAAAAAGGC GGCAGCCAAC TGCAACTGCA CGGCAACAAC ACCTATACGG
2501 GCAAAACCAT TATCGAAGGC GGTTCGCTGG TGTTGTACGG CAACAACAAA
2551 TCGGATATGC GCGTCGAAAC CAAAGGTGCG CTGATTTATA ACGGGGCGGC
2601 ATCCGGCGGC AGCCTGAACA GCGACGGCAT TGTCTATCTG GCAGATACCG
2651 ACCAATCCGG CGCAAACGAA ACCGTACACA TCAAAGGCAG TCTGCAGCTG
2701 GACGGCAAAG GTACGCTGTA CACACGTTTG GGCAAACTGC TGAAAGTGGA
2751 CGGTACGGCG ATTATCGGCG GCAAGCTGTA CATGTCGGCA CGCGGCAAGG
2801 GGGCAGGCTA TCTCAACAGT ACCGGACGAC GTGTTCCCTT CCTGAGTGCC
2851 GCCAAAATCG GGCAGGATTA TTCTTTCTTC ACAAACATCG AAACCGACGG
2901 CGGCCTGCTG GCTTCCCTCG ACAGCGTCGA AAAAACAGCG GGCAGTGAAG
2951 GCGACACGCT GTCCTATTAT GTCCGTCGCG GCAATGCGGC ACGGACTGCT
3001 TCGGCAGCGG CACATTCCGC GCCCGCCGGT CTGAAACACG CCGTAGAACA
3051 GGGCGGCAGC AATCTGGAAA ACCTGATGGT CGAACTGGAT GCCTCCGAAT
3101 CATCCGCAAC ACCCGAGACG GTTGAAACTG CGGCAGCCGA CCGCACAGAT
3151 ATGCCGGGCA TCCGCCCCTA CGGCGCAACT TTCCGCGCAG CGGCAGCCGT
3201 ACAGCATGCG AATGCCGCCG ACGGTGTACG CATCTTCAAC AGTCTCGCCG
3251 CTACCGTCTA TGCCGACAGT ACCGCCGCCC ATGCCGATAT GCAGGGACGC
3301 CGCCTGAAAG CCGTATCGGA CGGGTTGGAC CACAACGGCA CGGGTCTGCG
3351 CGTCATCGCG CAAACCCAAC AGGACGGTGG AACGTGGGAA CAGGGCGGTG
3401 TTGAAGGCAA AATGCGCGGC AGTACCCAAA CCGTCGGCAT TGCCGCGAAA
3451 ACCGGCGAAA ATACGACAGC AGCCGCCACA CTGGGCATGG GACGCAGCAC
3501 ATGGAGCGAA AACAGTGCAA ATGCAAAAAC CGACAGCATT AGTCTGTTTG
3551 CAGGCATACG GCACGATGCG GGCGATATCG GCTATCTCAA AGGCCTGTTC
3601 TCCTACGGAC GCTACAAAAA CAGCATCAGC CGCAGCACCG GTGCGGACGA
3651 ACATGCGGAA GGCAGCGTCA ACGGCACGCT GATGCAGCTG GGCGCACTGG
3701 GCGGTGTCAA CGTTCCGTTT GCCGCAACGG GAGATTTGAC GGTCGAAGGC
3751 GGTCTGCGCT ACGACCTGCT CAAACAGGAT GCATTCGCCG AAAAAGGCAG
3801 TGCTTTGGGC TGGAGCGGCA ACAGCCTCAC TGAAGGCACG CTGGTCGGAC
3851 TCGCGGGTCT GAAGCTGTCG CAACCCTTGA GCGATAAAGC CGTCCTGTTT

```
3901  GCAACGGCGG GCGTGGAACG CGACCTGAAC GGACGCGACT ACACGGTAAC
3951  GGGCGGCTTT ACCGGCGCGA CTGCAGCAAC CGGCAAGACG GGGGCACGCA
4001  ATATGCCGCA CACCCGTCTG GTTGCCGGCC TGGGCGCGGA TGTCGAATTC
4051  GGCAACGGCT GGAACGGCTT GGCACGTTAC AGCTACGCCG GTTCCAAACA
4101  GTACGGCAAC CACAGCGGAC GAGTCGGCGT AGGCTACCGG TTCCTCGAGC
4151  ACCACCACCA CCACCACTGA

   1  MATNDDDVKK AATVAIAAAY NNGQEINGFK AGETIYDIDE DGTITKKDAT
  51  AADVEADDFK GLGLKKVVTN LTKTVNENKQ NVDAKVKAAE SEIEKLTTKL
 101  ADTDAALADT DAALDATTNA LNKLGENITT FAEETKTNIV KIDEKLEAVA
 151  DTVDKHAEAF NDIADSLDET NTKADEAVKT ANEAKQTAEE TKQNVDAKVK
 201  AAETAAGKAE AAAGTANTAA DKAEAVAAKV TDIKADIATN KDNIAKKANS
 251  ADVYTREESD SKFVRIDGLN ATTEKLDTRL ASAEKSIADH DTRLNGLDKT
 301  VSDLRKETRQ GLAEQAALSG LFQPYNVGGS GGGGTSAPDF NAGGTGIGSN
 351  SRATTAKSAA VSYAGIKNEM CKDRSMLCAG RDDVAVTDRD AKINAPPPNL
 401  HTGDFPNPND AYKNLINLKP AIEAGYTGRG VEVGIVDTGE SVGSISFPEL
 451  YGRKEHGYNE NYKNYTAYMR KEAPEDGGGK DIEASFDDEA VIETEAKPTD
 501  IRHVKEIGHI DLVSHIIGGR SVDGRPAGGI APDATLHIMN TNDETKNEMM
 551  VAAIRNAWVK LGERGVRIVN NSFGTTSRAG TADLFQIANS EEQYRQALLD
 601  YSGGDKTDEG IRLMQQSDYG NLSYHIRNKN MLFIFSTGND AQAQPNTYAL
 651  LPFYEKDAQK GIITVAGVDR SGEKFKREMY GEPGTEPLEY GSNHCGITAM
 701  WCLSAPYEAS VRFTRTNPIQ IAGTSFSAPI VTGTAALLLQ KYPWMSNDNL
 751  RTTLLTTAQD IGAVGVDSKF GWGLLDAGKA MNGPASFPFG DFTADTKGTS
 801  DIAYSFRNDI SGTGGLIKKG GSQLQLHGNN TYTGKTIIEG GSLVLYGNNK
 851  SDMRVETKGA LIYNGAASGG SLNSDGIVYL ADTDQSGANE TVHIKGSLQL
 901  DGKGTLYTRL GKLLKVDGTA IIGGKLYMSA RGKGAGYLNS TGRRVPFLSA
 951  AKIGQDYSFF TNIETDGGLL ASLDSVEKTA GSEGDTLSYY VRRGNAARTA
1001  SAAAHSAPAG LKHAVEQGGS NLENLMVELD ASESSATPET VETAAADRTD
1051  MPGIRPYGAT FRAAAAVQHA NAADGVRIFN SLAATVYADS TAAHADMQGR
1101  RLKAVSDGLD HNGTGLRVIA QTQQDGGTWE QGGVEGKMRG STQTVGIAAK
1151  TGENTTAAAT LGMGRSTWSE NSANAKTDSI SLFAGIRHDA GDIGYLKGLF
1201  SYGRYKNSIS RSTGADEHAE GSVNGTLMQL GALGGVNVPF AATGDLTVEG
1251  GLRYDLLKQD AFAEKGSALG WSGNSLTEGT LVGLAGLKLS QPLSDKAVLF
1301  ATAGVERDLN GRDYTVTGGF TGATAATGKT GARNMPHTRL VAGLGADVEF
1351  GNGWNGLARY SYAGSKQYGN HSGRVGVGYR FLEHHHHHH*
```

**<u>961cL-ORF46.1</u>**

```
   1  ATGAAACACT TTCCATCCAA AGTACTGACC ACAGCCATCC TTGCCACTTT
  51  CTGTAGCGGC GCACTGGCAG CCACAAACGA CGACGATGTT AAAAAAGCTG
 101  CCACTGTGGC CATTGCTGCT GCCTACAACA ATGGCCAAGA AATCAACGGT
 151  TTCAAAGCTG GAGAGACCAT CTACGACATT GATGAAGACG GCACAATTAC
 201  CAAAAAAGAC GCAACTGCAG CCGATGTTGA AGCCGACGAC TTTAAAGGTC
 251  TGGGTCTGAA AAAAGTCGTG ACTAACCTGA CCAAAACCGT CAATGAAAAC
 301  AAACAAAACG TCGATGCCAA AGTAAAAGCT GCAGAATCTG AAATAGAAAA
 351  GTTAACAACC AAGTTAGCAG ACACTGATGC CGCTTTAGCA GATACTGATG
 401  CCGCTCTGGA TGCAACCACC AACGCCTTGA ATAAATTGGG AGAAAATATA
 451  ACGACATTTG CTGAAGAGAC TAAGACAAAT ATCGTAAAAA TTGATGAAAA
 501  ATTAGAAGCC GTGGCTGATA CCGTCGACAA GCATGCCGAA GCATTCAACG
 551  ATATCGCCGA TTCATTGGAT GAAACCAACA CTAAGGCAGA CGAAGCCGTC
 601  AAAACCGCCA ATGAAGCCAA ACAGACGGCC GAAGAAACCA AACAAAACGT
 651  CGATGCCAAA GTAAAAGCTG CAGAAACTGC AGCAGGCAAA GCCGAAGCTG
 701  CCGCTGGCAC AGCTAATACT GCAGCCGACA AGGCCGAAGC TGTCGCTGCA
 751  AAAGTTACCG ACATCAAAGC TGATATCGCT ACGAACAAAG ATAATATTGC
 801  TAAAAAAGCA AACAGTGCCG ACGTGTACAC CAGAGAAGAG TCTGACAGCA
 851  AATTTGTCAG AATTGATGGT CTGAACGCTA CTACCGAAAA ATTGGACACA
 901  CGCTTGGCTT CTGCTGAAAA ATCCATTGCC GATCACGATA CTCGCCTGAA
 951  CGGTTTGGAT AAAACAGTGT CAGACCTGCG CAAAGAAACC CGCCAAGGCC
1001  TTGCAGAACA AGCCGCGCTC TCCGGTCTGT TCCAACCTTA CAACGTGGGT
1051  GGATCCGGAG GAGGAGGATC AGATTTGGCA AACGATTCTT TTATCCGGCA
1101  GGTTCTCGAC CGTCAGCATT TCGAACCCGA CGGGAAATAC CACCTATTCG
1151  GCAGCAGGGG GGAACTTGCC GAGCGCAGCG GCCATATCGG ATTGGGAAAA
1201  ATACAAAGCC ATCAGTTGGG CAACCTGATG ATTCAACAGG CGGCCATTAA
1251  AGGAAATATC GGCTACATTG TCCGCTTTTC CGATCACGGG CACGAAGTCC
1301  ATTCCCCCTT CGACAACCAT GCCTCACATT CCGATTCTGA TGAAGCCGGT
1351  AGTCCCGTTG ACGGATTTAG CCTTTACCGC ATCCATTGGG ACGGATACGA
1401  ACACCATCCC GCCGACGGCT ATGACGGGCC ACAGGGCGGC GGCTATCCCG
```

```
1451  CTCCCAAAGG CGCGAGGGAT ATATACAGCT ACGACATAAA AGGCGTTGCC
1501  CAAAATATCC GCCTCAACCT GACCGACAAC CGCAGCACCG GACAACGGCT
1551  TGCCGACCGT TTCCACAATG CCGGTAGTAT GCTGACGCAA GGAGTAGGCG
1601  ACGGATTCAA ACGCGCCACC CGATACAGCC CCGAGCTGGA CAGATCGGGC
1651  AATGCCGCCG AAGCCTTCAA CGGCACTGCA GATATCGTTA AAAACATCAT
1701  CGGCGCGGCA GGAGAAATTG TCGGCGCAGG CGATGCCGTG CAGGGCATAA
1751  GCGAAGGCTC AAACATTGCT GTCATGCACG GCTTGGGTCT GCTTTCCACC
1801  GAAAACAAGA TGGCGCGCAT CAACGATTTG GCAGATATGG CGCAACTCAA
1851  AGACTATGCC GCAGCAGCCA TCCGCGATTG GGCAGTCCAA AACCCCAATG
1901  CCGCACAAGG CATAGAAGCC GTCAGCAATA TCTTTATGGC AGCCATCCCC
1951  ATCAAAGGGA TTGGAGCTGT TCGGGGAAAA TACGGCTTGG GCGGCATCAC
2001  GGCACATCCT ATCAAGCGGT CGCAGATGGG CGCGATCGCA TTGCCGAAAG
2051  GGAAATCCGC CGTCAGCGAC AATTTTGCCG ATGCGGCATA CGCCAAATAC
2101  CCGTCCCCTT ACCATTCCCG AAATATCCGT TCAAACTTGG AGCAGCGTTA
2151  CGGCAAAGAA AACATCACCT CCTCAACCGT GCCGCCGTCA AACGGCAAAA
2201  ATGTCAAACT GGCAGACCAA CGCCACCCGA AGACAGGCGT ACCGTTTGAC
2251  GGTAAAGGGT TTCCGAATTT TGAGAAGCAC GTGAAATATG ATACGTAACT
2301  CGAG
```

```
  1  MKHFPSKVLT TAILATFCSG ALAATNDDDV KKAATVAIAA AYNNGQEING
 51  FKAGETIYDI DEDGTITKKD ATAADVEADD FKGLGLKKVV TNLTKTVNEN
101  KQNVDAKVKA AESEIEKLTT KLADTDAALA DTDAALDATT NALNKLGENI
151  TTFAEETKTN IVKIDEKLEA VADTVDKHAE AFNDIADSLD ETNTKADEAV
201  KTANEAKQTA EETKQNVDAK VKAAETAAGK AEAAAGTANT AADKAEAVAA
251  KVTDIKADIA TNKDNIAKKA NSADVYTREE SDSKFVRIDG LNATTEKLDT
301  RLASAEKSIA DHDTRLNGLD KTVSDLRKET RQGLAEQAAL SGLFQPYNVG
351  GSGGGGSDLA NDSFIRQVLD RQHFEPDGKY HLFGSRGELA ERSGHIGLGK
401  IQSHQLGNLM IQQAAIKGNI GYIVRFSDHG HEVHSPFDNH ASHSDSDEAG
451  SPVDGFSLYR IHWDGYEHHP ADGYDGPQGG GYPAPKGARD IYSYDIKGVA
501  QNIRLNLTDN RSTGQRLADR FHNAGSMLTQ GVGDGFKRAT RYSPELDRSG
551  NAAEAFNGTA DIVKNIIGAA GEIVGAGDAV QGISEGSNIA VMHGLGLLST
601  ENKMARINDL ADMAQLKDYA AAIRDWAVQ NPNAAQGIEA VSNIFMAAIP
651  IKGIGAVRGK YGLGGITAHP IKRSQMGAIA LPKGKSAVSD NFADAAYAKY
701  PSPYHSRNIR SNLEQRYGKE NITSSTVPPS NGKNVKLADQ RHPKTGVPFD
751  GKGFPNFEKH VKYDT*
```

**961cL-741**

```
   1  ATGAAACACT TTCCATCCAA AGTACTGACC ACAGCCATCC TTGCCACTTT
  51  CTGTAGCGGC GCACTGGCAG CCACAAACGA CGACGATGTT AAAAAAGCTG
 101  CCACTGTGGC CATTGCTGCT GCCTACAACA ATGGCCAAGA AATCAACGGT
 151  TTCAAAGCTG GAGAGACCAT CTACGACATT GATGAAGACG GCACAATTAC
 201  CAAAAAAGAC GCAACTGCAG CCGATGTTGA AGCCGACGAC TTTAAAGGTC
 251  TGGGTCTGAA AAAAGTCGTG ACTAACCTGA CCAAAACCGT CAATGAAAAC
 301  AAACAAAACG TCGATGCCAA AGTAAAAGCT GCAGAATCTG AAATAGAAAA
 351  GTTAACAACC AAGTTAGCAG ACACTGATGC CGCTTTAGCA GATACTGATG
 401  CCGCTCTGGA TGCAACCACC AACGCCTTGA ATAAATTGGG AGAAAATATA
 451  ACGACATTTG CTGAAGAGAC TAAGACAAAT ATCGTAAAAA TTGATGAAAA
 501  ATTAGAAGCC GTGGCTGATA CCGTCGACAA GCATGCCGAA GCATTCAACG
 551  ATATCGCCGA TTCATTGGAT GAAACCAACA CTAAGGCAGA CGAAGCCGTC
 601  AAAACCGCCA ATGAAGCCAA ACAGACGGCC GAAGAAACCA AACAAAACGT
 651  CGATGCCAAA GTAAAAGCTG CAGAAACTGC AGCAGGCAAA GCCGAAGCTG
 701  CCGCTGGCAC AGCTAATACT GCAGCCGACA AGGCCGAAGC TGTCGCTGCA
 751  AAAGTTACCG ACATCAAAGC TGATATCGCT ACGAACAAAG ATAATATTGC
 801  TAAAAAAGCA AACAGTGCCG ACGTGTACAC CAGAGAAGAG TCTGACAGCA
 851  AATTTGTCAG AATTGATGGT CTGAACGCTA CTACCGAAAA ATTGGACACA
 901  CGCTTGGCTT CTGCTGAAAA ATCCATTGCC GATCACGATA CTCGCCTGAA
 951  CGGTTTGGAT AAAACAGTGT CAGACCTGCG CAAAGAAACC CGCCAAGGCC
1001  TTGCAGAACA AGCCGCGCTC TCCGGTCTGT TCCAACCTTA CAACGTGGGT
1051  GGATCCGGAG GGGGTGGTGT CGCCGCCGAC ATCGGTGCGG GGCTTGCCGA
1101  TGCACTAACC GCACCGCTCG ACCATAAAGA CAAAGGTTTG CAGTCTTTGA
1151  CGCTGGATCA GTCCGTCAGG AAAAACGAGA AACTGAAGCT GGCGGCACAA
1201  GGTGCGGAAA AAACTTATGG AAACGGTGAC AGCCTCAATA CGGGCAAATT
1251  GAAGAACGAC AAGGTCAGCC GTTTCGACTT TATCCGCCAA ATCGAAGTGG
1301  ACGGGCAGCT CATTACCTTG GAGAGTGGAG AGTTCCAAGT ATACAAACAA
1351  AGCCATTCCG CCTTAACCGC CTTTCAGACC GAGCAAATAC AAGATTCGGA
1401  GCATTCCGGG AAGATGGTTG CGAAACGCCA GTTCAGAATC GGCGACATAG
```

1451 CGGGCGAACA TACATCTTTT GACAAGCTTC CCGAAGGCGG CAGGGCGACA
1501 TATCGCGGGA CGGCGTTCGG TTCAGACGAT GCCGGCGGAA AACTGACCTA
1551 CACCATAGAT TTCGCCGCCA AGCAGGGAAA CGGCAAAATC GAACATTTGA
1601 AATCGCCAGA ACTCAATGTC GACCTGGCCG CCGCCGATAT CAAGCCGGAT
1651 GGAAAACGCC ATGCCGTCAT CAGCGGTTCC GTCCTTTACA ACCAAGCCGA
1701 GAAAGGCAGT TACTCCCTCG GTATCTTTGG CGGAAAAGCC CAGGAAGTTG
1751 CCGGCAGCGC GGAAGTGAAA ACCGTAAACG GCATACGCCA TATCGGCCTT
1801 GCCGCCAAGC AACTCGAGCA CCACCACCAC CACCACTGA

1 MKHFPSKVLT TAILATFCSG ALAATNDDDV KKAATVAIAA AYNNGQEING
51 FKAGETIYDI DEDGTITKKD ATAADVEADD FKGLGLKKVV TNLTKTVNEN
101 KQNVDAKVKA AESEIEKLTT KLADTDAALA DTDAALDATT NALNKLGENI
151 TTFAEETKTN IVKIDEKLEA VADTVDKHAE AFNDIADSLD ETNTKADEAV
201 KTANEAKQTA EETKQNVDAK VKAAETAAGK AEAAAGTANT AADKAEAVAA
251 KVTDIKADIA TNKDNIAKKA NSADVYTREE SDSKFVRIDG LNATTEKLDT
301 RLASAEKSIA DHDTRLNGLD KTVSDLRKET RQGLAEQAAL SGLFQPYNVG
351 GSGGGGVAAD IGAGLADALT APLDHKDKGL QSLTLDQSVR KNEKLKLAAQ
401 GAEKTYGNGD SLNTGKLKND KVSRFDFIRQ IEVDGQLITL ESGEFQVYKQ
451 SHSALTAFQT EQIQDSEHSG KMVAKRQFRI GDIAGEHTSF DKLPEGGRAT
501 YRGTAFGSDD AGGKLTYTID FAAKQGNGKI EHLKSPELNV DLAAADIKPD
551 GKRHAVISGS VLYNQAEKGS YSLGIFGGKA QEVAGSAEVK TVNGIRHIGL
601 AAKQLEHHHH HH*

**961cL-983**

1 ATGAAACACT TTCCATCCAA AGTACTGACC ACAGCCATCC TTGCCACTTT
51 CTGTAGCGGC GCACTGGCAG CCACAAACGA CGACGATGTT AAAAAAGCTG
101 CCACTGTGGC CATTGCTGCT GCCTACAACA ATGGCCAAGA AATCAACGGT
151 TTCAAAGCTG GAGAGACCAT CTACGACATT GATGAAGACG GCACAATTAC
201 CAAAAAAGAC GCAACTGCAG CCGATGTTGA AGCCGACGAC TTTAAAGGTC
251 TGGGTCTGAA AAAAGTCGTG ACTAACCTGA CCAAAACCGT CAATGAAAAC
301 AAACAAAACG TCGATGCCAA AGTAAAAGCT GCAGAATCTG AAATAGAAAA
351 GTTAACAACC AAGTTAGCAG ACACTGATGC CGCTTTAGCA GATACTGATG
401 CCGCTCTGGA TGCAACCACC AACGCCTTGA ATAAATTGGG AGAAAATATA
451 ACGACATTTG CTGAAGAGAC TAAGACAAAT ATCGTAAAAA TTGATGAAAA
501 ATTAGAAGCC GTGGCTGATA CCGTCGACAA GCATGCCGAA GCATTCAACG
551 ATATCGCCGA TTCATTGGAT GAAACCAACA CTAAGGCAGA CGAAGCCGTC
601 AAAACCGCCA ATGAAGCCAA ACAGACGGCC GAAGAAACCA AACAAAACGT
651 CGATGCCAAA GTAAAAGCTG CAGAAACTGC AGCAGGCAAA GCCGAAGCTG
701 CCGCTGGCAC AGCTAATACT GCAGCCGACA AGGCCGAAGC TGTCGCTGCA
751 AAAGTTACCG ACATCAAAGC TGATATCGCT ACGAACAAAG ATAATATTGC
801 TAAAAAAGCA AACAGTGCCG ACGTGTACAC CAGAGAAGAG TCTGACAGCA
851 AATTTGTCAG AATTGATGGT CTGAACGCTA CTACCGAAAA ATTGGACACA
901 CGCTTGGCTT CTGCTGAAAA ATCCATTGCC GATCACGATA CTCGCCTGAA
951 CGGTTTGGAT AAAACAGTGT CAGACCTGCG CAAAGAAACC CGCCAAGGCC
1001 TTGCAGAACA AGCCGCGCTC TCCGGTCTGT TCCAACCTTA CAACGTGGGT
1051 GGATCCGGCG GAGGCGGCAC TTCTGCGCCC GACTTCAATG CAGGCGGTAC
1101 CGGTATCGGC AGCAACAGCA GAGCAACAAC AGCGAAATCA GCAGCAGTAT
1151 CTTACGCCGG TATCAAGAAC GAAATGTGCA AAGACAGAAG CATGCTCTGT
1201 GCCGGTCGGG ATGACGTTGC GGTTACAGAC AGGGATGCCA AAATCAATGC
1251 CCCCCCCCCG AATCTGCATA CCGGAGACTT TCCAAACCCA AATGACGCAT
1301 ACAAGAATTT GATCAACCTC AAACCTGCAA TTGAAGCAGG CTATACAGGA
1351 CGCGGGGTAG AGGTAGGTAT CGTCGACACA GGCGAATCCG TCGGCAGCAT
1401 ATCCTTTCCC GAACTGTATG GCAGAAAAGA ACACGGCTAT AACGAAAATT
1451 ACAAAAACTA TACGGCGTAT ATGCGGAAGG AAGCGCCTGA AGACGGAGGC
1501 GGTAAAGACA TTGAAGCTTC TTTCGACGAT GAGGCCGTTA TAGAGACTGA
1551 AGCAAAGCCG ACGGATATCC GCCACGTAAA AGAAATCGGA CACATCGATT
1601 TGGTCTCCCA TATTATTGGC GGGCGTTCCG TGGACGGCAG ACCTGCAGGC
1651 GGTATTGCGC CCGATGCGAC GCTACACATA ATGAATACGA ATGATGAAAC
1701 CAAGAACGAA ATGATGGTTG CAGCCATCCG CAATGCATGG GTCAAGCTGG
1751 GCGAACGTGG CGTGCGCATC GTCAATAACA GTTTTGGAAC AACATCGAGG
1801 GCAGGCACTG CCGACCTTTT CCAAATAGCC AATTCGGAGG AGCAGTACCG
1851 CCAAGCGTTG CTCGACTATT CCGGCGGTGA TAAAACAGAC GAGGGTATCC
1901 GCCTGATGCA ACAGAGCGAT TACGGCAACC TGTCCTACCA CATCCGTAAT
1951 AAAAACATGC TTTTCATCTT TTCGACAGGC AATGACGCAC AAGCTCAGCC
2001 CAACACATAT GCCCTATTGC CATTTTATGA AAAAGACGCT CAAAAAGGCA
2051 TTATCACAGT CGCAGGCGTA GACCGCAGTG GAGAAAAGTT CAAACGGGAA

```
2101  ATGTATGGAG AACCGGGTAC AGAACCGCTT GAGTATGGCT CCAACCATTG
2151  CGGAATTACT GCCATGTGGT GCCTGTCGGC ACCCTATGAA GCAAGCGTCC
2201  GTTTCACCCG TACAAACCCG ATTCAAATTG CCGGAACATC CTTTTCCGCA
2251  CCCATCGTAA CCGGCACGGC GGCTCTGCTG CTGCAGAAAT ACCCGTGGAT
2301  GAGCAACGAC AACCTGCGTA CCACGTTGCT GACGACGGCT CAGGACATCG
2351  GTGCAGTCGG CGTGGACAGC AAGTTCGGCT GGGGACTGCT GGATGCGGGT
2401  AAGGCCATGA ACGGACCCGC GTCCTTTCCG TTCGGCGACT TTACCGCCGA
2451  TACGAAAGGT ACATCCGATA TTGCCTACTC CTTCCGTAAC GACATTTCAG
2501  GCACGGGCGG CCTGATCAAA AAGGCGGCA GCCAACTGCA ACTGCACGGC
2551  AACAACACCT ATACGGGCAA AACCATTATC GAAGGCGGTT CGCTGGTGTT
2601  GTACGGCAAC AACAAATCGG ATATGCGCGT CGAAACCAAA GGTGCGCTGA
2651  TTTATAACGG GGCGGCATCC GGCGGCAGCC TGAACAGCGA CGGCATTGTC
2701  TATCTGGCAG ATACCGACCA ATCCGGCGCA AACGAAACCG TACACATCAA
2751  AGGCAGTCTG CAGCTGGACG GCAAAGGTAC GCTGTACACA CGTTTGGGCA
2801  AACTGCTGAA AGTGGACGGT ACGGCGATTA TCGGCGGCAA GCTGTACATG
2851  TCGGCACGCG GCAAGGGGGC AGGCTATCTC AACAGTACCG GACGACGTGT
2901  TCCCTTCCTG AGTGCCGCCA AAATCGGGCA GGATTATTCT TTCTTCACAA
2951  ACATCGAAAC CGACGGCGGC CTGCTGGCTT CCCTCGACAG CGTCGAAAAA
3001  ACAGCGGGCA GTGAAGGCGA CACGCTGTCC TATTATGTCC GTCGCGGCAA
3051  TGCGGCACGG ACTGCTTCGG CAGCGGCACA TTCCGCGCCC GCCGGTCTGA
3101  AACACGCCGT AGAACAGGGC GGCAGCAATC TGGAAAACCT GATGGTCGAA
3151  CTGGATGCCT CCGAATCATC CGCAACACCC GAGACGGTTG AAACTGCGGC
3201  AGCCGACCGC ACAGATATGC CGGGCATCCG CCCCTACGGC GCAACTTTCC
3251  GCGCAGCGGC AGCCGTACAG CATGCGAATG CCGCCGACGG TGTACGCATC
3301  TTCAACAGTC TCGCCGCTAC CGTCTATGCC GACAGTACCG CCGCCCATGC
3351  CGATATGCAG GGACGCCGCC TGAAAGCCGT ATCGGACGGG TTGGACCACA
3401  ACGGCACGGG TCTGCGCGTC ATCGCGCAAA CCCAACAGGA CGGTGGAACG
3451  TGGGAACAGG GCGGTGTTGA AGGCAAAATG CGCGGCAGTA CCCAAACCGT
3501  CGGCATTGCC GCGAAAACCG GCGAAAATAC GACAGCAGCC GCCACACTGG
3551  GCATGGGACG CAGCACATGG AGCGAAAACA GTGCAAATGC AAAAAACCGAC
3601  AGCATTAGTC TGTTTGCAGG CATACGGCAC GATGCGGGCG ATATCGGCTA
3651  TCTCAAAGGC CTGTTCTCCT ACGGACGCTA CAAAAACAGC ATCAGCCGCA
3701  GCACCGGTGC GGACGAACAT GCGGAAGGCA GCGTCAACGG CACGCTGATG
3751  CAGCTGGGCG CACTGGGCGG TGTCAACGTT CCGTTTGCCG CAACGGGAGA
3801  TTTGACGGTC GAAGGCGGTC TGCGCTACGA CCTGCTCAAA CAGGATGCAT
3851  TCGCCGAAAA AGGCAGTGCT TTGGGCTGGA GCGGCAACAG CCTCACTGAA
3901  GGCACGCTGG TCGGACTCGC GGGTCTGAAG CTGTCGCAAC CCTTGAGCGA
3951  TAAAGCCGTC CTGTTTGCAA CGGCGGGCGT GGAACGCGAC CTGAACGGAC
4001  GCGACTACAC GGTAACGGGC GGCTTTACCG GCGCGACTGC AGCAACCGGC
4051  AAGACGGGGG CACGCAATAT GCCGCACACC CGTCTGGTTG CCGGCCTGGG
4101  CGCGGATGTC GAATTCGGCA ACGGCTGGAA CGGCTTGGCA CGTTACAGCT
4151  ACGCCGGTTC CAAACAGTAC GGCAACCACA GCGGACGAGT CGGCGTAGGC
4201  TACCGGTTCT GACTCGAG

   1  MKHFPSKVLT TAILATFCSG ALAATNDDDV KKAATVAIAA AYNNGQEING
  51  FKAGETIYDI DEDGTITKKD ATAADVEADD FKGLGLKKVV TNLTKTVNEN
 101  KQNVDAKVKA AESEIEKLTT KLADTDAALA DTDAALDATT NALNKLGENI
 151  TTFAEETKTN IVKIDEKLEA VADTVDKHAE AFNDIADSLD ETNTKADEAV
 201  KTANEAKQTA EETKQNVDAK VKAAETAAGK AEAAAGTANT AADKAEAVAA
 251  KVTDIKADIA TNKDNIAKKA NSADVYTREE SDSKFVRIDG LNATTEKLDT
 301  RLASAEKSIA DHDTRLNGLD KTVSDLRKET RQGLAEQAAL SGLFQPYNVG
 351  GSGGGGTSAP DFNAGGTGIG SNSRATTAKS AAVSYAGIKN EMCKDRSMLC
 401  AGRDDVAVTD RDAKINAPPP NLHTGDFPNP NDAYKNLINL KPAIEAGYTG
 451  RGVEVGIVDT GESVGSISFP ELYGRKEHGY NENYKNYTAY MRKEAPEDGG
 501  GKDIEASFDD EAVIETEAKP TDIRHVKEIG HIDLVSHIIG GRSVDGRPAG
 551  GIAPDATLHI MNTNDETKNE MMVAAIRNAW VKLGERGVRI VNNSFGTTSR
 601  AGTADLFQIA NSEEQYRQAL LDYSGGDKTD EGIRLMQQSD YGNLSYHIRN
 651  KNMLFIFSTG NDAQAQPNTY ALLPFYEKDA QKGIITVAGV DRSGEKFKRE
 701  MYGEPGTEPL EYGSNHCGIT AMWCLSAPYE ASVRFTRTNP IQIAGTSFSA
 751  PIVTGTAALL LQKYPWMSND NLRTTLLTTA QDIGAVGVDS KFGWGLLDAG
 801  KAMNGPASFP FGDFTADTKG TSDIAYSFRN DISGTGGLIK KGGSQLQLHG
 851  NNTYTGKTII EGGSLVLYGN NKSDMRVETK GALIYNGAAS GGSLNSDGIV
 901  YLADTDQSGA NETVHIKGSL QLDGKGTLYT RLGKLLKVDG TAIIGGKLYM
 951  SARGKGAGYL NSTGRRVPFL SAAKIGQDYS FFTNIETDGG LLASLDSVEK
1001  TAGSEGDTLS YYVRRGNAAR TASAAAHSAP AGLKHAVEQG GSNLENLMVE
1051  LDASESSATP ETVETAAADR TDMPGIRPYG ATFRAAAAVQ HANAADGVRI
1101  FNSLAATVYA DSTAAHADMQ GRRLKAVSDG LDHNGTGLRV IAQTQQDGGT
```

```
1151   WEQGGVEGKM RGSTQTVGIA AKTGENTTAA ATLGMGRSTW SENSANAKTD
1201   SISLFAGIRH DAGDIGYLKG LFSYGRYKNS ISRSTGADEH AEGSVNGTLM
1251   QLGALGGVNV PFAATGDLTV EGGLRYDLLK QDAFAEKGSA LGWSGNSLTE
1301   GTLVGLAGLK LSQPLSDKAV LFATAGVERD LNGRDYTVTG GFTGATAATG
1351   KTGARNMPHT RLVAGLGADV EFGNGWNGLA RYSYAGSKQY GNHSGRVGVG
1401   YRF*
```

It will be understood that the invention has been described by way of example only and medications may be made whilst remaining within the scope of the invention. For instance, the use of proteins from other strains is envisaged [*e.g.* see WO00/66741 for polymorphic sequences for ORF4, ORF40, ORF46, 225, 235, 287, 519, 726, 919 and 953].

## EXPERIMENTAL DETAILS

### *Cloning strategy and oligonucleotide design*

Genes coding for antigens of interest were amplified by PCR. using oligonucleotides designed on the basis of the genomic sequence of *N. meningitidis* B MC58. Genomic DNA from strain 2996 was always used as a template in PCR reactions, unless otherwise specified, and the amplified fragments were cloned in the expression vector pET21b+ (Novagen) to express the protein as C-terminal His-tagged product, or in pET-24b+(Novagen) to express the protein in 'untagged' form (*e.g.* ΔG 287K).

Where a protein was expressed without a fusion partner and with its own leader peptide (if present), amplification of the open reading frame (ATG to STOP codons) was performed.

Where a protein was expressed in 'untagged' form, the leader peptide was omitted by designing the 5'-end amplification primer downstream from the predicted leader sequence.

The melting temperature of the primers used in PCR depended on the number and type of hybridising nucleotides in the whole primer, and was determined using the formulae:

$$T_{m1} = 4\,(G+C) + 2\,(A+T) \qquad \text{(tail excluded)}$$

$$T_{m2} = 64.9 + 0.41\,(\%\,GC) - 600/N \qquad \text{(whole primer)}$$

The melting temperatures of the selected oligonucleotides were usually 65-70°C for the whole oligo and 50-60°C for the hybridising region alone.

Oligonucleotides were synthesised using a Perkin Elmer 394 DNA/RNA Synthesizer, eluted from the columns in 2.0ml $NH_4OH$, and deprotected by 5 hours incubation at 56°C. The oligos were precipitated by addition of 0.3M Na-Acetate and 2 volumes ethanol. The samples were centrifuged and the pellets resuspended in water.

The oligonucleotides used to make the 961 proteins and hybrids of the invention include:

| | | Sequences | Restriction site |
|---|---|---|---|
| **Orf46.1L** | Fwd | GGGAATTCCATATG-GGCATTTCCCGCAAAATATC | NdeI |
| | Rev | CCCGCTCGAG-TTACGTATCATATTTCACGTGC | XhoI |
| **orf46. (His-GST)** | Fwd | GGGAATTCCATATGCACGTGAAATATGATACGAAG | BamHI-NdeI |
| | Rev | CCCGCTCGAGTTTACTCCTATAACGAGGTCTCTTAAC | XhoI |
| **orf46.1-His** | Fwd | GGGAATTCCATATGTCAGATTTGGCAAACGATTCTT | NdeI |
| | Rev | CCCGCTCGAGCGTATCATATTTCACGTGC | XhoI |
| **287L** | Fwd | CTAGCTAGC-TTTAAACGCAGCGTAATCGCAATGG | NheI |
| | Rev | CCCGCTCGAG-TCAATCCTGCTCTTTTTTGCC | XhoI |
| **287** | Fwd | CTAGCTAGC-GGGGGCGGCGGTGGCG | NheI |
| | Rev | CCCGCTCGAG-TCAATCCTGCTCTTTTTTGCC | XhoI |
| **287LOrf4** | Fwd | CTAGCTAGCGCTCATCCTCGCCOCC-TGCGGGGGCGGCGGT | NheI |
| | Rev | CCCGCTCGAG-TCAATCCTGCTGTTTTTTGCC | XhoI |
| **287-fu** | Fwd | CGGGGATCC-GGGGGCGGCGGTGGGG | BamHI |
| | Rev | CCCGCTCGAG-TCAATCCTGCTCTTTTTTGCC | XhoI |
| **287-His** | Fwd | CTAGCTAGC-GGGGGCGGCGGTGGCG | NheI |
| | Rev | CCCGCTCGAG-ATCCTGCTCTTTTTTGCC* | XhoI |
| **287-His(2996)** | Fwd | CTAGCTAGC-TGCGGGGGCGGCGGTGGCG | NheI |
| | Rev | CCCGCTCGAG-ATCCTGCTCTTTTTTGCC | XhoI |
| **Δ1 287-His** | Fwd | CGCGGATCCGCTAGC-CCCGATGTTAAATCGGC § | NheI |
| **Δ2 287-His** | Fwd | CGCGGATCCGCTAGC-CAAGATATGGCGGCAGT§ | NheI |
| **Δ3 287-His** | Fwd | CGCGGATCCGCTAGC-GCCGAATCCGCAAATCA§ | NheI |
| **Δ4 287-His** | Fwd | CGCGCTAGC-GGAAGGGTTGATTTGGCTAATGG§ | NheI |
| **Δ4 287MC58-His** | Fwd | CGCGCTAGC-GGAAGGGTTGATTTGGCTAATGG§ | NheI |
| **287a-His** | Fwd | CGCCATATG-TTTAAACGCAGCGTAATCGC | NdeI |
| | Rev | CCCGCTCGAG-AAAATTGCTACCGCCATTCGCAGG | XhoI |

(continued)

| | | Sequences | Restriction site |
|---|---|---|---|
| **287b-His** | Fwd | CGCCATATG-GGAAGGGTTGATTTGGCTAATGG | Ndel |
| **Z87b-2996-His** | Rev | CCCGCTCGAG-CTTGTCTTTATAAATGATGACATATTTG | Xhol |
| **287b-MC58-His** | Rev | CCCGGTCGAG-TTTATAAAAGATAATATA7TGATTGATTCC | Xhol |
| **287c-2996-His** | Fwd | CGCGCTAGC-ATGCCGCTGATTCCCGTCAATC | Nhel |
| **'287$^{untagged}$'(2996)** | Fwd | CTAGCTAGC-GGGGGCGGCGGTGGCG | Nhel |
| | Rev | CCCGCTCGAG-TCAATCCTGCTCTTTTTTGCC | Xhol |
| **ΔG287-His*** | Fwd | CGCGGATCCGCTAGC-CCCGATGTTAAATCGGC | Nhel |
| | Rev | CCCGCTCGAG-ATCCTGCTCTTTTTTGCC | Xhol |
| **ΔG287K(2996)** | Fwd | CGCGGATCCGCTAGC-CCCGATGTTAAATCGGC | Nhel |
| | Rev | CCCGCTCGAG-TCAATCCTGCTCTTTTTTGCC | Xhol |
| **ΔG 287-L** | Fwd | CGCGGATCCGCTAGC-TTTGAACGCAGTGTGATTGCAATGGCTTGTATTTTTGCC CTTTCAGCCTGT TCGCCCGATGTTAAATCGGCG | Nhel |
| | Rev | GGCGCTCGAG-TCAATCCTGCTCTTTTTTGCC | Xhol |
| **ΔG 287-Orf4L** | Fwd | CGCGGATCCGCTAGC-AAAACCTTCTTCAAAACCCTTTCCGCCGCCGCACTCGCG CTCATCCTCGCCGCCTGC TCGCCCGATGTTAAATCG | Nhel |
| | Rev | CCCGCTCGAG-TCAATCCTGCTCTTTTTTGCC | Xhol |
| **741-His (MC58)** | Fwd | CGCGGATCCCATATG-AGCAGCGGAGGGGGTG | Ndel |
| | Rev | CCCGCTCGAG-TTGCTTGGCGGCAAGGC | Xhol |
| **ΔG741-His (MC58)** | Fwd | CGCGGATCCCATATG-GTCGCCGCCGACATCG | Ndel |
| | Rev | CCCGCTCGAG-TTGCTTGGCGGCAAGGC | Xhol |
| **919L** | Fwd | CGCGGATCCCATATG-AAAAAATACCTATTCCGC | Ndel |
| | Rev | CCCGCTCGAG-TTACGGGCGGTATTCGG | Xhol |
| **919** | Fwd | CGCGGATCCCATATG-CAAAGCAAGAGCATCCAAA | Ndel |
| | Rev | CCCGCTCGAG-TTACGGGCGGTATTCGG | Xhol |

(continued)

| | | Sequences | Restriction site |
|---|---|---|---|
| **919L Orf4** | Fwd | GGGAATTCCATATGAAAACCTTCTTCAAAACCCTTTCCG CCGCCGCGCTAGCGCTCATCCTCGCCGCC-TGCCAAAGCAAGAGCATC | NdeI-(NheI) |
| | Rev | CCCGCTCGAG-TTACGGGCGGTATTCGGGGTTCATACCG | XhoI |
| **953L** | Fwd | GGGAATTCCATATG-AAAAAAATCATCTTCGCCG | NdeI |
| | Rev | CCCGCTCGAG-TTATIGTITGGCTGCCTCGAT | XhoI |
| **953-fu** | Fwd | GGGAATTCATATG-GCCACCTACAAAGTGGACG | NdeI |
| | Rev | CGGGGGATCC-TTGTTTGGCTGCCTCGATTTG | BamHI |
| **961L** | Fwd | CGCGGATCCCATATG-AAACACTTTCCATCC | NdeI |
| | Rev | CCCGCTCGAG-TTACGACTCGTAATTGAC | XhoI |
| **961** | Fwd | CGCGGATCCCATATG-GCCACAAGCGACGAC | NdeI |
| | Rev | CCCGCTCGAG-TTACCACTCGTAATTGAC | XhoI |
| **961 c (His/GST)** | Fwd | CGCGGATCCCATATG-GCCACAAACGACG | BamHI-NdeI |
| | Rev | CCCGCTCGAG-ACCCACGTTGTAAGGTTG | XhoI |
| **961 c-(His/GST) (MC58)** | Fwd | CGCGGATCCCATATG-GCCACAAGCGACGACGA | BamHI-NdeI |
| | Rev | CCCGCTCGAG-ACCCACGTTGTAAGGTTG | XhoI |
| **961 c-L** | Fwd | CGCGGATCCCATATG-ATGAAACACTTTCCATCC | NdeI |
| | Rev | CCCGCTCGAG-TTAACCGACGTTGTAAGGT | XhoI |
| **961 c-L (MC58)** | Fwd | CGCGGATCCCATATG-ATGAAACACTTTCCATCC | NdeI |
| | Rev | CCCGCTCGAG-TTAACCCACGTTGTAAGGT | XhoI |
| **961 d (His/GST)** | Fwd | CGCGGATCCCATATG-GCCACAAACGACG | BamHI-NdeI |
| | Rev | CCCGCTCGAG-GTCTGACACTGTTTTATCC | XhoI |
| **961 Δ1-L** | Fwd | CGCGGATCCCATATG-ATGAAACACTTTCGATCC | NdeI |
| | Rev | CCCGCTCGAG-TTATGCTTTGGCGGCAAAG | XhoI |
| **fu 961-...** | Fwd | CGCGGATCCCATATG-GCCACAAACGACGAC | NdeI |
| | Rev | CGCGGATC-CCCACTCGTAATTGACGCC | BamHI |

(continued)

| | | Sequences | Restriction site |
|---|---|---|---|
| **fu 961-... (MC58)** | Fwd | CGCGGATCCCATATG-GCCACAAGCGACGAC | NdeI |
| | Rev | CGCGGATCC-CCACTCGTAATTGACGCC | BamHI |
| **fu 961 c -...** | Fwd | CGCGATCCCATATG-GCCACAAACGACGAC | NdeI |
| | Rev | CGCGGATCC-ACCCACGTTGTAAGGTTG | BamHI |
| **fu 961 c-L-...** | Fwd | CGCGGATCCCATATG-ATGAAACACTTTCCATCC | NdeI |
| | Rev | CGCGGATCC-ACCCACGTTGTAAGGTTG | BamHI |
| **fu (961)-741 (MC58)-His** | Fwd | CGCGGATCC-GGAGGGGGTGGTGTCG | BamHI |
| | Rev | CCCGCTCGAG-TTGCTTGGCGGCAAGGC | XhoI |
| **fu (961)-983-His** | Fwd | CGCGGATCC-GGCGGAGGCGGCACTT | BamHI |
| | Rev | CCCGCTCGAG-GAACCGGTAGCCTACG | XhoI |
| **fu (961)-Orf46.1-His** | Fwd | CGCGGATCCGGTGGTGGTGGT-TCAGATTTGGCAAACGATTC | BamHI |
| | Rev | CCCGCTCGAG-CGTATCATATTTCACGTGC | XhoI |
| **fu (961 c-L)-741(MC58)** | Fwd | CGCGGATCC -GGAGGGGGTGGTGTCG | BamHI |
| | Rev | CCCGCTCGAG-TTATTGCTTGGCGGCAAG | XhoI |
| **fu (96lc-L)-983** | Fwd | CGCGGATCC - GGCGGAGGCGGCACTT | BamHI |
| | Rev | CCCGCTCGAG-TCAGAACCGGTAGCCTAC | XhoI |
| **fu (961c-L)-Orf46.1** | Fwd | CGCGGATCCGGTGGTGGTGGT-TCAGATTTGGCAAACGATTC | BamHI |
| | Rev | CCCGCTCGAG-TTACGTATCATATTTCACGTGC | XhoI |
| **961-(His/GST)** | Fwd | CGCGGATCCCATATG-GCCACAAGCGACGACG | BamHI-NdeI |
| **(MC58)** | Rev | CCCGCTCGAG-CCACTCGTAATTGACGCC | XhoI |
| **961 Δ1-His** | Fwd | CGCGGATCCCATATG-GCCACAAACGACCAC | NdeI |
| | Rev | CCCGCTCGAG-TGCTTTGGCGGCAAAGTT | XhoI |
| **961a-(His/GST)** | Fwd | CGCGGATCCCATATG-GCCACAAACGACGAC | BamHI-NdeI |
| | Rev | CCCGCTCGAG-TTTAGCAATATTATCTTTGTTCGTAGC | XhoI |

(continued)

| | | Sequences | Restriction site |
|---|---|---|---|
| **961b-(His/GST)** | Fwd | CGCGGATCCCATATG-AAAGCAAACCGTGCCGA | BamHI-NdeI |
| | Rev | CCCGCTCGAG-CCACTCGTAATTGACGCC | XhoI |
| **961-His/GST$^{GATE}$** | Fwd | ggggacaagtttgtacaaaaaagcaggctGCAGCCACAAACGACGACG ATGTTAAAAAAGC | *attB1* |
| | Rev | ggggaccactttgtacaagaaagctgggtTTACCACTCGTAATTGACGC CGACATGGTAGG | *attB2* |
| **983-His(2996)** | Fwd | GGCGGATCCGCTAGC-TTAGGCGGCGGGGGAG | NheI |
| | Rev | CCCGCTCGAG-GAACCGGTAGCCTACG | XhoI |
| **ΔG983-His(2996)** | Fwd | CCCCTAGCTAGC-ACTTCTGCGCCCGACTT | NheI |
| | Rev | CCCGCTCGAG-GAACCGGTAGCCTACG | XhoI |
| **983-His** | Fwd | CGCGGATCCGCTAGC-TTAGGCGGCGGCGGAG | NheI |
| | Rev | CCCGCTCGAG-GAACCGCTAGCCTACG | XhoI |
| **ΔG983-His** | Fwd | GGCGGATCCGCTAGC-ACTTCTGCGCCCGACTT | NheI |
| | Rev | CCCGCTCGAG-GAACCGGTAGCCTACC | XhoI |
| **983L** | Fwd | CGCGGATCCGCTAGC-CGAACGACCCCAACCTTCCCTACAAAAACTTTCAA | NheI |
| | Rev | CCCGCTCGAG-TCAGAACCGACGTGCCAAGCCGTTC | XhoI |

* This primer was used as a Reverse primer for all the C terminal fusions of 287 to the His-tag.
§ Forward primers used in combination with the 287-His Reverse primer.
NB-All PCR reactions use strain 2996 unless otherwise specified (*e.g.* strain MC58)

In all constructs starting with an ATG not followed by a unique *Nhe*I site, the ATG codon is part of the *Nde*I site used for cloning. The constructs made using *Nhe*I as a cloning site at the 5' end (*e.g.* all those containing 287 at the N-terminus) have two additional codons (GCT AGC) fused to the coding sequence of the antigen.

***Preparation of chromosomal DNA templates***

*N.meningitidis* strains 2996, MC58, 394.98, 1000 and BZ232 (and others) were grown to exponential phase in 100ml of GC medium, harvested by centrifugation, and resuspended in 5ml buffer (20% w/v sucrose, 50mM Tris-HCl, 50mM EDTA, pH8). After 10 minutes incubation on ice, the bacteria were lysed by adding 10ml of lysis solution (50mM NaCl, 1% Na-Sarlcosyl, 50μg/ml Proteinase K), and the suspension incubated at 37°C for 2 hours. Two phenol extractions (equilibrated to pH 8) and one $CHCl_3$/isoamylalcohol (24:1) extraction were performed DNA was precipitated by addition of 0.3M sodium acetate and 2 volumes of ethanol, and collected by centrifugation. The pellet was washed once with 70%(v/v) ethanol and redissolved in 4.0ml TE buffer (10mM Tris-HCl, 1mM EDTA, pH 8.0). The DNA concentration was measured by reading $OD_{260}$.

***PCR Amplification***

The standard PCR protocol was as follows: 200ng of genomic DNA from 2996, MC58,1000, or BZ232 strains or 10ng of plasmid DNA preparation of recombinant clones were used as template in the presence of 40μM of each oligonucletide primer, 400-800 μM dNTPs solution, lx PCR buffer (including 1.5mM $MgCl_2$), 2.5 units *TaqI* DNA polymerase (using Perkin-Elmer AmpliTaQ, Boerhingher Mannheim Expand™ Long Template).

After a preliminary 3 minute incubation of the whole mix at 95°C, each sample underwent a two-step amplification: the first 5 cycles were performed using the hybridisation temperature that excluded the restriction enzyme tail of the primer ($T_{m1}$). This was followed by 30 cycles according to the hybridisation temperature calculated for the whole length oligos ($T_{m2}$). Elongation times, performed at 68°C or 72°C, varied according to the length of the Orf to be amplified. In the case of Orf1 the elongation time, starting from 3 minutes, was increased by 15 seconds each cycle. The cycles were completed with a 10 minute extension step at 72°C.

The amplified DNA was either loaded directly on a 1% agarose gel. The DNA fragment corresponding to the band of correct size was purified from the gel using the Qiagen Gel Extraction Kit, following the manufacturer's protocol.

***Digestion of PCR fragments and of the cloning vectors***

The purified DNA corresponding to the amplified fragment was digested with the appropriate restriction enzymes for cloning into pET-21b+, pET22b+ or pET-24b- Digested fragments were purified using the QIAquick PCR purification kit (following the manufacturer's instructions) and eluted with either $H_2O$ or 10mM Tris, pH 8.5. Plasmid vectors were digested with the appropriate restriction enzymes, loaded onto a 1.0% agarose gel and the band corresponding to the digested vector purified using the Qiagen QIAquack Gel Extraction Kit.

***Cloning***

The fragments corresponding to each gene, previously digested and purified, were ligated into pET21b+, pET22b+ or pET-24b+. A molar ratio of 3:1 fragment/vector was used with T4 DNA ligase in the ligation buffer supplied by the manufacturer.

Recombinant plasmid was transformed into competent *E.coli* DH5 or HB101 by incubating the ligase reaction solution and bacteria for 40 minutes on ice, then at 37°C for 3 minutes.

This was followed by the addition of 800μl LB broth and incubation at 37°C for 20 minutes. The cells were centrifuged at maximum speed in an Eppendorf microfuge, resuspended in approximately 200μl of the supernatant and plated onto LB ampicillin (100mg/ml) agar. Screening for recombinant clones was performed by growing randomly selected colonies overnight at 37°C in 4.0ml of LB broth + 100μg/ml ampicillin. Cells were pelleted and plasmid DNA extracted using the Qiagen QIAprep Spin Miniprep Kit, following the manufacturer's instructions. Approximately 1μg of each individual miniprep was digested with the appropriate restriction enzymes and the digest loaded onto a 1-1.5% agarose gel (depending on the expected insert size), in parallel with the molecular weight marker (1kb DNA Ladder, GIBCO). Positive clones were selected on the basis of the size of insert.

***Expression***

After cloning each gene into the expression vector, recombinant plasmids were transformed into *E.coli* strains suitable for expression of the recombinant protein. 1μl of each construct was used to transform *E.coli* BL21-DE3 as described above. Single recombinant colonies were inoculated into 2ml LB+Amp (100μg/ml), incubated at 37°C overnight, then diluted 1:30 in 20ml of LB+Amp (100μg/ml) in 100ml flasks, to give an $OD_{600}$ between 0.1 and 0.2. The flasks were incubated at 30°C or at 37°C in a gyratory water bath shaker until $OD_{600}$ indicated exponential growth suitable for induction of expression (0.4-0.8 OD). Protein expression was induced by addition of 1.0mM IPTG. After 3 hours incubation at 30°C or 37°C the $OD_{600}$ was measured and expression examined. 1.0ml of each sample was centrifuged in a microfuge, the pellet resuspended in PBS and analysed by SDS-PAGE and Coomassie Blue staining.

### *Gateway cloning and expression*

Sequences labelled GATE were cloned and expressed using the GATEWAY Cloning Technology (GIBCO-BRL). Recombinational cloning (RC) is based on the recombination reactions that mediate the integration and excision of phage into and from the *E.coli* genome, respectively. The integration involves recombination of the *attP* site of the phage DNA within the *attB* site located in the bacterial genome (BP reaction) and generates an integrated phage genome flanked by *attL* and *attR* sites. The excision recombines *attL* and *attR* sites back to *attP* and *attB* sites (LR reaction). The integration reaction requires two enzymes [the phage protein Integrase (Int) and the bacterial protein integration host factor (IHF)] (BP clonase). The excision reaction requires Int, IHF, and an additional phage enzyme, Excisionase (Xis) (LR clonase). Artificial derivatives of the 25-bp bacterial *attB* recombination site, referred to as B1 and B2, were added to the 5' end of the primers used in PCR reactions to amplify Neisserial ORFs. The resulting products were BP cloned into a "Donor vector" containing complementary derivatives of the phage *attP* recombination site (P1 and P2) using BP clonase. The resulting "Entry clones" contain ORFs flanked by derivatives of the *attL* site (L1 and L2) and were subcloned into expression "destination vectors" which contain derivatives of the *attL*-compatible *attR* sites (R1 and R2) using LR clonase. This resulted in "expression clones" in which ORFs are flanked by B1 and B2 and fused in frame to the GST or His N terminal tags. The *E. coli* strain used for GATEWAY expression is BL21-SI. Cells of this strain are induced for expression of the T7 RNA polymerase by growth in medium containing salt (0.3 M NaCl). Note that this system gives N-terminus His tags.

### *Preparation of membrane proteins.*

Fractions composed principally of either inner, outer or total membrane were isolated in order to obtain recombinant proteins expressed with membrane-localisation leader sequences. The method for preparation of membrane fractions, enriched for recombinant proteins, was adapted from Filip *et. al.* [*J.Bact.* (1973) 115:717-722] and Davies *et. al.* [*J.Immunol.Meth.* (1990) 143:215-225]. Single colonies harbouring the plasmid of interest were grown overnight at 37°C in 20 ml of LB/Amp (100 μg/ml) liquid culture. Bacteria were diluted 1:30 in 1.0 L of fresh medium and grown at either 30°C or 37°C until the $OD_{550}$ reached 0.6-0.8. Expression of recombinant protein was induced with IPTG at a final concentration of 1.0 mM. After incubation for 3 hours, bacteria were harvested by centrifugation at 8000*g* for 15 minutes at 4°C and resuspended in 20 ml of 20 mM Tris-HCl (pH 7.5) and complete protease inhibitors (Boehringer-Mannheim). All subsequent procedures were performed at 4°C or on ice.

Cells were disrupted by sonication using a Branson Sonifier 450 and centrifuged at 5000g for 20 min to sediment unbroken cells and inclusion bodies. The supernatant, containing membranes and cellular debris, was centrifuged at 50000g (Beckman Ti50, 29000rpm) for 75 min, washed with 20 mM Bis-tris propane (pH 6.5), 1.0 M NaCl, 10% (v/v) glycerol and sedimented again at 50000g for 75 minutes. The pellet was resuspended in 20mM Tris-HCl (pH 7.5), 2.0% (v/v) Sarkosyl, complete protease inhibitor (1.0 mM EDTA, final concentration) and incubated for 20 minutes to dissolve inner membrane. Cellular debris was pelleted by centrifugation at 5000g for 10 min and the supernatant centrifuged at 75000*g* for 75 minutes (Beckman Ti50, 33000rpm). Proteins 008L and 519L were found in the supernatant suggesting inner membrane localisation. For these proteins both inner and total membrane fractions (washed with NaCl as above) were used to immunise mice. Outer membrane vesicles obtained from the 75000*g* pellet were washed with 20 mM Tris-HCl (pH 7.5) and centrifuged at 75000g for 75 minutes or overnight. The OMV was finally resuspended in 500 μl of 20 mM Tris-HCl (pH 7.5), 10% v/v glycerol. Orf1L and Orf40L were both localised and enriched in the outer membrane fraction which was used to immunise mice. Protein concentration was estimated by standard Bradford Assay (Bio-Rad), while protein concentration of inner membrane fraction was determined with the DC protein assay (Bio-Rad). Various fractions from the isolation procedure were assayed by SDS-PAGE.

### *Purification of His-tagged proteins*

Various forms of 287 were cloned from strains 2996 and MC58. They were constructed with a C-terminus His-tagged fusion and included a mature form (aa 18-427), constructs with deletions (Δ1, Δ 2, Δ3 and Δ4) and clones composed of either B or C domains. For each clone purified as a His-fusion, a single colony was streaked and grown overnight at 37°C on a LB/Amp (100 μg/ml) agar plate. An isolated colony from this plate was inoculated into 20ml of

LB/Amp (100 μg/ml) liquid medium and grown overnight at 37°C with shaking. The overnight culture was diluted 1:30 into 1.0 L LB/Amp (100 μg/ml) liquid medium and allowed to grow at the optimal temperature (30 or 37°C) until the $OD_{550}$ reached 0.6-0.8. Expression of recombinant protein was induced by addition of IPTG (final concentration 1.0mM) and the culture incubated for a further 3 hours. Bacteria were harvested by centrifugation at 8000*g* for 15 min at 4°C. The bacterial pellet was resuspended in 7.5 ml of either (i) cold buffer A (300 mM NaCl, 50 mM phosphate buffer, 10 mM imidazole, pH 8.0) for soluble proteins or (ii) buffer B (10mM Tris-HCl, 100 mM phosphate buffer, pH 8.8 and, optionally, 8M urea) for insoluble proteins. Proteins purified in a soluble form included 287-His, Δ1, Δ2, Δ3 and Δ4287-His, Δ4287MC58-His, 287c-His and 287cMC58-His. Protein 287bMC58-His was insoluble and purified accordingly. Cells were disrupted by sonication on ice four times for 30 sec at 40W using a Branson sonifier 450 and centrifuged at 13000xg for 30 min at 4°C. For insoluble proteins, pellets were resuspended in 2.0 ml buffer C (6 M guanidine hydrochloride, 100 mM phosphate buffer, 10 mM Tris- HCl, pH 7.5 and treated with 10 passes of a Dounce homogenizer. The homogenate was centrifuged at 13000g for 30 min and the supernatant retained. Supernatants for both soluble and insoluble preparations were mixed with 150μl $Ni^{2+}$-resin (previously equilibrated with either buffer A or buffer B, as appropriate) and incubated at room temperature with gentle agitation for 30 min. The resin was Chelating Sepharose Fast Flow (Pharmacia), prepared according to the manufacturer's protocol. The batch-wise preparation was centrifuged at 700g for 5 min at 4°C and the supernatant discarded. The resin was washed twice (batch-wise) with 10ml buffer A or B for 10 min, resuspended in 1.0 ml buffer A or B and loaded onto a disposable column. The resin continued to be washed with either (i) buffer A at 4°C or (ii) buffer B at room temperature, until the $OD_{280}$ of the flow-through reached 0.02-0.01. The resin was further washed with either (i) cold buffer C (300mM NaCl, 50mM phosphate buffer, 20mM imidazole, pH 8.0) or (ii) buffer D (10mM Tris-HCl, 100mM phosphate buffer, pH 6.3 and, optionally, 8M urea) until $OD_{280}$ of the flow-through reached 0.02-0.01. The His-fusion protein was eluted by addition of 700μl of either (i) cold elution buffer A (300 mM NaCl, 50mM phosphate buffer, 250 mM imidazole, pH 8.0) or (ii) elution buffer B (10 mM Tris-HCl, 100 mM phosphate buffer, pH 4.5 and, optionally, 8M urea) and fractions collected until the $OD_{280}$ indicated all the recombinant protein was obtained. 20μl aliquots of each elution fraction were analysed by SDS-PAGE. Protein concentrations were estimated using the Bradford assay.

***Renaturation of denatured His-fusion proteins.***

Denaturation was required to solubilize 287bMC8, so a renaturation step was employed prior to immunisation. Glycerol was added to the denatured fractions obtained above to give a final concentration of 10% v/v. The proteins were diluted to 200 μg/ml using dialysis buffer I (10% v/v glycerol, 0.5M arginine, 50 mM phosphate buffer, 5.0 mM reduced glutathione, 0.5 mM oxidised glutathione, 2.0M urea, pH 8.8) and dialysed against the same buffer for 12-14 hours at 4°C. Further dialysis was performed with buffer II (10% v/v glycerol, 0.5M arginine, 50mM phosphate buffer, 5.0mM reduced glutathione, 0.5mM oxidised glutathione, pH 8.8) for 12-14 hours at 4°C. Protein concentration was estimated using the formula:

$$Protein\ (mg/ml) = (1.55 \times OD_{280}) - (0.76 \times OD_{260})$$

***Amino acid sequence analysis.***

Automated sequence analysis of the $NH_2$-terminus of proteins was performed on a Beckman sequencer (LF 3000) equipped with an on-line phenylthiohydantoin-amino acid analyser (System Gold) according to the manufacturer's recommendations.

***Immunization***

Balb/C mice were immunized with antigens on days 0, 21 and 35 and sera analyzed at day 49.

***Sera analysis - ELISA***

The acapsulated MenB M7 and the capsulated strains were plated on chocolate agar plates and incubated overnight at 37°C with 5% $CO_2$. Bacterial colonies were collected from the agar plates using a sterile dracon swab and inoculated into Mueller-Hinton Broth (Difco) containing 0.25% glucose. Bacterial growth was monitored every 30 minutes by following $OD_{620}$. The bacteria were let to grow until the OD reached the value of 0.4-0.5. The culture was centrifuged for 10 minutes at 4000rpm. The supernatant was discarded and bacteria were washed twice with PBS, resuspended in PBS containing 0.025% formaldehyde, and incubated for 1 hour at 37°C and then overnight at 4°C with stirring. 100μl

bacterial cells were added to each well of a 96 well Greiner plate and incubated overnight at 4°C. The wells were then washed three times with PBT washing buffer (0.1% Tween-20 in PBS). 200μl of saturation buffer (2.7% polyvinylpyrrolidone 10 in water) was added to each well and the plates incubated for 2 hours at 37°C. Wells were washed three times with PBT. 200μl of diluted sera (Dilution buffer: 1% BSA, 0.1% Tween-20, 0.1% $NaN_3$ in PBS) were added to each well and the plates incubated for 2 hours at 37°C. Wells were washed three times with PBT. 100μl of HRP-conjugated rabbit anti-mouse (Dako) serum diluted 1:2000 in dilution buffer were added to each well and the plates were incubated for 90 minutes at 37°C. Wells were washed three times with PBT buffer. 100μl of substrate buffer for HRP (25ml of citrate buffer pH5, 10mg of O-phenildiamine and 10μl of $H_2O_2$) were added to each well and the plates were left at room temperature for 20 minutes. 100μl 12.5% $H_2SO_4$ was added to each well and $OD_{490}$ was followed. The ELISA titers were calculated abitrarely as the dilution of sera which gave an $OD_{490}$ value of 0.4 above the level of preimmune sera. The ELISA was considered positive when the dilution of sera with $OD_{490}$ of 0.4 was higher than 1:400.

***Sera analysis - FACS Scan bacteria binding assay***

The acapsulated MenB M7 strain was plated on chocolate agar plates and incubated overnight at 37°C with 5% $CO_2$. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000rpm. The supernatant was discarded and the pellet was resuspended in blocking buffer (1% BSA in PBS, 0.4% $NaN_3$) and centrifuged for 5 minutes at 4000rpm. Cells were resuspended in blocking buffer to reach $OD_{620}$ of 0.05. 100μl bacterial cells were added to each well of a Costar 96 well plate. 100μl of diluted (1:100, 1:200, 1:400) sera (in blocking buffer) were added to each well and plates incubated for 2 hours at 4°C. Cells were centrifuged for 5 minutes at 4000rpm, the supernatant aspirated and cells washed by addition of 200μl/well of blocking buffer in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/well of PBS, 0.25% formaldehyde. Samples were transferred to FACScan tubes and read. The condition for FACScan (Laser Power 15mW) setting were: FL2 on; FSC-H threshold:92; FSC PMT Voltage: E 01; SSC PMT: 474; Amp. Gains 6.1; FL-2 PMT: 586; compensation values: 0.

***Sera analysis - bactericidal assay***

*N. meningitidis* strain 2996 was grown overnight at 37°C on chocolate agar plates (starting from a frozen stock) with 5% $CO_2$. Colonies were collected and used to inoculate 7ml Mueller-Hinton broth, containing 0.25% glucose to reach an $OD_{620}$ of 0.05-0.08. The culture was incubated for approximately 1.5 hours at 37 degrees with shacking until the $OD_{620}$ reached the value of 0.23-0.24. Bacteria were diluted in 50mM Phosphate buffer pH 7.2 containing 10mM $MgCl_2$, 10mM $CaCl_2$ and 0.5% (w/v) BSA (assay buffer) at the working dilution of $10^5$ CFU/ml. The total volume of the final reaction mixture was 50 μl with 25 μl of serial two fold dilution of test serum, 12.5 μl of bacteria at the working dilution, 12.5 μl of baby rabbit complement (final concentration 25%).

Controls included bacteria incubated with complement serum, immune sera incubated with bacteria and with complement inactivated by heating at 56°C for 30'. Immediately after the addition of the baby rabbit complement, 10μl of the controls were plated on Mueller-Hinton agar plates using the tilt method (time 0). The 96-wells plate was incubated for 1 hour at 37°C with rotation. 7μl of each sample were plated on Mueller-Hinton agar plates as spots, whereas 10μl of the controls were plated on Mueller-Hinton agar plates using the tilt method (time 1). Agar plates were incubated for 18 hours at 37 degrees and the colonies corresponding to time 0 and time 1 were counted.

***Sera analysis - western blots***

Purified proteins (500ng/lane), outer membrane vesicles (5μg) and total cell extracts (25μg) derived from MenB strain 2996 were loaded onto a 12% SDS-polyacrylamide gel and transferred to a nitrocellulose membrane. The transfer was performed for 2 hours at 150mA at 4°C, using transfer buffer (0.3% Tris base, 1.44% glycine, 20% (v/v) methanol). The membrane was saturated by overnight incubation at 4°C in saturation buffer (10% skimmed milk, 0.1% Triton X100 in PBS). The membrane was washed twice with washing buffer (3% skimmed milk, 0.1% Triton X100 in PBS) and incubated for 2 hours at 37°C with mice sera diluted 1:200 in washing buffer. The membrane was washed twice and incubated for 90 minutes with a 1:2000 dilution of horseradish peroxidase labelled anti-mouse Ig. The membrane was washed twice with 0.1% Triton X100 in PBS and developed with the Opti-4CN Substrate Kit (Bio-Rad). The reaction was stopped by adding water.

The OMVs were prepared as follows: *N. meningitidis* strain 2996 was grown overnight at 37 degrees with 5%

$CO_2$ on 5 GC plates, harvested with a loop and resuspended in 10 ml of 20mM Tris-HCl pH 7.5, 2 mM EDTA. Heat inactivation was performed at 56°C for 45 minutes and the bacteria disrupted by sonication for 5 minutes on ice (50% duty cycle, 50% output, Branson sonifier 3 mm microtip). Unbroken cells were removed by centrifugation at 5000*g* for 10 minutes, the supernatant containing the total cell envelope fraction recovered and further centrifuged overnight at 50000*g* at the temperature of 4°C. The pellet containing the membranes was resuspended in 2% sarkosyl, 20mM Tris-HCl pH 7.5, 2 mM EDTA and incubated at room temperature for 20 minutes to solubilise the inner membranes. The suspension was centrifuged at 10000g for 10 minutes to remove aggregates, the supernatant was further centrifuged at 50000g for 3 hours. The pellet, containing the outer membranes was washed in PBS and resuspended in the same buffer. Protein concentration was measured by the D.C. Bio-Rad Protein assay (Modified Lowry method), using BSA as a standard.

Total cell extracts were prepared as follows: *N. meningitidis* strain 2996 was grown overnight on a GC plate, harvested with a loop and resuspended in 1ml of 20mM Tris-HCl. Heat inactivation was performed at 56°C for 30 minutes.

***961 domain studies***

Cellular fractions preparation Total lysate, periplasm, supernatant and OMV of *E.coli* clones expressing different domains of 961 were prepared using bacteria from over-night cultures or after 3 hours induction with IPTG. Briefly, the periplasm were obtained suspending bacteria in saccarose 25% and Tris 50mM (pH 8) with polimixine 100μg/ml. After 1hr at room temperature bacteria were centrifuged at 13000rpm for 15 min and the supernatant were collected. The culture supernatant were filtered with 0.2μm and precipitated with TCA 50% in ice for two hours. After centrifugation (30 min at 13000 rp) pellets were rinsed twice with ethanol 70% and suspended in PBS. The OMV preparation was performed as previously described. Each cellular fraction were analyzed in SDS-PAGE or in Western Blot using the polyclonal anti-serum raised against GST-961.

Adhesion assay Chang epithelial cells (Wong-Kilbourne derivative, clone 1-5c-4, human conjunctiva) were maintained in DMEM (Gibco) supplemented with 10% heat-inactivated FCS, 15mM L-glutamine and antibiotics.

For the adherence assay, sub-confluent culture of Chang epithelial cells were rinsed with PBS and treated with trypsin-EDTA (Gibco), to release them from the plastic support. The cells were then suspended in PBS, counted and dilute in PBS to $5x10^5$ cells/ml.

Bacteria from over-night cultures or after induction with IPTG, were pelleted and washed twice with PBS by centrifuging at 13000 for 5 min. Approximately $2\text{-}3x10^8$ (cfu) were incubated with 0.5 mg/ml FITC (Sigma) in 1ml buffer containing 50mM $NaHCO_3$ and 100mM NaCl pH 8, for 30 min at room temperature in the dark. FITC-labeled bacteria were wash 2-3 times and suspended in PBS at $1\text{-}1.5x10^9$/ml. 200μl of this suspension ($2\text{-}3x10^8$) were incubated with 200μl ($1x10^5$) epithelial cells for 30min a 37°C. Cells were than centrifuged at 2000rpm for 5 min to remove non-adherent bacteria, suspended in 200μl of PBS, transferred to FACScan tubes and read

## Claims

1. A method for the heterologous expression of *N.meningitidis* protein '961', wherein:

   (a) protein 961 has the amino acid sequence '961' in strain MC58:

```
MSMKHFPAKVLTTAILATFCSGALAATSDDDVKKAATVAIVAAYNNGQEINGFKAGETIYDIGE
DGTITQKDATAADVEADDFKGLGLKKVVTNLTKTVNENKQNVDAKVKAAESEIEKLTTKLADTD
AALADTDAALDETTNALNKLGENITTFAEETKTNIVKIDEKLEAVADTVDKHAEAFNDIADSLD
ETNTKADEAVKTANEAKQTAEETKQNVDAKVKAAETAAGKAEAAAGTANTAADKAEAVAAKVTD
IKADIATNKADIAKNSARIDSLDKNVANLRKETRQGLAEQAALSGLFQPYNVGRFNVTAAVGGY
KSESAVAIGTGFRFTENFAAKAGVAVGTSSGSSAAYHVGVNYEW
```

   and wherein
   (b) at least one domain in the protein is deleted, wherein the domains of '961' in strain MC58 are as follows: (1) amino acids 1-23; (2) amino acids 24-268; (3) amino acids 269-307; and (4) amino acids 308-364,

   and wherein the '961' protein is expressed in a host cell.

2. The method of claim 1, wherein no fusion partner is used for protein expression.

3. The method of claim 1, wherein the protein includes a C-terminal His-tag.

4. The method of claim 1, wherein the protein includes a N-terminal GST.

5. The method of claim 1, wherein the protein '961' is the N-terminal portion of a hybrid protein.

6. A protein expressed by the method of any preceding claim.

## Patentansprüche

1. Verfahren zur heterologen Expression von *N. meningitidis*-Protein '961', wobei:

   (a) Protein 961 die Aminosäuresequenz '961' in Stamm MC58 hat:

```
MSMKHFPAKVLTTAILATFCSGALAATSDDDVKKAATVAIVAAYNNGQEINGFKAGETIYDIGE
DGTITQKDATAADVEADDFKGLGLKKVVTNLTKTVNENKQNVDAKVKAAESEIEKLTTKLADTD
AALADTDAALDETTNALNKLGENITTFAEETKTNIVKIDEKLEAVADTVDKHAEAFNDIADSLD
ETNTKADEAVKTANEAKQTAEETKQNVDAKVKAAETAAGKAEAAAGTANTAADKAEAVAAKVTD
IKADIATNKADIAKNSARIDSLDKNVANLRKETRQGLAEQAALSGLFQPYNVGRFNVTAAVGGY
KSESAVAIGTGFRFTENFAAKAGVAVGTSSGSSAAYHVGVNYEW
```

   und wobei

(b) zumindest eine Domäne des Proteins deletiert wurde, wobei die Domänen von '961' in Stamm MC58 folgendermaßen sind: (1) Aminosäuren 1 bis 23; (2) Aminosäuren 24 bis 268; (3) Aminosäuren 269 bis 307; und (4) Aminosäuren 308 bis 364,

und wobei das '961'-Protein in einer Wirtszelle exprimiert wird.

**2.** Verfahren nach Anspruch 1, wobei kein Fusionspartner zur Proteinexpression verwendet wird.

**3.** Verfahren nach Anspruch 1, wobei das Protein eine C-terminale His-Markierung umfasst.

**4.** Verfahren nach Anspruch 1, wobei das Protein eine N-terminale GST umfasst.

**5.** Verfahren nach Anspruch 1, wobei das Protein '961' der N-terminale Teil eines Hybridproteins ist.

**6.** Protein, exprimiert durch das Verfahren nach einem der vorhergehenden Ansprüche.

## Revendications

**1.** Méthode d'expression hétérologue de la protéine '961' de *N. meningitidis,* dans laquelle :

(a) la protéine '961' présente la séquence d'acides aminés '961' dans la souche MC58 :

MSMKHFPAKVLTTAILATFCSGALAATSDDDVKKAATVAIVAAYNNGQEINGFKAGETIYDIGE
DGTITQKDATAADVEADDFKGLGLKKVVTNLTKTVNENKQNVDAKVKAAESEIEKLTTKLADTD
AALADTDAALDETTNALNKLGENITTFAEETKTNIVKIDEKLEAVADTVDKHAEAFNDIADSLD
ETNTKADEAVKTANEAKQTAEETKQNVDAKVKAAETAAGKAEAAAGTANTAADKAEAVAAKVTD
IKADIATNKADIAKNSARIDSLDKNVANLRKETRQGLAEQAALSGLFQPYNVGRFNVTAAVGGY
KSESAVAIGTGFRFTENFAAKAGVAVGTSSGSSAAYHVGVNYEW

et dans laquelle
(b) au moins un domaine de la protéine est délété, les domaines de la protéine '961' dans la souche MC58 étant comme suit : (1) acides aminés 1-23 ; (2) acides aminés 24-268 ; (3) acides aminés 269-307 ; et (4) acides aminés 308-364,

et dans laquelle la protéine '961' s'exprime dans une cellule hôte.

**2.** Méthode selon la revendication 1, dans laquelle aucun partenaire de fusion n'est utilisé pour l'expression de la protéine.

**3.** Méthode selon la revendication 1, dans laquelle la protéine comprend un His-tag C-terminal.

**4.** Méthode selon la revendication 1, dans laquelle la protéine comprend une GST N-terminale.

**5.** Méthode selon la revendication 1, dans laquelle la protéine '961' correspond à la portion N-terminale d'une protéine hybride.

**6.** Protéine exprimée par la méthode selon l'une quelconque des revendications précédentes.

# *FIGURE 4*

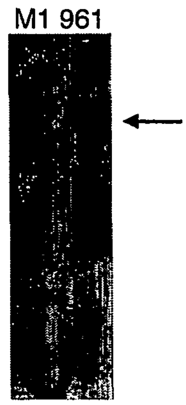


***PURIFICATION***

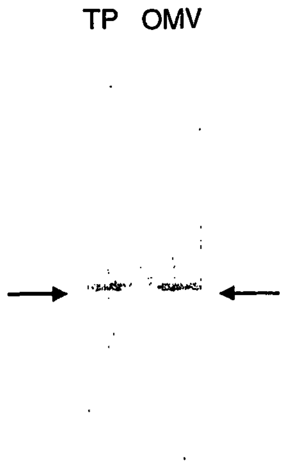


***WESTERN BLOT***

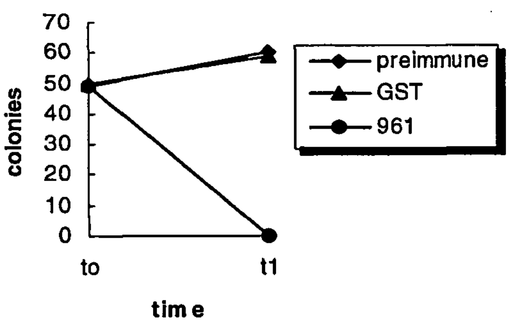


***BACTERICIDAL ASSAY***

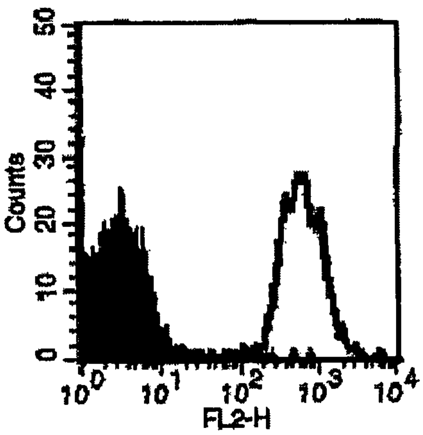


***FACS***

***ELISA:*** *POSITIVE*

## *FIGURE 12*

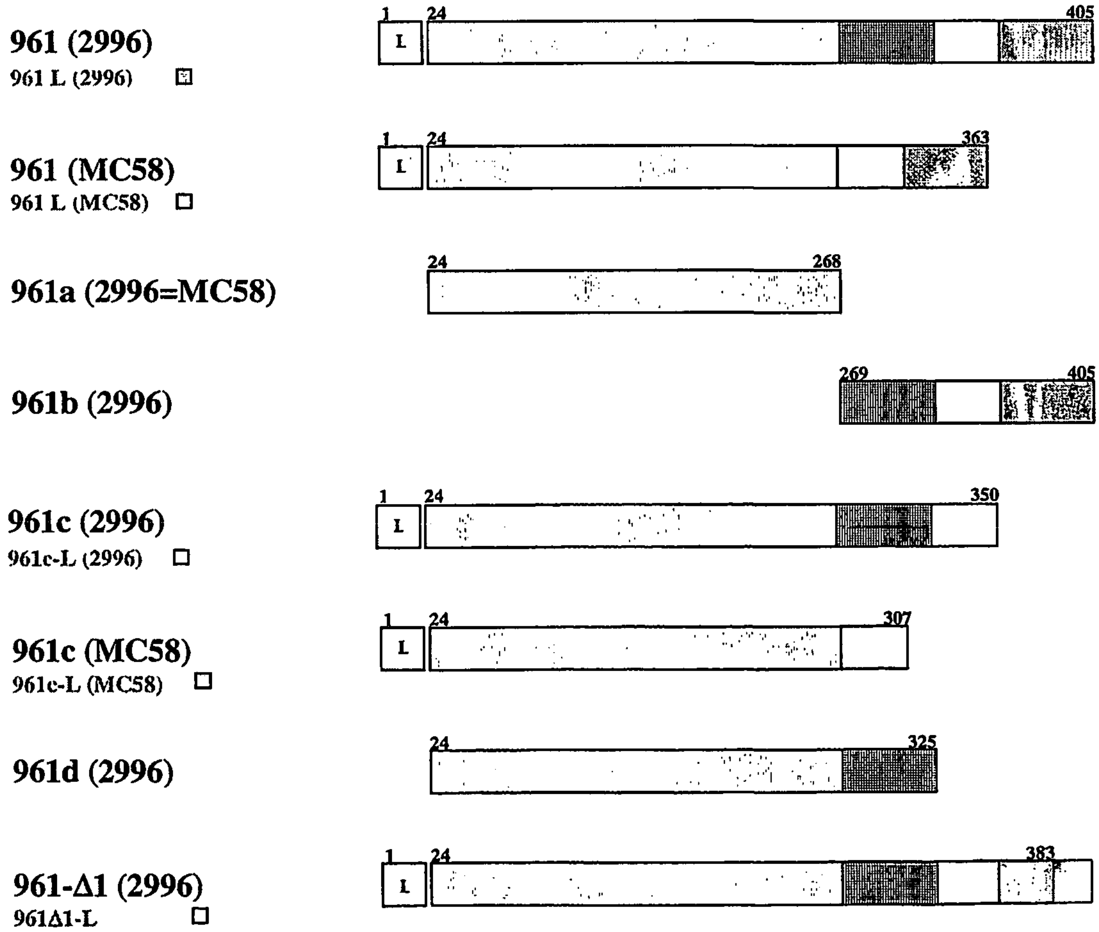
961 (2996)
961 L (2996)
1
24
405
L
961 (MC58)
961 L (MC58)
1
24
363
L
961a (2996=MC58)
24
268
961b (2996)
269
405
961c (2996)
961c-L (2996)
1
24
350
L
961c (MC58)
961c-L (MC58)
1
24
307
L
961d (2996)
24
325
961-Δ1 (2996)
961Δ1-L
1
24
383
L

## FIGURE 14

### FIGURE 14A — ΔG287—919

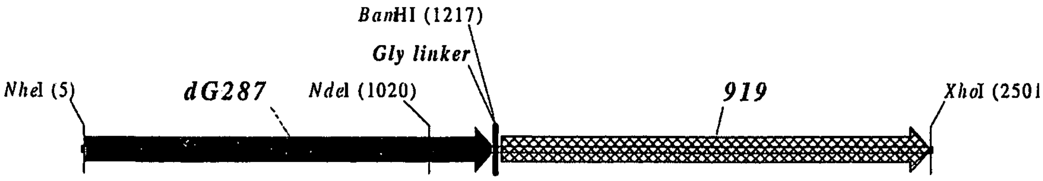


### FIGURE 14B — ΔG287—953

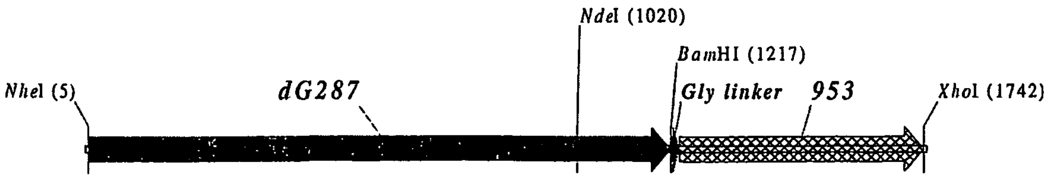

## FIGURE 14C — ΔG287—961

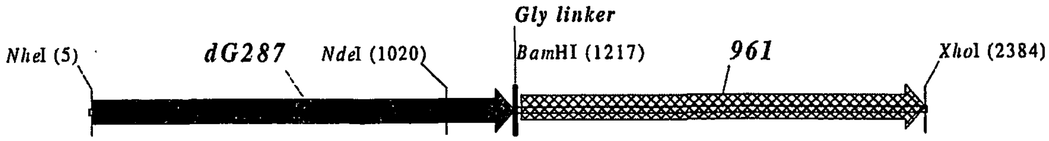


## FIGURE 14D — ΔG287NZ—919

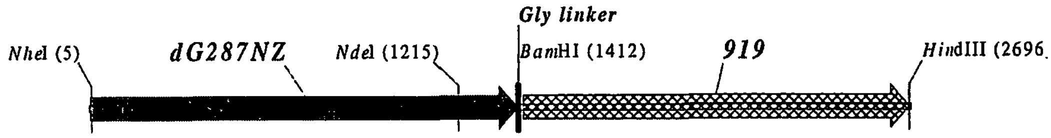


## FIGURE 14E — ΔG287NZ—953

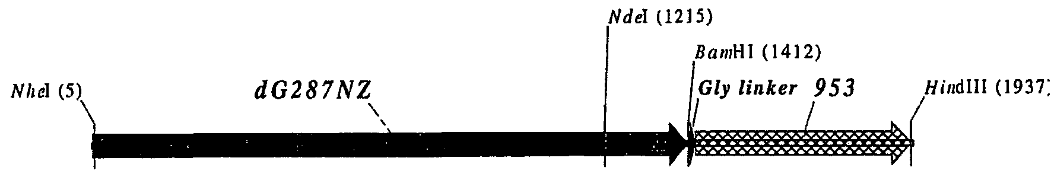


## FIGURE 14F — ΔG287NZ—961

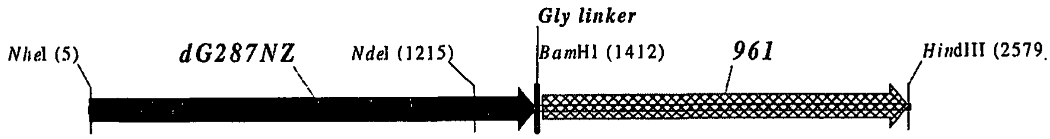


## FIGURE 14G — ΔG983-ORF46.1

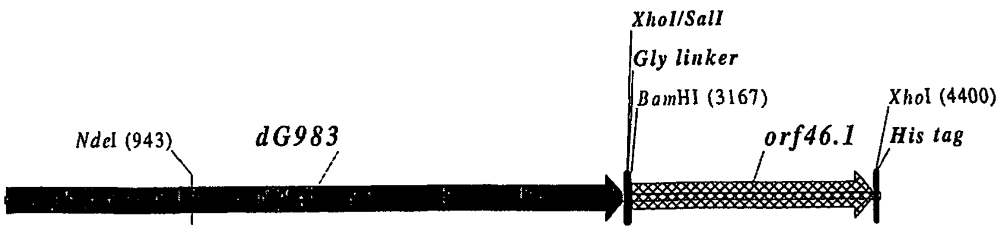

## FIGURE 14H — ΔG983-741

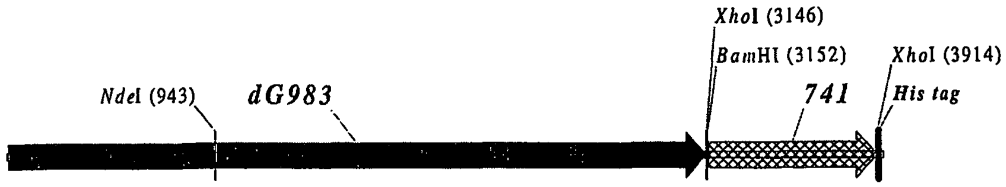


## FIGURE 14I — ΔG983-961

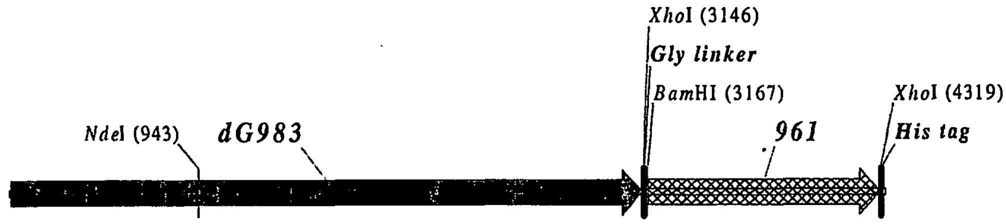


## FIGURE 14J — ΔG983-961c

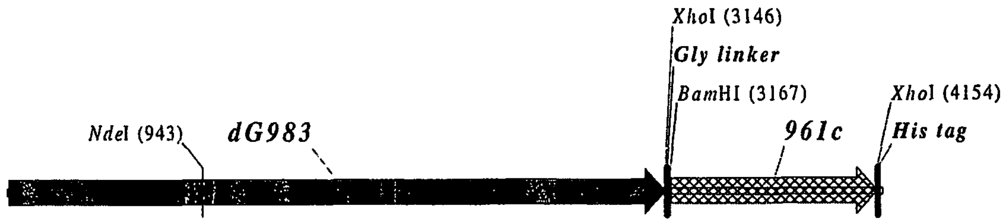


## FIGURE 14K — ΔG741-961

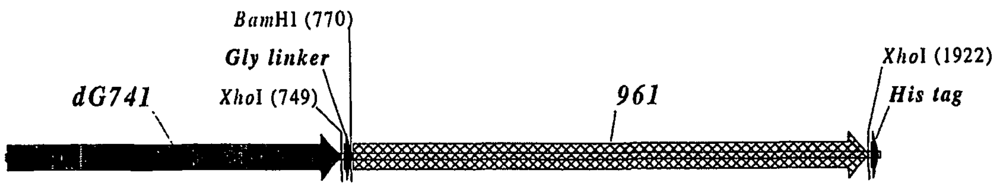


## FIGURE 14L — ΔG741-961c

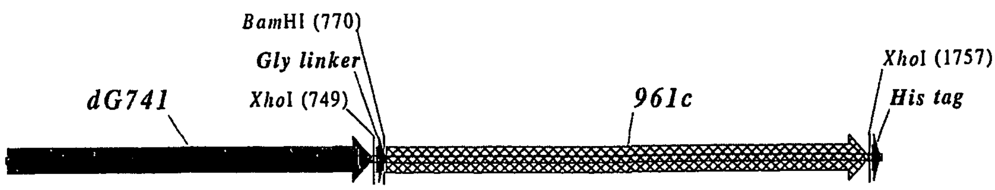

## FIGURE 14M — ΔG741-983

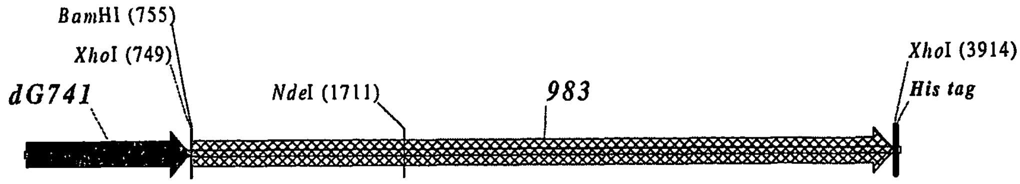


## FIGURE 14N — ΔG741-ORF46.1

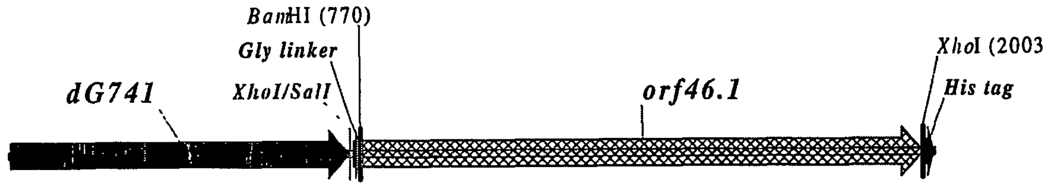


## FIGURE 14O — ORF46.1-741

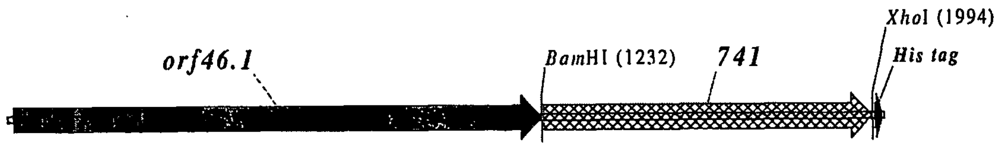


## FIGURE 14P — ORF46.1-961

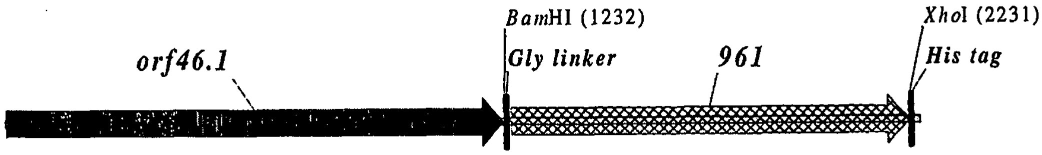


## FIGURE 14Q — ORF46.1—961c

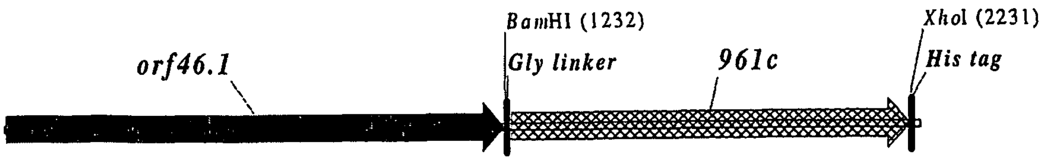

## FIGURE 14R — 961-ORF46.1

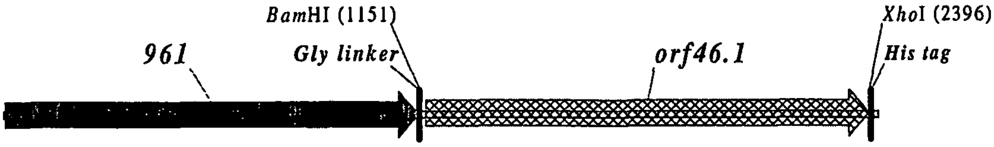


## FIGURE 14S — 961-741

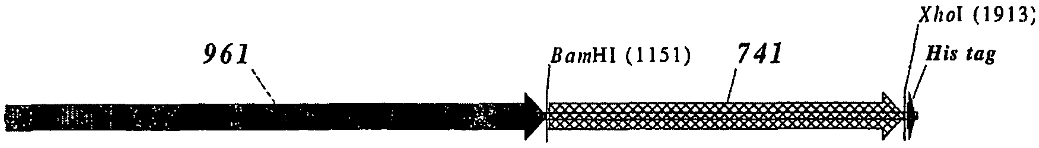


## FIGURE 14T — 961-983

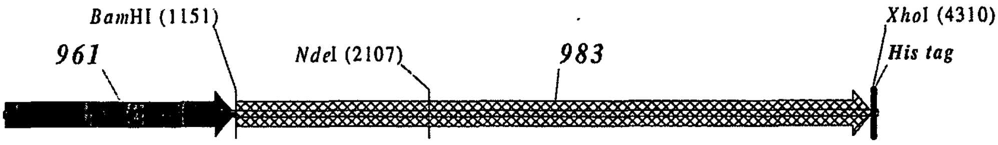


## FIGURE 14U — 961c-ORF46.1

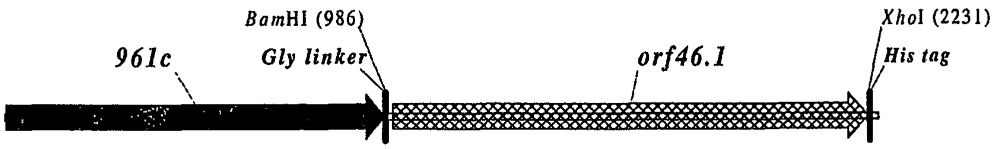


## FIGURE 14V — 961c-741

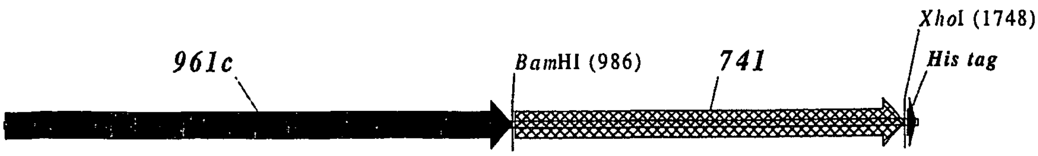

**FIGURE 14W — 961c-983**

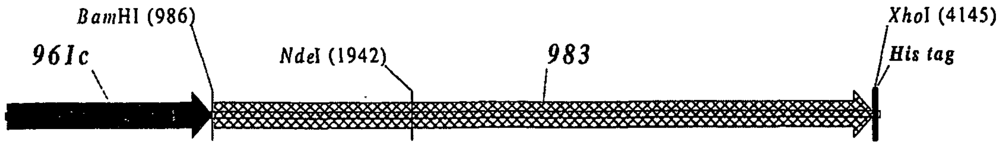


**FIGURE 14X — 961cL-ORF46.1**

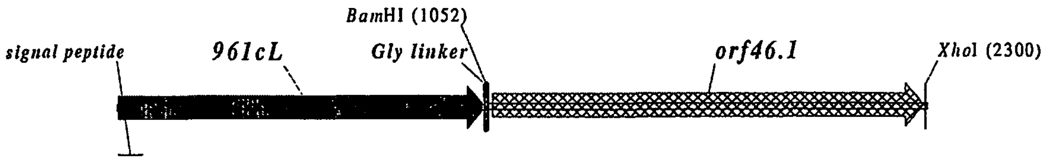


**FIGURE 14Y — 961cL-741**

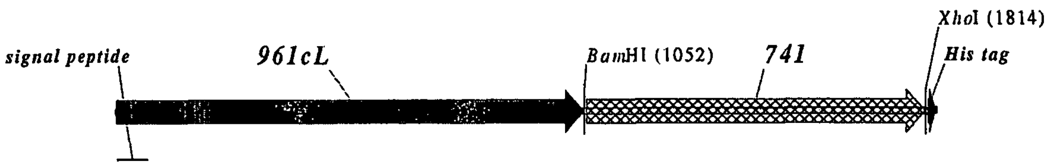


**FIGURE 14Z — 961cL-983**